# FASCICULE DE BREVET EUROPEEN

(11) **EP 3 700 911 B1**
(45) Date de publication et mention de la délivrance du brevet: **06.09.2023**
(21) Numéro de dépôt: 18796605.6
(22) Date de dépôt: 24.10.2018
(51) Int. Cl.: C07D 498/22, A61P 35/00, A61K 31/407

(54) **NOUVEAUX DERIVES MACROCYCLIQUES, LEUR PROCEDE DE PREPARATION ET LES COMPOSITIONS PHARMACEUTIQUES QUI LES CONTIENNENT**
MAKROCYCLISCHE DERIVATE, VERFAHREN ZU IHRER HERSTELLUNG UND DIESE ENTHALTENDE PHARMAZEUTISCHE ZUSAMMENSETZUNGEN
NOVEL MACROCYCLIC DERIVATIVES, PROCESS FOR PREPARING SAME AND PHARMACEUTICAL COMPOSITIONS CONTAINING SAME

(30) Priorité: 25.10.2017 FR 1760078
(43) Date de publication de la demande: 02.09.2020
(73) Titulaire: Les Laboratoires Servier, 92284 Suresnes (FR); Vernalis (R&D) Limited, Cambridge CB21 6GB (GB)
(72) Inventeur: STARCK, Jérôme-Benoît, 92500 Rueil-Malmaison (FR); DURAND, Didier, 78240 Chambourcy (FR); CHEN, I-Jen, Cambridge Cambridgeshire CB1 3JJ (GB); LE TIRAN, Arnaud, 78290 Croissy Sur Seine (FR); ORTUNO, Jean-Claude, 78390 Bois D'Arcy (FR); NYERGES, Miklós, 2016 Leanyfalu (HU); LIGETI, Melinda, 1133 Budapest (HU); FEJES, Imre, 1037 Budapest (HU)
(86) Numéro de dépôt international: PCT/EP2018/079113
(87) Numéro de publication internationale: WO 2019/081559

(56) Documents cités:
- WO-A1-2014/022752
- WO-A1-2015/011397

## Description

La présente invention concerne de nouveaux dérivés macrocycliques, leur procédé de préparation et les compositions pharmaceutiques qui les contiennent.

Les composés de la présente invention sont nouveaux et présentent des caractéristiques pharmacologiques très intéressantes dans le domaine de l'apoptose et de l'oncologie.

L'apoptose, ou mort cellulaire programmée, est un processus physiologique crucial pour le développement embryonnaire et le maintien de l'homéostasie tissulaire.

La mort cellulaire de type apoptotique fait intervenir des changements morphologiques, tels que la condensation du noyau, la fragmentation de l'ADN, ainsi que des phénomènes biochimiques, tels que l'activation des caspases qui vont dégrader des composants structuraux clés de la cellule pour induire son désassemblage et sa mort. La régulation du processus d'apoptose est complexe et implique l'activation ou la répression de plusieurs voies de signalisation intracellulaire (Cory S. et al., Nature Review Cancer, 2002, 2, 647-656).

Des dérégulations de l'apoptose sont impliquées dans certaines pathologies. Une apoptose accrue est liée aux maladies neurodégénératives telles que la maladie de Parkinson, la maladie d'Alzheimer et l'ischémie. Inversement, des déficiences dans l'exécution de l'apoptose jouent un rôle important dans le développement des cancers et leur chimiorésistance, des maladies auto-immunes, des maladies inflammatoires et des infections virales. Ainsi, l'échappement à l'apoptose fait partie des signatures phénotypiques du cancer (Hanahan D. et al., Cell 2000, 100, 57-70).

Les protéines anti-apoptotiques de la famille Bcl-2 sont associées à de nombreuses pathologies. L'implication des protéines de la famille Bcl-2 est décrite dans de nombreux types de cancer, tel que le cancer colorectal, le cancer du sein, le cancer du poumon à petites cellules, le cancer du poumon non à petites cellules, le cancer de la vessie, le cancer de l'ovaire, le cancer de la prostate, la leucémie lymphoïde chronique, le lymphome folliculaire, le myélome... La surexpression des protéines anti-apoptotiques de la famille Bcl-2 est impliquée dans la tumorogenèse, dans la résistance à la chimiothérapie et dans le pronostique clinique des patients atteints de cancer. Il existe donc un besoin thérapeutique de composés inhibant l'activité anti-apoptotique des protéines de la famille Bcl-2. Parmi les inhibiteurs Bcl-2 connus dans la littérature, WO2015/011397 décrit des dérivés de 3-[2-(3,4-dihydroisoquinoléine-2(1H)-carbonyl)phenyl]-5,6,7,8-tetrahydroindolizine-1-carboxamide et leurs propriétés pharmacologiques d'intérêt pour le traitement du cancer. Par ailleurs, WO2014/022752 divulgue des inhibiteurs PIM, également utiles dans le traitement du cancer et présentant une structure macrocyclique.

Les composés de la présente invention outre leur nouveauté, présentent des propriétés proapoptotiques permettant de les utiliser dans les pathologies impliquant un défaut d'apoptose, comme par exemple dans le traitement du cancer, des maladies auto-immunes et du système immunitaire.

L'invention concerne dans le premier mode de réalisation (E1) les composés de formule (I): 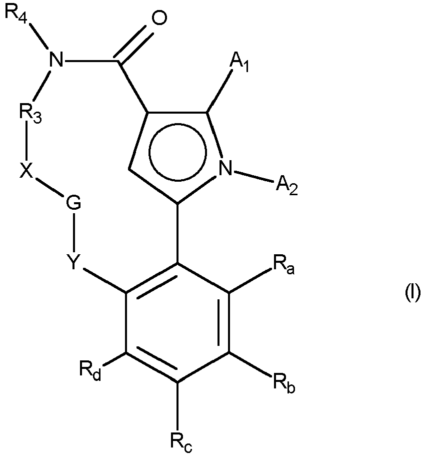 dans laquelle :
◆ A₁ et A₂ représentent chacun un groupement méthyle,
◆ G représente un groupe -NR₇-, un groupe 1,2,3,4-tétrahydroisoquinoléinylène éventuellement substitué par un groupement T, un groupe 2,3-dihydro-1*H-*isoindolylène éventuellement substitué par un groupement T, ou un groupe pipéridinylène,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ X représente un groupe (C₂-C₈)alkylène, dont 1 à 3 chaînons peuvent être remplacés par un hétéroatome choisi parmi oxygène, soufre et N-R₅, ou par un groupe arylène ou hétéroarylène,
◆ Y représente un groupe -CH₂- ou -CO-,
◆ R₁ et R₂ représentent indépendamment l'un de l'autre un atome d'hydrogène ou un groupement alkyle (C₁-C₆) linéaire ou ramifié,
   ou bien R₁ et R₂ forment avec l'atome d'azote qui les porte un hétérocycloalkyle,
◆ R₃ et R₄ sont tels que :
   - l'un d'entre eux représente un groupement phényl de formule suivante: 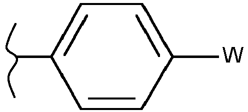 dans lequel W représente un groupe hydroxy ou un groupe phosphate choisi parmi - OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]nCH₃), et -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chaînons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5,
   - tandis que l'autre représente un groupement aryle, hétéroaryle, hétérocycloalkyle, cycloalkyle ou alkyle (C₁-C₆) linéaire ou ramifié, étant entendu qu'un ou plusieurs atomes de carbone des groupements précédents, ou de leurs éventuels substituants, peut(vent) être deutéré(s),
◆ R₅ représente un atome d'hydrogène ou un groupement alkyle (C₁-C₆) linéaire ou ramifié,
◆ R₆ et R₇ représentent indépendamment les uns des autres un atome d'hydrogène ou un groupement alkyle (C₁-C₆) linéaire ou ramifié,
◆ Rₐ, R_{c} et R_{d} représentent chacun un atome d'hydrogène et R_{b} représente un atome d'hydrogène ou d'halogène,

étant entendu que :
   - par "aryle", on entend un groupement phényle, naphtyle, biphényle ou indényle,
   - par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre, azote et azote quaternaire,
   - par "cycloalkyle", on entend tout groupement carbocyclique non aromatique, mono ou bi-cyclique, contenant 3 à 10 chaînons,
   - par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
   - par arylène, hétéroarylène, 1,2,3,4-tétrahydroisoquinoléinylène, 2,3-dihydro-1*H-*isoindolylène ou pipéridinylène, on entend un groupe aryl, hétéroaryl, 1,2,3,4-tétrahydroisoquinoléine ou pipéridine divalent,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi : alkyle (C₁-C₆) linéaire ou ramifié éventuellement substitué par un groupe hydroxy, morpholinyl, 3-3-difluoropiperidinyl ou 3-3-difluoropyrrolidinyl ; spiro (C₃-C₆) ; alkoxy (C₁-C₆) linéaire ou ramifié éventuellement substitué par un groupe morpholinyl ; (C₁-C₆)alkyl-S- ; hydroxy ; oxo ; A-oxyde ; nitro ; cyano ; -COOR' ; -OCOR' ; NR'R" ; polyhalogénoalkyle (C₁-C₆) linéaire ou ramifié ; trifluorométhoxy ; (C₁-C₆)alkylsulfonyl ; halogène ; aryle éventuellement substitué par un ou plusieurs atomes d'halogène ; hétéroaryle ; aryloxy ; arylthio ; cycloalkyle ; hétérocycloalkyle éventuellement substitué par un ou plusieurs atomes d'halogène ou groupements alkyles (C₁-C₆) linéaires ou ramifiés ; étant entendu que R' et R" représentent, indépendamment l'un de l'autre, un atome d'hydrogène ou un groupement alkyle (C₁-C₆) linéaire ou ramifié éventuellement substitué par un groupe méthoxy,
ses énantiomères, diastéréoisomères, ou ses sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc.

Parmi les bases pharmaceutiquement acceptables, on peut citer à titre non limitatif l'hydroxyde de sodium, l'hydroxyde de potassium, la triéthylamine, la *tert*butylamine, etc.

Différents modes de réalisation spécifiques de l'invention (E) sont détaillés ci-dessous. A noter que les caractéristiques des différents modes de réalisation peuvent être combinée entre elles pour générer de nouveaux modes de réalisations :
**E2.** Composé de formule (I) selon E1 dans lequel Y représente un groupe -CO-.
**E3.** Composé de formule (I) selon E1 ou E2 dans lequel G représente un groupe choisi parmi les groupes suivants: 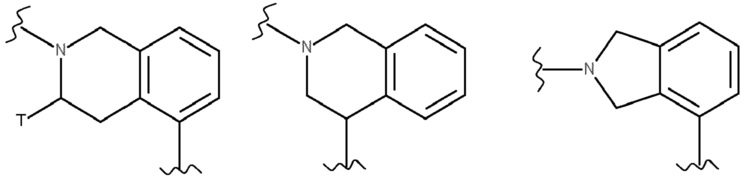 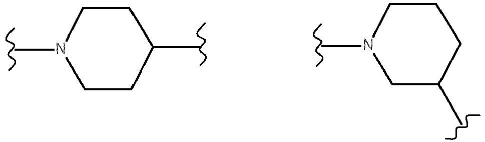 où T représente un groupe méthyle ou un groupe (4-morpholinyl)méthyle.
**E4.** Composé de formule (I) selon l'un des modes E1 à E3 dans lequel X représente un groupe choisi parmi les groupes suivants : 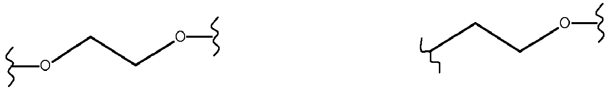 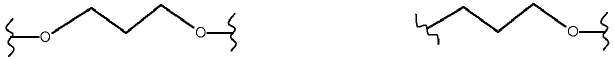 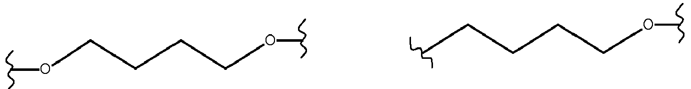 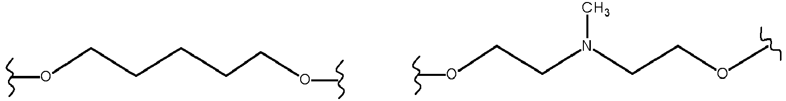 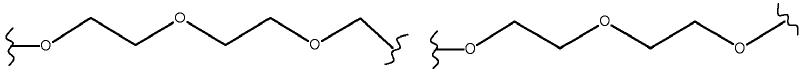 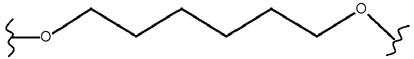
**E5.** Composé de formule (I) selon l'un des modes E1 à E4 dans lequel l'un des groupes R₃ ou R₄ représente un groupe 4-hydroxyphényl tandis que l'autre représente un groupe choisi dans la liste suivante :
   - un groupe phényl éventuellement substituté par un groupement cyano,
   - un groupe pyrazolyl,
   - un groupe 1-méthyl-1*H-*pyrazolyl,
   - un groupe 1-(tétrahydrofuran-3-yl)-1*H*-pyrazolyl,
   - un groupe 5-méthyl-2-cyano-1*H*-pyrrolyl,
   - un groupe 1-méthyl-2-cyano-1*H*-pyrrolyl,
   - un groupe 1,2-diméthyl-1*H*-pyrrolyl,
   - un groupe 1,5-diméthyl-2-cyano-1*H*-pyrrolyl,
   - un groupe pyrimidinyl,
   - un groupe éthyl,
   - un groupe pyridinium.
   Dans certains modes de réalisation de l'invention, R₃ ou R₄ représente un groupe 4-[(NaO)₂OPO]phényl.
**E6.** Composé de formule (I) selon le mode E5 dans lequel R₄ représente un groupe 4-hydroxyphényl.
**E7.** Composé de formule (I) selon le mode E5 dans lequel R₃ représente un groupe 4-hydroxyphényl.
**E8.** Composé de formule (I) selon le mode E7 dans lequel R₃ représente un groupe 4-hydroxyphényl et G représente un groupe pipéridinylène.
**E9.** Composé de formule (I) selon le mode de réalisation E1 choisi parmi le groupe suivant :
   - 6-chloro-14-(4-hydroxyphényl)-10,11-diméthyl-2,13-dioxo-20,23-dioxa-1,10,14-triazahexacyclo[26.3.1.1-9,12-.1-15,19-.0-3,8-.0-24,29-]tétratriaconta-3,5,7,9(34),11,15(33),16,18,24,26,28-undécaène-17-carbonitrile,
   - 11-chloro-4-(4-hydroxyphényl)-1,7,8-triméthyl-4,16,17,23,24,25-hexahydro-1H,14H-15,18-méthano-6,9-(méthéno)dibenzo[b,h]pyrazolo[4,3-p][1,6,11,15]oxatriazacycloicosine-5,14(8H)-dione,
   - 6-chloro-14-(4-hydroxyphényl)-10,11-diméthyl-2,13-dioxo-23-oxa-1,10,14-triazahexacy clo[26.3.1.1-9,12-. 1-15,19~.0~3,8~.0-24,29~]tétratriaconta-3,5,7,9(34),11,15(33),16,18,24,26,28-undécaène-17-carbonitrile,
   - 11-chloro-4-(4-hydroxyphényl)-1,7,8-triméthyl-5,14-dioxo-4,5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile,
   - 11-chloro-4-(4-hydroxyphényl)-1,7,8-triméthyl-5,14-dioxo-1,4,5,8,16,17,23,24-octahydro-14H-15,18-méthano-6,9-(méthéno)dibenzo[l,r]pyrrolo[2,3-d][1,6,10,15]oxatriazacyclononadécine-2-carbonitrile,
   - 11-chloro-4-(4-hydroxyphényl)-1,7,8-triméthyl-1,4,16,21,22,23-hexahydro-14H-15,21-méthano-6,9-(méthéno)dibenzo[j,o]pyrazolo[3,4-b][1,4,8,13]oxatriazacyclononadécine-5,14(8H)-dione,
   - (16S ou R)-11-chloro-4-(4-hydroxyphényl)-1,7,8,16-tétraméthyl-5,14-dioxo-4, 5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile,
   - 4-[16-chloro-3-hydroxy-19,20-diméthyl-13,22-dioxo-6,7,8,9,10,11,19,22-octahydro-13H,23H-8,12-méthano-21,18-(méthéno)dibenzo[b,j][1,4,8,13]oxatriazacyclononadécin-23-yl]-1,5-diméthyl-1H-pyrrole-2-carbonitrile,
   - 10-fluoro-2-(4-hydroxyphényl)-5,6-diméthyl-24,27,30-trioxa-2,6,15,32,35-pentaazahexacyclo[29.2.2.1~4,7~.1~15,19~.0~8,13~.0~18,23~]heptatriaconta-1(33),4,7(37),8,10,12,18,20,22,31,34-undécaène-3,14-dione,
   - 10-chloro-2-(4-hydroxyphényl)-5,6-diméthyl-24,30-dioxa-2,6,15,32,35-pentaazahexacyclo[29.2.2.1~4,7~.1-15,19~.0~8,13~.0~18,23~]heptatriaconta-1(33),4,7(37),8,10,12,18,20,22,31,34-undécaène-3,14-dione,
   - 10-chloro-2-(4-hydroxyphényl)-5,6,27-triméthyl-24,30-dioxa-2,6,15,27,32,35-hexaazahexacyclo[29.2.2.1~4,7~.1~15,19~.0~8,13~.0~18,23~]heptatriaconta-1(33),4,7(37),8,10,12,18,20,22,31,34-undécaène-3,14-dione,
   - 11-chloro-4-(4-hydroxyphényl)-1,7,8-triméthyl-1,4,16,17,23,24-hexahydro-14H-15,18-méthano-6,9-(méthéno)dibenzo[l,r]pyrazolo[3,4-d][1,6,10,15]oxatriazacyclononadécine-5,14(8H)-dione.
**E10.** Procédé de préparation d'un composé de formule (I) selon le mode de réalisation E1 caractérisé en ce que l'on utilise comme produit de départ le composé de formule (II): 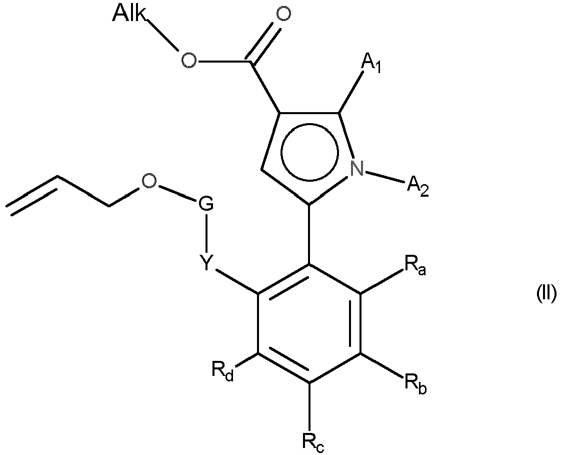
   dans lequel A₁, A₂, Rₐ, R_{b}, R_{c}, R_{d}, Y et G ont la même signification que dans la formule (I) définie dans E1, et Alk représente un groupe (C₁-C₆)alkyl linéaire ou ramifié,
   composé de formule (II) dont la fonction ester -OAlk est hydrolysée pour conduire à l'acide carboxylique ou au carboxylate correspondant, lequel peut être converti en le chlorure d'acyle ou l'anhydride correspondant, avant d'être couplé avec une amine NHR_{3A}R₄, dans laquelle R₄ a la même signification que dans la formule (I), et R_{3A} représente :
      - un groupement R₃ tel que défini dans la formule (I)
      - ou un groupement R₃-O-Alk'-Z, R₃-Alk'-Z ou R₃-Z dans lequel Alk' représente un groupe (C₁-C₆)alkyl linéaire ou ramifié, et Z représente un atome d'halogène ou un groupe -OH,
   pour former le composé de formule (III) : 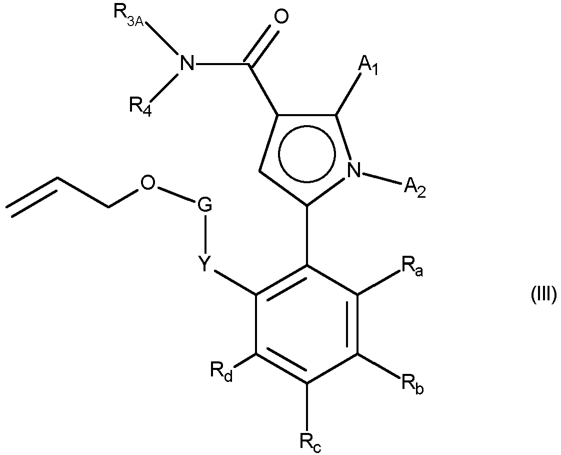
   dans lequel A₁, A₂, Rₐ, R_{b}, R_{c}, R_{d}, R₄, Y et G ont la même signification que dans la formule (I),
   lequel est soumis à une réaction de déprotection de la fonction alcool, suivie soit d'une substitution nucléophile intramoléculaire, soit d'une réaction de Mitsunobu, ou bien d'une substitution nucléophile aromatique,
   pour conduire au composé de formule (I),
   composé de formule (I) qui peut être purifié selon une technique classique de séparation, qui peut être transformé en ses sels d'addition à un acide ou à une base pharmaceutiquement acceptable et dont on sépare éventuellement les isomères selon une technique classique de séparation,
   étant entendu qu'à tout moment jugé opportun au cours du procédé précédemment décrit, les groupements hydroxy et amino des réactifs ou intermédiaires de synthèse peuvent être protégés puis déprotégés pour les besoins de la synthèse.
**E11.** Procédé de préparation d'un composé de formule (I) selon le mode de réalisation E1 caractérisé en ce que l'on utilise comme produit de départ le composé de formule (IV): 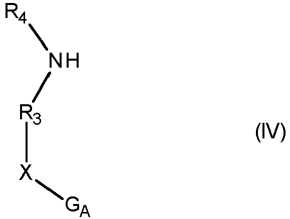
   dans lequel R₃, R₄ et X ont la même signification que dans la formule (I), et G_{A} représente un groupe choisi parmi la liste suivante : 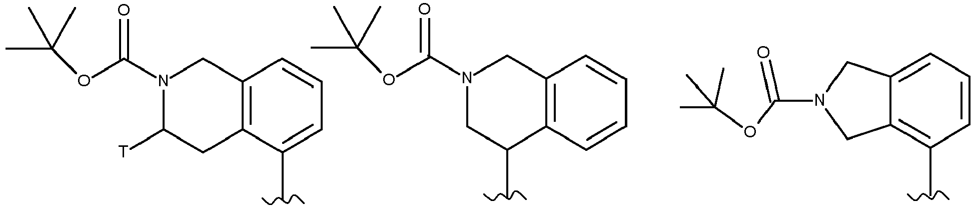 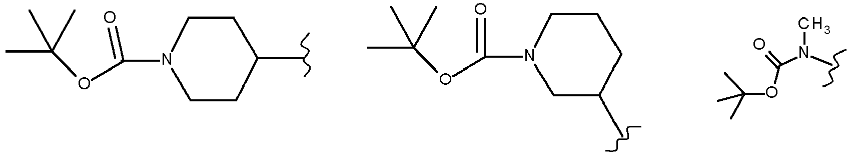
   composé de formule (IV) qui est ensuite couplé à un composé de formule (V) : 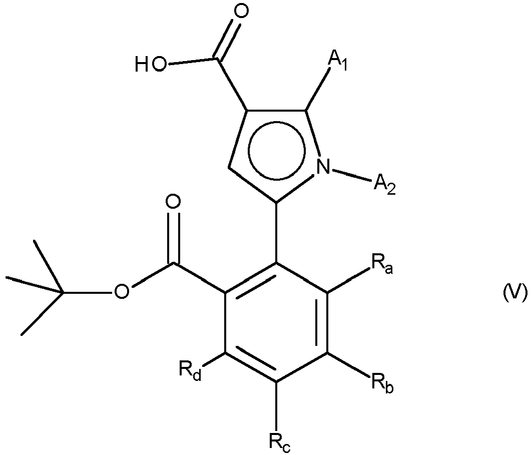
   dans lequel A₁, A₂, Rₐ, R_{b}, R_{c}, et R_{d} ont la même signification que dans la formule (I), pour conduire au composé de formule (VI) : 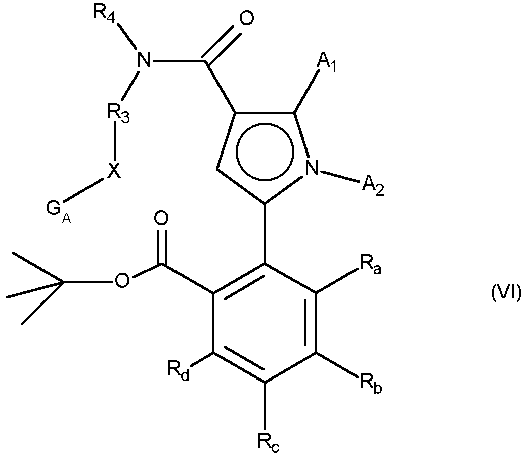
   dans lequel A₁, A₂, Rₐ, R_{b}, R_{c}, R_{d}, R₃, R₄ et X ont la même signification que dans la formule (I),
   lequel est soumis à une réaction de déprotection suivie d'un couplage intramoléculaire pour conduire au composé de formule (I),
   composé de formule (I) qui peut être purifié selon une technique classique de séparation, que peut être transformé en ses sels d'addition à un acide ou à une base pharmaceutiquement acceptable et dont on sépare éventuellement les isomères selon une technique classique de séparation,
   étant entendu qu'à tout moment jugé opportun au cours du procédé précédemment décrit, les groupements hydroxy et amino des réactifs ou intermédiaires de synthèse peuvent être protégés puis déprotégés pour les besoins de la synthèse.
**E12.** Composition pharmaceutique contenant un composé de formule (I) selon l'un des modes E1 à E9, ou un de ses sels d'addition avec un acide ou une base pharmaceutiquement acceptable, en combinaison avec un ou plusieurs excipients pharmaceutiquement acceptables.
**E13.** Composition pharmaceutique selon le mode de réalisation E12 pour son utilisation en tant qu'agent pro-apoptotique.
**E14.** Composition pharmaceutique selon le mode de réalisation E12 pour son utilisation dans le traitement des cancers, des maladies auto-immunes et du système immunitaire.
**E15.** Composition pharmaceutique selon le mode de réalisation E14 dans laquelle le cancer est choisi parmi la liste suivante : cancer de la vessie, du cerveau, du sein, de l'utérus, leucémie lymphoïde chronique, cancer colorectal, cancer de l'œsophage, du foie, leucémie lymphoblastique, lymphome non hodgkinien, mélanome, hémopathie maligne, myélome, cancer de l'ovaire, cancer du poumon non à petites cellules, cancer de la prostate et cancer du poumon à petites cellules.
**E16.** Utilisation d'un composé de formule (I) selon l'un des modes E1 à E9 pour la fabrication d'un médicament utile en tant qu'agent pro-apoptotique.
**E17.** Utilisation d'un composé de formule (I) selon l'un des modes E1 à E9 pour la fabrication d'un médicament destiné au traitement des cancers, des maladies immunitaires et auto-immunes.
**E18.** Utilisation d'un composé de formule (I) selon le mode de réalisation E17 dans laquelle le cancer est choisi parmi la liste suivante : cancer de la vessie, du cerveau, du sein, de l'utérus, leucémie lymphoïde chronique, cancer colorectal, cancer de l'oesophage, du foie, leucémie lymphoblastique, lymphome non hodgkinien, mélanome, hémopathie maligne, myélome, cancer de l'ovaire, cancer du poumon non à petites cellules, cancer de la prostate et cancer du poumon à petites cellules.
**E19.** Association d'un composé de formule (I) selon l'un des modes E1 à E9 avec un agent anticancéreux choisi parmi les agents génotoxiques, les poisons mitotiques, les antimétabolites, les inhibiteurs du protéasome, les inhibiteurs de kinases ou les anticorps.
**E20.** Composition pharmaceutique contenant une association selon le mode de réalisation E19 en combinaison avec un ou plusieurs excipients pharmaceutiquement acceptables.
**E21.** Association selon le mode de réalisation E19 pour son utilisation dans le traitement des cancers.
**E22.** Utilisation d'une association selon le mode de réalisation E19 pour la fabrication d'un médicament utile dans le traitement des cancers.
**E23.** Composé de formule (I) selon l'un des modes E1 à E9 pour son utilisation en association avec une radiothérapie dans le traitement des cancers.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en une ou plusieurs prises.

Enfin, les composés de l'invention peuvent être liés à des anticorps monoclonaux ou à des fragments de ceux-ci ou peuvent être liés à des protéines de charpente qui peuvent être apparentées, ou non, à des anticorps monoclonaux.

Par fragments d'anticorps, on doit entendre des fragments du type Fv, scFv, Fab, F(ab')2, F(ab'), scFv-Fc, ou des diabodies, qui en général ont la même spécificité de liaison que l'anticorps duquel ils sont issus. Selon la présente invention, les fragments d'anticorps de l'invention peuvent être obtenus à partir d'anticorps par des méthodes telles que digestion par enzymes telles que la pepsine ou la papaïne, et/ou par clivage des ponts disulfures par réduction chimique. D'autre manière, les fragments d'anticorps compris dans la présente invention peuvent être obtenus par des techniques de recombinaison génétique également bien connues à l'homme du métier ou bien par synthèse de peptides au moyen de synthétiseurs automatiques de peptides par exemple, tels que ceux fournis par la société Applied Biosystems etc...

Par protéines de charpente qui peuvent être apparentées, ou non, à des anticorps monoclonaux, on entend une protéine qui comprend, ou non, un repliement d'immunoglobuline et qui livre une capacité de liaison similaire à celle d'un anticorps monoclonal. L'homme du métier sait comment sélectionner la protéine de charpente. Plus particulièrement, il est connu que, pour être sélectionné, une telle charpente devrait présenter plusieurs caractéristiques comme suivant (Skerra A., J. Mol. Recogn. 13, 2000, 167-187): une bonne conservation phylogénétique, une architecture robuste avec une organisation moléculaire en trois dimensions bien connue (telle que la cristallographie ou la RMN, par exemple), une petite taille, aucune ou peu de modification(s) post-traductionnelle(s), la facilité de production, d'expression et de purification. Une telle protéine de charpente peut être, sans s'y limiter, une structure choisie parmi le groupe consistant en la fibronectine et de préférence le dixième domaine type III de la fibronectine (FNfn10), la lipocaline, l'anticaline (Skerra A., J. Biotechnol., 2001, 74(4):257-75), la protéine Z dérivée du domaine B de la protéine A des staphylocoques, la thiorédoxine A ou une protéine quelconque avec un domaine répété tel qu'une "répétition ankyrine" (Kohl et al., PNAS, 2003, vol.100, no.4, 1700-1705), une "répétition armadillo", une "répétition riche en leucine" ou une "répétition tétratricopeptide". On pourrait aussi mentionner une charpente dérivée de toxines (telles que des toxines de scorpions, d'insectes, de plantes ou des mollusques, par exemple) ou des protéines inhibitrices de l'oxyde nitrique synthase (PIN).

Les Préparations et Exemples suivants illustrent l'invention et ne la limitent en aucune façon.

### Préparation 1a: acide -4-chloro-2-[4-(éthoxycarbonyl}-1,5-diméthyl-1H-pyrrol-2 yl]benzoïque

### Stade A : 1,2-diméthyl-1H-pyrrole-3-carboxylate d'éthyle

A une solution de 2-méthyl-1*H*-pyrrole-3-carboxylate d'éthyle (10 g ; 65,3 mmol) et d'iodure de méthyle (8,95 mL ; 130,6 mmol) dans le *N,N*-diméthylformamide (70 mL) placée à 0°C, est additionné, en 3 portions, l'hydrure de sodium à 60 % (2,61 g ; 65,3 mmol). L'ensemble est ensuite agité à 0°C pendant 1 heure. Le milieu réactionnel est hydrolysé par l'addition d'eau glacée (420 mL) puis dilué avec de l'acétate d'éthyle. Après décantation, la phase organique est lavée successivement avec une solution aqueuse d'acide chlorhydrique 0,1 N, une solution aqueuse saturée en chlorure de lithium, et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 6.65 (d, 1H), 6.3 (1d, 1H), 4.1 (1q, 2H) ; 3.5 (s, 3H), 2.4 (s, 3H), 1.5 (1t, 3H).

**IR :** v : >C=O : 1688 cm⁻¹ ; C-O-C : 1172 cm⁻¹.

### Stade B : 5-(5-chloro-2-formylphényl)-1,2-diméthyl-1H-pyrrole-3-carboxylate d'éthyle

A une solution du composé obtenu au Stade A (10 g ; 62,8 mmol) dans le *N,N-*diméthylacétamide (65 mL), sont additionnés successivement du 2-bromo-4-chlorobenzaldéhyde (15,2 g ; 69 mmol), de l'acétate de potassium (12,3 g ; 125,6 mmol), puis l'ensemble est agité sous argon pendant 20 minutes. On ajoute alors du dichlorobis(triphénylphosphine)palladium(II) (2,2 g ; 3,14 mmol). Le milieu réactionnel est ensuite chauffé à 130 °C pendant la nuit. Après retour à température ambiante, le milieu réactionnel est dilué dans du dichlorométhane, puis on y ajoute du noir animal (2 g). L'ensemble est agité à température ambiante pendant 1 heure, puis filtré. La phase organique est alors lavée avec de l'eau, séchée sur sulfate de magnésium et concentrée à sec. Le résidu est purifié par chromatographie sur gel de silice en utilisant le dichlorométhane et l'éthanol comme éluants pour obtenir le produit du titre.

**RMN ¹H** (400 MHz, dmso-d6, 300 K) δ ppm : 9.8 (s, 1H), 7.91-7.69-7,61 (d, 3H), 6.5 (s, 1H), 4.2 (q, 2H,), 3.4 (s, 3H), 2.55 (s, 3H), 1.28 (t, 3H).

### Stade C : acide 4-chloro-2-[4-(éthoxycarbonyl)-1,5-diméthyl-1H-pyrrol-2-yl]benzoïque

Le composé obtenu au Stade B (12,85 g ; 42 mmol) et le 2-méthyl-2-butène (35,7 mL ; 336 mmol) sont mis en solution dans un mélange d'acétone (20 mL) et de tétrahydrofurane (20 mL).200 mL d'une solution aqueuse contenant un mélange de chlorite de sodium (13,3 g ; 147 mmol) et d'hydrogénophosphate de sodium (14,5 g ; 105 mmol) y sont additionnés goutte à goutte. L'ensemble est ensuite agité énergiquement à température ambiante durant 7 heures. Le milieu réactionnel est concentré pour éliminer l'acétone puis dilué avec de l'acétate d'éthyle. Après décantation, la phase organique est lavée à l'eau et concentrée à sec. Le résidu est ensuite repris dans un minimum d'éther éthylique. Le solide alors obtenu est filtré, lavé à l'éther éthylique puis séché sous vide à 40 °C pendant une nuit. Le produit du titre est utilisé pour la suite sans autre purification.

**RMN ¹H** (400 MHz, dmso-d6, 300 K) δ ppm : 13 (m, 1H), 7.85 (d, 1H), 7.60 (dd, 1H), 7.41 (d, 1H), 6.3 (s, 1H), 4.15 (q, 2H), 3.25 (s, 3H), 2.5 (s, 3H), 1.25 (t, 3H).

**IR :** v : -OH : 3100-2500 cm⁻¹; >C=O : 1681 cm⁻¹.

### Préparation 2a : acide 5-(5-chloro-2-formylphényl)-1,2-diméthyl-1H-pyrrole-3-carboxylique

### Stade A : 1,2-diméthyl-1H-pyrrole-3-carboxylate d'éthyle

A une solution d'acétoacétate d'éthyle (1000 g ; 7,68 mol) dans du tétrahydrofurane (3 L) placée à -10 °C, est additionnée sur une période d' 1 heure une solution aqueuse 50 % massique de chloroacétaldéhyde (1206 g ; 7,68 mol). Une solution aqueuse 40% massique de méthylamine (1495 g ; 19,2 mol) est ensuite additionnée sur une période de 3 heures à - 10°C. Puis, le mélange réactionnel est chauffé à 30 °C sur une période d' 1,5 heure et agité à cette température pendant 16 heures. Après retour à température ambiante, le mélange est dilué dans l'acétate d'éthyle (3 L) et les phases sont séparées. La phase aqueuse basique est conservée pour extraction. La phase organique est refroidie à 10°C et une solution aqueuse d'acide chlorhydrique 1 N (2,5 L) est ajoutée sur une période de 15 minutes. Les phases sont séparées et la phase organique est à nouveau lavée avec une solution aqueuse d'acide chlorhydrique 1 N (2,5 L), puis avec une solution aqueuse saturée en chlorure de sodium (1 L). Les phases aqueuses basiques et acides sont réunies et lavées avec de l'acétate d'éthyle (1,5 L). Les phases organiques sont réunies et lavées avec une solution aqueuse saturée en chlorure de sodium (1 L), séchées sur sulfate de sodium, filtrées et concentrées à sec. Le produit est purifié par distillation sous vide pour obtenir le produit du titre.

**RMN ¹H** (400 MHz, CDCl₃, 300 K) δ ppm : 6.50 (d, 1 H), 6.45 (d, 1 H), 4.25 (q, 2 H), 3.51 (s, 3 H), 2.49 (s, 3 H), 1.33 (t, 3 H).

### Stade B : acide 1,2-diméthyl-1H-pyrrole-3-carboxylique

A une solution du composé obtenu au Stade A (500 g ; 2,99 mol) dans de l'eau (5 L) est ajouté de l'hydroxyde de lithium monohydraté (251 g ; 5,98 mmol) et le mélange est chauffé à 100°C pendant 2 heures. Après retour à température ambiante, le mélange réactionnel est lavé avec du toluène (1 L) et du méthyl-*tert*-butyléther (1 L). La phase aqueuse est acidifiée avec une solution aqueuse d'acide chlorhydrique (530 mL) à pH=1 à une température comprise entre 10 et 15 °C, agitée pendant 1 heure puis filtrée. Le solide obtenu est lavé trois fois avec de l'eau et séché sous vide à une température comprise entre 60 et 65 °C pendant 36 heures.

**RMN ¹H** (400 MHz, CDCl₃, 300 K) δ ppm : 11.52 (s, 1 H), 6.62 (d, 1 H), 6.29 (d, 1 H), 3.50 (s, 3 H), 2.41 (s, 3 H).

### Stade C : acide 5-(5-chloro-2-formylphényl)-1,2-diméthyl-1H-pyrrole-3-carboxylique

Le composé du titre est obtenu selon le procédé décrit au Stade B de la Préparation 1a en utilisant l'acide 2- bromo-4-chlorobenzaldéhyde et le composé obtenu au stade précédent.

**RMN ¹H** (400 MHz, dmso-d6, 300 K) δ ppm : 9.8 (s, 1 H), 7.91 (d, 1 H), 7.69 (dd, 1 H), 7.61 (d, 1 H), 6.5 (s, 1 H), 4.2 (quad., 2 H), 3.4 (s, 3 H), 2.55 (s, 3 H), 1.28 (t, 3 H).

### Préparation 1a': 5-(prop-2-én-1-yloxy)-1,2,3,4-tétrahydroisoquinoléine, chlorhydrate (1:1)

### Stade A : 5-hydroxy-3,4-dihydroisoquinoléine-2(1H)-carboxylate de tert-butyle

A une solution de la 5-hydroxyisoquinoléine (20 g ; 137 mmol) dans l'acide acétique (120 mL), est additionné le dioxyde de platine (2 g ; 8,8 mmol). L'ensemble est placé sous atmosphère d'hydrogène (2 bars) pendant 24 heures. Le milieu réactionnel est filtré et le catalyseur est lavé au toluène. Le filtrat ainsi obtenu est concentré à sec. Le résidu obtenu est utilisé pour la suite sans autre purification.

A une solution du résidu obtenu (1,95 g ; 13 mmol) dans le dichlorométhane (110 mL), sont additionnés la diisoprolyléthylamine (9,7 mL ; 57 mmol) et le di-*tert-*butyldicarbonate (3,69 g, 16,9 mmol), puis l'ensemble est agité pendant 2 heures à température ambiante. Le milieu réactionnel est dilué avec une solution aqueuse saturée en chlorure d'ammonium. Après décantation, la phase organique est lavée avec une solution aqueuse saturée en hydrogénocarbonate de sodium, puis avec une solution aqueuse saturée en chlorure de sodium. Elle est ensuite séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 9.41 (m, 1 H), 6.97 (t, 1 H), 6.64/6.54 (2d, 2 H), 4.42 (m, 2 H), 3.53 (t, 2 H), 2.59 (t, 2 H), 1.42 (s, 9 H).

**IR :** v : -OH : 3294 cm⁻¹ ; >C=O : 1652 cm⁻¹.

### Stade B : 5-(prop-2-én-1-yloxy)-3,4-dihydroisoquinoléine-2(1H)-carboxylate de tert-butyle

A une solution du composé obtenu au Stade A (10 g ; 40,1 mmol) dans l'acétonitrile (110 mL), sont additionnés le bromure d'allyle (6 mL ; 60,2 mmol) et le carbonate de potassium (14,6 g, 120,3 mmol), puis l'ensemble est agité pendant 20 heures à température ambiante. Le milieu réactionnel est dilué avec de l'acétate d'éthyle. Après décantation, la phase organique est lavée avec de l'eau et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.8 (d, 1 H), 7.12 (t, 1 H), 6.75 (d, 1 H), 6.05 (m, 1 H), 5.41 (dquad, 1 H), 5.28 (dquad, 1 H), 4.58 (dt, 2 H), 4.49 (s, 2 H), 3.55 (t, 2 H), 2.68 (t, 2 H), 1.43 (s, 9 H).

**IR :** v : >C=O : 1691 cm⁻¹; >C-O-C< : 1162 cm⁻¹.

### Stade C : 5-(prop-2-én-1-yloxy)-1,2,3,4-tétrahydroisoquinoléine, chlorhydrate (1:1)

A une solution du composé obtenu au Stade B (9,7 g ; 33,4 mmol) dans le dioxane (30 mL), est additionnée une solution d'acide chlorhydrique 4 N dans le dioxane (33,4 mL ; 133,5 mmol), puis l'ensemble est agité pendant 48 heures à température ambiante. Le milieu réactionnel est concentré à sec et le résidu obtenu est repris dans l'acétate d'éthyle puis filtré.

Le produit du titre est obtenu sous la forme d'un solide, qui est utilisé sans autre purification.

**RMN ¹H** (400 MHz, dmso-d6, 300 K) δ ppm : 9.4 (s, 2 H), 7.21 (t, 1 H), 6.92 (d, 1 H), 6.8 (d, 1 H), 6.08 (m, 1 H), 5.41 (dt, 1 H), 5.29 (dt, 1 H), 4.62 (m, 2 H), 4.25 (s, 2 H), 3.35 (t, 2 H), 2.86 (t, 2 H).

**IR :** v : >NH₂+ : 3250-2250 cm⁻¹.

### Préparation 2a': 1,2,3,4-tétrahydroisoquinoléin-4-ylméthanol

### Stade A : 1,2,3,4-tétrahydroisoquinoléine-4-carboxylate de méthyle

A une solution de l'acide 1,2,3,4-tétrahydro-4-isoquinolinecarboxylique (5 g ; 28,2 mmol) dans le méthanol (40 mL), est additionné le triméthylchlorosilane (5,4 mL ; 42,3 mmol), puis l'ensemble est agité pendant 16 heures à température ambiante. Après un second ajout de triméthylchlorosilane (4 mL ; 31,35 mmol), on laisse le milieu réactionnel sous agitation pendant 16 heures à température ambiante. Le milieu réactionnel est ensuite concentré à sec, et le résidu est repris dans le méthanol, puis le milieu est de nouveau concentré. Cette opération est effectuée à deux reprises pour fournir le produit du titre, qui est utilisé pour la suite sans autre purification.

**RMN ¹H** (400 MHz, dmso-d6, 300 K) δ ppm : 9.75-9.6 (massif, 2 H), 7.4-7.25 (massif, 4 H), 4.3 (s, 2 H), 4.29 (m, 1 H), 3.72 (s, 3 H), 3.65/3.55 (ABx, 2 H).

**IR :** v : -NH2+ : 3200-2150 cm⁻¹; >C=O : 1731 cm⁻¹.

### Stade B : 1,2,3,4-tétrahydroisoquinoléin-4-ylméthanol

A un mélange d'hydrure d'aluminium lithium (1,24 g ; 32,7 mmol) dans le tétrahydrofurane (50 mL) placé à 0 °C, est additionné au goutte à goutte à cette même température le composé obtenu au Stade A (3,47 g ; 18,1 mmol) en solution dans le tétrahydrofurane (50 mL). Le milieu réactionnel est agité 2 heures à 0 °C, puis hydrolysé avec un mélange d'eau (11 mL) et d'une solution aqueuse de soude 1 N (15 mL). Après ajout d'acétate d'éthyle, on laisse l'ensemble sous agitation pendant 16 heures à température ambiante. L'insoluble est ensuite filtré et le filtrat est extrait à l'acétate d'éthyle. Les phases organiques sont réunies et lavées avec une solution aqueuse saturée en chlorure de sodium, puis séchées sur sulfate de magnésium, filtrées et concentrées à sec pour fournir le produit du titre, qui est utilisé pour la suite sans autre purification.

**RMN ¹H** (400 MHz, dmso-d6, 300 K) δ ppm : 7.2-6.95 (m, 4 H), 3.8 (s, 2 H), 3.6 (d, 2 H), 3.3 (m, 2 H), 3.18/2.85 (2dd, 2 H), 2.65 (m, 1 H).

**IR** : v : -NH/-OH+ : 3295 cm⁻¹ ; >C=C< : 1626 cm⁻¹.

### Préparation 1a" : N-(4-{[tert-butyl(diméthyl)silyl]oxy}phényl)-3-(2-chloroéthoxy)aniline

### Stade A : 1-bromo-3-(2-chloroéthoxy)benzène

A une solution de 3-bromophénol (5 g ; 18,9 mmol) et de bromochloroéthane (3,7 mL ; 43,3 mmol) dans l'acétonitrile (80 mL), est additionné le carbonate de potassium (12 g ; 86,7 mmol), puis l'ensemble est agité à 80 °C pendant 24 heures. Le milieu réactionnel est dilué avec un mélange d'acétate d'éthyle et d'eau. Après décantation, la phase organique est lavée avec de l'eau, et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, CDCl₃, 300 K) δ ppm : 7.15 (m, 2 H), 7.09 (m, 1 H), 6.85 (dt, 1 H), 4.21 (t, 2 H), 3.8 (t, 2 H).

**IR :** v : Ar : 1588 cm⁻¹ ; >C-O-C< : 1227 cm⁻¹ ; γ : >CH-Ar : 764 et 678 cm⁻¹.

### Stade B : 4-{[tert-butyl(diméthyl)silyl]oxy}aniline

Le composé du titre est obtenu à partir de la 4-aminophénol dans le tétrahydrofurane en présence d'imidazole et de chlorure de tert-butyl(diméthyl)silyle selon le protocole décrit dans la littérature (S. Knaggs et al, Organic & Biomolecular Chemistry, 3(21), 4002-4010; 2005**)**.

**RMN ¹H** (400 MHz, dmso-d6, 300 K) δ ppm : 6.45-6.55 (dd, 4H), 4.60 (m, 2H), 0.90 (s, 9H), 0.10 (s, 6H).

**IR :** v : -NH₂⁺ : 3300-3400 cm⁻¹

### Stade C : N-(4-{[tert-butyl(diméthyl)silyl]oxy}phényl)-3-(2-chloroéthoxy)aniline

Une solution des composés obtenus au Stade A (4,8 g ; 20,8 mmol) et au Stade B (5,6 g ; 24,9 mmol) dans le toluène (70 mL) est dégazée par bullage d'argon pendant 10 minutes. Y sont additionnés le *tert*-butylate de sodium (2,4 g ; 24,9 mmol) et le chloro(2-di-*tert-*butylphosphino-2',4',6'-triisopropyl-1,1'-biphényl)[2-(2-aminoéthyl)phényl]palladium(II) (0,7 g ; 1 mmol). L'ensemble est ensuite agité à 80 °C pendant 1 heure. Le milieu réactionnel est filtré sur Célite^{®}. Après un rinçage à l'acétate d'éthyle, le filtrat est lavé avec de l'eau et une solution aqueuse saturée en chlorure de sodium, puis séché sur sulfate de magnésium, filtré et concentré à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.88 (s, 1 H), 7.08 (t, 1 H), 7 (d, 2 H), 6.79 (d, 2 H), 6.54 (dd, 1 H), 6.49 (t, 1 H), 6.32 (dd, 1 H), 4.19 (t, 2 H), 3.91 (t, 2 H), 0.95 (s, 9 H), 0.2 (s, 6 H)
**IR :** v : >NH : 3393 cm⁻¹ ; δ : Si-CH₃ : 1250 cm⁻¹.

### Préparation 2a" : N-(4-{[tert-butyl(diméthyl)silyl]oxy}phényl)-3-(3-chloropropoxy) aniline

Le composé du titre est obtenu selon le procédé de la Préparation 1a" en remplaçant le bromochloroéthane par le bromochloropropane au Stade A.

**RMN ¹H** (400 MHz, dmso-d6, 300 K) δ ppm : 7.85 (s, 1 H), 7.05 (t, 1 H), 6.99 (d, 2 H), 6.77 (d, 2 H), 6.52 (dd, 1 H), 6.48 (t, 1 H), 6.31 (dd, 1 H), 4.02 (t, 2 H), 3.8 (t, 2 H), 2.13 (quint., 2 H), 0.95 (s, 9 H), 0.2 (s, 6 H).

**IR :** v : >NH : 3398 cm⁻¹; δ : NH : 1504 cm⁻¹ ; δ : Si-CH₃ : 1250 cm⁻¹.

### Préparation 3a" : N-(4-{[tert-butyl(diméthyl)silyl]oxy}phényl)-3-(4-chlorobutoxy)aniline

Le composé du titre est obtenu selon le procédé de la Préparation 1a" en remplaçant le bromochloroéthane par le bromochlorobutane au Stade A.

**RMN ¹H** (400 MHz, dmso-d6, 300 K) δ ppm : 7.81 (m, 1 H), 7.05 (m, 1 H), 7 (m, 2 H), 6.77 (m, 2 H), 6.5 (m, 1 H), 6.47 (m, 1 H), 6.3 (m, 1 H), 3.92 (m, 2 H), 3.7 (m, 2 H), 1.8 (m, 4 H), 0.97 (m, 9 H), 0.2 (m, 6 H).

**IR :** v : >NH : 3401 cm⁻¹.

### Préparation 4a": 3-[(4-{[tert-butyl(diméthyl)silyl]oxy}phényl)anùno]-5-(2-chloroéthoxy) benzonitrile

### Stade A : (3-bromo-5-méthoxyphényl)méthanol

A une solution d'acide 3-bromo-5-méthoxybenzoïque (10 g ; 43,3 mmol) dans le tétrahydrofurane (280 mL), est additionné goutte à goutte le complexe de boranediméthylsulfure (32,5 mL ; 64,9 mmol), puis l'ensemble est agité pendant 2 heures. Le milieu réactionnel est acidifié goutte à goutte par une solution aqueuse d'acide chlorhydrique 2 Njusqu'à pH=1. Après extraction à l'éther, la phase organique est lavée avec une solution aqueuse de soude 1 N et une solution aqueuse saturée en chlorure de sodium, puis séchée sur sulfate de magnésium, filtrée et concentrée à sec. Le produit du titre est obtenu sous forme d'une huile qui est utilisée au stade suivant sans purification.

**RMN ¹H** (400 MHz, dmso-d6, 300 K) δ ppm : 7.08 (m, 1 H), 6.99 (m, 1 H), 6.89 (m, 1 H), 5.3 (br. s, 1 H), 4.47 (s, 2 H), 3.75 (s, 3 H). "br." signifie "large".

**IR :** v : -OH : 1588 cm⁻¹ ; >C-O : 1268 et 1038 cm⁻¹ ; γ : >CH-Ar : 811 cm⁻¹.

### Stade B : 3-bromo-5-méthoxybenzaldéhyde

A une solution du composé obtenu au Stade A (8,6 g ; 39,8 mmol) dans le dichlorométhane (400 mL), est additionné le réactif de Dess Martin (20,3 mL ; 47,8 mmol), puis l'ensemble est agité pendant 2 heures. Après ajout d'éther, le milieu réactionnel est filtré sur un lit de silice. Le filtrat est concentré, repris dans un mélange d'heptane et d'acétate d'éthyle, puis filtré à nouveau sur un lit de silice. Après concentration du filtrat, le produit du titre est obtenu sous forme d'un solide jaune pâle qui est utilisé au stade suivant sans purification.

**RMN ¹H** (400 MHz, dmso-d6, 300 K) δ ppm : 9.5 (s, 1 H), 7.69 (t, 1 H), 7.5 (t, 1 H), 7.42 (t, 1 H), 3.85 (s, 3 H).

**IR :** v : >C=O : 1691 cm⁻¹.

### Stade C : (E)-1-(3-bromo-5-méthoxyphényl)-Nhydroxyméthanimine

A une solution du composé obtenu au Stade B (7,8 g ; 36,4 mmol) dans l'éthanol (10 mL), sont additionnés successivement le chlorhydrate d'hydroxylamine (12,6 g ; 182 mmol) et la pyridine (6,27 mL ; 87,4 mmol), puis l'ensemble est agité à 65 °C pendant 1 heure. Après retour à température ambiante, le milieu réactionnel est dilué avec un mélange d'acétate d'éthyle et d'eau. Après décantation, la phase organique est lavée avec de l'eau et une solution aqueuse saturée en chlorure de sodium, puis séchée sur sulfate de magnésium, filtrée et concentrée à sec. Le produit du titre est obtenu sous forme d'un solide blanc qui est utilisé au stade suivant sans purification.

**RMN ¹H** (400 MHz, dmso-d6, 300 K) δ ppm : 11.45 (s, 1 H), 8.1 (s, 1 H), 7.35 (t, 1 H), 7.16 (d, 2 H), 3.8 (s, 3 H).

**IR :** v : -OH : 3300-3000 cm⁻¹ ; Ar : 1600 et 1564 cm⁻¹ ; >C-O : 1220 et 1059 cm⁻¹ ; -N-O : 960 cm⁻¹ ; γ : >CH-Ar : 831 cm⁻¹.

### Stade D : 3-bromo-5-méthoxybenzonitrile

A une solution du composé obtenu au Stade C (8,1 g ; 35,2 mmol) dans le dioxane (70 mL), sont additionnés à 0 °C la pyridine (22 mL ; 211 mmol) et, goutte à goutte, l'anhydride d'acide trifluoroacétique (1,4 mL; 70,4 mmol), puis l'ensemble est agité à température ambiante pendant 24 heures. Le milieu réactionnel est placé à 0°C, puis on additionne goutte à goutte une deuxième portion d'anhydride trifluoroacétique (1,4 mL ; 70,4 mmol). L'ensemble est ensuite agité à température ambiante pendant 24 heures. Le milieu réactionnel est de nouveau placé à 0°C, puis on additionne goutte à goutte une troisième portion d'anhydride trifluoroacétique (1,4 mL ; 70,4 mmol). L'ensemble est agité à 60 °C pendant 1 heure. Après retour à température ambiante, le milieu réactionnel est dilué avec un mélange de dichlorométhane et d'eau. Après décantation, la phase organique est lavée avec une solution aqueuse d'acide chlorhydrique 1 N et une solution aqueuse saturée en chlorure de sodium, puis séchée sur sulfate de magnésium, filtrée et concentrée à sec. Le produit du titre est obtenu sous forme d'un solide jaune pâle qui est utilisé au stade suivant sans purification.

**RMN ¹H** (400 MHz, dmso-d6, 300 K) δ ppm : 7.69 (t, 1 H), 7.53 (dd, 1 H), 7.5 (dd, 1 H), 3.82 (s, 3 H).

**IR :** v : -CN : 2232 cm⁻¹; Ar : 1597 et 1562 cm⁻¹; >C-O-C< : 1278 et 1051 cm⁻¹ ; γ : >CH-Ar : 848, 814 et 671 cm⁻¹.

### Stade E : 3-bromo-5-hydroxybenzonitrile

A une solution du composé obtenu au Stade D (5,9 g ; 27,9 mmol) dans la 2,4,6-collidine (55 mL) est additionné l'iodure de lithium (11,2 g ; 83,7 mmol), puis l'ensemble est agité à 150 °C pendant 16 heures. Après retour à température ambiante, le milieu réactionnel est versé dans de l'eau glacé. Après extraction au dichlorométhane, les phases organiques sont réunies, lavées avec de l'eau, puis séchées sur sulfate de magnésium, filtrées et concentrées à sec. Le produit du titre est obtenu sous forme d'un solide brun orangé qui est utilisé au stade suivant sans purification.

**RMN ¹H** (400 MHz, dmso-d6, 300 K) δ ppm : 10.7 (br. s, 1 H), 7.51 (t, 1 H), 7.3 (t, 1 H), 7.18 (dd, 1H).

**IR :** v : -OH : 3283 cm⁻¹ ; -CN : 2245 cm⁻¹.

### Stade F : 3-[(4-{[tert-butyl(diméthyl)silyl]oxy}phényl)amino]-5-(2-chloroéthoxy) benzonitrile

Le composé du titre est obtenu selon les procédés des Stades A et C de la Préparation 1a" en utilisant le composé obtenu au stade précédent et le bromochloroéthane scomme produits de départ.

**RMN ¹H** (400 MHz, dmso-d6, 300 K) δ ppm : 8.29 (s, 1 H), 7.04 (d, 2 H), 6.82 (d, 2 H), 6.79/6.75/6.67 (3^{∗}m, 3 H), 4.24 (dd, 2 H), 3.91 (dd, 2 H), 1.19 (s, 6 H), 0.95 (s, 9 H).

**IR :** v : >NH : 3332 cm⁻¹; -CN : 2232 cm⁻¹ ; Ar : 1595 et 1504 cm⁻¹; >C-O-C< : 1250 cm⁻¹ ; γ : -Si-C : 828 cm⁻¹.

### Préparation 5a": 3-[(4-{[tert-butyl(diméthyl)silyl]oxy}phényl)amino]-5-(3-chloropropoxy)benzonitrile

Le composé du titre est obtenu selon le procédé de la Préparation 4a" en remplaçant le bromochloroéthane par le bromochloropropane au Stade F.

**RMN ¹H** (400 MHz, dmso-d6, 300 K) δ ppm : 8.3 (m, 1 H), 7.05-6.85 (m, 4 H), 6.8-6.6 (m, 3 H), 4.1 (m, 2 H), 3.8 (m, 2 H), 2.05 (m, 2 H), 0.95 (m, 9 H), 0.2 (m, 6 H).

**IR :** v : >NH : 3345 cm⁻¹ ; -CN : 2229 cm⁻¹.

### Préparation 6a": N-(4-{[tert-butyl(diméthyl)silyl]oxy}phényl)-5-(3-chloropropyl)-1-méthyl-1H-pyrazol-4-amine

### Stade A : 5-(3-{[tert-butyl(diméthyl)silyljoxy}propyl)-1-méthyl-1H-pyrazole

A une solution du *N*-méthylpyrazole (3,2 g ; 39 mmol) dans le tétrahydrofurane (65 mL), est additionnée goutte à goutteà -78 °C une solution de n-butyllithium dans l'hexane (26,8 mL ; 42,9 mmol), puis l'ensemble est agité pendant 1 heure jusqu'à ce qu'il retrouve une température de 0 °C. Le milieu réactionnel est ensuite placé à -78 °C, et on ajoute le (3-bromopropoxy)-*tert*-butyldiméthylsilane (10,6 mL ; 46,8 mmol). L'ensemble est agité à température ambiante pendant 16 heures et versé dans un mélange d'eau glacée et d'acétate d'éthyle. Après extraction à l'acétate d'éthyle, la phase organique est lavée avec une solution aqueuse saturée en chlorure de sodium, puis séchée sur sulfate de magnésium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.35 (d, 1 H), 7 (d, 1 H), 3.8 (s, 3 H), 3.65 (t, 2 H), 2.7 (m, 2 H), 1.85 (m, 2 H), 0.9 (s, 9 H), 0.5 (s, 6 H).

**IR :** γ : CH₃ : 1254 cm⁻¹ ; v : -Si-O- : 1098 cm⁻¹ ; -Si-C- : 834 et 772 cm⁻¹.

### Stade B : 3-(4-bromo-1-méthyl-1H-pyrazol-5-yl)propan-1-ol

A une solution du composé obtenu au Stade A (4,8 g; 18,9 mmol) dans le méthanol (200 mL), est additionné à 0 °C le tribromure de pyridinium (6,6 g ; 20,8 mmol). L'ensemble est agité pendant 1 heure à 0 °C, puis pendant 16 heures à température ambiante. Après concentration du milieu réactionnel, le résidu est repris dans un mélange d'une solution aqueuse à 10 % en carbonate de potassium et de dichlorométhane. Après extraction au dichlorométhane, la phase organique est lavée avec une solution aqueuse saturée en chlorure de sodium, puis séchée sur sulfate de magnésium, filtrée et concentrée à sec. Le résidu est purifié par chromatographie sur gel de silice en utilisant le dichlorométhane et le méthanol ammoniacal comme éluants pour obtenir le produit du titre.

**RMN ¹H** (400 MHz, dmso-d6, 300 K) δ ppm : 7.45 (s, 1 H), 4.59 (t, 1 H), 3.79 (s, 3 H), 3.4 (quad, 2 H), 2.7 (t, 2 H), 1.65 (m, 2 H).

**IR :** v : -OH : 3348 cm⁻¹.

### Stade C : 4-bromo-5-(3-chloropropyl)-1-méthyl-1H-pyrazole

A une solution du composé obtenu au Stade B (3,9 g ; 17,2 mmol) dans le tétrahydrofurane (40 mL), est additionné goutte à goutte à 0 °C le chlorure de thionyle (2,6 mL ; 36,4 mmol), puis l'ensemble est agité pendant 1 heure à 50 °C. Après concentration du milieu réactionnel, le résidu est repris dans un mélange d'eau et d'acétate d'éthyle. Après extraction à l'acétate d'éthyle, la phase organique est lavée avec une solution aqueuse saturée en chlorure de sodium, puis séchée sur sulfate de magnésium, filtrée et concentrée à sec. Le résidu est purifié par chromatographie sur gel de silice en utilisant le dichlorométhane et le méthanol ammoniacal comme éluants pour obtenir le produit du titre.

**RMN ¹H** (400 MHz, dmso-d6, 300 K) δ ppm : 7.48 (s, 1 H), 3.8 (s, 3 H), 3.69 (t, 2 H), 2.8 (t, 2 H), 1.95 (m, 2 H).

### Stade D : N-(4-{[tert-butyl(diméthyl)silyl]oxy}phényl)-5-(3-chloropropyl)-1-méthyl-1H-pyrazol-4-amine

Une solution des composés obtenus au Stade C (3,6 g ; 15,2 mmol) et au Stade B (3,4 g ; 15,2 mmol) de la Préparation 1a" dans un mélange de toluène (25 mL) et de tétrahydrofurane (25 mL) est dégazée par bullage d'argon pendant 10 minutes. Y sont additionnés le *tert*-butylate de sodium (1,75 g ; 18,2 mmol) et le chloro(2-di-*tert-*butylphosphino-2',4',6'-triisopropyl-1,1'-biphényl)[2-(2-aminoéthyl)phényl]palladium(II) (0,5 g ; 0,76 mmol), puis l'ensemble est agité à température ambiante pendant 2 heures. Le milieu réactionnel est filtré sur Célite^{®}, puis concentré après un rinçage au tétrahydrofurane. Le résidu est repris avec un mélange d'eau et de dichlorométhane, puis extrait au dichlorométhane, séché sur sulfate de magnésium, filtré et concentré à sec. Le résidu est purifié par une première chromatographie sur gel de silice en utilisant l'heptane et l'acétate d'éthyle comme éluants, puis une deuxième chromatographie en utilisant le dichlorométhane et l'acétate d'éthyle comme éluants pour obtenir le produit du titre.

**RMN ¹H** (400 MHz, dmso-d6, 300 K) δ ppm : 7.21 (s, 1 H), 6.59 (d, 2 H), 6.45 (d, 2 H), 3.72 (s, 3 H), 3.55 (t, 2 H), 2.89 (quint, 2 H), 2.65 (t, 2 H), 0.91 (s, 9 H), 0.1 (s, 6 H).

### Préparation 7a" : 4-[(4-{[tert-butyl(diméthyl)silyl]oxy}phényl)amino]-1-(4-chlorobutyl)-5-méthyl-1H-pyrrole-2-carbonitrile

### Stade A : 5-méthyl-1H-pyrrole-2-carbonitrile

A une solution d'acétamidocyanoacétate d'éthyle (50,0 g ; 0,29 mol) dans l'éthanol (1,25 L), est additionné de l'éthanoate de sodium (100 g ; 1,47 mol), puis l'ensemble est agité pendant 10 minutes à 30 °C, puis pendant 10 minutes à 50 °C. A cette température, on ajoute goutte à goutte sur une période de 2 heures une solution de 1,4-dichloro-2-butyne (72,3 g ; 0,587 mol) dans l'éthanol (250 mL), puis l'ensemble est agité au reflux pendant 100 minutes. Après retour à température ambiante, on ajoute une solution aqueuse d'acide chlorhydrique 2 N (588 mL), puis l'éthanol est concentré. Après extraction à l'acétate d'éthyle, la phase organique est lavée avec une solution aqueuse saturée en chlorure de sodium, puis séchée sur sulfate de magnésium, filtrée et concentrée à sec. Le résidu obtenu est distillé sous vide pour obtenir le produit du titre.

**RMN ¹H** (400 MHz, CDCl₃, 300 K) δ ppm : 8.76 (br. s, 1 H), 6.76 (t, 1 H), 5.93-5.96 (m, 1 H), 2.3 (s, 3 H).

### Stade B : 4-bromo-5-méthyl-1H-pyrrole-2-carbonitrile

A une solution du composé obtenu au Stade A (40,7 g ; 0,341 mol) dans un mélange d'acide acétique (325 mL) et de dichlorométhane (122 mL), est additionnée à 10°C sur une période de 75 minutes une solution de brome (59,9 g ; 0,374 mol) dans l'acide acétique (163 mL). L'ensemble est ensuite agité pendant 30 minutes à cette température, puis pendant 1 heure à température ambiante. Le milieu réactionnel est hydrolysé (200 mL) et le dichlorométhane est concentré. Après retour à température ambiante, on ajoute de l'eau (400 mL) et la suspension obtenue est agitée pendant 2 heures à 0 °C. Le précipité est filtré et séché sous vide pour obtenir le produit du titre sans purification.

**RMN ¹H** (400 MHz, CDCl₃, 300 K) δ ppm : 9.37 (br. s, 1 H), 6.78 (d, 1 H), 2.27 (s, 3 H).

### Stade C : 4-bromo-1-(4-chlorobutyl)-5-méthyl-1H-pyrrole-2-carbonitrile

A un mélange d'hydrure de sodium à 60 % dans l'huile (0,95 g ; 23,8 mmol) dans un minimum de *N,N*-diméthylformamide, est additionné le composé obtenu au Stade B (4 g ; 21,6 mmol) dans du N,N-diméthylformamide (120 mL), puis l'ensemble est agité pendant 15 minutes à température ambiante avant d'ajouter le 1,4-dichlorobutane (4,7 mL ; 43,2 mol). On laisse le milieu réactionnel agir à température ambiante pendant 3 jours, avant de le diluer dans de l'eau (1,5 L). Le produit est ensuite extrait à l'acétate d'éthyle. Les phases organiques sont lavées avec une solution aqueuse saturée en chlorure de sodium, puis séchées sur sulfate de magnésium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 6.76 (s, 1 H), 4.05 (t, 2 H), 3.56 (t, 2 H), 2.27 (s, 3 H), 1.87-1.95 (m, 2 H).

### Stade D : 4-[(4-{[tert-butyl(diméthyl)silyl]oxy}phényl)anùno]-1-(4-chlorobutyl)-5-méthyl-1H-pyrrole-2-carbonitrile

Le composé du titre est obtenu selon le procédé décrit au Stade C de la Préparation 1a" à partir du composé obtenu au stade précédent.

**RMN ¹H** (400 MHz, CDCl₃, 300 K) δ ppm : 6.64-6.69 (m, 3 H), 6.46-6.51 (m, 2 H), 4.67 (br. s, 1 H), 4.04 (t, 2 H), 3.57 (t, 2 H), 2.15 (s, 3 H), 1.89-1.99 (m, 2 H), 1.79-1.88 (m, 2 H), 0.96 (s, 9 H), 0.15 (s, 6 H).

### Préparation 8a": 4-[(4-{[tert-butyl(diméthyl)silyl]oxy}phényl)amino]-1-(3-chloropropyl)-5-méthyl-1H-pyrrole- 2-carbonitrile

Le composé du titre est obtenu selon le procédé de la Préparation 7a" en remplaçant le 1,4-dichlorobutane par le bromochloropropane au Stade C.

**RMN** ¹H (400 MHz, CDCl₃, 300 K) δ ppm : 6.68 (s, 1 H), 6.67 (d, 2 H), 6.48 (d, 2 H), 4.67 (br. s, 1 H), 4.18 (t, 2 H), 3.56 (t, 2 H), 2.26 (quint., 2 H), 2.18 (m, 3 H), 0.96 (s, 9 H), 0.15 (s, 6 H).

### Préparation 9a": 3-[(4-{[tert-butyl(diméthyl)silyl]oxy}phényl)amino]-5-(3-chloropropyl)benzonitrile

### Stade A : (3-bromo-5-iodophényl)méthanol

A une solution d'acide 3-bromo-5-iodobenzoïque (10 g ; 30,58 mmol) dans le tétrahydrofurane (70 mL), est additionné au goutte à goutte à 0 °C le complexe de boranetétrahydrofurane 1 M dans le tétrahydrofurane (61,1 mL ; 61,1 mmol), puis l'ensemble est agité pendant 16 heures à température ambiante. Le milieu réactionnel est dilué dans le méthanol (10 mL) et hydrolysé par une solution aqueuse de soude 1 M (100 mL). Après extraction au dichlorométhane, la phase organique est lavée avec une solution aqueuse saturée en chlorure de sodium, puis séchée sur sulfate de magnésium, filtrée et concentrée à sec. Le produit du titre est obtenu sous forme d'un solide brun pâle qui est utilisé au stade suivant sans purification.

**RMN ¹H** (400 MHz, CDCl₃, 300 K) δ ppm : 7.76-7.79 (m, 1 H), 7.63-7.66 (m, 1 H), 7.47-7.50 (m, 1 H), 4.64 (s, 2 H).

### Stade B : 3-bromo-5-iodobenzaldéhyde

A une solution du composé obtenu au Stade A (7,89 g ; 25,2 mmol) dans le dichlorométhane (80 mL), est additionné du pyridinium dichromate (12,3 g ; 32,8 mmol), puis l'ensemble est agité pendant 16 heures à température ambiante. Le milieu réactionnel est filtré sur silice, puis le filtrat est concentré pour fournir le produit du titre sans purification.

**RMN ¹H** (400 MHz, CDCl₃, 300 K) δ ppm : 9.87 (s, 1 H), 8.12 (t, 1 H), 8.10 (t, 1 H), 7.96 (t, 1 H).

### Stade C : 3-bromo-5-iodobenzonitrile

A une solution du composé obtenu au Stade B (6,95 g ; 22,3 mmol) dans le tétrahydrofurane (60 mL), sont additionnés une solution aqueuse d'hydroxyde d'ammonium à 28 % (30 mL) et de l'iode (6,81 g ; 26,8 mmol), puis l'ensemble est agité jusqu'à disparition du composé de départ. Le milieu réactionnel est dilué par une solution aqueuse de sulfite de sodium jusqu'à disparition de la couleur orangée. Après extraction à l'acétate d'éthyle, la phase organique est lavée avec une solution aqueuse saturée en chlorure de sodium, puis séchée sur sulfate de sodium, filtrée et concentrée à sec avec de la silice. Le produit ainsi déposé sur silice est purifié par chromatographie sur gel de silice en utilisant le dichlorométhane et l'heptane comme éluants pour obtenir le produit du titre.

**RMN ¹H** (400 MHz, CDCl₃, 300 K) δ ppm : 8.10 (s, 1 H), 7.92 (s, 1 H), 7.76 (s, 1 H).

### Stade D : 3-bromo-5-(3-oxopropyl)benzonitrile

A une solution du composé obtenu au Stade C (6,05 g; 19,6 mmol) dans le *N,N-*diméthylformamide (85 mL), sont additionnés de l'alcool allylique (2,78 mL ; 39,3 mmol), du chlorure de benzyltriéthylammonium (4,47 g ; 19,6 mmol) et du bicarbonate de sodium (3,30 g; 39,3 mmol). Après une purge à l'azote, l'acétate de palladium(II) (0,13 g; 0,59 mmol) est ajouté, puis l'ensemble est porté à 40 °C pendant 16 heures. Le milieu réactionnel est dilué dans un mélange d'eau (200 mL) et d'acétate d'éthyle (100 mL). Après extraction à l'acétate d'éthyle, la phase organique est lavée avec une solution aqueuse saturée en chlorure de sodium, puis séchée sur sulfate de sodium, filtrée et concentrée à sec. Le résidu est purifié par chromatographie sur gel de silice en utilisant l'acétate d'éthyle et l'heptane comme éluants pour obtenir le produit du titre.

**RMN ¹H** (400 MHz, CDCl₃, 300 K) δ ppm : 9.81 (t, 1 H), 7.64 (t, 1 H), 7.59-7.61 (m, 1 H), 7.44 (t, 1 H), 2.93-3.02 (m, 2 H), 2.81-2.88 (m, 2 H).

### Stade E : 3-bromo-5-(3-hydroxypropyl)benzonitrile

A une solution du composé obtenu au Stade D (2,98 g ; 12,52 mmol) dans le méthanol (30 mL), est additionné par portions le borohydrure de sodium (0,62 g; 16,27 mmol). L'ensemble est agité à température ambiante pendant 30 minutes. Le milieu réactionnel est dilué avec une solution aqueuse de soude 1 M (50 mL). Après extraction au dichlorométhane, la phase organique est lavée avec une solution aqueuse saturée en chlorure de sodium, puis séchée sur sulfate de sodium, filtrée et concentrée à sec pour obtenir le produit du titre sans purification.

**RMN** ¹H (400 MHz, CDCl₃, 300 K) δ ppm : 7.61-7.64 (m, 1 H), 7.59-7.61 (m, 1 H), 7.44 (t, 1 H), 3.68 (t, 2 H), 2.72-2.79 (m, 2 H), 1.83-1.93 (m, 2 H).

### Stade F : 3-bromo-5-(3-chloropropyl)benzonitrile

A une solution du composé obtenu au Stade E (2,57 g ; 10,7 mmol) et de triéthylamine (3,43 mL ; 24,6 mmol) dans le dichlorométhane (30 mL) à 0 °C, est additionné le chlorure de méthanesulfonyle (1,65 mL ; 21,4 mmol). L'ensemble est agité à température ambiante pendant 2 heures, puis le chlorure de tétrabutylammonium (8,92 g ; 32,1 mmol) est ajouté. Le milieu réactionnel est agité pendant 16 heures, puis dilué dans un mélange d'eau et de dichlorométhane. Après extraction à l'acétate d'éthyle, la phase organique est lavée avec une solution aqueuse saturée en chlorure de sodium, puis séchée sur sulfate de sodium, filtrée et concentrée à sec. Le résidu est purifié par chromatographie sur gel de silice en utilisant l'acétate d'éthyle et l'heptane comme éluants pour obtenir le produit du titre.

**RMN ¹H** (400 MHz, CDCl₃, 300 K) δ ppm : 7.65 (t, 1 H), 7.60 (s, 1 H), 7.44 (s, 1 H), 3.53 (t, 2 H), 2.78-2.86 (m, 2 H), 2.04-2.14 (m, 2 H).

### Stade G : 3-[(4-{[tert-butyl(diméthyl)silyl]oxy}phényl)amino]-5-(3-chloropropyl)benzonitrile

Le composé du titre est obtenu selon le procédé décrit Stade C de la Préparation 1a" à partir du composé obtenu au stade précédent.

**RMN ¹H** (400 MHz, CDCl₃, 300 K) δ ppm : 6.78-7.07 (m, 7 H), 5.60 (br. s, 1 H), 3.52 (t, 2 H), 2.71 (t, 2 H), 2.00-2.10 (m, 2 H), 0.99 (s, 9 H), 0.27 (s, 6 H).

### Préparation 10a": N-(4-{[tert-butyl(diméthyl)silyl]oxy}phényl)-1-méthyl-5-[2-(tétrahydro-2H-pyran-2-yloxy)éthyl]-1H-pyrazol-4-amine

### Stade A : 5-(2-{[tert-butyl(diméthyl)silyl]oxy}éthyl)-1-méthyl-1H-pyrazole

Le composé du titre est obtenu selon le procédé décrit au Stade A de la Préparation 6a" en remplaçant le (3-bromopropoxy)-*tert*-butyldiméthylsilane par le (2-bromoéthoxy)-*tert-*butyldiméthylsilane.

**RMN ¹H** (400/500 MHz, CDCl₃, 300 K) δ ppm : 7.35 (d, 1 H), 7 (d, 1 H), 3.8 (s, 3 H), 3.65 (t, 2 H), 2.7 (m, 2 H), 1.85 (m, 2 H), 0.9 (s, 9 H), 0.5 (s, 6 H)
**IR :** v : -Si-O- : 1098 cm⁻¹; -Si-C- : 834 et 772 cm⁻¹.

### Stade B : 2-(4-bromo-1-méthyl-1H-pyrazol-5-yl)éthanol

Le composé du titre est obtenu selon le procédé décrit au Stade B de la Préparation 6a".

**RMN ¹H** (400 MHz, dmso-d6, 300 K) δ ppm : 7.45 (s, 1 H), 4.59 (t, 1 H), 3.79 (s, 3 H), 3.4 (quad, 2 H), 2.7 (t, 2 H), 1.65 (m, 2 H).

**IR :** v : -OH : 3348 cm⁻¹.

### Stade C : 4-bromo-1-méthyl-5-[2-(tétrahydro-2H-pyran-2-yloxy)éthyl]-1H-pyrazole

A une solution du composé obtenu au stade précédent (5,34 g ; 2.4 mmol) dans le dichlorométhane (40 mL), sont additionnés le 3,4-dihyro-2H-pyrane (7 mL ; 6 mmol) et l'acide para-toluènesulfonique (4,6 g; 2,4 mmol), puis l'ensemble est agité pendant 16 heures. Le milieu réactionnel est dilué dans une solution aqueuse saturée en hydrogénocarbonate de sodium. Après extraction au dichlorométhane, la phase organique est séchée sur sulfate de magnésium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.42 (s, 1 H), 4.55 (t, 1 H), 3.8-3.3 (m, 4 H), 3.8 (s, 3 H), 2.71 (m, 2 H), 1.78 (m, 2 H), 1.7-1.4 (m, 6 H).

### Stade D : N-(4-{[tert-butyl(diméthyl)silyl]oxy}phényl)-1-méthyl-5-[2-(tétrahydro-2H-pyran-2-yloxy)éthyll-1H-pyrazol-4-amine

Le composé du titre est obtenu selon le procédé décrit au Stade D de la Préparation 6a" à partir du composé bromé du stade précédent.

**RMN ¹H** (500 MHz, dmso-d6, 300 K) δ ppm : 7.2 (s, 1 H), 6.6 (s, 1 H), 6.55 (d, 2 H), 6.45 (d, 2 H), 4.4 (t, 1 H), 3.7 (s, 3 H), 3.65-3.2 (4m, 4 H), 2.58 (m, 2 H), 1.68 (m, 2 H), 1.6-1.3 (m, 6 H), 0.92 (s, 9 H), 0.1 (s, 6 H).

**IR :** v : >NH : 3356 cm⁻¹; ->C-C-O- : 1240 cm⁻¹.

### Préparation 11a" :

### Stade A : 4-(prop-2-én-1-yloxy)aniline

A une solution du *N*-(allyloxyphényl)acétamide (4 g ; 20,9 mmol) dans l'éthanol (30 mL) est additionnée une solution de soude concentrée (7 mL ; 83,6 mmol). L'ensemble est agité à 100 °C pendant 24 heures. Après retour à température ambiante, l'éthanol du milieu réactionnel est concentré, puis le résidu est repris dans l'eau (100 mL). Après extraction au dichlorométhane, les phases organiques sont séchées sur sulfate de magnésium, filtrées et concentrées à sec pour obtenir le produit du titre sans purification.

**RMN ¹H** (400 MHz, dmso-d6, 300 K) δ ppm : 6.66 (d, 2 H), 6.49 (d, 2 H), 6 (m, 1 H), 5.34 (dq, 1 H), 5.2 (dq, 1 H), 4.59 (s, 2 H), 4.4 (dt, 2 H).

**IR :** v : -NH2 : 3428, 3354 et 3220 cm⁻¹.

### Stade B : 4-fluoro-3-{[4-(prop-2-én-1-yloxy)phényl]amino}benzonitrile

Le composé du titre est obtenu selon le procédé du Stade C de la Préparation 1a" en utilisant le composé obtenu au stade précédent et le 3-bromo-4-fluorobenzonitrile.

**RMN ¹H** (400 MHz, dmso-d6, 300 K) δ ppm : 8.08 (s, 1 H), 7.36 (dd, 1 H), 7.24 (dd, 1 H), 7.19 (m, 1 H), 7.12 (d, 2 H), 6.96 (d, 2 H), 6.05 (m, 1 H), 5.4 (ddt, 1 H), 5.26 (ddt, 1 H), 4.56 (dt, 2 H).

**IR :** v : -NH : 3327 cm⁻¹ ; >CN : 2235 cm⁻¹.

### Préparation 1b : acide 5-[2-(tert-butoxycarbonyl)-5-chlorophényl]-1 2-diméthyl-1H-pyrrole-3-carboxylique

### Stade A : 1,2-diméthyl-1H-pyrrole-3-carboxylate d'éthyle

Le mode opératoire est identique à celui décrit au Stade A de la Préparation 1a.

**RMN ¹H** (400 MHz, CDCl₃, 300 K) δ ppm : 6.50 (d, 1 H), 6.45 (d, 1 H), 4.25 (q, 2 H), 3.51 (s, 3 H), 2.49 (s, 3 H), 1.33 (t, 3 H).

### Stade B : acide 1,2-diméthyl-1H-pyrrole-3-carboxylique

Le mode opératoire est identique à celui décrit au Stade B de la Préparation 1a.

**RMN ¹H** (400 MHz, CDCl₃, 300 K) δ ppm : 11.52 (s, 1 H), 6.62 (d, 1 H), 6.29 (d, 1 H), 3.50 (s, 3 H), 2.41 (s, 3 H).

### Stade C : 2-bromo-4-chlorobenzoate de tert-butyle

A une solution de sulfate de magnésium (1431 g; 11,9 mol) dans du dichlorométhane (10,5 L), sont additionnés de l'acide sulfurique (287 g ; 2,97 mol) sur une période de 30 minutes, puis de l'acide 2- bromo-4-chlorobenzoique (700 g ; 2,97 mol) et 700 mL de *tert-*butanol. Le mélange réactionnel est agité pendant 4 jours à température ambiante, puis filtré. Le filtrat est lavé avec une solution aqueuse d'hydrogénocarbonate de potassium 5 %, puis la phase organique est séchée avec du sulfate de sodium et concentrée à sec puis reconcentrée dans l'heptane (1 L) pour obtenir le produit du titre.

**RMN ¹H** (400 MHz, CDCl₃, 300 K) δ ppm : 7.65 (d+d, 2 H), 7.30 (dd, 1 H), 1.65 (s, 9 H).

### Stade D : acide 5-[2-(tert-butoxycarbonyl)-5-chlorophényl]-1,2-diméthyl-1H-pyrrole-3-carboxylique

A une solution dégazée (par barbotage d'azote pendant 15 minutes) du résidu obtenu au Stade C (187,5 g ; 0,643 mol) et du composé obtenu au Stade B (89,6 g ; 0,643 mol) dans le *N,N*-diméthylformamide (1,87 L), est ajouté du carbonate de potassium (178 g ; 1,29 mol ; préalablement pulvérisé à l'aide d'un broyeur Ultra-Turrax^{®}) en suspension dans de l'acétate d'éthyle. Puis la suspension est dégazée pendant 15 minutes supplémentaires. L'acétate de palladium(II) (7,2 g, 0,003 mol) est ajouté, puis la suspension est chauffée à 100 °C et agitée pendant 18 heures. Après retour à température ambiante, le mélange réactionnel est dilué avec de l'eau (950 mL). Cette même opération est effectuée une seconde fois avec la même quantité du composé obtenu au Stade C.

Les deux solutions sont réunies et lavées avec du méthyl-*tert*-butyléther. Les phases aqueuses à pH=10 sont acidifiées à pH=2 avec une solution aqueuse d'acide chlorhydrique 12 N à une température comprise entre 10 et 20 °C. La suspension obtenue est refroidie à 0 °C, agitée pendant 1 heure puis filtrée. Le solide est lavé avec de l'eau (2 L), puis essoré pendant 1 heure. A une solution du résidu repris dans du méthanol (12 L) est additionné du charbon (375 g).La suspension est chauffée à 40 °C et agitée pendant 2 heures. Le mélange est filtré sur célite^{®} (375 g) et le solide est lavé avec du méthanol. Le filtrat est concentré à sec et le résidu obtenu est dilué dans un mélange d'éthanol (1,3 L) et de méthanol (400 mL). La suspension est distillée ; 400 mL de distillat sont collectés. De l'éthanol (1 L) est chargé afin de poursuivre la distillation jusqu'à ce que 1 L de distillat soit collecté. Après retour à température ambiante, la suspension est agitée pendant 16 heures, puis refroidie à 0 °C et agitée de nouveau pendant 2 heures. Le produit est filtré et lavé avec de l'éthanol froid puis séché sous vide à 60 °C pendant 16 heures. Le produit du titre est obtenu sous la forme d'un solide blanc.

**RMN ¹H** (400 MHz, dmso-d6, 300 K) δ ppm : 10.87-12.20 (s, 1 H), 7.77 (d, 1H), 7.57 (dd, 1 H), 7.44 (d, 1 H), 6.25 (s, 1 H), 3.25 (s, 3 H), 2.51 (s, 3 H), 1.25 (t, 9 H).

### Préparation 2b : acide 5-[2-(tert-butoxycarbonyl)-5-fluorophényl]-1,2-diméthyl-1H-pyrrole-3-carboxylique

Le composé du titre est obtenu selon le procédé de la Préparation 1b en utilisant l'acide 2-bromo-4-fluorobenzoique au Stade C.

**RMN ¹H** (400 MHz, CDCl₃, 300 K) δ ppm : 12.18 (s, 1 H), 7.93 (dd, 1H), 7.14 (td, 1 H), 7.03 (dd, 1 H), 6.52 (s, 1 H), 3.27 (s, 3 H), 2.60 (s, 3 H), 1.33 (t, 9 H).

### Préparation 1b': 5-(3-hydroxypropyl)-3,4-dihydroisoquinoléine-2(1H)-carboxylate de tert-butyle

### Stade A : (2E)-3-(isoquinoléin-5-yl)prop-2-énoate de méthyle

Dans un tube scellé contenant une solution de méthyl acrylate (5,4 mL ; 60,2 mmol), de la triphénylphosphine (0,63 g ; 2,4 mmol), de la triéthylamine (13,4 mL ; 96,0 mmol) et du palladium(II) acétate (0,27 g ; 1,2 mmol) dans du N,N-diméthylformamide (30 mL), est additionné du 5-bromoisoquinoléine (5 g ; 24,1 mmol). De l'azote est barboté dans le mélange pendant 10 minutes, puis le tube est scellé et plongé dans un bain d'huile à 120 °C. Le mélange réactionnel est agité pendant 1,5 heures, puis hydrolysé après retour à température ambiante. Le produit est extrait avec de l'acétate d'éthyle puis les phases organiques sont lavées avec une solution aqueuse saturée en chlorure de sodium, séchées sur sulfate de magnésium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 9.29 (s, 1 H), 8.62 (d, 1H), 8.42 (d, 1 H), 8.03 (d, 1 H), 7.94-8.01 (m, 2 H), 7.64 (t, 1 H), 6.57 (d, 1 H), 3.88 (s, 3 H).

### Stade B : 5-[(1E)-3-méthoxy-3-oxoprop-1-én-1-yl]-3,4-dihydroisoquinoléine-2(1H)-carboxylate de tert-butyle

Une solution du composé obtenu au Stade A (7,24 g ; 34 mmol) et de cyanoborohydrure de sodium (9,6 g ; 152,8 mmol) dans du méthanol (200 mL), est placée à 45°C. Le diéthylétherate de trifluorure de bore (18,9 mL ; 152,8 mmol) est additionné goutte à goutte, puis le mélange réactionnel est agité pendant 20 minutes à cette température, et du di-*tert-*butylcarbonate (8,15 g ; 37,4 mmol) puis de la triéthyamine (14,2 mL ; 101,9 mmol) sont ajoutés. Le milieu réactionnel est agité pendant 15 minutes à 45 °C.Après retour à température ambiante, il est hydrolysé par de l'eau et une solution aqueuse de soude 1 N. Le produit est extrait avec de l'acétate d'éthyle, puis les phases organiques sont lavées avec une solution aqueuse d'acide chlorhydrique et de l'eau, séchées sur sulfate de magnésium, filtrées et concentrées à sec pour obtenir le produit du titre.

**RMN ¹H** (400 MHz, CDCl₃, 300 K) δ ppm : 7.94 (d, 1 H), 7.44 (d, 1 H), 7,21 (t, 1 H), 7.11-7.17 (m, 1 H), 6.35 (d, 1 H), 4.58 (s, 2 H), 3.81 (s, 3 H), 3.67 (t, 2 H), 2.93 (t, 2 H), 1.49 (s, 9 H).

### Stade C : 5-[(1E)-3-hydroxyprop-1-én-1-yl]-3,4-dihydroisoquinoléine-2(1H)-carboxylate de tert-butyle

A une solution d'hydrure de diisobutylaluminium à 25 % massique dans le toluène (50 mL ; 74,7 mmol), est additionnée lentement à -78 °C une solution du composé obtenu au Stade B (10,78 g ; 34 mmol) dans du toluène (200 mL). Le mélange réactionnel est agité pendant 20 minutes à cette température puis du méthanol est ajouté lentement. Après retour à température ambiante, le milieu réactionnel est hydrolysé avec de l'eau et une solution aqueuse de soude 1 N. Le produit est extrait avec de l'acétate d'éthyle, puis les phases organiques sont lavées par une solution aqueuse saturée en chlorure de sodium, séchées sur sulfate de magnésium, filtrées et concentrées à sec pour obtenir le produit du titre.

**RMN ¹H** (400 MHz, CDCl₃, 300 K) δ ppm : 7.33 (d, 1 H), 7.17 (t, 1 H), 7,03 (d, 1 H), 6.80 (d, 1 H), 6.24 (dt, 1 H), 4.54-4.60 (m, 2 H), 4.35 (d, 2 H), 3.65 (t, 2 H), 2.84 (t, 2 H), 1.49 (s, 9 H).

### Stade D : 5-(3-hydroxypropyl)-3,4-dihydroisoquinoléine-2(1H)-carboxylate de tert-butyle

A une solution du composé obtenu au Stade C (9,82 g ; 34,0 mmol) dans du méthanol (300 mL), est additionné du palladium sur charbon (1 g ; 10 % massique). Le mélange réactionnel est hydrogéné pendant 16 heures puis filtré sur Célite^{®}. Le filtrat est concentré à sec pour obtenir le produit du titre.

**RMN ¹H** (400 MHz, CDCl₃, 300 K) δ ppm : 7.32 (d, 1 H), 7.16 (t, 1 H), 7,02 (d, 1 H), 6.79 (d, 1 H), 6.24 (dt, 1 H), 4.57 (s, 2 H), 4.34 (d, 2 H), 3.62-3.67 (m, 2 H), 3.49 (s, 2 H), 2.84 (t, 2 H), 1.49 (s, 9 H).

### Préparation 2b': 5-(4-hydroxybutoxy)-3,4-dihydroisoquinoléine-2(1H)-carboxylate de tert-butyle

### Stade A : 5-hydroxy-3,4-dihydroisoquinoléine-2(1H)-carboxylate de tert-butyle

A une solution de 5-hydroxy-isoquinoléine (20 g ; 137 mmol) dans l'acide acétique (120 mL), est additionné le dioxyde de platine (2 g ; 8,8 mmol). L'ensemble est placé sous atmosphère d'hydrogène (2 bars) pendant 24 heures. Le milieu réactionnel est filtré et le catalyseur est lavé avec du toluène. Le filtrat ainsi obtenu est concentré à sec. Le produit du titre est obtenu sous la forme d'une huile, qui est utilisée pour la suite sans purification.

A une solution du résidu obtenu (1,95 g ; 13 mmol) dans le dichlorométhane (110 mL), on ajoute la diisoprolyléthylamine (9,7 mL ; 57 mmol) et le di-tert-butyldicarbonate (3,69 g, 16,9 mmol), puis l'ensemble est agité pendant 2 heures à température ambiante. Le milieu réactionnel est dilué avec une solution aqueuse saturée en chlorure d'ammonium. Après décantation, la phase organique est lavée avec une solution aqueuse saturée en hydrogénocarbonate de sodium, et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 9.41 (m, 1 H), 6.97 (t, 1 H), 6.64/6.54 (2d, 2 H), 4.42 (m, 2 H), 3.53 (t, 2 H), 2.59 (t, 2 H), 1.42 (s, 9 H).

**IR :** v : -OH : 3294 cm⁻¹ ; >C=O : 1652 cm⁻¹.

### Stade B : 5-[4-(tétrahydro-2H-pyran-2-yloxy)butoxy]-3,4-dihydroisoquinoléine-2(1H)-carboxylate de tert-butyle

A une solution du composé obtenu au Stade A (1 g ; 4 mmol) dans l'acétonitrile (15 mL), sont additionnés le 2-(4-bromobutoxy)-tétrahydropyrane (0,77 mL ; 4,2 mmol) et le carbonate de césium (1,4 g, 4,2 mmol), puis l'ensemble est agité pendant 18 heures à 70 °C. Le milieu réactionnel est dilué avec de l'acétate d'éthyle et de l'eau. Après décantation, la phase organique est lavée avec une solution aqueuse saturée en chlorure de sodium, puis séchée sur sulfate de magnésium, filtrée et contrée à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.12 (dd, 1 H), 6.79 (d, 1 H), 6.72 (d, 1 H), 4.55 (t, 1 H), 4.45 (s, 2 H), 3.98 (t, 2 H), 3.74/3.42 (2*m, 2 H), 3.68/3.41 (2*m, 2 H), 3.54 (t, 2 H), 2.63 (t, 2 H), 1.79 (m, 2 H), 1.74-1.4 (m, 6 H), 1.68 (m, 2 H), 1.42 (s, 9 H).

**IR :** v : >C=O : 1693 cm⁻¹ ; >C-O-C< : 1033 cm⁻¹.

### Stade C : 5-(4-hydroxybutoxy)-3,4-dihydroisoquinoléine-2(1H)-carboxylate de tert-butyle

A une solution du composé obtenu au Stade B (1,29 g; 3,18 mmol) dans le méthanol (50 mL), est additionné le *para*-toluènesulfonate de pyridinium (0,16 g ; 0,64 mmol), puis l'ensemble est agité pendant 8 heures à 60 °C. Le milieu réactionnel est dilué avec du dichlorométhane et une solution aqueuse saturée en chlorure d'ammonium. Après décantation, la phase organique est lavée avec de l'eau, puis séchée sur sulfate de magnésium, filtrée et contrée à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.12 (dd, 1 H), 6.78 (d, 1 H), 6.72 (d, 1 H), 4.45 (s, 2 H), 4.42 (t, 1 H), 3.96 (t, 2 H), 3.54 (t, 2 H), 3.45 (m, 2 H), 2.62 (t, 2 H), 1.75 (m, 2 H), 1.58 (m, 2 H), 1.42 (s, 9 H).

**IR :** v : -OH : 3600-3100 cm⁻¹; >C=O : 1693 cm⁻¹.

### Préparation 3b': 5-hydroxy-3,4-dihydroisoquinoléine-2(1H)-carboxylate de tert-butyle

A une solution de 5-hydroxy-isoquinoléine (20 g ; 137 mmol) dans l'acide acétique (120 mL), est additionné le dioxyde de platine (2 g ; 8,8 mmol). L'ensemble est placé sous atmosphère d'hydrogène (2 bars) pendant 24 heures. Le milieu réactionnel est filtré et le catalyseur est lavé avec du toluène. Le filtrat ainsi obtenu est concentré à sec. Le produit du titre est obtenu sous la forme d'une huile, qui est utilisée pour la suite sans purification.

A une solution du résidu obtenu (1,95 g ; 13 mmol) dans le dichlorométhane (110 mL), sont additionnés la diisoprolyléthylamine (9,7 mL ; 57 mmol) et le di-tert-butyldicarbonate (3,69 g, 16,9 mmol), puis l'ensemble est agité pendant 2 heures à température ambiante. Le milieu réactionnel est dilué avec une solution aqueuse saturée en chlorure d'ammonium. Après décantation, la phase organique est lavée avec une solution aqueuse saturée en hydrogénocarbonate de sodium, et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 9.41 (m, 1 H), 6.97 (t, 1 H), 6.64/6.54 (2d, 2 H), 4.42 (m, 2 H), 3.53 (t, 2 H), 2.59 (t, 2 H), 1.42 (s, 9 H).

**IR :** v : -OH : 3294 cm-1 ; >C=O : 1652 cm-1.

### Préparation 4b' : 5-hydroxy-3-(morpholin-4-ylméthyl)-3,4-dihydroisoquinoléine-2(1H)-carboxylate de tert-butyle

### Stade A : 3-méthoxy-2-méthylbenzoate de méthyle

A une solution d'acide 2-méthyl-3-méthoxybenzoïque (20 g ; 0,12 mol) dans le méthanol (200 mL), est additionné à 0 °C goutte à goutte le chlorure de thionyle (17,5 mL ; 0,24 mol). Le milieu réactionnel est porté à reflux pendant 2 heures. Après retour à température ambiante, le milieu réactionnel est concentré puis dilué dans un mélange d'acétate d'éthyle et d'une solution aqueuse de soude 1 N. Après décantation, la phase organique est lavée à l'eau et une solution aqueuse saturée en chlorure de sodium, puis séchée sur sulfate de magnésium, filtrée et concentrée à sec. Le produit du titre est obtenu sous la forme d'une huile, qui est utilisée au stade suivant sans purification.

**RMN ¹H** (400 MHz, CDCl₃, 300 K) δ ppm : 7.39 (dd, 1 H), 7.19 (t, 1 H), 6.98 (d, 1 H), 3.89 (s, 3 H), 3.84 (s, 3 H), 2.42 (s, 3 H).

**IR :** v : >C=O : 1719 ; >C-O-C 1254 et 1066 cm⁻¹.

### Stade B : 2-(bromométhyl)-3-méthoxybenzoate de méthyle

A une solution du composé obtenu au Stade A (19,8 g ; 0,11 mol) dans le tétrachlorure de carbone (100 mL), sont additionnés le *N*-bromosuccinimide (19,56 g; 0,18 mol) et l'azoisobutyronitrile (2 g ; 0,012 mol). Le milieu réactionnel est porté à à reflux pendant 3 heures. Après retour à température ambiante, le milieu réactionnel est dilué dans un mélange de dichlorométhane et d'eau. Après décantation, la phase organique est lavée avec une solution aqueuse saturée en chlorure de sodium, puis séchée sur sulfate de sodium, filtrée et concentrée à sec. Le résidu est repris dans le dichlorométhane pour fournir le produit du titre sous la forme d'un solide blanc, qui est utilisé au stade suivant sans purification.

**RMN ¹H** (400 MHz, CDCl₃, 300 K) δ ppm : 7.51 (d, 1 H), 7.31 (t, 1 H), 7.08 (d, 1 H), 5.05 (s, 2 H), 3.91 (2s, 6 H).

**IR :** v : >C=O : 1713 cm⁻¹.

### Stade C : (acétylamino)[2-méthoxy-6-(méthoxycarbonyl)benzyl]propanedioate de diéthyle

A une suspension d'hydrure de sodium (3,42 g ; 85,6 mmol) dans le *N,N-*diméthylformamide, est additionnée, goutte à goutte, à une température inférieure à 30 °C une solution d'acétamidomalonate de diéthyle (16,9 g; 77,8 mmol) dans le *N,N-*diméthylformamide (100 mL). Le milieu réactionnel est agité pendant 15 minutes, puis on additionne goutte à goutte à température ambiante une solution du composé obtenu au Stade B (21,2 g ; 81,67 mmol). Après un contact de 18 heures, le milieu réactionnel est concentré puis dilué dans un mélange d'acétate d'éthyle et d'une solution aqueuse saturée en hydrogénocarbonate de sodium. Après extraction à l'acétate d'éthyle, les phases organiques sont réunies, lavées à l'eau et une solution aqueuse saturée en chlorure de lithium, puis séchées sur sulfate de sodium, filtrées et concentrées à sec. Le résidu est repris dans l'éther de diisopropyle pour fournir le produit du titre sous la forme d'un solide blanc cassé, qui est utilisé au stade suivant sans purification.

**RMN ¹H** (400 MHz, dmso-d6, 300 K) δ ppm : 7.68 (s, 1 H), 7.3 (t, 1 H), 7.1 (2d, 2 H), 4.15/4.05 (2m, 4 H), 3.81 (s, 2 H), 3.71/3.7 (2s, 6 H), 1.79 (s, 3 H), 1.15 (t, 6 H).

**IR :** v : -NH : 3367 ; >C=O : 1755, 1732 et 1707 ; >C=O : 1668 ; >C=C< : 1600 cm⁻¹.

### Stade D : acide 5-méthoxy-1-oxo-1,2,3,4-tétrahydroisoquinoléine-3-carboxylique

A une solution du composé obtenu au Stade C (8,1 g ; 20 mmol) dans une solution aqueuse d'acide chlorhydrique 5 N, est additionné l'acide acétique (40 mL). Le milieu réactionnel est porté à reflux pendant 18 heures, puis il est filtré après retour à température ambiante. Le précipité est rincé avec une solution aqueuse d'acide chlorhydrique 5 N et du toluène. Après séchage sous vide, le produit du titre est obtenu sous la forme d'un solide crème, lequel est utilisé au stade suivant sans purification.

**RMN ¹H** (400 MHz, dmso-d6, 300 K) δ ppm : 12.75 (s, 1 H), 7.98 (d, 1 H), 7.48 (d, 1 H), 7.3 (t, 1 H), 7.15 (d, 1 H), 4.2 (m, 1 H), 3.8 (s, 3 H), 3.28/3.05 (m, 2 H).

**IR :** v : -NH/OH : 3215 et 3000 à 2000 ; >C=O : 1715 et 1627 cm⁻¹.

### Stade E : 5-méthoxy-3-(morpholin-4-ylcarbonyl)-3,4-dihydroisoquinoléin-1(2H)-one

A une solution du composé obtenu au Stade D (11,1 g ; 50 mmol) dans du dichlorométhane (150 mL), sont additionnés successivement la morpholine (4,4 mL ; 50 mmol), le 1-hydroxybenzotriazole (6,7 g ; 50 mmol), le chlorhydrate de 1-(3-diméthylaminopropyl)-3-éthylcarbodiimide (9,6 g; 50 mmol) et la diisopropyléthylamine (20 mL ; 115,2 mmol). L'ensemble est ensuite agité pendant une nuit à température ambiante. Le milieu réactionnel est dilué avec un mélange de dichlorométhane et d'eau. Après décantation, la phase organique est lavée avec une solution aqueuse saturée en chlorure d'ammonium et une solution aqueuse saturée en chlorure de sodium, puis séchée sur sulfate de magnésium, filtrée et concentrée à sec. Le résidu est repris dans le dichlorométhane, filtré et rincé à l'isopropanol chaud pour obtenir le produit du titre, lequel est utilisé au stade suivant sans purification.

**RMN ¹H** (400 MHz, dmso-d6, 300 K) δ ppm : 7.71 (d, 1 H), 7.45 (d, 1 H), 7.29 (t, 1 H), 7.11 (d, 1 H), 4.7 (m, 1 H), 3.81 (s, 3 H), 3.65-3.3 (massif, 8 H), 3.05/2.95 (2*dd, 2 H). **IR :** v : -NH : 3284 ; >C=O : 1676 ; >C-O-C< : 1268 et 1248 cm⁻¹.

### Stade F : 5-méthoxy-3-(morpholin-4-ylméthyl)-1,2,3,4-tétrahydroisoquinoléine

A une solution du composé obtenu au Stade E (5 g ; 17,2 mmol) dans du tétrahydrofurane (300 mL), est additionnée goutte à goutte une solution 2 M du complexe borane / diméthylsulfure dans le tétrahydrofurane (43 mL ; 86 mmol). L'ensemble est ensuite agité à reflux pendant 5 heures puis à température ambiante pendant une nuit. Une solution aqueuse d'acide chlorhydrique 5 N est additionnée goutte à goutte et le milieu réactionnel est porté à reflux pendant 8 heures. Enfin, une solution aqueuse de soude est additionnée à 0 °C jusqu'à atteindre un pH basique, et le milieu réactionnel est dilué avec du dichlorométhane. Après extraction, les phases organiques sont lavées avec de l'eau, puis séchées sur sulfate de magnésium, filtrées et concentrées à sec. Le résidu est purifié par chromatographie sur gel de silice en utilisant le dichlorométhane et l'éthanol ammoniacal comme éluants pour obtenir le produit du titre.

**RMN ¹H** (400 MHz, dmso-d6, 300 K) δ ppm : 7.61 (d, 1 H), 7.09 (t, 1 H), 6.72 (d, 1 H), 3.9 (s, 2 H), 3.75 (s, 3 H), 3.6 (t, 4 H), 2.9 (m, 1 H), 2.62/2.05 (2dd, 2 H), 2.5-2.3 (m, 6 H). **IR :** v : -NH : 3203 cm⁻¹.

### Stade G : 3-(morpholin-4-ylméthyl)-1,2,3,4-tétrahydroisoquinoléin-5-ol

A une solution du composé obtenu au Stade F (5,6 g ; 21 mmol) dans du dichlorométhane (60 mL), est additionnée goutte à goutte à une tempéarture de -10 °C une solution 1 M de tribromoborane dans le dichlorométhane (100 mL ; 100 mmol). L'ensemble est ensuite agité à cette température pendant 3 heures, puis réchauffé à 10 °C en 1 heure. Le milieu réactionnel est dilué à 0 °C avec du dichlorométhane et une solution aqueuse saturée en hydrogénocarbonate de sodium, puis filtré pour obtenir un solide blanc. Après décantation, la phase aqueuse est extraite avec de l'acétate d'éthyle puis les phases organiques sont concentrées. Le résidu obtenu ainsi que le précipité blanc sont réunis pour obtenir le produit du titre, lequel est utilisé au stade suivant sans purification.

**RMN ¹H** (400 MHz, dmso-d6, 300 K) δ ppm : 9.2 (br. s, 1 H), 6.89 (t, 1 H), 6.59 (d, 1 H), 6.48 (d, 1 H), 3.81 (s, 2 H), 3.6 (t, 4 H), 2.89 (m, 1 H), 2.6/2.02 (2dd, 2 H), 2.5-2.25 (m, 6 H).

**IR :** v : -NH/OH : 3412 et 3000 à 2500 ; >C=C< : 1615 cm⁻¹.

### Stade H : 5-hydroxy-3-(morpholin-4-ylméthyl)-3,4-dihydroisoquinoléine-2(1H)-carboxylate de tert-butyle

A une solution du composé obtenu au Stade G (4,1 g ; 16,5 mmol) dans le dichlorométhane (100 mL), sont additionnés la diisoprolyléthylamine (7,4 mL ; 72,6 mmol) et le di-*tert-*butyldicarbonate (7,9 g, 36,3 mmol), puis l'ensemble est agité pendant 18 heures à température ambiante. Le milieu réactionnel est dilué avec une solution aqueuse saturée en chlorure d'ammonium. Après décantation, la phase organique est lavée avec une solution aqueuse saturée en hydrogénocarbonate de sodium et une solution aqueuse saturée en chlorure de sodium, puis séchée sur sulfate de magnésium, filtrée et concentrée à sec. Le résidu est dilué dans une solution 1 M de potasse dans le méthanol. Après un contact de 2 heures à température ambiante, le milieu réactionnel est dilué avec du dichlorométhane et une solution aqueuse saturée en chlorure d'ammonium. Après extraction au dichlorométhane, les phases organiques sont lavées avec de l'eau, séchées sur sulfate de magnésium, filtrées et concentrées. Le résidu est purifié par chromatographie sur gel de silice en utilisant le dichlorométhane et le méthanol comme éluants pour obtenir le produit du titre.

**RMN ¹H** (400 MHz, dmso-d6, 300 K) δ ppm : 9.41 (br. s, 1 H), 6.97 (t, 1 H), 6.62 (d, 1 H), 6.55 (d, 1 H), 4.7-4.4 (d+m, 2 H), 4.08 (m, 1 H), 3.52 (m, 4 H), 2.75-2.2 (m, 7 H), 2.08 (dd, 1 H), 1.42 (br. s, 9 H).

**IR :** v : -OH : 3295 ; >C=O : 1689 et 1656 cm⁻¹.

### Préparation 5b': 4-(2-hydroxyéthyl)-3,4-dihydroisoquinoléine-2(1H)-carboxylate de tert-butyle

### Stade A : 4-(prop-2-én-1-yl)isoquinoléine

Une solution de 4-bromoisoquinoléine (25 g ; 0,12 mol) et de carbonate de potassium (50 g ; 0,36 mol) dans un mélange d'eau (125 mL) et de diméthoxyéthane (375 mL) est dégazée à l'aide d'un filet d'azote. On additionne ensuite du tétrakis(triphénylphosphine)palladium(0) (7 g ; 0,006 mol) et du pinacolate d'allylboronique (35 mL ; 0,18 mol). De l'azote est barboté dans le mélange pendant 30 minutes, puis celui-ci est porté à reflux et agité pendant 18 heures. Après retour à température ambiante, le milieu réactionnel est hydrolysé. Le produit est extrait avec de l'acétate d'éthyle, puis les phases organiques sont lavées avec une solution aqueuse saturée en chlorure de sodium, séchées sur sulfate de magnésium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 9.15 (s, 1 H), 8.39 (s, 1H), 7.98 (d, 2 H), 7.71 (ddd, 1 H), 7.60 (ddd, 1 H), 6.08 (dddd, 1 H), 5.05-5.15 (m, 2 H), 3.78 (d, 2 H).

### Stade B : 2-(isoquinoléin-4-yl)éthanol

Le composé obtenu au Stade A (14 g ; 78 mmol) est mis en solution dans un mélange de dichlorométhane (180 mL) et de méthanol (180 mL). Dans la solution ainsi obtenue, on fait buller de l'ozone à l'aide d'un diffuseur de gaz à -78 °C pendant 1,5 heures, puis de l'air pendant 10 minutes, et enfin de l'azote pendant la même durée. Le mélange réactionnel est maintenu à 0 °C, et du borohydrure de sodium (8,83 g ; 233 mmol) est ajouté par portions. Après un temps de contact de 18 heures à température ambiante, le milieu est dilué dans un mélange d'eau et d'une solution aqueuse saturée en chlorure d'ammonium. Le produit est extrait avec de l'acétate d'éthyle, puis les phases organiques sont lavées par une solution aqueuse saturée en chlorure de sodium, séchées sur sulfate de magnésium, filtrées et concentrées à sec. Le résidu est purifié par chromatographie sur gel de silice en utilisant l'acétate d'éthyle et le méthanol comme éluants pour obtenir le produit du titre.

**RMN ¹H** (400 MHz, CDCl₃, 300 K) δ ppm : 9.00 (s, 1 H), 8.37 (s, 1H), 8.03 (d, 1 H), 7.92 (d, 1 H), 7.74 (ddd, 1 H), 7.61 (ddd, 1 H), 4.01 (t, 2 H), 3.29 (t, 2 H), 2.35 (s, 1 H).

### Stade C : 4-(2-hydroxyéthyl)-3,4-dihydroisoquinoléine-2(1H)-carboxylate de tert-butyle

Le composé du titre est obtenu selon le procédé décrit au Stade B de la Préparation 1b' en utilisant le composé du stade précédent comme produit de départ.

**RMN ¹H** (400 MHz, CDCl₃, 300 K) δ ppm : 7.16-7.22 (m, 3 H), 7.07-7.13 (m, 1H), 4.87 (d, 1 H), 4.32 (d, 1 H), 4.19 (s, 1 H), 3.73-3.87 (m, 2 H), 3.19 (d, 1 H), 2.97-3.04 (m, 1 H), 1.81 (q, 2 H), 1.65 (s, 1 H), 1.50 (s, 9 H).

### Préparation 6b': 4-hydroxy-1,3-dihydro-2H-isoindole-2-carboxylate de tert-butyle

### Stade A : 2,2-diméthylpropanoate de furan-2-yle

A une solution de 2-(5H)-furanone (22 g ; 0,26 mol) et de chlorure de triméthylacétyle (38 g ; 0,31 mol) dans l'acétonitrile (50 mL), est additionnée une solution de triéthylamine (43.5 mL ; 0,31 mol) dans l'acétonitrile (11 mL). Le mélange réactionnel est agité pendant 3 jours à température ambiante, puis la suspension résultante est filtrée et rincée avec de l'éther méthyl-tert-butylique. La phase organique est lavée avec une solution aqueuse saturée en bicarbonate de sodiumet une solution aqueuse saturée en chlorure de sodium, puis séchée sur sulfate de magnésium, filtrée et concentrée sous vide. L'huile résultante est distillée sous vide (15 Torr, fractions collectées à 76-78 °C) pour obtenir le produit du titre.

**RMN ¹H** (400 MHz, CDCl₃, 300 K) δ ppm : 7.05 (dd, 1 H), 6.36 (dd, 1 H), 5.86 (dd, 1 H), 1.34 (s, 9 H).

### Stade B : 2,2-diméthylpropanoate de 1,3-dioxo-3,3a,7,7a-tétrahydro-4,7-epoxy-2-benzofuran-4(1H)-yle

A une solution d'anhydride maléique (24,9 g ; 0,25 mol) fraîchement broyé au mortier dans de l'éther diéthylique (207 mL), est additionné le composé obtenu au Stade A (38,76 g ; 0,23 mol). Le mélange réactionnel est agité à température ambiante pendant 16 heures. La suspension beige résultante est filtrée, et le filtrat est concentré jusqu'à environ 50 mL puis filtré à nouveau. Les solides ainsi obtenus sont réunis et séchés sous vide pour donner le produit du titre.

**RMN ¹H** (400 MHz, dmso-d6, 300 K) δ ppm : 6.77 (dd, 1 H), 6.69 (d, 1 H), 5.33 (d, 1 H), 3.66 (s, 2 H), 1.22 (s, 9 H).

### Stade C : 4-hydroxy-2-benzofurane-1,3-dione

A une solution d'acide sulfurique concentré (80 mL) refroidie à -15 °C, est additionné par portions le composé obtenu au Stade B (36,5 g ; 0,16 mol). Le mélange est agité pendant 15 minutes à -15 °C, puis versé sur de l'eau glacée. Le solide formé est ensuite filtré, rincé avec de l'eau et séché sous vide pour donner le produit du titre.

**RMN ¹H** (400 MHz, dmso-d6, 300 K) δ ppm : 11.72 (s, 1 H), 7.77 (dd, 1 H), 7.45 (d, 1 H), 7.33 (d, 1 H).

### Stade D : 4-hydroxy-2-(4-méthoxybenzyl)-1H-isoindole-1,3(2H)-dione

A une solution du composé obtenu au Stade C (24,76 g ; 0,15 mol) dans l'acide acétique (150 mL), est additionnée la 4-méthoxybenzylamine (21,7 mL ; 0,17 mol), puis le mélange est porté à reflux pendant 5 heures. Après retour à température ambiante, de l'eau (200 mL) est ajouté au mélange, lequel est encore agité pendant 1 heure. La suspension est filtrée et le solide est rincé avec de l'eau. Le produit brut est dissous dans de l'acétate d'éthyle et la phase organique est lavée avec une solution aqueuse saturée en bicarbonate de sodium, une solution aqueuse saturée en chlorure d'ammonium et enfin avec une solution aqueuse saturée en chlorure de sodium, séchée sur sulfate de sodium, filtrée et concentrée sous vide pour donner le produit du titre.

**RMN ¹H** (400 MHz, CDCl₃, 300 K) δ ppm : 7.54 (t, 1 H), 7.32-7.40 (m, 3 H), 7.12 (d, 1 H), 6.84 (d, 2 H), 4.73 (s, 2 H), 3.77 (s, 3 H).

### Stade E : 2-(4-méthoxybenzyl)-2,3-dihydro-1H-isoindol-4-ol

A une suspension d'hydrure d'aluminum lithium (11,9 g; 0,31 mol) dans le tétrahydrofurane (150 mL) à 0 °C, est additionnée goutte à goutte, tout en conservant la température interne du mélange en dessous de à 20 °C, une solution du composé obtenu au Stade D (35,5 g ; 0,13 mol) dans le tétrahydrofurane (250 mL). Une fois l'addition terminée, le mélange est chauffé au reflux. Le mélange est agité pendant 2 heures à reflux, puis refroidi jusqu'à 0°C. De l'acétate d'éthyle est ajouté lentement, tout en maintenant la température interne du mélange en dessous de 20 °C. Lorsque l'on n'observe plus d'effet exothermique lors de l'ajout d'acétate d'éthyle, le mélange est dilué avec de l'acétate d'éthyle et une solution aqueuse 1,5 N de sel de Rochelle. Le mélange est agité vigoureusement pendant 2 heures à température ambiante. Après décantation, la phase aqueuse est lavée avec de l'acétate d'éthyle. Les phases organiques sont réunies et lavées avec une solution aqueuse saturée en chlorure de sodium, séchées sur sulfate de magnésium, filtrées et concentrées sous vide pour donner le produit du titre.

**RMN ¹H** (400 MHz, CDCl₃, 300 K) δ ppm : 7.27-7.34 (m, 2 H), 6.95 (t, 1 H), 6.81-6.90 (m, 2 H), 6.68 (d, 1 H), 6.39 (d, 1 H), 3.89 (d, 4 H), 3.83 (s, 2 H), 3.80 (s, 3 H).

### Stade F : 4-hydroxy-1,3-dihydro-2H-isoindole-2-carboxylate de tert-butyle

A une solution du composé obtenu au Stade E (18,2 g ; 71,3 mmol) dans le méthanol (325 mL) et l'acide acétique (8,2 mL), est additionné du palladium sur charbon (10 % massique). Le réacteur est scellé, purgé à l'azote puis à l'hydrogène. Le milieu réactionnel est soumis à une pression d'hydrogène de 45 psi et agité pendant 4 heures à température ambiante. Le mélange réactionnel est filtré et rincé avec du méthanol, puis le filtrat est concentré sous vide. A une solution du produit brut dans le méthanol (200 mL), sont additionnés la triéthylamine (40 mL ; 0,29 mol) et le di-tert-butyl dicarbonate (15,6 g; 71,3 mmol). Le milieu est agité à température ambiante pendant 16 heures. Le solvant est évaporé sous vide et le résidu est dilué dans de l'acétate d'éthyle. La phase organique est lavée avec une solution aqueuse 2 N d'acide chlorhydrique, une solution aqueuse saturée en bicarbonate de sodium, et avec une solution aqueuse saturée en chlorure de sodium. Puis, elle est séchée sur sulfate de magnésium, filtrée et concentrée sous vide. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.11-7.17 (m, 1 H), 7.02 (s, 0.5 H), 6.68-6.84 (m, 2 H), 5.98 (s, 0.5 H), 4.63-4.85 (m, 4 H), 1.51-1.55 (m, 9 H).

### Préparation 7b': 5-hydroxy-3-méthyl-3,4-dihydroisoquinoléine-2(1H)-carboxylate de tert-butyle

### Stade A : 3-(benzyloxy)-2-(prop-2-én-1-yl)benzaldéhyde

A une solution du 2-allyl-3-hydroxy-benzaldéhyde (20 g ; 0,12 mol) dans l'acétonitrile (400 mL), sont additionnés le bromure de benzyle (16 mL ; 0,13 mol) et le carbonate de potassium (18 g ; 0,13 mol). Le mélange réactionnel est agité pendant 3 jours à température ambiante, puis versé dans un mélange de glace et d'une solution aqueuse saturée en hydrogénocarbonate de sodium. Après extraction à l'acétate d'éthyle, les phases organiques sont séchées sur sulfate de magnésium, filtrées et concentrées sous vide. Le résidu est purifié par chromatographie sur gel de silice en utilisant le dichlorométhane comme éluant pour obtenir le produit du titre.

**RMN ¹H** (400 MHz, dmso-d6, 300 K) δ ppm : 10.2 (s, 1 H), 7.5-7.3 (m, 8 H), 6 (ddt, 1 H), 5.2 (s, 2 H), 5-4.85 (m, 2 H), 3.85 (dt, 2 H).

**IR :** v : >C=O : 1681 cm⁻¹.

### Stade B : N-benzyl-1-[3-(benzyloxy)-2-(prop-2-én-1-yl)phényl]méthananùne

A une solution du composé obtenu au Stade A (20 g ; 0,078 mol) dans le dichlorométhane (800 mL), sont additionnés la benzylamine (10 mL ; 0,078 mol) et par portions le triacétoxyborohydrure de sodium (25 g; 0,118 mol). Le mélange réactionnel est agité pendant 16 heures à température ambiante, puis une solution aqueuse de soude 1 N et de glace est additionnée. Après extraction dans le dichlorométhane, les phases organiques sont lavées avec une solution aqueuse de soude 1 N, puis avec une solution aqueuse saturée en chlorure de sodium, et séchées sur sulfate de magnésium, filtrées et concentrées sous vide. Le résidu est purifié par chromatographie sur gel de silice en utilisant le dichlorométhane et l'éthanol ammoniacal comme éluants pour obtenir le produit du titre.

**RMN ¹H** (400 MHz, dmso-d6, 300 K) δ ppm : 7.48-7.22 (m, 10 H), 7.14 (t, 1 H), 6.99 (d, 1 H), 6.95 (d, 1 H), 5.85 (m, 1 H), 5.09 (s, 2 H), 4.85 (m, 1 H), 4.76 (m, 1 H), 3.7 (s, 2 H), 3.61 (s, 2 H), 3.44 (d, 2 H), 2.35 (br. s, 1H).

### Stade C : 2-benzyl-5-(benzyloxy)-3-méthyl-1,2,3,4-tétrahydroisoquinoléine

A une solution du composé obtenu au Stade B (18,9 g ; 0,055 mol) dans le tétrahydrofurane (1 L), est additionnée goutte à goutte à 60 °C une solution de *n*-butyllithium 1,5 N dans l'hexane (40 mL ; 0,06 mol). A la fin de l'addition, le mélange réactionnel est refroidi jusqu'à température ambiante puis neutralisé avec de l'eau. Après extraction à l'éther, les phases organiques sont lavées avec une solution aqueuse saturée en chlorure de sodium et séchées sur sulfate de magnésium, filtrées et concentrées sous vide. Le résidu est purifié par chromatographie sur gel de silice en utilisant l'heptane et l'éthanol ammoniacal comme éluant pour obtenir le produit du titre.

**RMN ¹H** (400 MHz, dmso-d6, 300 K) δ ppm : 7.5-7.2 (m, 5 H), 7.5-7.2 (m, 5 H), 7.03 (t, 1 H), 6.83 (d, 1 H), 6.58 (d, 1 H), 5.1 (s, 2 H), 3.76 (d, 1 H), 3.53 (d, 1 H), 3.58 (d, 1 H), 3.49 (d, 1 H), 3.05 (m, 1 H), 2.8 (dd, 1 H), 2.49 (dd, 1 H), 1.09 (d, 3 H).

### Stade D : 3-méthyl-1,2,3,4-tétrahydroisoquinoléin-5-ol

A une solution du composé obtenu au Stade C (13,47 g ; 39,22 mmol) dans du méthanol (250 mL), sont additionnés une solution aqueuse d'acide chlorhydrique 1 N (58,8 mL ; 58,8 mmol), puis du palladium sur charbon (10 % massique). Le ballon est mis sous pression d'hydrogène et le milieu réactionnel est agité pendant 48 heures à température ambiante. Le mélange réactionnel est filtré et rincé avec du méthanol, puis le filtrat est concentré sous vide. Le résidu est repris dans l'éthanol puis filtré pour obtenir le produit du titre.

**RMN ¹H** (400 MHz, dmso-d6, 300 K) δ ppm : 9.75 (s, 1 H), 9.3 (br. s, 1 H), 7.05 (t, 1 H), 6.75 (d, 1 H), 6.65 (d, 1 H), 4.21 (m, 2 H), 3.48 (m, 1 H), 2.95 (dd, 1 H), 2.47 (dd, 1 H), 1.4 (d, 3 H).

**IR :** v : -OH : 3226 cm⁻¹ ; -NH₂⁺ : 3300-3400 cm⁻¹.

### Stade E : 5-hydroxy-3-méthyl-3,4-dihydroisoquinoléine-2(1H)-carboxylate de tert-butyle

A une solution du composé obtenu au Stade D (5 g ; 25,2 mmol) dans le dichlorométhane (250 mL), sont additionnés à 0 °C la triéthylamine (7,38 mL ; 52,9 mmol) et le di-tert-butyl dicarbonate par portions (5,5 g ; 25,2 mmol). Le milieu réactionnel est agité à cette température pendant 3 heures, puis à température ambiante pendant 20 heures. Le solvant est évaporé sous vide et le résidu est dilué dans de l'acétate d'éthyle. La phase organique est lavée avec une solution aqueuse saturée en chlorure d'ammonium, de l'eau, une solution aqueuse saturée en chlorure de sodium, puis séchée sur sulfate de magnésium, filtrée et concentrée sous vide. Le résidu est purifié par chromatographie sur gel de silice en utilisant le cyclohexane et l'acétate d'éthyle comme éluants pour obtenir le produit du titre.

**RMN ¹H** (400 MHz, dmso-d6, 300 K) δ ppm : 9.4 (br. s, 1 H), 6.98 (t, 1 H), 6.67 (d, 1 H), 6.6 (d, 1 H), 4.59 (d, 1 H), 4.15 (d, 1 H), 4.45 (m, 1 H), 2.65 (d, 2 H), 1.42 (s, 9 H), 0.99 (d, 3 H).

**IR :** v : -OH : 3308 cm⁻¹ ; >C=O : 1655 cm⁻¹.

### Préparation 8b' : 5-(3-iodopropoxy)-3,4-dihydroisoquinoléine-2(1H)-carboxylate de tert-butyle

### Stade A : 5-(3-chloropropoxy)-3,4-dihydroisoquinoléine-2(1H)-carboxylate de tert-butyle

A une solution du composé obtenu à la Préparation 3b' (1 g ; 4 mmol) dans l'acétonitrile (20 mL), sont additionnés le bromo-chloropropane (0,48 mL ; 4,8 mmol) et le carbonate de potassium (1,1 g, 8 mmol), puis l'ensemble est agité pendant 18 heures à 70 °C. Le milieu réactionnel est dilué avec de l'acétate d'éthyle et de l'eau. Après décantation, la phase organique est lavée avec de l'eau, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.14 (t, 1 H), 6.82 (d, 1 H), 6.75 (d, 1 H), 4.46 (s, 2 H), 4.08 (t, 2 H), 3.82 (t, 2 H), 3.55 (t, 2 H), 2.64 (t, 2 H), 2.18 (quint, 2 H), 1.42 (s, 9 H).

**IR :** v : >C=O : 1692 cm⁻¹ ; >C-O-C< : 1241/1164/1112 cm⁻¹.

### Stade B : 5-(3-iodopropoxy)-3,4-dihydroisoquinoléine-2(1H)-carboxylate de tert-butyle

A une solution du composé obtenu au Stade A (1 g ; 3,07 mmol) dans l'acétone (30 mL), est additionné l'iodure de sodium (2,3 g ; 15,3 mmol), puis l'ensemble est agité à reflux pendant 24 heures. Le milieu réactionnel est concentré puis dilué avec de l'acétate d'éthyle et de l'eau. Après extraction à l'acétate d'éthyle, les phases organiques sont lavées avec une solution aqueuse saturée en chlorure de sodium, puis séchées sur sulfate de magnésium, filtrées et concentrées à sec pour obtenir le produit du titre.

**RMN ¹H** (400 MHz, dmso-d6, 300 K) δ ppm : 7.12 (t, 1 H), 6.8 (d, 1 H), 6.72 (d, 1 H), 4.45 (s, 2 H), 4 (t, 2 H), 3.55 (t, 2 H), 3.41 (t, 2 H), 2.63 (t, 2 H), 2.2 (m, 2 H), 1.41 (s, 9 H). **IR :** v : >C=O : 1692 cm⁻¹.

### Préparation 9b' : 5-[2-(2-hydroxyéthoxy)éthoxy]-3,4-dihydroisoquinoléine-2(1H)-carboxylate de tert-butyle

A une solution du composé obtenu à la Préparation 3b' (5 g ; 20 mmol) dans le *N,N-*diméthylformamide (20 mL), sont additionnés le 2-(2-chloroéthoxy)éthanol (6,25 mL ; 60 mmol) et le carbonate de potassium (8,3 g, 60 mmol), puis l'ensemble est agité pendant 5 heures à 125 °C. Le milieu réactionnel est dilué avec de l'acétate d'éthyle et de l'eau. Après décantation, la phase organique est lavée avec une solution aqueuse saturée en chlorure de lithium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.13 (t, 1 H), 6.81 (d, 1 H), 6.75 (d, 1 H), 4.58 (m, 1 H), 4.46 (s, 2 H), 4.09 (m, 2 H), 3.75 (m, 2 H), 3.54 (t, 2 H), 3.51 (massif, 4 H), 2.63 (t, 2 H), 1.42 (s, 9 H).

**IR** : v : -OH : 3450 cm⁻¹; >C=O : 1690 cm⁻¹.

### Préparation 10b': 5-(but-3-yn-1-yl)-3,4-dihydroisoquinoléine-2(1H)-carboxylate de tert-butyle

### Stade A : 5-(3-méthoxy-3-oxopropyl)-3,4-dihydroisoquinoléine-2(1H)-carboxylate de tert-butyle

A une solution du composé obtenu au Stade B de la Préparation 1b' (19,74 g ; 62,3 mmol) dans du méthanol (150 mL), est additionnée du palladium sur charbon (10 % massique). On fait barboter de l'hydrogène dans la suspension pendant 10 minutes, puis le mélange est agité sous atmosphère d'hydrogène (1 bar) pendant 18 heures. Le mélange réactionnel est filtré sur un lit de Célite^{®} et le filtrat est concentré à sec pour donner le produit du titre, lequel est utilisé pour la suite sans purification.

**RMN ¹H** (400 MHz, CDCl₃, 300 K) δ ppm : 7.10 - 7.16 (m, 1 H), 7.04 (d, 1 H), 6.98 (d, 1 H), 4.56 (s, 2 H), 3.68 (s, 3 H), 3.66 (br. s, 2 H), 2.90 - 2.96 (m, 2 H), 2.80 (t, 2 H), 2.55 - 2.61 (m, 2H), 1.48 (s, 9 H).

### Stade B : 5-(but-3-yn-1-yl)-3,4-dihydroisoquinoléine-2(1H)-carboxylate de tert-butyle

A une solution du produit obtenu au Stade A (10 g ; 31,3 mmol) dans du dichlorométhane (100 mL) à -78 °C, est additionnée lentement une solution d'hydrure de diisobutylaluminum à 25 % massique dans le toluène (25,2 mL, 37,6 mmol) sur une période de 45 minutes. Le débit d'addition est déterminé de façon à maintenir la température du mélange réactionnel en-dessous de -75 °C. A la fin de l'addition, le mélange est agité à -78 °C pendant 30 minutes, puis du méthanol (50 mL) est ajouté lentement à la réaction. Le mélange est ensuite réchauffé progressivement jusqu'à 0 °C. Du méthanol (50 mL) est ajouté à nouveau, ainsi que du carbonate de potassium (8,65 g ; 62,6 mmol) puis du diméthyl-(1-diazo-2-oxopropyl)phosphonate (7,22 g ; 37,6 mmol). Le mélange est agité à température ambiante pendant 48 heures et est dilué avec du méthyl-tert-butyl éther (400 mL). On ajoute ensuite une solution aqueuse 1 N de potassium sodium tartrate tétrahydrate (250 mL). Les phases sont séparées, et la phase aqueuse est lavée avec du méthyl-*tert*-butyl éther. Les phases organiques sont réunies et lavées avec une solution aqueuse saturée en chlorure de sodium, séchées avec du sulfate de sodium, filtrées et évaporées sous vide. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.12 - 7.18 (m, 1 H), 7.09 (d, 1 H), 7.00 (d, 1 H), 4.57 (s, 2 H), 3.61 - 3.73 (m, 2 H), 2.78 - 2.89 (m, 4 H), 2.44 (td, 2 H), 1.98 (t, 1 H), 1.49 (s, 9 H).

### Préparation 1b": 5-(3-{3-[(4-{[tert-butyl(diméthyl)silyl]oxy}phényl)amino]-5-cyanophénoxy}propyl)-3,4-dihydroisoquinoléine-2(1H)-carboxylate de tert-butyle

### Stade A : 5-[3-(3-bromo-5-cyanophénoxy)propyl]-3,4-dihydroisoquinoléine-2(1H)-carboxylate de tert-butyle

A une solution du composé obtenu à la Préparation 1b' (9,89 g ; 34,0 mmol) et de 3-bromo-5-fluorobenzonitrile (27,2 g ; 135,8 mmol) dans du *N*,*N*-diméthylformamide (80 mL), est additionné par portions de l'hydrure de sodium à 60 % dans l'huile (1,77 g ; 44,1 mmol). L'ensemble est ensuite agité pendant 45 minutes à température ambiante puis hydrolysé lentement. Après extraction à l'acétate d'éthyle, les phases organiques sont lavées avec une solution aqueuse saturée de chlorure de sodium, puis séchées sur sulfate de magnésium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.36 (t, 1 H), 7.26-7.28 (m, 1 H), 7.12-7.17 (m, 1 H), 7,06-7.09 (m, 1 H), 6.98-7.05 (m, 2 H), 4.58 (s, 2 H), 3.98 (t, 2 H), 3.62-3.68 (m, 2 H), 2.76-2.82 (m, 4 H), 2.02-2.09 (m, 2 H), 1.49 (s, 9 H).

### Stade B : 4-{[tert-bbutyl(diméthyl)silyl]oxy}aniline

Le composé du titre est obtenu à partir de 4-aminophénol dans le tétrahydrofurane en présence d'imidazole et de chlorure de tert-butyl(diméthyl)silyle selon le protocole décrit dans la littérature (S. Knaggs et al, Organic & Biomolecular Chemistry, 3(21), 4002-4010; 2005**).**
**RMN ¹H** (400 MHz, dmso-d6, 300 K) δ ppm : 6.45-6.55 (dd, 4H), 4.60 (m, 2H), 0.90 (s, 9H), 0.10 (s, 6H).
**IR :** v : -NH₂⁺ : 3300-3400 cm⁻¹

### Stade C : 5-(3-{3-[(4-{[tert-butyl(diméthyl)silyl]oxy}phényl)amino]-5-cyanophénoxby}propyl)-3,4-dihydroisoquinoléine-2(1H)-carboxylate de tert-butyle

Une solution des composés obtenus au Stade A (8,11 g ; 17,2 mmol) et au Stade B (4,23 g ; 17,2 mmol) dans le toluène (110 mL) est dégazée à l'argon pendant 10 minutes., On additionne le *tert*-butylate de sodium (1,82 g ; 18,9 mmol), le 2-di-*tert*-butylphosphino-2',4',6'-triisopropyl-1,1'-biphényle (0,73 g; 1,72 mmol) et le tris(dibenzylidènacétone)dipalladium(0) (0,86 g ; 0,86 mmol), puis l'ensemble est agité à 80°C pendant 30 minutes. Le milieu réactionnel est filtré sur céliteⓇ. Après un rinçage à l'acétate d'éthyle, de la silice est ajouté au filtrat, puis le mélange est concentré et purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.13 (t, 1 H), 6.95-7.06 (m, 4 H), 6.83 (d, 2 H), 6.69 (br. s, 1 H), 6.51-6.59 (m, 2 H), 5.59 (br. s, 1 H), 4.57 (s, 2 H), 3.92 (t, 2 H), 3.58-3.68 (m, 2 H), 2.61-2.83 (m, 4 H), 2.02 (quint., 2 H), 1.49 (s, 9 H), 1.00 (s, 9 H), 0.21 (s, 6 H).

### Préparation 2b" : 5-(4-{4-[(4-{[tert-butyl(diméthyl)silyl]oxy}phényl)amino]-1H-pyrazol-1-yl}butoxy)-3,4-dihydroisoquinoléine-2(1H)-carboxylate de tert-butyle

### Stade A : 5 -[4-(4-bromo-1H-pyrazol-1-yl)butoxy]-3,4-dihydroisoquinoléine-2(1H)-carboxylate de tert-butyle

A une solution du composé de la Préparation 2b' (0,786 g ; 2,44 mmol) et de triphénylphosphine (0,768 mg ; 2,93 mmol) dans le tétrahydrofurane (5 mL), est additionnée goutte à goutte une solution de 4-bromopyrazole (0,359 mg ; 2,44 mmol) et d'azodicarboxylate de diisopropyle (0,58 mL ; 2,93 mmol) dans le tétrahydrofurane (5 mL). Le milieu réactionnel est agité à température ambiante pendant 2 heures puis dilué dans un mélange d'acétate d'éthyle et d'eau. Après extraction de la phase aqueuse à l'acétate d'éthyle, les phases organiques sont réunies, lavées avec une solution aqueuse saturée en chlorure d'ammonium, une solution aqueuse saturée en chlorure de sodium, puis séchées sur sulfate de magnésium, filtrées et concentrées à sec. Le résidu est purifié par chromatographie sur phase OasisⓇ en utilisant l'acétonitrile et l'eau comme éluants pour obtenir le produit du titre.

**RMN ¹H** (400 MHz, dmso-d6, 300 K) δ ppm : 8.02 (s, 1 H), 7.53 (s, 1 H), 7.12 (dd, 1 H), 6.81-6.68 (2*d, 2 H), 4.46 (s, 2 H), 4.16 (t, 2 H), 3.95 (t, 2 H), 3.54 (t, 2 H), 2.61 (t, 2 H), 1.93 (m, 2 H), 1.65 (m, 2 H), 1.41 (s, 9 H).

**IR :** v : >C=O : 1688 cm⁻¹.

### Stade B : 5-(4-{4-[(4-{[tert-butyl(diméthyl)silyl]oxy}phényl)amino]-1H-pyrazol-1-yl}butoxy)-3,4-dihydroisoquinoléine-2(1H)-carboxylate de tert-butyle

Le composé du titre est obtenu selon le procédé décrit au Stade C de la Préparation 1b" en utilisant le composé obtenu au stade précédent, le composé du Stade B de la Préparation 1b" et le chloro(2-di-*tert*-butylphosphino-2',4',6'-triisopropyl-1,1'-biphényl)[2-(2-aminoéthyl)phényl]palladium(II) comme catalyseur et ligand.

**RMN ¹H** (400 MHz, dmso-d6, 300 K) δ ppm : 7.63 (d, 1 H), 7.26 (d, 1 H), 7.2 (s, 1 H), 7.11 (t, 1 H), 6.75 (dd, 2 H), 6.64 (m, 4 H), 4.45 (s, 2 H), 4.1 (t, 2 H), 3.96 (t, 2 H), 3.53 (t, 2 H), 2.62 (t, 2 H), 1.93 (m, 2 H), 1.68 (m, 2 H), 1.41 (s, 9 H), 0.93 (s, 9 H), 0.12 (s, 6 H).

**IR :** v : -NH : 3340 cm⁻¹ ; >C=O : 1690 cm⁻¹.

### Préparation 3b": 5-(2-{4-[(4-{[tert-butyl(diméthyl)silyl]oxy}phényl)amino]-1-méthyl-1H-pyrazol-5-yl}éthoxy)-3,4-dihydroisoquinoléine-2(1H)-carboxylate de tert-butyle

### Stade A : 5-(2-{[tert-butyl(diméthyl)silyl]oxy}éthyl)-1-méthyl-1H-pyrazole

A une solution de *N*-méthylpyrazole (10,95 g ; 133 mmol) dans le tétrahydrofurane (200 mL), est additionnée goutte à goutte à -78 °C une solution de *n*-butyllithium dans l'hexane (100 mL ; 160 mmol), puis la température est remontée à 0 °C en 1 heure. Le milieu réactionnel est de nouveau refroidi jusqu'à -78 °C, et on additionne une solution de (3-bromoéthoxy)-*tert*-butyldiméthylsilane (34,2 mL ; 160 mmol) dans le tétrahydrofurane (50 mL). Le milieu réactionnel est ensuite agité à température ambiante pendant 18 heures et versé dans un mélange d'eau glacée et d'acétate d'éthyle. Après extraction à l'acétate d'éthyle, la phase organique est lavée avec une solution aqueuse saturée en chlorure de sodium, puis séchée sur sulfate de magnésium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.28 (d, 1 H), 6.04 (d, 1 H), 3.79 (t, 2 H), 3.74 (s, 3 H), 2.81 (t, 2 H), 0.84 (s, 9 H), 0.1 (s, 6 H).

### Stade B : 2-(4-bromo-1-méthyl-1H-pyrazol-5-yl)éthanol

A une solution du composé obtenu au Stade A (9,8 g; 41,1 mmol) dans le méthanol (400 mL), est additionné à 0°C le tribromure de pyridinium (14 g ; 43 mmol), puis l'ensemble est agité pendant 1 heure à 0 °C,puis pendant 2 heures à température ambiante. Le milieu réactionnel est ensuite concentré et le résidu est repris dans un mélange d'une solution aqueuse à 10 % en carbonate de potassium et de dichlorométhane. Après extraction au dichlorométhane, la phase organique est lavée avec une solution aqueuse saturée en chlorure de sodium, puis séchée sur sulfate de magnésium, filtrée et concentrée à sec. Le résidu est purifié par chromatographie sur gel de silice en utilisant le dichlorométhane et le méthanol ammoniacal comme éluants pour obtenir le produit du titre.

**RMN ¹H** (400 MHz, dmso-d6, 300 K) δ ppm : 7.45 (s, 1 H), 4.88 (t, 1 H), 3.81 (s, 3 H), 3.55 (q, 2 H), 2.8 (t, 2 H).

**IR :** v : -OH : 3350 cm⁻¹ ; >C-C-O- : 1049 cm⁻¹.

### Stade C : 5-[2-(4-bromo-1-méthyl-1H-pyrazol-5-yl)éthoxy]-3,4-dihydroisoquinoléine-2(1H)-carboxylate de tert-butyle

Le composé du titre est obtenu selon le procédé décrit au Stade A de la Préparation 2b", en utilisant l'alcool obtenu au stade précédent et le composé de la Préparation 3b'.

**RMN ¹H** (400 MHz, dmso-d6, 300 K) δ ppm : 7.49 (s, 1 H), 7.13 (t, 1 H), 6.82 (d, 1 H), 6.75 (d, 1 H), 4.45 (s, 2 H), 4.15 (t, 2 H), 3.87 (s, 3 H), 3.51 (t, 2 H), 3.16 (t, 2 H), 2.54 (t, 2 H), 1.41 (s, 9 H).

IR : v : >C=O : 1689 cm⁻¹.

### Stade D : 5-(2-{4-f(4-{[tert-butyl(diméthyl)silyl]oxy}phényl)amino]-1-méthyl-1H-pyrazol-5-yl}éthoxy)-3,4-dihydroisoquinoléine-2(1H)-carboxylate de tert-butyle

Le composé du titre est obtenu selon le procédé décrit au Stade C de la Préparation 1b", en utilisant le composé obtenu au stade précédent, le composé du Stade B de la Préparation 1b" et le chloro(2-di-*tert*-butylphosphino-2',4',6'-triisopropyl-1,1'-biphényl)[2-(2-aminoéthyl)phényl]palladium(II) comme catalyseur et ligand.

**RMN ¹H** (400 MHz, dmso-d6, 300 K) δ ppm : 7.28 (s, 1 H), 7.06 (t, 1 H), 6.71/6.67 (2*dd, 2 H), 6.7 (s, 1 H), 6.57 (d, 2 H), 6.48 (d, 2 H), 4.43 (s, 2 H), 4.05 (t, 2 H), 3.82 (s, 3 H), 3.48 (t, 2 H), 3.03 (t, 2 H), 2.5 (t, 2 H), 1.4 (s, 9 H), 0.9 (s, 9 H), 0.09 (s, 6 H).

**IR :** v : >NH : 3321 cm⁻¹ ; >C=O : 1677 cm⁻¹.

### Préparation 4b" : N-(4-[[tert-butyl(diméthyl)silyl)oxy}phényl)-1-méthyl-5-[3-(tétrahydro-2H-pyran-2-yloxy)propyl]-1H-pyrazol-4-amine

### Stade A : 5-(3-{[tert-butyl(diméthyl)silyl]oxy}propyl)-1-méthyl-1H-pyrazole

Le composé du titre est obtenu selon le procédé décrit au Stade A de la Préparation 3b", en utilisant (3-bromopropoxy)-*tert*-butyldiméthylsilane.

**RMN ¹H** (400/500 MHz, CDCl₃, 300 K) δ ppm : 7.35 (d, 1 H), 7 (d, 1 H), 3.8 (s, 3 H), 3.65 (t, 2 H), 2.7 (m, 2 H), 1.85 (m, 2 H), 0.9 (s, 9 H), 0.5 (s, 6 H).

**IR :** v : -Si-O- : 1098 cm⁻¹ ; -Si-C- : 834 et 772 cm⁻¹.

### Stade B : 3-(4-bromo-1-méthyl-1H-pyrazol-5-yl)propan-1-ol

Le composé du titre est obtenu selon le procédé décrit au Stade B de la Préparation 3b" en utilisant le composé du stade précédent.

**RMN ¹H** (400 MHz, dmso-d6, 300 K) δ ppm : 7.45 (s, 1 H), 4.59 (t, 1 H), 3.79 (s, 3 H), 3.4 (quad, 2 H), 2.7 (t, 2 H), 1.65 (m, 2 H).

**IR :** v : -OH : 3348 cm⁻¹.

### Stade C : 4-bromo-1-méthyl-5-[3-(tétrahydro-2H-pyran-2-yloxy)propyl]-1H-pyrazole

A une solution du composé obtenu au Stade B (5,34 g ; 2.4 mmol) dans le dichlorométhane (40 mL), sont additionnés le 3,4-dihyro-2*H*-pyrane (7 mL ; 6 mmol) et l'acide *para*toluènesulfonique (4,6 g ; 2,4 mmol), puis l'ensemble est agité pendant 16 heures. Le milieu réactionnel est dilué dans une solution aqueuse saturée en hydrogénocarbonate de sodium. Après extraction au dichlorométhane, la phase organique est séchée sur sulfate de magnésium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.42 (s, 1 H), 4.55 (t, 1 H), 3.8-3.3 (m, 4 H), 3.8 (s, 3 H), 2.71 (m, 2 H), 1.78 (m, 2 H), 1.7-1.4 (m, 6 H).

### Stade D : N-(4-{[tert-butyl(diméthyl)silyl]oxy}phényl)-l-méthyl-5-[3-(tétrahydro-2H-pyran-2-yloxy)propyl]-1H-pyrazol-4-amine

Le composé du titre est obtenu selon le procédé décrit au Stade D de la Préparation 3b" en utilisant le composé du stade précédent.

**RMN ¹H** (500 MHz, dmso-d6, 300 K) δ ppm : 7.2 (s, 1 H), 6.6 (s, 1 H), 6.55 (d, 2 H), 6.45 (d, 2 H), 4.4 (t, 1 H), 3.7 (s, 3 H), 3.65-3.2 (4m, 4 H), 2.58 (m, 2 H), 1.68 (m, 2 H), 1.6-1.3 (m, 6 H), 0.92 (s, 9 H), 0.1 (s, 6 H).

**IR :** v : >NH : 3356 cm⁻¹; ->C-C-O- : 1240 cm⁻¹.

### Préparation 5b" : 5-(3-{3-[(4-{[tert-butyl(diméthyl)silyl]oxy}phényl)amino]-5-cyano-1-méthyl-1H-pyrrol-2-yl}propoxy)-3,4-dihydroisoquinoléine-2(1H)-carboxylate de tert-butyle

### Stade A : (2E)-3-(1-méthyl-1H-pyrrol-2-yl)prop-2-énoate d'éthyle

A une solution de phosphonoacétate de triéthyle (14,2 mL ; 71,5 mmol) dans le tétrahydrofurane (300 mL), est additionné le *tert*-butoxyde de potassium (9,25 g ; 82,5 mmol), puis l'ensemble est agité à 0 °C pendant 45 minutes. Une solution de *N*-méthyl-2-pyrrolecarboxaldéhyde (6 g ; 55,0 mmol) dans le tétrahydrofurane (20 mL) est ajoutée et l'ensemble est agité à température ambiante pendant 16 heures, puis le solvant est concentré. Le résidu est dilué dans de l'eau et de l'acétate d'éthyle. Après extraction à l'acétate d'éthyle, la phase organique est lavée avec une solution aqueuse saturée en chlorure de sodium, séchée sur sulfate de sodium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.59 (d, 1 H), 6.73-6.76 (m, 1 H), 6.65 (dd, 1 H), 6.11-6.18 (m, 2 H), 4.23 (q, 2 H), 3.71 (s, 3 H), 1.32 (t, 3 H).

### Stade B : 3-(1-méthyl-1H-pyrrol-2-yl)propanoate d'éthyle

A une solution du composé obtenu au Stade A (8,1 g ; 45,1 mmol) dans l'éthanol (70 mL), est additionné du palladium sur charbon (10 % massique), puis l'ensemble est hydrogéné pendant 6 heures et 30 minutes. Le mélange réactionnel est filtré sur céliteⓇ et le filtrat est concentré pour obtenir le produit du titre, lequel est utilisé pour la suite sans purification.

**RMN ¹H** (400 MHz, CDCl₃, 300 K) δ ppm : 6.52-6.56 (m, 1 H), 6.01-6.06 (m, 1 H), 5.87 (ddt, 1 H), 4.15 (q, 2 H), 3.55 (s, 3 H), 2.83-2.91 (m, 2 H), 2.60-2.68 (m, 2 H), 1.26 (t, 3 H).

### Stade C : 3-(5-cyano-1-méthyl-1H-pyrrol-2-yl)propanoate d'éthyle

A une solution du composé obtenu au Stade B (12 g ; 66,2 mmol) dans l'acétonitrile (300 mL) à -20 °C, est additionné goutte à goutte en maintenant cette température l'isocyanate de chlorosulfonyle (6,92 mL ; 79,5 mmol), puis l'ensemble est agité à -20 °C pendant 30 minutes. Du *N*,*N*-diméthylformamide (10,3 mL ; 132,4 mmol) puis de la triéthylamine (18,5 mL ; 132,4 mmol) sont ajoutés à une température maintenue à -10 °C, et l'ensemble est agité jusqu'à ce qu'il atteigne la température ambiante. Le milieu réactionnel est dilué dans une solution aqueuse d'acide chlorhydrique 1 M (500 mL). Après extraction à l'acétate d'éthyle, la phase organique est lavée avec une solution aqueuse d'acide chlorhydrique 1 M, puis avec une solution aqueuse saturée en chlorure de sodium, séchée sur sulfate de sodium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 6.72 (d, 1 H), 5.93 (d, 1 H), 4.15 (q, 2 H), 3.68 (s, 3 H), 2.86-2.93 (m, 2 H), 2.63-2.69 (m, 2 H), 1.26 (t, 3 H).

### Stade D : 4-bromo-5-(3-hydroxypropyl)-1-méthyl-1H-pyrrole-2-carbonitrile

A une solution du composé obtenu au Stade C (9,74 g ; 47,2 mmol) dans le *N,N-*diméthylformamide (125 mL) à 0 °C, est additionné par portions le *N*-bromosuccinimide (8,82 g ; 49,6 mmol), puis l'ensemble est agité à température ambiante pendant 30 minutes. Le milieu réactionnel est dilué dans de l'eau et du *tert*-butylméthyléther. Après extraction au *tert*-butylméthyléther, la phase organique est lavée avec de l'eau et une solution aqueuse saturée en chlorure de sodium, séchée sur sulfate de sodium, filtrée et concentrée à sec pour obtenir le produit, lequel est directement engagé dans l'étape suivante.

A une solution de ce composé (3,13 g ; 10,97 mmol) dans le tétrahydrofurane (30 mL) à 0 °C, est additionnée une solution de borohydrure de lithium 2 M dans le tétrahydrofurane (11 mL ; 21,95 mmol), puis l'ensemble est agité à température ambiante pendant 6 heures. Le milieu réactionnel est dilué lentement par une solution aqueuse de soude 1 M (60 mL). Après extraction au *tert*-butylméthyléther, la phase organique est lavée avec une solution aqueuse saturée en chlorure de sodium, séchée sur sulfate de sodium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 6.75 (s, 1 H), 3.73 (s, 3 H), 3.62-3.69 (m, 2 H), 2.78 (t, 2 H), 1.75-1.85 (m, 2 H), 1.38-1.45 (m, 1 H).

### Stade E : 5-[3-(3-bromo-5-cyano-1-méthyl-1H-pyrrol-2-yl)propoxy]-3,4-dihydroisoquinoléine-2(1H)-carboxylate de tert-butyle

Le composé du titre est obtenu selon le procédé décrit au Stade A de la Préparation 2b", en utilisant l'alcool obtenu au stade précédent et le composé de la Préparation 3b'.

**RMN ¹H** (400 MHz, dmso-d6, 300 K) δ ppm : 7.11 (t, 1 H), 7.06 (s, 1 H), 6.75 (2d, 2 H), 4.49 (s, 2 H), 3.99 (t, 2 H), 3.7 (s, 3 H), 3.55 (t, 2 H), 2.88 (t, 2 H), 2.61 (t, 2 H), 1.98 (m, 2 H), 1.41 (s, 9 H).

**IR :** v : >CN : 2218 cm⁻¹; ->C=O : 1681 cm⁻¹.

### Stade F : 5-(3-{3-[(4-{[tert-butyl(diméthyl)silyl]oxy}phényl)amino]-5-cyano-1-méthyl-1H-pyrrol-2-yl}propoxy)-3,4-dihydroisoquinoléine-2(lH)-carboxylate de tert-butyle

Le composé du titre est obtenu selon le procédé décrit au Stade D de la Préparation 3b" en utilisant le composé du stade précédent.

**RMN ¹H** (400 MHz, dmso-d6, 300 K) δ ppm : 7.05 (t, 1 H), 6.75 (s, 1 H), 6.75 (s, 1 H), 6.7/6.6 (2d, 2 H), 6.52 (d, 2 H), 6.45 (d, 2 H), 4.42 (s, 2 H), 3.88 (t, 2 H), 3.68 (s, 3 H), 3.5 (t, 2 H), 2.75 (t, 2 H), 2.51 (t, 2 H), 1.89 (m, 2 H), 1.41 (s, 9 H), 0.9 (s, 9 H), 0.1 (s, 6 H).

**IR :** v : >NH : 3364 cm⁻¹ ; >CN : 2208 cm⁻¹ ; >C=O : 1690 cm⁻¹.

### Préparation 6b" : 5-(2-{3-[(4-{[tert-butyl(diméthyl)silyl]oxy}phényl)amino]-5-cyano-1-méthyl-1H-pyrrol-2-yl}éthoxy)-3,4-dihydroisoquinoléine-2(1H)-carboxylate de tert-butyle

### Stade A : acide (1-méthyl-1H-pyrrol-2-yl)(oxo)acétique

A une solution de chlorure d'oxalyle (20,9 g ; 0,25 mol) dans le dichlorométhane à -10 °C, est additionné le *N*-méthylpyrrole (20 g ; 0,25 mol) en maintenant une température inférieure à 0 °C, puis l'ensemble est agité à 0 °C pendant 1 heure. Le milieu réactionnel est dilué dans une solution aqueuse d'hydroxyde de potassium à 25 % à 0 °C. Après décantation, la phase aqueuse est lavée avec du dichlorométhane et acidifiée jusqu'à pH=1 par une solution aqueuse d'acide sulfurique à 20 %. Le précipité est filtré et séché pour obtenir le produit du titre.

**RMN ¹H** (400 MHz, CDCl₃, 300 K) δ ppm : 8.01 (dd, 1 H), 7.10 (t, 1 H), 6.27 (dd, 1 H), 3.99 (s, 3 H).

### Stade B : acide (1-méthyl-1H-pyrrol-2-yl)acétique

A une solution aqueuse d'hydrazine monohydratée à 65 % (15,5 mL ; 0,21 mol), est additionné le composé obtenu au Stade A (29 g; 0,19 mol), puis l'ensemble est agité quelques minutes. Une solution aqueuse de soude à 20 % (326 mL) est ajoutée lentement et l'ensemble est agité à reflux pendant 4 heures. Après retour à température ambiante, une solution aqueuse d'acide chlorhydrique 6 N (25 mL) est ajoutée. Après extraction au dichlorométhane, la phase organique est lavée avec de l'eau et une solution aqueuse saturée en chlorure de sodium, séchée sur sulfate de sodium, filtrée et concentrée jusqu'à un volume de 100 mL. Après un ajout lent d'heptane, le précipité est filtré pour obtenir le produit du titre.

**RMN ¹H** (400 MHz, CDCl₃, 300 K) δ ppm : 6.60-6.64 (m, 1 H), 6.04-6.12 (m, 2 H), 3.68 (s, 2 H), 3.59 (s, 3 H).

### Stade C : (1-méthyl-1H-pyrrol-2-yl)acétate de méthyle

A une solution du composé obtenu au Stade B (10,2 g ; 73.4 mmol) et de carbonate de potassium (15,2 g ; 110,1 mmol) dans du dichlorométhane (100 mL), est additionné le diméthylsulfate (7 mL ; 73,4 mmol), puis l'ensemble est agité vigoureusement à 30 °C pendant 4 heures. Une solution aqueuse d'hydroxyde d'ammonium à 5 % (326 mL) est ajoutée. Après décantation, la phase organique est lavée avec une solution aqueuse d'hydroxyde d'ammonium à 5 % et une solution aqueuse saturée en chlorure de sodium, séchée sur sulfate de sodium, filtrée et concentrée à sec jusqu'à un volume de 100 mL. Après un ajout lent d'heptane, le précipité est filtré pour obtenir le produit du titre. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 6.58-6.62 (m, 1 H), 6.06-6.09 (m, 1 H), 6.03-6.06 (m, 1 H), 3.71 (s, 3 H), 3.64 (s, 2 H), 3.58 (s, 3 H).

### Stade D : (5-cyano-1-méthyl-1H-pyrrol-2-yl)acétate de méthyle

Le composé du titre est obtenu selon le procédé décrit au Stade C de la Préparation 5b" en utilisant le composé du stade précédent.

**RMN ¹H** (400 MHz, CDCl₃, 300 K) δ ppm : 6.74 (d, 1 H), 6.08 (d, 1 H), 3.73 (s, 3 H), 3.69 (s, 3 H), 3.66 (s, 2 H).

### Stade E : 5-(2-hydroxyéthyl)-1-méthyl-1H-pyrrole-2-carbonitrile

A une solution du composé obtenu au Stade D (11,4 g; 10,97 mmol) dans le tétrahydrofurane (115 mL) à 0 °C, est additionnée une solution de borohydrure de lithium 2 M dans le tétrahydrofurane (47,7 mL ; 95,4 mmol) à une température inférieure à 5 °C, puis l'ensemble est agité à température ambiante pendant 16 heures. Le milieu réactionnel est dilué lentement avec une solution aqueuse saturée en chlorure d'ammonium (150 mL). Après extraction au *tert*-butylméthyléther, la phase organique est lavée avec une solution aqueuse saturée en chlorure de sodium, séchée sur sulfate de sodium, filtrée et concentrée à sec. Le résidu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 6.75 (d, 1 H), 6.02 (d, 1 H), 3.88 (t, 2 H), 3.69 (s, 3 H), 2.86 (t, 2 H).

### Stade F : 4-bromo-5-(2-hydroxyéthyl)-1-méthyl-1H-pyrrole-2-carbonitrile

A une solution du composé obtenu au Stade E (4,25 g ; 28,3 mmol) dans le *N,N-*diméthylformamide (43 mL) à 0 °C, est additionné par portions le *N*-bromosuccinimide (5,04 g ; 28,3 mmol), puis l'ensemble est agité à température ambiante pendant 3 heures. Le milieu réactionnel est dilué dans de l'eau et du *tert*-butylméthyléther. Après extraction au *tert*-butylméthyléther, la phase organique est lavée avec de l'eau et une solution aqueuse saturée en chlorure de sodium, séchée sur sulfate de sodium, filtrée et concentrée à sec pour obtenir le produit du titre, lequel est utilisé pour la suite sans purification.

**RMN** ¹**H** (400 MHz, CDCl₃, 300 K) δ ppm : 6.76 (d, 1 H), 3.83 (t, 2 H), 3.75 (s, 3 H), 2.91 (t, 2 H), 1.64 (s, 1 H).

### Stade G : 5-[2-(3-bromo-5-cyano-1-méthyl-1H-pyrrol-2-yl)éthoxy]-3,4-dihydroisoquinoléine-2(1H)-carboxylate de tert-butyle

Le composé du titre est obtenu selon le procédé décrit au Stade A de la Préparation 2b", en utilisant l'alcool obtenu au stade précédent et le composé de la Préparation 3b'.

**RMN ¹H** (400 MHz, dmso-d6, 300 K) δ ppm : 7.12 (t, 1 H), 7.11 (s, 1 H), 6.8 (d, 1 H), 6.72 (d, 1 H), 4.45 (s, 2 H), 4.15 (t, 2 H), 3.78 (s, 3 H), 3.52 (t, 2 H), 3.18 (t, 2 H), 2.55 (t, 2 H), 1.41 (s, 9 H).

**IR :** v : >CN : 2215 cm⁻¹; ->C=O : 1686 cm⁻¹.

### Stade H : 5-(2-{3-[(4-{[tert-butyl(diméthyl)silyl]oxy}phényl)amino]-5-cyano-l-méthyl-1H-pyrrol-2-yl}éthoxy)-3,4-dihydroisoquinoléine-2(1H)-carboxylate de tert-butyle

Le composé du titre est obtenu selon le procédé décrit au Stade D de la Préparation 3b" en utilisant le composé du stade précédent.

**RMN ¹H** (400 MHz, dmso-d6, 300 K) δ ppm : 7.05 (t, 1 H), 6.82 (s, 1 H), 6.78 (s, 1 H), 6.7 (2d, 2 H), 6.55 (2d, 4 H), 4.4 (s, 2 H), 4.02 (t, 2 H), 3.7 (s, 3 H), 3.48 (t, 2 H), 3.05 (t, 2 H), 2.51 (t, 2 H), 1.41 (s, 9 H), 0.91 (s, 9 H), 0.12 (s, 6 H).

**IR :** v : >NH : 3315 cm-1 ; >CN : 2212 cm-1 ; >C=O : 1655 cm-1.

### Préparation 7b" : 5-[2-({4-[(4-{[tert-butyl(diméthyl)silyl]oxy}phényl)amino]-1-méthyl-1H pyrazol5-yl}oxy)éthoxy]-3,4-dihydroisoquinoléine-2(1H)-carboxylate de tert-butyle

### Stade A : 5-{2-[(4-bromo-1-méthyl-1H-pyrazol-5-yl)oxy]éthoxy}-3,4-dihydroisoquinoléine-2(1H)-carboxylate de tert-butyle

A une solution du composé obtenu à la Préparation 3b' (4,06 g ; 16,28 mmol) dans l'acétonitrile (75 mL), sont additionnés le bromo-chloroéthane (2 mL ; 24,43 mmol) et le carbonate de potassium (3,35 g ; 24,43 mmol), puis l'ensemble est agité pendant 2 jours à 70 °C. Après concentration au 2/3 du milieu réactionnel, celui-ci est dilué avec de l'acétate d'éthyle et de l'eau. Après extraction à l'acétate d'éthyle, les phases organiques sont lavées avec une solution aqueuse saturée en hydrogénocarbonate de sodium, de l'eau, puis avec une solution saturée en chlorure de sodium, et séchées sur sulfate de magnésium, filtrées et concentrées à sec. Le résidu est purifié par chromatographie sur gel de silice en utilisant le dichlorométhane comme éluant pour obtenir une huile incolore.

A une solution du résidu ainsi obtenu (4,4 g ; 14,1 mmol) dans l'acétone (80 mL), est additionné l'iodure de sodium (21,15 g ; 141.1 mmol), puis l'ensemble est agité à reflux pendant 5 jours. Le milieu réactionnel est concentré puis dilué avec de l'acétate d'éthyle et de l'eau. Après extraction à l'acétate d'éthyle, les phases organiques sont lavées avec une solution saturée en chlorure de sodium, puis séchées sur sulfate de magnésium, filtrées et concentrées à sec pour obtenir une huile brune, qui est utilisée au stade suivant sans purification.

A une solution du résidu ainsi obtenu (5,29 g ; 13,13 mmol) dans l'acétonitrile (100 mL), sont additionnés le 5-hydroxy-*N*-méthylpyrazole (1,29 g ; 13,13 mmol), le carbonate de césium (4,7 g, 24,43 mmol) et l'iodure de sodium (0,39 g ; 2,6 mmol), puis l'ensemble est agité pendant 5 heures à 90 °C. Après concentration du milieu réactionnel, le résidu est dilué avec de l'acétate d'éthyle et de l'eau. Après extraction à l'acétate d'éthyle, les phases organiques sont lavées avec une solution aqueuse saturée en hydrogénocarbonate de sodium, de l'eau, une solution saturée en chlorure de sodium, puis séchées sur sulfate de magnésium, filtrées et concentrées à sec. Le résidu est purifié par chromatographie sur gel de silice en utilisant l'heptane et l'acétate d'éthyle comme éluants pour obtenir une huile incolore.

A une solution du résidu ainsi obtenu (2,8 g ; 7,5 mmol) dans le méthanol (200 mL), est additionné par portions à 0°C le tribromure de pyridinium (2,4 g ; 7,5 mmol). L'ensemble est agité pendant 1 heure à 0 °C, puis pendant 2 heures à température ambiante. Après concentration du milieu réactionnel, le résidu est repris dans un mélange d'eau et de dichlorométhane. Après extraction au dichlorométhane, les phases organiques sont lavées avec une solution aqueuse d'acide chlorhydrique 1 M, de l'eau, puis séchées sur sulfate de sodium, filtrées et concentrées à sec. Le résidu est purifié par chromatographie sur phase RP-18 en utilisant l'acétonitrile et l'eau comme éluants pour obtenir le produit du titre.

**RMN ¹H** (400 MHz, dmso-d6, 300 K) δ ppm : 7.41 (s, 1 H), 7.14 (m, 1 H), 6.88 (d, 1 H), 6.84 (d, 1 H), 4.56/4.28 (2t, 4 H), 4.45 (s, 2 H), 3.62 (s, 3 H), 3.52 (t, 2 H), 2.55 (t, 2 H), 1.42 (s, 9 H).

**IR :** v : >C=O : 1689 cm⁻¹.

### Stade B : 5-[2-({4-[(4-{[tert-butyl(diméthyl)silyl]oxy}phényl)amino]-1-méthyl-1H-pyrazol-5-yl}oxy)éthoxy]-3,4-dihyroisoquinoléine-2(1H)-carboxylate de tert-butyle

Une solution du composé obtenu au Stade A (2 g ; 4,42 mmol) et du composé obtenu au Stade B de la Préparation 1b" (1,48 g ; 6,63 mmol) dans le toluène (15 mL) est dégazée à l'argon pendant 10 minutes. On additionne le *tert*-butylate de sodium (0,51 g ; 5,3 mmol), le 2-di-*tert*-butylphosphino-2',4',6'-triisopropylbiphényle (0,187 g; 0,44 mmol) et le chloro(2-di-*tert*-butylphosphino-2',4',6'-triisopropyl-1,1'-biphényl)[2-(2-aminoéthyl)phényl]palladium(II) (0,3 g ; 0,44 mmol), puis l'ensemble est agité à 100 °C pendant 2 heures au micro-onde (300 W). Le milieu réactionnel est filtré sur CéliteⓇ. Après un rinçage à l'acétate d'éthyle, le filtrat est lavé avec de l'eau et une solution aqueuse saturée en chlorure de sodium, puis séché sur sulfate de magnésium, filtré et concentré à sec. Le résidu est purifié par chromatographie sur gel de silice en utilisant l'heptane et l'acétate d'éthyle comme éluants, puis par chromatographie sur phase RP-18 en utilisant l'acétonitrile et l'eau comme éluants pour obtenir le produit du titre.

**RMN ¹H** (400 MHz, dmso-d6, 300 K) δ ppm : 7.18 (s, 1 H), 7.1 (m, 1 H), 6.75 (d, 2 H), 6.7 (s, 1 H), 6.6 (d, 2 H), 6.48 (d, 2 H), 4.45 (s, 2 H), 4.42/4.15 (2t, 4 H), 3.53 (s, 3 H), 3.51 (t, 2 H), 2.52 (t, 2 H), 1.42 (s, 9 H), 0.92 (s, 9 H), 0.11 (s, 6 H).

**IR :** v : >NH : 3485-3182 ; >C=O : 1689 cm⁻¹.

### Préparation 8b" : 4-[2-({4-[(4-{[tert-butyl(diméthyl)silyl]oxy}phényl)amino]-1-méthyl-1H-pyrazol-5-yl}oxy)éthyl]-3,4-dihydroisoquinoléine-2(1H)-carboxylate de tert-butyle

### Stade A : 5-chloro-1-méthyl-4-nitro-1H-pyrazole

A une solution de 1-méthyl-4-nitro-1*H*-pyrazole (5 g ; 39,34 mmol) dans le tétrahydrofurane (50 mL), sont additionnés goutte à goutte à -78 °C une solution 1,3 M de bis(triméthylsilyl)amidure de lithium dans le tétrahydrofurane (82 mL ; 106,22 mmol) et l'hexachloroéthane (14 g, 59,01 mmol), puis l'ensemble est agité pendant 1 heure à -78 °C. Le milieu réactionnel est transvasé sur une solution aqueuse saturée en chlorure d'ammonium et de la glace. Le produit est extrait au dichlorométhane, puis les phases organiques sont séchées sur sulfate de sodium, filtrées et concentrées à sec. Le résidu est purifié par chromatographie sur gel de silice en utilisant le dichlorométhane et l'acétate d'éthyle comme éluants pour obtenir le produit du titre.

**RMN ¹H** (400 MHz, dmso-d6, 300 K) δ ppm : 8.41 (s, 1 H), 3.90 (s, 3 H).

**IR :** v : >CH : 3122 ; -NO₂ : 1521+1312 cm⁻¹.

### Stade B : 4-{2-[(1-méthyl-4-nitro-1H-pyrazol-5-yl)oxy]éthyl}-3,4-dihydroisoquinoléine-2(1H)-carboxylate de tert-butyle

A une solution du composé obtenu à la Préparation 5b' (2,8 g ; 10,09 mmol) dans le tétrahydrofurane (50 mL), est additionné l'hydrure de sodium à 60 % (240 mg ; 10,09 mmol), puis l'ensemble est agité pendant 1 heure à température ambiante. Une solution du composé obtenu au Stade A (1,4 g ; 8,66 mmol) dans le tétrahydrofurane (25 mL) est ajoutée, et le milieu réactionnel est agité pendant 16 heures puis transvasé sur une solution aqueuse saturée en chlorure d'ammonium et de la glace. Le produit est extrait à l'acétate d'éthyle puis les phases organiques sont séchées sur sulfate de sodium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 8.15 (s, 1 H), 7.20 (m, 4 H), 4.80 (m, 1H), 4.51 (m, 2H), 4.30 (d, 1H), 4.08 (m, 1H), 3.71 (s, 3H), 3.25 (m, 1H), 3.10 (m, 1H), 2.03 (m, 1H), 1.90 (m, 1H), 1.40 (s, 9 H).

**IR :** v : >C=O : 1687 ; -NO₂ : 1567+1329 cm⁻¹.

### Stade C : 4-{2-[(4-amino-1-méthyl-1H-pyrazol-5-yl)oxy]éthyl}-3,4-dihydroisoquinoléine-2(1H)-carboxylate de tert-butyle

A une solution du composé obtenu au Stade B (2,4 g ; 5,96 mmol) dans le méthanol (75 mL), est additionné le palladium sur charbon à (15 % massique) puis l'ensemble est hydrogéné pendant 24 heures à température ambiante sous une pression de 1 bar. Le milieu réactionnel est filtré et concentré à sec. Le résidu est purifié par chromatographie sur gel de silice en utilisant le dichlorométhane et l'éthanol ammoniacal comme éluants pour obtenir le produit du titre.

**RMN ¹H** (400 MHz, dmso-d6, 300 K) δ ppm : 7.19 (m, 4 H), 6.88 (s, 1H), 4.76 (m, 1H), 4.39- 4.21 (m, 3H), 4.01 (dd, 1H), 3.52 (s, 3H), 3.40 (s, 2H), 3.24 (m, 1H), 3.04 (m, 1H), 1.91 (m, 1H), 1.82 (m, 1H), 1.42 (s, 9 H).

**IR :** v : -NH₂ et -NH : 3390, 3327 et 3240 ; >C=O : 1684 cm⁻¹.

### Stade D : tert-butyl(4-iodophénoxy)diméthylsilane

A une solution du *para*-iodophénol (20 g ; 90 mmol) dans le dichlorométhane (50 mL), sont additionnés la triéthylamine (15,2 mL ; 109 mmol) et le chlorure de *tert*butyldiméthylchlorosilane (16,4 g ; 109 mmol), puis l'ensemble est agité pendant 1 heure à température ambiante. Après hydrolyse, le produit est extrait à l'acétate d'éthyle puis les phases organiques sont lavées avec de l'eau, une solution aqueuse saturée en chlorure de sodium, puis séchées sur sulfate de magnésium, filtrées et concentrées à sec. Le résidu est purifié par chromatographie sur gel de silice en utilisant l'éther de pétrole et l'acétate d'éthyle comme éluants pour obtenir le produit du titre.

**RMN ¹H** (400 MHz, CDCl₃, 300 K) δ ppm : 7.5 (d, 2H), 6.67 (d, 2H), 0.95 (s, 9H), 0.20 (s, 6H).

**IR :** v : >C-O-C< : 1252 ; -Si-O-C- : 905 ; -Si-C- : 822 cm⁻¹.

### Stade E : 4-[2-({4-[(4-{[tert-butyl(diméthyl)silyl]oxy}phényl)amino]-1-méthyl-1H-pyrazol-5-yl}oxy)éthyl]-3,4-dihydroisoquinoléine-2(1H)-carboxylate de tert-butyle

Une solution du composé obtenu au Stade C (1,05 g ; 2,81 mmol) et du composé obtenu au Stade D (1,48 g ; 3,14 mmol) dans le toluène (15 mL) est dégazée à l'argon pendant 10 minutes. On additionne le *tert*-butylate de sodium (300 mg ; 3,1 mmol) et le chloro(2-di-*tert*-butylphosphino-2',4',6'-triisopropyl-1,1'-biphényl)[2-(2-aminoéthyl)phényl]palladium(II) (387 mg ; 0,56 mmol), puis l'ensemble est agité à 100 °C pendant 2,5 heures au micro-onde (300 W). Le milieu réactionnel est filtré sur CéliteⓇ. Après un rinçage avec du dichlorométhane, le filtrat est concentré à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.2-7.20 (m, 5 H), 6.60 (d, 2H), 6.5 (d, 2H), 6.4 (s, 1H), 4.65 (d, 1H), 4.25 (d, 1H), 4.20 (m, 2H), 3.60 (s, 3H), 3.30 (dd, 1H), 3.20 (dd, 1H), 2.88 (m, 1H), 1.80 (m, 2H), 1.40 (s, 9 H), 0.9 (s, 9H), 0.01 (s, 6H).

**IR :** v : >NH : 3328 ; >C=O : 1693 cm⁻¹.

### Préparation 9b" : 5-(2-{4-[(4-{[tert-butyl(diméthyl)silyl]oxy}phényl)amino]-1-(tétrahydrofuran-3-yl)-1H-pyrazol-5-yl}éthoxy)-3,4-dihydroisoquinoléine-2(1H)-carboxylate de tert-butyle

### Stade A : méthanesulfonate de tétrahydrofuran-3-yle

A une solution de 3-hydroxytétrahydrofurane (14,8 g ; 0,25 mol) et de triéthylamine (35 mL ; 0,25 mol) dans le dichlorométhane (200 mL), est additionné le chlorure de méthanesulfonyle (14,6 mL ; 0,18 mol), puis l'ensemble est agité à température ambiante pendant 16 heures. Le milieu réactionnel est dilué dans de l'eau. Après extraction au dichlorométhane, les phases organiques sont lavées avec de l'eau, puis séchées sur sulfate de sodium, filtrées et concentrées à sec pour obtenir le produit du titre, lequel est utilisé au stade suivant sans purification.

**RMN ¹H** (400 MHz, CDCl₃, 300 K) δ ppm : 5.24-5.32 (m, 1 H), 3.81-4.04 (m, 4 H), 3.02 (s, 3 H), 2.17-2.26 (m, 2 H).

### Stade B : 1-(tétrahydrofuran-3-yl)-1H-pyrazole-5-carboxylate de méthyle

A une solution de 1*H*-pyrazol-5-carboxylate de méthyle (20 g ; 0,16 mol) et du composé obtenu au Stade A (28,9 g ; 0,18 mol) dans du *N*,*N*-diméthylformamide (400 mL), est additionné le carbonate de potassium (33 g ; 0,24 mol), puis l'ensemble est agité à 80 °C pendant 48 heures. Après retour à température ambiante, le milieu réactionnel est filtré et le filtrat est concentré. Le résidu est repris dans un mélange 15 % d'acétate d'éthyle dans l'heptane puis filtré sur gel de silice. Le filtrat est concentré et le résidu obtenu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.50 (d, 1 H), 6.83 (d, 1 H), 5.85-5.94 (m, 1 H), 4.08-4.20 (m, 2 H), 4.01 (dd, 1 H), 3.95 (td, 1 H), 3.86 (s, 3 H), 2.32-2.54 (m, 2 H).

### Stade C : [1-(tétrahydrofuran-3-yl)-1H-pyrazol-5-yl]méthanol

A une suspension d'hydrure d'aluminium lithium (3,38 g ; 86,0 mmol) dans le tétrahydrofurane (100 mL) refroidie à 0°C, est additionnée une solution du composé obtenu au Stade B (8,43 g ; 49,0 mmol) dans le tétrahydrofurane (50 mL), puis l'ensemble est agité à température ambiante pendant 16 heures. Le milieu réactionnel est refroidi jusqu'à 0 °C et dilué lentement avec de l'eau (3,4 mL), une solution aqueuse d'hydroxyde de sodium à 15 % (6,8 mL) et enfin avec de l'eau (6,8 mL). Du sulfate de magnésium est ajouté au milieu.

Après filtration et concentration du filtrat, le produit du titre est obtenu, lequel est utilisé pour la suite sans purification.

**RMN** ¹**H** (400 MHz, CDCl₃, 300 K) δ ppm : 7.43 (s, 1 H), 6.17 (s, 1 H), 5.05-5.18 (m, 1 H), 4.69 (s, 2 H), 4.19 (q, 1 H), 4.06-4.13 (m, 1 H), 3.91-4.02 (m, 2 H), 2.32-2.50 (m, 2 H), 2.10-2.32 (m, 1 H).

### Stade D : [4-bromo-1-(tétrahydrofuran-3-yl)-1H-pyrazol-5-yl]méthanol

A une solution du composé obtenu au Stade C (7,9 g ; 47 mmol) dans du dichlorométhane (100 mL) à 0 °C, est additionné par portions le N-bromosuccinimide (8,78 g ; 49,3 mmol), puis l'ensemble est agité à température ambiante pendant 1,25 heures. Le milieu réactionnel est dilué dans une solution aqueuse d'hydroxyde de sodium 1 M (100 mL). Après extraction au dichlorométhane, les phases organiques sont réunies, lavées avec de l'eau et séchées sur sulfate de sodium, filtrées et concentrées à sec. Le résidu obtenu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.44 (s, 1 H), 5.08-5.19 (m, 1 H), 4.69 (d, 2 H), 4.19 (q, 1 H), 4.09 (dd, 1 H), 3.89-4.01 (m, 2 H), 2.32-2.48 (m, 3 H).

### Stade E : 4-bromo-5-(chlorométhyl)-1-(tétrahydrofuran-3-yl)-1H-pyrazole

A une solution du composé obtenu au Stade D (8,93 g ; 36,1 mmol) et de triéthyamine (7,5 mL ; 54,2 mmol) dans du dichlorométhane (100 mL), est additionné du chlorure de méthanesulfonyle (2,85 mL ; 36,1 mmol), puis l'ensemble est agité à température ambiante pendant 16 heures. Le milieu réactionnel est dilué dans de l'eau. Après extraction au dichlorométhane, les phases organiques sont réunies, lavées avec de l'eau et séchées sur sulfate de sodium, filtrées et concentrées à sec pour obtenir le produit du titre, lequel est utilisé pour la suite sans purification.

**RMN ¹H** (400 MHz, CDCl₃, 300 K) δ ppm : 7.49 (s, 1 H), 4.97-5.05 (m, 1 H), 4.64 (s, 2 H), 4.09-4.22 (m, 2 H), 3.93-4.05 (m, 2 H), 2.42 (q, 2 H).

### Stade F : [4-bromo-1-(tétrahydrofuran-3-yl)-1H-pyrazol-5-yl]acétonitrile

A une solution du composé obtenu au Stade E (7,92 g ; 29,8 mmol) dans un mélange d'acétonitrile (80 mL) et d'eau (80 mL), est additionné du cyanure de potassium (3,88 g ; 59,7 mmol), puis l'ensemble est agité à 60 °C pendant 16 heures. L'acétonitrile est évaporé et la phase aqueuse est extraite à l'acétate d'éthyle. Les phases organiques sont réunies, lavées avec de l'eau, une solution aqueuse saturée en chlorure de sodium, puis séchées sur sulfate de sodium, filtrées et concentrées à sec. Le résidu obtenu est purifié par chromatographie sur gel de silice en utilisant l'heptane et l'acétate d'éthyle comme éluants pour obtenir le produit du titre.

**RMN ¹H** (400 MHz, CDCl₃, 300 K) δ ppm : 7.53 (s, 1 H), 4.92-5.00 (m, 1 H), 4.14-4.25 (m, 2 H), 4.03-4.10 (m, 1 H), 3.97 (td, 1 H), 3.85 (d, 2 H), 2.36-2.54 (m, 2 H).

### Stade G : 2-[4-bromo-1-(tétrahydrofuran-3-yl)-1H-pyrazol-5-yl]éthanol

A une solution du composé obtenu au Stade F (6,83 g ; 26,7 mmol) dans du dichlorométhane (133 mL), est additionnée à -78 °C une solution d'hydrure de diisobutylaluminium 1 M dans le dichlorométhane (53,3 mL ; 53,3 mmol), puis l'ensemble est agité à -78 °C pendant 3 heures. Après un ajout lent de méthanol (10 mL) et de borohydrure de sodium (3,04 g ; 80,1 mmol), le milieu réactionnel est agité à température ambiante pendant 16 heures, puis dilué avec une solution aqueuse d'acide chlorhydrique 1 N (50 mL). Après un contact de 10 minutes, les solvants sont évaporés et le produit est extrait à l'acétate d'éthyle. Les phases organiques sont réunies, lavées avec une solution aqueuse saturée en chlorure de sodium, séchées sur sulfate de magnésium, filtrées et concentrées à sec. Le résidu obtenu est purifié par chromatographie sur gel de silice en utilisant le dichlorométhane et le méthanol comme éluants pour obtenir le produit du titre.

**RMN ¹H** (400 MHz, CDCl₃, 300 K) δ ppm : 7.48 (s, 1 H), 5.04 (tt, 1 H), 4.13-4.21 (m, 1 H), 4.06-4.12 (m, 1 H), 3.91-4.00 (m, 2 H), 3.80-3.89 (m, 2 H), 2.96 (t, 2 H), 2.29-2.44 (m, 2 H).

### Stade H : 5-{2-[4-bromo-1-(tétrahydrofuran-3-yl)-1H-pyrazol-5-yl]éthoxy}-3,4-dihydroisoquinoléine-2(1H)-carboxylate de tert-butyle

Une solution de triphénylphosphine (2,64 g; 10,1 mmol) et d'azodicarboxylate de diisopropyle (1,97 mL ; 10,1 mmol) dans le tétrahydrofurane à 0 °C est préparée. Après décoloration et apparition d'un précipité blanchâtre (au bout de 5 minutes), on additionne une solution du composé obtenu au Stade G (2,02 g ; 7,74 mmol) dans du tétrahydrofurane (10 mL), puis l'ensemble est agité à température ambiante pendant 1 heure. Après ajout du composé de la Préparation 3b' (2,5 g; 10,1 mmol) en solution dans le tétrahydrofurane (10 mL), le milieu réactionnel est agité à température ambiante pendant 4 heures puis dilué avec du diméthylsulfoxyde (10 mL). Après concentration du milieu réactionnel, le résidu est purifié par chromatographie sur phase RP-18 en utilisant le méthanol et l'eau comme éluants, puis par chromatographie sur gel de silice en utilisant le dichlorométhane et le méthanol comme éluants pour obtenir le produit du titre.

**RMN ¹H** (400 MHz, CDCl₃, 300 K) δ ppm : 7.49 (s, 1 H), 7.12 (t, 1 H), 6.74 (d, 1 H), 6.66 (d, 1 H), 4.99-5.09 (m, 1 H), 4.54 (s, 2 H), 4.12-4.23 (m, 3 H), 4.09 (dd, 1 H), 3.93-4.02 (m, 2 H), 3.54-3.69 (m, 2 H), 3.21 (td, 2 H), 2.63-2.71 (m, 2 H), 2.28-2.47 (m, 2 H), 1.48 (s, 9 H).

### Stade I: 5-(2-{4-[(4-{[tert-butyl(diméthyl)silyl]oxy}phényl)amino]-1-(tétrahydrofuran-3-yl)-1H-pyrazol-5-yl}éthoxy)-3,4-dihydroisoquinoléine-2(1H)-carboxylate de tert-butyle

Le composé du titre est obtenu selon le procédé décrit au Stade B de la Préparation 7b" en utilisant le composé du stade précédent.

**RMN ¹H** (400 MHz, CDCl₃, 300 K) δ ppm : 7.48 (s, 1 H), 7.08 (t, 1 H), 6.73 (d, 1 H), 6.62-6.67 (m, 2 H), 6.56 (d, 1 H),6.48-6.52 (m, 2 H), 4.96-5.04 (m, 1 H), 4.75 (s, 1 H), 4.54 (s, 2 H), 4.17-4.24 (m, 1 H), 4.02-4.15 (m, 4 H), 3.94-4.02 (m, 1 H), 6.61 (br. s, 1 H), 3.11 (t, 2 H), 2.67 (br. s, 2 H), 2.45-2.54 (m, 1 H), 2.31-2.42 (m, 1 H), 1.49 (s, 9 H), 0.96 (s, 9 H), 0.15 (s, 6 H).

### Préparation 10b" : 4-(2-[4-[(4-[[tert-butyl(diméthyl)silyl]oxy}phényl)amino]-1-méthyl-1H-pyrazol-5-yl}éthoxy)-1,3-dihydro-2H-isoindole-2-carboxylate de tert-butyle

### Stade A : 4-[2-(4-bromo-1-méthyl-1H-pyrazol-5-yl)éthoxy]-1,3-dihydro-2H-isoindole-2-carboxylate de tert-butyle

Une solution du composé obtenu au Stade B de la Préparation 3b" (1 g ; 4,92 mmol) et du composé de la Préparation 6b' (1,54 g ; 6,54 mmol) dans un mélange de tétrahydrofurane (10 mL) et de toluène (10 mL) est dégazée à l'argon pendant 10 minutes. On additionne le cyanométhylène tri-*n*-butylphosphorane (2,58 mL ; 9,84 mmol), puis l'ensemble est scellé et agité à 110 °C pendant 48 heures. Le milieu réactionnel est concentré puis dilué dans un mélange d'acétate d'éthyle et d'eau. Après décantation, la phase organique est lavée avec de l'eau et une solution aqueuse saturée en chlorure de sodium, puis séchée sur sulfate de magnésium, filtrée et concentrée à sec. Le résidu est trituré dans le cyclohexane pour obtenir le produit du titre.

**RMN ¹H** (400 MHz, dmso-d6, 300K) δ ppm : 7.49 (s, 1 H), 7.24 (t, 1 H), 6.89 (m, 2 H), 4.55 (d, 2 H), 4.41 (m, 2 H), 4.19 (t, 2 H), 3.87 (d, 3 H), 3.16 (t, 2 H), 1.45 (d, 9 H).

**IR :** v : >C=C< : 1686 cm⁻¹.

### Stade B : 4-(2-{4-[(4-[[tert-butyl(diméthyl)silyl]oxy}phényl)amino]-1-méthyl-1H-pyrazol-5-yl}éthoxy)-l,3-dihydro-2H-isoindole-2-carboxylate de tert-butyle

Une solution du composé obtenu au Stade A (0,95 g ; 2,25 mmol) et du composé obtenu au Stade B de la Préparation 1b" (0,74 g ; 3,37 mmol) dans le tétrahydrofurane (50 mL) est dégazée à l'argon pendant 10 minutes. On additionne le tert-butylate de sodium (281 mg ; 2,9 mmol) et le chloro(2-di-*tert*-butylphosphino-2',4',6'-triisopropyl-1,1'-biphényl)[2-(2-aminoéthyl)phényl]palladium(II) (154 mg ; 0,22 mmol), puis l'ensemble est agité à 50 °C pendant 3 heures. Le milieu réactionnel est filtré sur Célite^{®} Après un rinçage avec de l'acétate d'éthyle, le produit est extrait à l'acétate d'éthyle, puis les phases organiques sont lavées avec une solution aqueuse saturée en chlorure d'ammonium, de l'eau et une solution aqueuse saturée en chlorure de sodium, puis séchées sur sulfate de magnésium, filtrées et concentrées à sec. Le résidu est purifié par chromatographie sur gel de silice en utilisant le dichlorométhane et l'acétate d'éthyle comme éluants pour obtenir le produit du titre.

**RMN ¹H** (400 MHz, dmso-d6, 300 K) δ ppm : 7.28 (s, 1 H), 7.18 (t, 1 H), 6.88 (2d, 1 H), 6.75 (d, 1 H), 6.69 (2s, 1 H), 6.58 (d, 2 H), 6.49 (d, 2 H), 4.55 (d, 2 H), 4.41 (s, 2 H), 4.1 (t, 2 H), 3.81 (s, 3 H), 3.01 (t, 2 H), 1.48 (s, 9 H), 0.91 (s, 9 H), 0.11 (s, 6 H).

**IR :** v : >NH : 3308 cm⁻¹ ; >C=O : 1681 cm⁻¹ ; >C=C< : 1617 cm⁻¹.

### Préparation 11b" : (3R ou 3S)-5-(3-{3-[(4-{[tert-butyl(diméthyl)silyl]oxy}phényl)amino]-5-cyano-1-méthyl-1H-pyrrol-2-yl}propoxy)-3-méthyl-3,4-dihydroisoquinoléine-2(1H)-carboxylate de tert-butyle

### Stade A : 5-[3-(3-bromo-5-cyano-1-méthyl-1H-pyrrol-2-yl)propoxy]-3-méthyl-3,4-dihydroisoquinoléine-2(1H)-carboxylate de tert-butyle

Une solution du composé obtenu au Stade D de la Préparation 5b" (3,19 g ; 13,12 mmol) et du composé obtenu à la Préparation 7b' (3,14 g; 11,93 mmol) dans un mélange de tétrahydrofurane (30 mL) et de toluène (30 mL) est dégazée à l'argon. On additionne le cyanométhylène tri-*n*-butylphosphorane (6,26 mL ; 23,86 mmol). Le ballon est scellé et le milieu réactionnel est agité à 110 °C pendant 20 heures, puis concentré. Le résidu ainsi obtenu est dilué dans un mélange d'acétate d'éthyle et d'eau. Après extraction de la phase aqueuse à l'acétate d'éthyle, les phases organiques sont réunies, lavées avecde l'eau, une solution aqueuse saturée en chlorure de sodium, puis séchées sur sulfate de magnésium, filtrées et concentrées à sec. Le résidu est purifié par chromatographie sur gel de silice en utilisant l'heptane et l'acétate d'éthyle comme éluants pour obtenir le produit du titre.

**RMN ¹H** (400 MHz, dmso-d6, 300 K) δ ppm : 7.11 (t, 1 H), 7.09 (s, 1 H), 6.78 (t, 2 H), 4.55 (d, 2 H), 4.5 (m, 1 H), 3.95 (m, 2 H), 3.7 (m, 3 H), 2.85 (t, 2 H), 2.69 (d, 2 H), 1.95 (t, 2 H), 1.4 (s, 9 H), 1 (d, 3 H).

**IR :** v : -CN : 2215 cm⁻¹ ; >C=O : 1686 cm⁻¹.

### Stade B : 5-(3-{3-[(4-{[tert-butyl(diméthyl)silyl]oxy}phényl)amino]-5-cyano-1-méthyl-1H-pyrrol-2-yl}propoxy)-3-méthyl-3,4-dihydroisoquinoléine-2(1H)-carboxylate de tert-butyle

Le composé du titre est obtenu selon le procédé décrit au Stade D de la Préparation 3b" en utilisant le composé du stade précédent.

**RMN ¹H** (400 MHz, dmso-d6, 300 K) δ ppm : 7.05 (t, 1 H), 6.8 (m, 2 H), 6.75 (d, 1 H), 6.65 (d, 1 H), 6.55 (d, 2 H), 6.5 (d, 2 H), 4.65 (d, 1 H), 4.15 (d, 1 H), 4.45 (m, 1 H), 3.88 (t, 2 H), 3.65 (s, 3 H), 2.75 (t, 2 H), 2.55 (d, 2 H), 1.9 (m, 2 H), 1.45 (s, 9 H), 0.98 (d, 3 H), 0.95 (s, 6 H), 0.1 (s, 9 H).

**IR :** v : >NH : 3400 cm⁻¹; -CN : 2208 cm⁻¹ ; >C=O : 1688 cm⁻¹.

### Stade C : (3R ou 3S)-5-(3-{3-[(4-{[tert-butyl(diméthyl)silyl]oxy}phényl)amino]-5-cyano-1-méthyl-1H-pyrrol-2-yl}propoxy)-3-méthyl-3,4-dihydroisoquinoléine-2(1H)-carboxylate de tert-butyle

Le composé du titre est obtenu par chromatographie en phase chirale (S,S) Whelk-01 en utilisant l'heptane et l'isopropanol comme éluants pour obtenir le produit du titre.

**RMN ¹H** (400 MHz, dmso-d6, 300 K) δ ppm : 7.07 (t, 1 H), 6.77 (s, 1 H), 6.77 (m, 1 H), 6.73 (d, 1 H), 6.66 (d, 1 H), 6.56 (d, 2 H), 6.49 (d, 2 H), 4.62 (d, 1 H), 4.14 (d, 1 H), 4.46 (m, 1 H), 3.87 (t, 2 H), 3.66 (s, 3 H), 2.74 (t, 2 H), 2.57 (m, 2 H), 1.89 (m, 2 H), 1.43 (s, 9 H), 0.95 (d, 3 H), 0.92 (s, 9 H), 0.11 (s, 6 H).

### Préparation 12b": (3S ou 3R)-5-(3-{3-[(4-{[tert-butyl(diméthyl)silyl]oxy}phényl)amino]-5-cyano-1-méthyl-1H-pyrrol- 2-yl}propoxy)-3-méthyl-3,4-dihydroisoquinoléine-2(1H)-carboxylate de tert-butyle

Le composé du titre est obtenu selon le procédé de la Préparation 11b".

**RMN ¹H** (400 MHz, dmso-d6, 300 K) δ ppm : 7.07 (t, 1 H), 6.77 (s, 1 H), 6.77 (m, 1 H), 6.73 (d, 1 H), 6.66 (d, 1 H), 6.56 (d, 2 H), 6.49 (d, 2 H), 4.62 (d, 1 H), 4.14 (d, 1 H), 4.46 (m, 1 H), 3.87 (t, 2 H), 3.66 (s, 3 H), 2.74 (t, 2 H), 2.57 (m, 2 H), 1.89 (m, 2 H), 1.43 (s, 9 H), 0.95 (d, 3 H), 0.92 (s, 9 H), 0.11 (s, 6 H).

### Préparation 13b": 4-(2-{5-(benzyloxy)-2-[(5-cyano-l,2-diméthyl-1H-pyrrol-3-yl)amino]phénoxy}éthyl)pipéridine-1-carboxylate de tert-butyle

### Stade A : 4-(benzyloxy)-2-fluoro-1-nitrobenzène

A une solution de 3-fluoro-4-nitrophénol (12,76 g ; 81,2 mmol) dans l'acétone (165 mL), sont additionnés le carbonate de potassium (13,47 g ; 97,5 mmol) et le bromure de benzyle (9,75 mL ; 82,0 mmol). Le milieu réactionnel est agité à reflux pendant 14 heures, puis après retour à température ambiante, il est dilué dans de l'eau. Après extraction de la phase aqueuse à l'acétate d'éthyle, les phases organiques sont réunies, lavées avec une solution aqueuse saturée en chlorure de sodium, puis séchées sur sulfate de magnésium, filtrées et concentrées à sec. Le résidu est repris dans un minimum d'acétate d'éthyle et du pentane, et le précipité obtenu est filtré pour obtenir le produit du titre.

**RMN ¹H** (400 MHz, dmso-d6, 300 K) δ ppm : 8.15 (t, 1 H), 7.25 (dd, 1 H), 7.05 (dd, 1 H), 5.25 (s, 2 H), 7.3-7.5 (m, 5 H).

**IR :** v : -NO2 : 1510 et 1500 cm⁻¹; -NO2 : 1329 cm⁻¹.

### Stade B : 4-{2-[5-(benzyloxy)-2-nitrophénoxy]éthyl}pipéridine-1-carboxylate de tert-butyle

A un mélange d'hydrure de sodium (1,52 g ; 38,1 mmol) dans du tétrahydrofurane (30 mL) à 0 °C, est additionnée goutte à goutte une solution de 4-(2-hydroxyéthyl)pipéridine-1-carboxylate de *tert*-butyle (8 g ; 35 mmol) dans le tétrahydrofurane (30 mL). Le milieu réactionnel est agité pendant 15 minutes à 0 °C, puis pendant 30 minutes à température ambiante. Le milieu réactionnel est de nouveau refroidi jusqu'à 0 °C, puis on additionne goutte à goutte une solution du composé obtenu au Stade A (7,86 g ; 31,79 mmol) dans le tétrahydrofurane (30 mL). Le milieu réactionnel est agité à température ambiante pendant 17 heures puis hydrolysé. Après extraction de la phase aqueuse à l'acétate d'éthyle, les phases organiques sont réunies, puis séchées sur sulfate de magnésium, filtrées et concentrées à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.95 (d, 1 H), 7.4 (m, 5 H), 6.9 (d, 1 H), 6.7 (dd, 1 H), 5.25 (s, 2 H), 4.2 (t, 2 H), 3.9/2.7 (m, 4 H), 1.7 (m, 3 H), 1.7/1.05 (m, 4 H), 1.4 (s, 9 H).

**IR :** v : ; >C=O : 1683 cm⁻¹; -NO2 : 1513 et 1255 cm⁻¹.

### Stade C : 4-{2-[2-amino-5-(benzyloxy)phénoxy]éthyl}pipéridine-1-carboxylate de tert-butyle

A une solution du composé obtenu au Stade B (9,4 g ; 22 mmol) dans un mélange de tétrahydrofurane (70 mL) et d'acide acétique glacial (70 mL), est additionné du fer (12,9 g ; 23,1 mmol) puis l'ensemble est chauffé à 65 °C pendant 18 heures. Le milieu réactionnel est filtré sur Célite^{®}, puis après l'ajout d'une solution aqueuse de soude 5 N pour atteindre un pH=7, il est concentré de moitié. Après extraction de la phase aqueuse au dichlorométhane, les phases organiques sont réunies, puis séchées sur sulfate de magnésium, filtrées et concentrées à sec. Le résidu est purifié par chromatographie sur gel de silice en utilisant le dichlorométhane et l'acétate d'éthyle comme éluants pour obtenir le produit du titre.

**RMN ¹H** (400 MHz, dmso-d6, 300 K) δ ppm : 7.4-7.3 (m, 5 H), 6.55 (m, 2 H), 6.35 (dd, 1 H), 4.95 (s, 2 H), 4.22 (m, 2 H), 3.95 (m, 4 H), 2.7 (m, 2 H), 1.65 (m, 3 H), 1.65/1.05 (m, 4 H), 1.4 (s, 9 H).

**IR :** v : ; -NH2 : 3450-3365 cm⁻¹; >C=O : 1669 cm⁻¹.

### Stade D : 4-bromo-1,5-diméthyl-1H-pyrrole-2-carbonitrile

A une solution de 1,5-diméthyl-1*H*-pyrrole-2-carbonitrile (15 g ; 124,8 mmol) dans l'acide acétique glacial (300 mL), est additionnée goutte à goutte une solution de brome (24 mL ; 457,74 mmol) dans l'acide acétique glacial (60 mL). Le milieu réactionnel est agité pendant 16 heures à température ambiante. Après concentration du milieu réactionnel de moitié, la même quantité d'eau est additionnée (180 mL) et le précipité formé est filtré, puis solubilisé dans le dichlorométhane. La phase organique est lavée par une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.05 (s, 1 H), 3.65 (s, 3 H), 2.23 (s, 3 H). **IR :** v : ; >CH: 3131 cm⁻¹; >CN : 2220 cm⁻¹.

### Stade E : 4-(2-{5-(benzyloxy)-2-[(5-cyano-l,2-diméthyl-1H-pyrrol-3-yl)amino]phénoxy}éthyl)pipéridine-1-carboxylate de tert-butyle

Le composé du titre est obtenu selon le procédé décrit au Stade B de la Préparation 2b" en utilisant le composé du stade précédent.

**RMN ¹H** (400 MHz, dmso-d6, 300 K) δ ppm : 7.4 (d, 2 H), 7.35 (t, 2 H), 7.3 (f, 1 H), 6.7 (s, 1 H), 6.6 (d, 1 H), 6.35 (dd, 1 H), 6.3 (d, 1 H), 5.85 (s, 1 H), 4.95 (s, 2 H), 4.05 (t, 2 H), 3.9/2.7 (m+m, 2+2 H), 3.6 (s, 3 H), 2.05 (s, 3 H), 1.7 (m, 5 H), 1.4 (s, 9 H), 1.05 (m, 2 H).

**IR :** v : ; >NH : 3404 cm⁻¹ ; >CN : 2207 cm⁻¹ ; >C=O : 1684 cm⁻¹.

### Préparation 14b" : 3-(2-{5-(benzyloxy)-2-[(5-cyano-1,2-diméthyl-1H-pyrrol-3 yl)amino]phénoxy}éthyl)pipéridine-1-carboxylate de tert-butyle

Le composé du titre est obtenu selon le procédé de la Préparation 13b" en utilisant le 3-(2-hydroxyéthyl)-pipéridine-1-carboxylate de *tert*-butyle au Stade B.

**RMN ¹H** (400 MHz, dmso-d6, 300 K) δ ppm : 7.4 (d, 2 H), 7.35 (t, 1 H), 7.3 (t, 2 H), 6.75 (s, 1 H), 6.62 (d, 1 H), 6.35 (dd, 1 H), 6.25 (d, 1 H), 5.85 (s, 1 H), 4.95 (s, 2 H), 4.05-2.5 (m, 4 H), 4.05 (t, 2 H), 3.63 (s, 3 H), 2.1 (s, 3 H), 1.8-1.3 (m, 7 H), 1.35 (br. s, 9 H).

**IR :** v : ; >NH : 3400 cm⁻¹ ; >CN : 2207 cm⁻¹ ; >C=O : 1684 cm⁻¹.

### Préparation 15b" : iodure de ]-(3-{[2-(tert-butoxycarbonyl)-1,2,3,4-tétrahydroisoquinoléin-5-yl]oxy}propyl)-3-{[4-(prop-2-én-1-yloxy)phényl]amino}pyridinium

### Stade A : 1-nitro-4-(prop-2-én-1-yloxy)benzène

A une solution de 4-nitrophénol (20 g; 0,144 mol) dans l'acétonitrile (500 mL), sont additionnés le bromure d'allyle (15 mL ; 0,173 mol) et le carbonate de césium (52 g, 0,158 mol), puis l'ensemble est agité pendant 16 heures à température ambiante et pendant 2 heures à 70 °C. Après filtration de l'insoluble et concentration du filtrat, le résidu est dilué avec du dichlorométhane et de l'eau. Après décantation, la phase organique est lavée avec de l'eau, puis séchée sur sulfate de magnésium, filtrée et concentrée à sec. Le résidu est purifié par chromatographie sur gel de silice en utilisant l'acétate d'éthyle et l'éther de pétrole comme éluants pour obtenir le produit du titre.

**RMN ¹H** (400 MHz, CDCl₃, 300 K) δ ppm : 8.2 (d, 2 H), 7 (d, 2 H), 6 (m, 1 H), 5.45 (tdd, 1 H), 5.4 (tdd, 1 H), 4.6 (m, 2 H).

**IR :** v : >NO2 : 1590 et 1331 cm⁻¹.

### Stade B : 4-(prop-2-én-1-yloxy)aniline

Le composé du titre est obtenu selon le procédé décrit au Stade C de la Préparation 13b" en utilisant le composé du stade précédent.

**RMN ¹H** (400 MHz, dmso-d6, 300 K) δ ppm : 6.65 (d, 2 H), 6.49 (d, 2 H), 6 (m, 1 H), 5.34/5.2 (dd, 2 H), 4.59 (s, 2 H), 4.4 (d, 2 H).

**IR :** v : >NH2 : 3429 et 3350 cm⁻¹.

### Stade C : N-[4-(prop-2-én-1-yloxy)phényl]pyridin-3-amine

Le composé du titre est obtenu selon le procédé décrit au Stade D de la Préparation 3b" en utilisant la 3-bromopyridine et le composé du stade précédent.

**RMN 1H** (400 MHz, dmso-d6, 300 K) δ ppm : 8.22 (d, 1 H), 8.05 (s, 1 H), 7.92 (dd, 1 H), 7.31-7.11 (2*dd, 2 H), 7.05 (d, 2 H), 6.91 (d, 2 H), 6.04 (m, 1 H), 5.46-5.19 (2*dd, 2 H), 4.52 (d, 2 H).

**IR :** v : >NH : 3250 et 3184 cm⁻¹.

### Stade D : iodure de 1-(3-{[2-(tert-butoxycarbonyl)-1,2,3,4-tétrahydroisoquinoléin-5-yl]oxy}propyl)-3-{[4-(prop-2-én-1-yloxy)phényl]amino}pyridinium

A une solution du composé obtenu au Stade C (740 mg ; 3,27 mmol) dans le dioxane (13 mL), est additionné le composé de la Préparation 8b' (1,05 g ; 2,5 mmol), puis l'ensemble est agité pendant 18 heures à 70 °C. Après concentration, le résidu est purifié par chromatographie sur gel de silice en utilisant le dichlorométhane et le méthanol comme éluant pour obtenir le produit du titre.

**RMN ¹H** (400 MHz, dmso-d6, 300 K) δ ppm : 9.1 (s, 1 H), 8.34 (m, 2 H), 7.81 (m, 2 H), 7.14 (t, 1 H), 7.12 (d, 2 H), 6.91 (d, 2 H), 6.78 (t, 2 H), 6.04 (m, 1 H), 5.4/5.27 (2*dd, 2 H), 4.67 (t, 2 H), 4.54 (d, 2 H), 4.46 (s, 2 H), 4.04 (t, 2 H), 3.5 (t, 2 H), 2.5 (d, 2 H), 2.37 (t, 2 H), 1.42 (s, 9 H).

**IR :** v : >NH : 3500-2700 cm⁻¹; >C=O : 1686 cm⁻¹.

### Préparation 16b ": 2-méthyl-N-[4-(prop-2-én-1-yloxy)phényl]-4-[2-(tétrahydro-2H-pyran-2-yloxy)éthoxy]pyrimidin-5-amine

### Stade A : 4-chloro-2-méthyl-6-[2-(tétrahydro-2H-pyran-2-yloxy)éthoxy]pyrimidin-5-amine

A un mélange d'hydrure de sodium (1,5 g ; 36,4 mmol) dans du tétrahydrofurane (10 mL) à 0 °C, est additionnée goutte à goutte une solution de 2-tétrahydropyran-2-yloxyéthanol (4,6 mL ; 33,6 mmol) dans le tétrahydrofurane (60 mL). Le milieu réactionnel est agité 15 minutes à 0 °C, puis 30 minutes à température ambiante. Le milieu réactionnel est de nouveau refroidi jusqu'à 0 °C, puis on additionne goutte à goutte une solution de 5-amino-4,6-dichloro-2-méthylpyrimidine (5 g ; 28 mmol) dans le tétrahydrofurane (70 mL). Le milieu réactionnel est agité à température ambiante pendant 17 heures puis hydrolysé. Après extraction de la phase aqueuse à l'acétate d'éthyle, les phases organiques sont réunies, puis séchées sur sulfate de magnésium, filtrées et concentrées à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 5.01 (s, 2 H), 4.66 (m, 1 H), 4.49 (m, 2 H), 3.94 (2*m, 2 H), 3.77/3.44 (2*m, 2 H), 2.34 (s, 3 H), 1.76-1.35 (m, 4 H), 1.61/1.45 (2*m, 2 H).

**IR :** v : -NH2 : 3464 et 3340 cm⁻¹.

### Stade B : 2-méthyl-4-[2-(tétrahydro-2H-pyran-2-yloxy)éthoxy]pyrimidin-5-amine

A une solution du composé obtenu au Stade A (6,9 g ; 24,0 mmol) dans l'éthanol (120 mL), est additionné du palladium sur charbon (10 % massique), puis l'ensemble est hydrogéné pendant 6 heures à température ambiante sous 1 bar. Le milieu réactionnel est filtré et concentré à sec. Le résidu est dilué dans un mélange d'une solution aqueuse saturée en bicarbonate de sodium et de dichlorométhane. Après décantation et extraction au dichlorométhane, les phases organiques sont réunies, puis séchées sur sulfate de magnésium pour fournir le produit du titre.

**RMN ¹H** (400 MHz, dmso-d6, 300 K) δ ppm : 7.76 (s, 1 H), 4.76 (s, 2 H), 4.65 (m, 1 H), 4.46 (m, 2 H), 3.93/3.75 (2m, 2 H), 3.75/3.44 (2m, 2 H), 2.34 (s, 3 H), 1.75-1.35 (m, 6 H).

**IR :** v : -NH₂: 3600-3100 cm⁻¹.

### Stade C : 1-bromo-4-(prop-2-én-1-yloxy)benzène

A une solution de 4-bromophénol (10 g ; 57,8 mmol) dans l'acétone (290 mL), sont additionnés le bromure d'allyle (5,5 mL ; 63,6 mmol) et le carbonate de potassium (16 g, 116 mmol), puis l'ensemble est agité pendant 6 heures à 85 °C, puis pendnat 16 heures à température ambiante. Après filtration de l'insoluble et concentration du filtrat, le résidu est dilué avec de l'acétate d'éthyle et de l'eau. Après décantation, la phase organique est lavée avec de l'eau et une solution aqueuse saturée en chlorure de sodium, puis séchée sur sulfate de magnésium, filtrée et concentrée à sec. Le résidu est purifié par chromatographie sur gel de silice en utilisant l'acétate d'éthyle et l'éther de pétrole comme éluants pour obtenir le produit du titre.

**RMN ¹H** (400 MHz, CDCl3, 300 K) δ ppm : 7.36 (d, 2 H), 6.79 (d, 2 H), 6.01 (m, 1 H), 5.4 (d, 1 H), 5.3 (d, 1 H), 4.5 (d, 2 H).

**IR :** v : >CH-Ar : 821 cm⁻¹ ; >C=C< : 1590, 1578 et 1488 cm⁻¹.

### Stade D : 2-méthyl-N-[4-(prop-2-én-1-yloxy)phényl]-4-[2-(tétrahydro-2H-pyran-2-yloxy)éthoxy]pyrimidin-5-amine

Le composé du titre est obtenu selon le procédé décrit au Stade B de la Préparation 2b" en utilisant les composés obtenus aux Stades B et C précédents.

**RMN ¹H** (400 MHz, dmso-d6, 300 K) δ ppm : 8.07 (s, 1 H), 7.17 (s, 1 H), 6.98 (d, 2 H), 6.86 (d, 2 H), 6.03 (m, 1 H), 5.38/5.24 (2*dd, 2 H), 4.62 (m, 1 H), 4.52 (m, 2 H), 4.5 (m, 2 H), 3.94/3.76 (2*m, 2 H), 3.74/3.4 (2*m, 2 H), 2.43 (s, 3 H), 1.61 (m, 2 H), 1.42 (m, 4 H).

**IR :** v : ; >NH : 3415 cm⁻¹; >C=C< : 1649 cm⁻¹.

### Préparation 17b" : [2-(3-cyano-5-{[4-(prop-2-én-1-yloxy)phényl]amino}phénoxy)éthyl] méthylcarbamate de tert-butyle

### Stade A : (3-bromo-5-méthoxyphényl)méthanol

A une solution de l'acide 3-bromo-5-méthoxybenzoïque (10 g ; 43,3 mmol) dans le tétrahydrofurane (280 mL), est additionné goutte à goutte le complexe de boranediméthylsulfure (32,5 mL ; 64,9 mmol), puis l'ensemble est agité pendant 2 heures. Le milieu réactionnel est acidifié goutte à goutte par une solution aqueuse d'acide chlorhydrique 2 N jusqu'à pH=1. Après extraction à l'éther, la phase organique est lavée avec une solution aqueuse de soude 1 N et une solution aqueuse saturée en chlorure de sodium, puis séchée sur sulfate de magnésium, filtrée et concentrée à sec. Le produit du titre est obtenu sous forme d'une huile, qui est utilisée au stade suivant sans purification.

**RMN** ¹**H** (400 MHz, dmso-d6, 300 K) δ ppm : 7.08 (m, 1 H), 6.99 (m, 1 H), 6.89 (m, 1 H), 5.3 (br. s, 1 H), 4.47 (s, 2 H), 3.75 (s, 3 H).

**IR :** v : -OH : 1588 cm⁻¹ ; >C-O : 1268 et 1038 cm⁻¹ ; γ : >CH-Ar : 811 cm⁻¹.

### Stade B : 3-bromo-5-méthoxybenzaldéhyde

A une solution du composé obtenu au Stade A (8,6 g ; 39,8 mmol) dans le dichlorométhane (400 mL) est additionné le réactif de Dess-Martin (20,3 mL ; 47,8 mmol), puis l'ensemble est agité pendant 2 heures. Après ajout d'éther, le milieu réactionnel est filtré sur un lit de silice. Le filtrat est concentré, repris dans un mélange d'heptane et d'acétate d'éthyle, puis filtré à nouveau sur un lit de silice. Après concentration du filtrat, le produit du titre est obtenu sous forme d'un solide jaune pâle, lequel est utilisé au stade suivant sans purification.

**RMN** ¹**H** (400 MHz, dmso-d6, 300 K) δ ppm : 9.5 (s, 1 H), 7.69 (t, 1 H), 7.5 (t, 1 H), 7.42 (t, 1 H), 3.85 (s, 3 H).

**IR :** v : >C=O : 1691 cm⁻¹.

### Stade C : (Z}-1-(3-bromo-5-méthoxyphényl)-N-hydroxyméthanimine

A une solution du composé obtenu au Stade B (7,8 g ; 36,4 mmol) dans l'éthanol (10 mL), sont additionnés successivement le chlorhydrate d'hydroxylamine (12,6 g ; 182 mmol) et la pyridine (6,27 mL ; 87,4 mmol), puis l'ensemble est agité à 65 °C pendant 1 heure. Après retour à température ambiante, le milieu réactionnel est dilué avec un mélange d'acétate d'éthyle et d'eau. Après décantation, la phase organique est lavée avec de l'eau et une solution aqueuse saturée en chlorure de sodium, puis séchée sur sulfate de magnésium, filtrée et concentrée à sec. Le produit du titre est obtenu sous forme d'un solide blanc, lequel est utilisé au stade suivant sans purification.

**RMN** ¹**H** (400 MHz, dmso-d6, 300 K) δ ppm : 11.45 (s, 1 H), 8.1 (s, 1 H), 7.35 (t, 1 H), 7.16 (d, 2 H), 3.8 (s, 3 H).

**IR :** v : -OH : 3300-3000 cm⁻¹; Ar : 1600 et 1564 cm⁻¹; >C-O : 1220 et 1059 cm⁻¹ ; -N-O : 960 cm⁻¹ ; γ : >CH-Ar : 831 cm⁻¹.

### Stade D : 3-bromo-5-méthoxybenzonitrile

A une solution du composé obtenu au Stade C (8,1 g ; 35,2 mmol) dans le dioxane (70 mL), sont additionnés à 0 °C la pyridine (22 mL ; 211 mmol) et, goutte à goutte, l'anhydride de l'acide trifluoroacétique (1,4 mL; 70,4 mmol), puis l'ensemble est agité à température ambiante pendant 24 heures. Après retour à 0 °C, on additionne goutte à goutte une deuxième portion d'anhydride de l'acide trifluoroacétique (1,4 mL ; 70,4 mmol), puis l'ensemble est agité à température ambiante pendant 24 heures. Après retour à 0 °C, on additionne goutte à goutte une troisième portion d'anhydride de l'acide trifluoroacétique (1,4 mL ; 70,4 mmol), puis l'ensemble est agité à 60 °C pendant 1 heure. Après retour à température ambiante, le milieu réactionnel est dilué avec un mélange de dichlorométhane et d'eau. Après décantation, la phase organique est lavée avec une solution aqueuse d'acide chlorhydrique 1 N et une solution aqueuse saturée en chlorure de sodium, puis séchée sur sulfate de magnésium, filtrée et concentrée à sec. Le produit du titre est obtenu sous forme d'un solide jaune pâle, lequel est utilisé au stade suivant sans purification.

**RMN** ¹**H** (400 MHz, dmso-d6, 300 K) δ ppm : 7.69 (t, 1 H), 7.53 (dd, 1 H), 7.5 (dd, 1 H), 3.82 (s, 3 H).

**IR :** v : -CN : 2232 cm⁻¹; Ar : 1597 et 1562 cm⁻¹; >C-O-C< : 1278 et 1051 cm⁻¹ ; γ : >CH-Ar : 848, 814 et 671 cm⁻¹.

### Stade E : 3-bromo-5-hydroxybenzonitrile

A une solution du composé obtenu au Stade D (5,9 g ; 27,9 mmol) dans la collidine (55 mL), est additionné l'iodure de lithium (11,2 g ; 83,7 mmol), puis l'ensemble est agité à 150 °C pendant 16 heures. Après retour à température ambiante, le milieu réactionnel est versé dans de l'eau glacé. Après extraction au dichlorométhane, les phases organiques sont réunies, lavées avec de l'eau, puis séchées sur sulfate de magnésium, filtrées et concentrées à sec. Le produit du titre est obtenu sous forme d'un solide brun orangé, lequel est utilisé au stade suivant sans purification.

**RMN** ¹**H** (400 MHz, dmso-d6, 300 K) δ ppm : 10.7 (br. s, 1 H), 7.51 (t, 1 H), 7.3 (t, 1 H), 7.18 (dd, 1H).

**IR :** v : -OH : 3283 cm⁻¹ ; -CN : 2245 cm⁻¹.

### Stade F : (2-hydroxyéthyl)méthylcarbamate de tert-butyle

A une solution du 2-(méthylamino)éthanol (30 g ; 0,399 mol) dans le dichlorométhane (800 mL), est additionné par portions à température ambiante le di-*tert*-butyl dicarbonate (87,3 g ; 0,399 mol). Le milieu est agité à cette température pendant 16 heures. Le solvant est évaporé sous vide et le résidu est purifié par chromatographie sur gel de silice en utilisant l'éther de pétrole et l'acétate d'éthyle comme éluants pour obtenir le produit du titre.

**RMN ¹H** (400 MHz, CDCl₃, 300 K) δ ppm : 3.75 (t, 2 H), 3.39 (t, 2 H), 2.91 (s, 3 H), 2.85 (m, 1 H), 1.45 (s, 9 H).

**IR :** v : -OH : 3431 cm⁻¹ ; >C=O : 1692 et 1668 cm⁻¹.

### Stade G : [2-(3-bromo-5-cyanophénoxy)éthyl]méthylcarbamate de tert-butyle

Le composé du titre est obtenu selon le procédé décrit au Stade H de la Préparation 9b" en utilisant les composés obtenus aux Stades E et F précédents.

**RMN ¹H** (400 MHz, dmso-d6, 300 K) δ ppm : 7.7 (br. s, 1 H), 7.52 (br. s, 1 H), 7.5 (br. s, 1 H), 4.2 (m, 2 H), 3.5 (t, 2 H), 2.88 (br. s, 3 H), 1.35 (2br. s, 9 H).

**IR :** v : -CN : 2235 cm⁻¹ ; >C=O : 1687 cm⁻¹.

### Stade H : [2-(3-cyano-5-{[4-(prop-2-én-1-yloxy)phényl]amino}phénoxy)éthyl]méthyl carbamate de tert-butyle

Le composé du titre est obtenu selon le procédé décrit au Stade B de la Préparation 2b" en utilisant le composé du stade précédent et le composé obtenu au Stade B de la Préparation 15b".

**RMN ¹H** (400 MHz, dmso-d6, 300 K) δ ppm : 7.07 (d, 2 H), 6.94 (d, 2 H), 6.73 (dd, 2 H), 6.63 (t, 1 H), 6.05 (m, 1 H), 5.4 (dd, 1 H), 5.26 (dd, 1 H), 4.54 (td, 2 H), 4.06 (t, 2 H), 3.5 (t, 2 H), 2.83 (s, 3 H), 1.34 (s, 9 H).

**IR :** v : -NH : 3344 cm⁻¹; -CN : 2231 cm⁻¹ ; >C=O : 1741 cm⁻¹; >C=O : 1673 cm⁻¹ ; >C=C< : 1591 cm⁻¹.

### Préparation 18b ": [4-(3-cyano-5-{[4-(prop-2-én-1-yloxy)phényl]amino}phénoxy)butyl ]méthylcarbamate de tert-butyle

Le composé du titre est obtenu selon le procédé de la Préparation 17b" en utilisant le 4-(méthylamino)butanol au Stade F.

**RMN ¹H** (400 MHz, dmso-d6, 300 K) δ ppm : 8.23 (s, 1 H), 7.07 (d, 2 H), 6.94 (d, 2 H), 6.73 (t, 1 H), 6.68 (t, 1 H), 6.63 (t, 1 H), 6.05 (m, 1 H), 5.39/5.26 (2dquad, 2 H), 4.54 (dt, 2 H), 3.96 (t, 2 H), 3.19 (t, 2 H), 2.76 (br. s, 3 H), 1.59 (m, 4 H), 1.37 (br. s, 9 H).

**IR :** v : -NH : 3340 cm⁻¹ ; -CN : 2229 cm⁻¹ ; >C=O : 1687 cm⁻¹ ; >C=O : 1670 cm⁻¹.

### Préparation 19b" : 5-(2-{2-[(4-{[tert-butyl(diméthyl)silyl]oxy}phényl)amino]éthoxy} éthoxy) -3,4-dihydroisoquinoléine-2(1H)-carboxylate de tert-butyle

### Stade A : 5-[2-(2-oxoéthoxy)éthoxy]-3,4-dihydroisoquinoléine-2(1H)-carboxylate de tert-butyle

A une solution de chlorure d'oxalyle (0,2 mL ; 2,22 mmol) dans le tétrahydrofurane (7 mL) est additionné à -78 °C le diméthylsulfoxyde (0,2 mL). Après un contact de 30 minutes à cette température, on ajoute goutte à goutte à -78 °C une solution du composé obtenu à la Préparation 9b' (0,5 g ; 1,48 mmol) dans le tétrahydrofurane (8 mL) et, dans les mêmes conditions, la triéthylamine (0,77 mL ; 5,93 mmol). L'ensemble est agité pendant 1 heure à -78 °C, puis pendant 2 heures à 0 °C. Le milieu réactionnel est dilué avec un mélange d'acétate d'éthyle et d'eau. Après décantation, la phase organique est lavée avec une solution aqueuse saturée en chlorure de sodium, puis séchée sur sulfate de magnésium, filtrée et concentrée à sec. Le produit du titre est obtenu sous forme d'une huile marron, qui est utilisée au stade suivant sans purification.

**RMN** ¹**H** (400 MHz, dmso-d6, 300 K) δ ppm : 9.6 (s, 1 H), 7.13 (t, 1 H), 6.82 (d, 1 H), 6.75 (d, 1 H), 4.46 (br. s, 2 H), 4.29 (s, 2 H), 4.12 (m, 2 H), 3.85 (m, 2 H), 3.54 (m, 2 H), 2.64 (t, 2 H), 1.41 (s, 9 H).

**IR :** v : >C=O : 1689 cm⁻¹.

### Stade B : 5-(2-{2-[(4-{[tert-butyl(diméthyl)silyl]oxy}phényl)amino]éthoxy}éthoxy)-3,4-dihydroisoquinoléine-2(1H)-carboxylate de tert-butyle

A une solution du composé obtenu au Stade A (0,5 g ; 1,49 mmol) dans le dichlorométhane (10 mL), sont additionnés le composé obtenu au Stade B de la Préparation 1b" (0,4 g ; 1,79 mmol) et le triacétoxy-borohydrure de sodium (0,63 g ; 2,98 mmol). L'ensemble est agité pendant 16 heures à température ambiante. Le milieu réactionnel est dilué dans une solution aqueuse saturée en hydrogénocarbonate de sodium. Après extraction au dichlorométhane, les phases organiques sont lavées avec une solution aqueuse saturée en chlorure de sodium, puis séchées sur sulfate de magnésium, filtrées et concentrées à sec. Le résidu est purifié par chromatographie sur gel de silice en utilisant l'acétate d'éthyle et l'éther de pétrole comme éluants pour obtenir le produit du titre.

**RMN ¹H** (400 MHz, dmso-d6, 300 K) δ ppm : 7.13 (t, 1 H), 6.81 (d, 1 H), 6.75 (d, 1 H), 6.59 (d, 2 H), 6.47 (d, 2 H), 5.05 (t, 1 H), 4.46 (s, 2 H), 4.1 (t, 2 H), 3.77 (t, 2 H), 3.62 (t, 2 H), 3.52 (t, 2 H), 3.13 (quad, 2 H), 2.63 (t, 2 H), 1.42 (s, 9 H), 0.92 (s, 9 H), 0.11 (s, 6 H).

**IR :** v : >NH : 3387 cm⁻¹ ; >C=O : 1693 cm⁻¹.

### Préparation 20b" : 5-[2-(1-{3-[(4-{[tert-butyl(diméthyl)silyl]oxy}phényl)amino]-5-cyanophényl}-1H-1,2,3-triazol-4-yl)éthyl]-3,4-dihydroisoquinoléine-2(1H)-carboxylate de tert-butyle

### Stade A : 3-bromo-5-nitrobenzonitrile

A une solution de tert-butyl nitrite (8,19 mL ; 62 mmol) dans le *N,N-* diméthylformamide (35 mL) à 50 °C, est additionnée goutte à goutte une solution de 2-amino-3-bromo-5-nitrobenzonitrile (10 g ; 41,3 mmol) dans du *N*,*N*-diméthylformamide (85 mL). Le mélange est agité à 50 °C jusqu'à ce que le dégagement gazeux s'arrête. Le mélange brut est ensuite versé dans une solution aqueuse 0,5 N d'acide chlorhydrique (1 L), puis le tout est extrait avec du méthyl-*tert*-butyléther. Les phases organiques sont réunies et lavées avec une solution aqueuse saturée de bicarbonate de sodium, puis avec une solution aqueuse saturée en chlorure de sodium, séchées avec du sulfate de sodium, filtrées et concentrées sous vide. Le résidu est purifié par chromatographie sur gel de silice en utilisant l'acétate d'éthyle et l'heptane comme éluants pour obtenir le produit du titre.

**RMN ¹H** (400 MHz, CDCl₃, 300 K) δ ppm 8.61 (t, 1 H), 8.47 (t, 1 H), 8.12 (t, 1 H).

### Stade B : 3-amino-5-bromobenzonitrile

A une solution du composé obtenu au Stade A (6,85 g ; 30 mmol) dans de l'acétate d'éthyle (20 mL) et de l'éthanol (82 mL), est additionnépar portions du chlorure d'étain dihydraté (33,85 g ; 150 mmol). Le mélange réactionnel est ensuite chauffé à 70 °C pendant 30 minutes et concentré jusqu'à un volume d'environ 40 mL, puis versé sur de la glace. Le mélange ainsi obtenu est alcalinisé avec une solution aqueuse 2 N d'hydroxyde de sodium, et le tout est agité pendant 20 minutes. Le produit est extrait avec de l'acétate d'éthyle. Les phases organiques sont réunies et lavées avec une solution aqueuse saturée en chlorure de sodium, séchées avec du sulfate de sodium, filtrées et concentrées sous vide. Le résidu est purifié par chromatographie sur gel de silice en utilisant l'acétate d'éthyle et l'heptane comme éluants pour obtenir le produit du titre.

**RMN ¹H** (400 MHz, CDCl₃, 300 K) δ ppm : 7.12 (t, 1 H), 7.01 (t, 1 H), 6.80 - 6.83 (m, 1 H), 3.97 (br. s, 2 H).

### Stade C : 3-azido-5-bromobenzonitrile

A une solution du composé obtenu au Stade B (3,96 g ; 20 mmol) dans l'acétate d'éthyle (60 mL) à 0 °C, sont additionnés goutte à goutte de l'acide chlorhydrique concentré (12 mL) en maintenant la température inférieure à 5 °C, puis goutte à goutte une solution de sodium nitrite (1,66 g ; 24 mmol) dans de l'eau (25 mL) en maintenant également la température inférieure à 5 °C. Après 1 heure d'agitation à 0 °C, une solution d'azidure de sodium (1,56 g ; 24 mmol) dans de l'eau (25 mL) est ajoutée goutte à goutte en maintenant la température inférieure à 5 °C. Le mélange réactionnel est agité pendant 3 heures jusqu'à ce qu'il revienneà température ambiante , puis il est dilué avec de l'eau. Le produit est extrait avec de l'acétate d'éthyle. Les phases organiques sont réunies et lavées avec une solution aqueuse saturée en chlorure de sodium, séchées avec du sulfate de sodium, filtrées et concentrées sous vide pour fournir le composé du titre sans purification.

**RMN ¹H** (400 MHz, CDCl₃, 300 K) δ ppm : 7.55 (t, 1 H), 7.41 (t, 1 H), 7.20 - 7.24 (m, 1 H).

### Stade D : 5-{2-[1-(3-bromo-5-cyanophényl)-1H-l,2,3-triazol-4-yl]éthyl}-3,4-dihydroisoquinoléine-2(1H)-carboxylate de tert-butyle

A une solution du composé obtenu au Stade C (1,56 g ; 7 mmol) et du composé obtenu à la Préparation 10b' (2 g ; 7 mmol) dans du tétrahydrofurane (30 mL), sont additionnés de l'iodure de cuivre (133 mg ; 0,7 mmol) et de la triéthylamine (1,16 mL ; 8,4 mmol). Le mélange réactionnel est agité à température ambiante pendant 16 heures. La réaction est ensuite concentrée jusqu'à un volume d'environ 15 mL, et le tout est dilué avec de l'acétate d'éthyle. La phase organique est lavée avec une solution aqueuse 2 N d'acide chlorhydrique, puis avec une solution aqueuse saturée en chlorure de sodium. La phase organique est séchée avec du sulfate de sodium, filtrée et concentrée sous vide. Le résidu est purifié par chromatographie sur gel de silice en utilisant l'acétate d'éthyle et l'heptane comme éluants pour obtenir le produit du titre.

**RMN ¹H** (400 MHz, CDCl₃, 300 K) δ ppm : 8.17 (s, 1 H), 7.91 - 7.99 (m, 1 H), 7.79 - 7.86 (m, 1 H), 7.64 (br. s, 1 H), 7.09 - 7.20 (m, 1 H), 7.03 (dd, 2 H), 4.58 (s, 2 H), 3.65 (br. s, 2 H), 3.06 (br. s, 4 H), 2.82 (br. s, 2 H), 1.49 (s, 9 H).

### Stade E : 5-[2-(1-{3-[(4-{[tert-butyl(diméthyl)silyl]oxy}phényl)amino]-5-cyanophényl}-1H-1,2,3-triazol-4-yl)éthyl]-3,4-dihydroisoquinoléine-2(1H)-carboxylate de tert-butyle

Le composé du titre est obtenu selon le procédé décrit au Stade B de la Préparation 2b" en utilisant le composé du stade précédent et le composé du Stade B de la Préparation 1b".

**RMN ¹H** (400 MHz, CDCl₃, 300 K) δ ppm : 7.40 - 7.76 (m, 2 H), 7.19 (s, 1 H), 7.05 - 7.16 (m, 4 H), 6.95 - 7.05 (m, 2 H), 6.82 - 6.90 (m, 2 H), 6.67 (br. s, 1 H), 4.57 (s, 2 H), 3.58 - 3.67 (m, 2 H), 2.96 - 3.07 (m, 4 H), 2.70 - 2.81 (m, 2 H), 1.47 (s, 9 H), 1.00 (s, 9 H), 0.22 (s, 6 H).

### Préparation 21b": 4-(2-{2-[(5-{[4-(prop-2-én-1-yloxy)phényl]amino}pyrimidin-2-yl)oxy]éthoxy}éthoxy)-l,3-dihydro-2H-isoindole-2-carboxylate de tert-butyle

### Stade A : 5-bromo-2-[2-(2-chloroéthoxy)éthoxy]pyrimidine

A une solution de 5-bromo-2-chloropyrimidine (3,48 g ; 18 mmol) et de 2-(2-chloroéthoxy)-éthanol (3,59 g; 28,8 mmol) dans l'acétonitrile (50 mL), est additionné le carbonate de césium (11,73 g, 36 mmol), puis l'ensemble est agité pendant 14 heures à 60 °C. Après refroidissement, le milieu réactionnel est filtré, puis le filtrat est concentré. Le résidu est purifié par chromatographie sur gel de silice en utilisant l'heptane et l'acétate d'éthyle comme éluants pour obtenir le produit du titre.

**RMN ¹H** (500 MHz, dmso-d6, 300 K) δ ppm : 8.75 (s, 2 H), 4.41 (m, 2 H), 3.8 (m, 2 H), 3.72 (s, 4 H).

### Stade B : 4-{2-[2-({5-[(4-hydroxyphényl)amino]pyrimidin-2-yl}oxy)éthoxy]éthoxy}1-1,3-dihydro-2H-isoindole-2-carboxylate de tert-butyle

A une solution du composé obtenu au Stade A (2,81 g ; 10 mmol) et du composé de la Préparation 6b' (2,5 g ; 10 mmol) dans l'acétonitrile (100 mL), sont additionnés le carbonate de césium (3,9 g, 12 mmol) et l'iodure de potassium (0,35 g ; 2,1 mmol), puis l'ensemble est agité pendant 14 heures à 60 °C. Après retour à température ambiante, le milieu réactionnel est concentré. Le résidu est purifié par chromatographie sur gel de silice en utilisant l'heptane et l'acétate d'éthyle comme éluants.

Une solution du résidu ainsi obtenu (3,3 g ; 6,87 mmol) et du composé du Stade B de la Préparation 15b" (1,44 g ; 9,6 mmol) dans le tétrahydrofurane (60 mL) est dégazée à l'argon pendant 10 minutes. On additionne le carbonate de césium (2,91 g ; 8,9 mmol) et le chloro(2-di-*tert*-butylphosphino-2',4',6'-triisopropyl-1, 1'-biphényl)[2-(2-aminoéthyl)phényl]palladium(II) (180 mg ; 0,26 mmol), puis l'ensemble est agité à reflux pendant 5 heures, puis à 50 °C pendant 15 heures. Après refroidissement, le milieu réactionnel est concentré. Le résidu est purifié par chromatographie sur gel de silice en utilisant l'heptane et l'acétate d'éthyle comme éluants pour obtenir le produit du titre.

**RMN ¹H** (500 MHz, dmso-d6, 300 K) δ ppm : 9.00 (1H, s), 8.22 (1H, s), 8.21 (1H, s), 7.65 (1H, s), 6.90 (1H, d), 6.89 (1H, d), 7.24 (1H, t), 6.86 (2H, m), 6.68 (2H, m), 4.45 - 4.57 (4H, m), 4.33 (2H, m), 4.16 (2H, m), 3.81 (4H, m), 1.44 (9H, s).

### Stade C : 4-(2-{2-[(5-{[4-(prop-2-én-1-yloxy)phényl]amino}pyrimidin-2-yl)oxy]éthoxy}éthoxy)-1,3-dihydro-2H-isoindole-2-carboxylate de tert-butyle

Le composé du titre est obtenu selon le procédé décrit au Stade C de la Préparation 16b" en utilisant le phénol du stade B.

**RMN ¹H** (500 MHz, dmso-d6, 300 K) δ ppm : 8.29/8.28 (s, 2 H), 7.81 (brs, 1 H), 7.24 (t, 1 H), 6.93 (m, 2 H), 6.9 (m, 1 H), 6.88 (m, 1 H), 6.86 (m, 2 H), 6.03 (m, 1 H), 5.37/5.24 (m+m, 2 H), 4.57/4.55 (brs, 2 H), 4.48/4.45 (brs, 2 H), 4.38-3.78 (m, 8 H), 1.44 (s, 9 H).

### Préparation 22b": 5-(2-{2-[(5-{[4-(prop-2-én-1-yloxy)phényl]amino}pyrimidin-2-yl)oxy]éthoxy}éthoxy)-3,4-dihydroisoquinoléine-2(1H)-carboxylate de tert-butyle

Le composé du titre est obtenu selon le procédé de la Préparation 21b" en remplaçant au Stade B le composé de la Préparation 6b' par le composé de la Préparation 3b'.

**RMN** ¹**H** (500 MHz, dmso-d6, 300 K) δ ppm : 8.29 (s, 2 H), 7.82 (s, 1 H), 7.12 (t, 1 H), 6.94 (m, 2 H), 6.86 (m, 2 H), 6.8 (d, 1 H), 6.73 (d, 1 H), 6.02 (m, 1 H), 5.37/5.23 (m+m, 2 H), 4.49 (m, 2 H), 4.45 (br., 2 H), 4.36 (m, 2 H), 4.1 (m, 2 H), 3.81 (m, 2 H), 3.81 (m, 2 H), 3.51 (t, 2 H), 2.61 (t, 2 H), 1.4 (s, 9 H).

### Préparation 23b ": 5-({6-[(5-{[4-(prop-2-én-1-yloxy)phényl]amino}pyrimidin-2-yl)oxy]hexyl}oxy)-3,4-dihydroisoquinoléine-2(1H)-carboxylate de tert-butyle

Le composé du titre est obtenu selon le procédé de la Préparation 22b" en remplaçant au Stade A le 2-(2-chloroéthoxy)-éthanol par le 6-chlorohexanol.

### Masse haute résolution (ESI+) :

Formule brute :

   C₃₃H₄₂N₄O₅
[M+H]+ calculé 575.32,
[M+H]+ mesuré 575.4.

### Préparation 24b": 5-({5-[(5-{[4-(prop-2-én-1-yloxy)phényl]amino}pyrimidin-2-yl)oxy]pentyl}oxy)-3,4-dihydroisoquinoléine-2(1H)-carboxylate de tert-butyle

Le composé du titre est obtenu selon le procédé de la Préparation 22b" en remplaçant au Stade A le 2-(2-chloroéthoxy)-éthanol par le 6-chloropentanol.

**RMN ¹H** (500 MHz, dmso-d6) δ ppm : 8.29 (s, 2 H), 7.79 (s, 1 H), 7.12 (t, 1 H), 6.93 (m, 2 H), 6.86 (m, 2 H), 6.79 (d, 1 H), 6.72 (d, 1 H), 6.03 (m, 1 H), 5.38/5.24 (m, 2 H), 4.49 (dt, 2 H), 4.45 (br., 2 H), 4.24 (t, 2 H), 3.98 (t, 2 H), 3.53 (t, 2 H), 2.61 (t, 2 H), 1.78 (m, 2 H), 1.78 (m, 2 H), 1.56 (m, 2 H), 1.41 (s, 3 H).

### Préparation 25b": 5-{4-[(5-{[4-(prop-2-én-1-yloxy)phényl]amino}pyrimidin-2 yl)oxy]butoxy}-3,4-dihydroisoquinoléine-2(1H)-carboxylate de tert-butyle

Le composé du titre est obtenu selon le procédé de la Préparation 22b" en remplaçant au Stade A le 2-(2-chloroéthoxy)-éthanol par le 6-chlorobutanol.

**RMN ¹H** (500 MHz, dmso-d6) δ ppm 8.29 (s, 2 H), 7.8 (s, 1 H), 7.12 (t, 1 H), 6.93 (d, 2 H), 6.86 (d, 2 H), 6.8 (d, 1 H), 6.72 (d, 1 H), 6.07-5.98 (m, 1 H), 5.4-5.21 (m, 2 H), 4.48 (dt, 2 H), 4.45 (br, 2 H), 4.28 (t, 2 H), 4.02 (t, 2 H), 3.53 (t, 2 H), 2.62 (t, 2 H), 1.87 (br, 4 H), 1.4 (s, 9 H).

### Préparation 26b": 3-[(2-{2-[(5-{[4-(prop-2-én-1-yloxy)phényl]amino}pyrimidin-2-yl)oxy]éthoxy}éthoxy)méthyl]pipéridine-1-carboxylate de tert-butyle

### Stade A : 3-({2-[2-(benzyloxy)éthoxy]éthoxy}méthyl)pipéridine-1-carboxylate de tert-butyle

A une solution de 3-(hydroxyméthyl)pipéridine-1-carboxylate de *tert*-butyle (7,54 g ; 35 mmol) dans du *N,N*-diméthylformamide à 0 °C, est additionné l'hydrure de sodium à 60 % dans l'huile (1,6 g ; 40,2 mmol). L'ensemble est ensuite agité pendant 60 minutes. Après retour à température ambiante, une solution de 2-(2-benzyloxyéthoxy)éthyl-4-méthylbenzènesulfonate (12,26 g, 35 mmol) dans du *N,N-*diméthylformamide (20 mL) est ajoutée. L'ensemble est ensuite agité pendant 60 minutes, puis hydrolysé lentement. Après extraction au dichlorométhane, les phases organiques sont évaporées et le résidu est purifié par chromatographie sur gel de silice en utilisant le dichlorométhane et le méthanol comme éluant pour obtenir le produit du titre.

**RMN ¹H** (500 MHz, dmso-d6) δ ppm 7.36-7.29 (m, 4 H), 7.27 (m, 1 H), 4.48 (s, 2 H), 4-3.77/2.7-2.36 (br, 2 H), 3.75/2.74 (br+td, 2 H), 3.59-3.44 (m, 8 H), 3.27/3.21 (dd, 2 H), 1.66/1.11 (br, 2H), 1.62 (br, 1 H), 1.55/1.29 (m, 2 H), 1.37 (s, 9 H).

### Stade B : 3-{[2-(2-hydroxyéthoxy)éthoxy]méthyl}pipéridine-1-carboxylate de tert-butyle

A une solution du composé obtenu au Stade A (11,0 g ; 27,95 mmol) dans l'éthanol (85 mL) est additionné du palladium sur charbon (10 % massique). L'ensemble est hydrogéné pendant 10 heures à température ambiante sous 4 bars. Le milieu réactionnel est filtré puis concentré à sec pour obtenir le produit du titre.

**RMN ¹H** (500 MHz, dmso-d6) δ ppm 4.45 (t, 1 H), 3.87/2.57 (brm+brs, 2 H), 3.74/2.78 (m+m, 2 H), 3.56-3.46 (m, 4 H), 3.49 (m, 2 H), 3.43 (t, 2 H), 3.28/3.23 (dd+dd, 2 H), 1.68/1.15 (m+m, 2 H), 1.64 (m, 1 H), 1.58/1.32 (m+m, 2 H), 1.39 (s, 9 H).

### Stade C : 3-[(2-{2-[(5-bromopyrimidin-2-yl)oxy]éthoxy}éthoxy)méthyl]pipéridine-1-carboxylate de tert-butyle

A une solution du composé obtenu au Stade B (8,34 g, 27,5 mmol) et de 5-bromo-2-chloropyrimidine (5,31 g; 27 mmol) dans l'acétonitrile (160 mL), est additionné le carbonate de césium (17,9 g, 55 mmol), puis l'ensemble est agité pendant 7 heures à 85 °C. Après refroidissement, le milieu réactionnel est filtré, puis le filtrat est concentré. Le résidu est purifié par chromatographie sur gel de silice en utilisant l'heptane et l'acétate d'éthyle comme éluants pour obtenir le produit du titre.

**RMN ¹H** (500 MHz, dmso-d6) δ ppm 8.75 (s, 2 H), 4.4 (m, 2 H), 4.04-2.27 (brm, 4 H), 3.75 (m, 2 H), 3.57 (t, 2 H), 3.49/3.47 (m+m, 2 H), 3.25/3.2 (dd+dd, 2 H), 1.64/1.1 (m+m, 2 H), 1.6 (m, 1 H), 1.55/1.29 (m+m, 2 H), 1.37 (s, 9 H).

### Stade D : 3-[(2-{2-[(5-{[4-(prop-2-én-1-yloxy)phényl]amino}pyrimidin-2-yl)oxy]éthoxy}éthoxy)méthyl]pipéridine-1-carboxylate de tert-butyle

Le composé du titre est obtenu selon le procédé décrit au Stade B de la Préparation 2b" en utilisant le composé du stade précédent et le composé obtenu au Stade B de la Préparation 15b".

**RMN ¹H** (500 MHz, dmso-d6) δ ppm 8.29 (s, 2 H), 7.81 (s, 1 H), 6.94 (m, 2 H), 6.86 (m, 2 H), 4.33 (m, 2 H), 4-3.73/2.71-2.34 (br+br., 2 H), 3.73/2.74 (br+m, 2 H), 3.73 (m, 2 H), 3.58 (t, 2 H), 3.49 (m, 2 H), 3.27/3.21 (dd+dd, 2 H), 1.66/1.1 (brd+br., 2 H), 1.62 (br., 1 H), 1.55/1.29 (m+m, 2 H), 1.37 (s, 9 H).

### Préparation 27b": 4-[(2-{2-[(5-{[4-(prop-2-én-1-yloxy)phényl]amino}pyrimidin-2-yl)oxy]éthoxy}éthoxy)méthyl]pipéridine-1-carboxylate de tert-butyle

Le composé du titre est obtenu selon le procédé de la Préparation 26b" en remplaçant au Stade A le 3-(hydroxyméthyl)pipéridine-1-carboxylate de *tert*-butyle par le 4-(hydroxyméthyl)pipéridine-1-carboxylate de *tert*-butyle.

**RMN ¹H** (500 MHz, dmso-d6) δ ppm 8.29 (s, 2 H), 7.82 (s, 1 H), 6.94 (m, 2 H), 6.86 (m, 2 H), 6.03 (m, 1 H), 5.38/5.24 (m+m, 2 H), 4.49 (m, 2 H), 4.32 (m, 2 H), 3.91/2.67 (br+br., 4 H), 3.73 (m, 2 H), 3.57 (m, 2 H), 3.49 (m, 2 H), 3.23 (d, 2 H), 1.67 (m, 1 H), 1.61/0.98 (brd+qd, 4H), 1.37 (s, 9 H).

### Préparation 28b": 5-[2-(méthyl{2-[(5-{[4-(prop-2-én-1-yloxy)phényl]amino}pyrimidin-2-yl)oxy]éthyl}amino)éthoxy]-3,4-dihydroisoquinoléine-2(1H)-carboxylate de tert-butyle

### Stade A : 2-{[2-(benzyloxy)éthyl](méthyl)amino}éthanol

A une solution de 2-chloroéthoxyméthylbenzène (4,6 mL ; 30 mmol) et de 2-(méthylamino)éthanol (3,1 mL ; 39 mmol) dans le *N*,*N*-diméthylacétamide (40 mL), est additionné l'iodure de potassium (1,0 g, 6 mmol), puis est ajoutée la triéthylamine (5,4 mL ; 39 mmol). L'ensemble est agité à 120 °C pendant toute la nuit. Après filtration du précipité, le filtrat est concentré à sec, puis purifié par chromatographie sur gel de silice en utilisant le dichlorométhane et le méthanol comme éluants pour obtenir le produit du titre.

**RMN ¹H** (500 MHz, dmso-d6) δ ppm 7.38-7.24 (m, 5 H), 4.46 (s, 2 H), 4.34 (brt, 1 H), 3.51 (t, 2 H), 3.45 (brt, 2 H), 2.58 (t, 2 H), 2.45 (t, 2 H), 2.21 (s, 3 H).

### Stade B : 5-(2-{[2-(benzyloxy)éthyl](méthyl)amino}léthoxy)-3,4-dihydroisoquinoléine-2(1H)-carboxylate de tert-butyle

A une solution du composé obtenu au Stade A (6,28 g, 30 mmol) et du composé obtenu à la Préparation 3b' (4,99 g, 20 mmol) dans le tétrahydrofurane (150 mL), sont aditionnées sous atmosphère d'azote la triphénylphosphine (7,87 g, 30 mmol) à 0 °C, puis goutte à goutte une solution d'azodicarboxylate de diéthyle à 40 % dans le toluène (14 mL, 30 mmol). Après retour à température ambiante, le mélange est agité pendant 1 heure. Après évaporation des solvants, le résidu est repris 2 fois dans le diisopropyléther, puis il est purifié par chromatographie sur RP-18 en utilisant l'acétonitrile, l'eau et le carbonate d'ammonium comme éluants pour obtenir le produit du titre.

**RMN ¹H** (500 MHz, dmso-d6) δ ppm 7.35-7.24 (m, 5 H), 7.11 (t, 1 H), 6.79 (dd, 1 H), 6.72 (dd, 1 H), 4.46 (s, 2 H), 4.45 (s, 2 H), 4.02 (t, 2 H), 3.53 (t, 2 H), 3.51 (t, 2 H), 2.7 (t, 2 H), 2.65 (t, 2 H), 2.6 (t, 2 H), 2.3 (s, 3 H), 1.41 (s, 9 H).

### Stade C : 5-{2-[(2-hydroxyéthyl)(méthyl)amino]éthoxy}-3,4-dihydroisoquinoléine-2(1H)-carboxylate de tert-butyle

A une solution du composé obtenu au Stade B (3,88 g ; 8,8 mmol) dans le tétrahydrofurane (50 mL) et l'éthanol (80 mL), sont additionnés l'acide chlorhydrique 4 M dans le dioxane (2,4 mL), puis le palladium sur charbon (570 mg). Le mélange est hydrogéné à température ambiante sous 4 bars pendant 15 heures. Après filtration et évaporation, le résidu est dissous dans l'eau, puis une solution aqueuse de soude 2 M est additionnée jusqu'à atteindre un pH=12. Après extraction à l'acétate d'éthyle et évaporation des solvants, on obtient le produit du titre.

**RMN ¹H** (400 MHz, dmso-d6) δ ppm 7.12 (t, 1 H), 6.8 (dd, 1 H), 6.73 (dd, 1 H), 4.45 (s, 2 H), 4.34 (t, 1 H), 4.03 (t, 2 H), 3.53 (t, 2 H), 3.47 (t, 2 H), 2.77 (t, 2 H), 2.62 (t, 2 H), 2.51 (t, 2 H), 2.28 (s, 3 H), 1.41 (s, 3 H)

### Stade D : 5-{2-[{2-[(5-bromopyrimidin-2-yl)oxy]éthyl}(méthyl)amino]éthoxy}-3,4-dihydroisoquinoléine-2(1H)-carboxylate de tert-butyle

Le composé du titre est obtenu selon le procédé décrit au Stade B de la Préparation 28b" en utilisant le composé du stade précédent et le 5-bromopyrimidin-2-ol.

**RMN ¹H** (500 MHz, dmso-d6) δ ppm 8.72 (s, 1 H), 7.11 (t, 1 H), 6.78 (d, 1 H), 6.72 (d, 1 H), 4.45 (s, 2 H), 4.38 (t, 2 H), 4.03 (t, 2 H), 3.49 (t, 2 H), 2.84 (t, 2 H), 2.83 (t, 2 H), 2.57 (t, 2 H), 2.35 (s, 3 H), 1.41 (s, 9 H)

### Stade E : 5-[2-(méthyl{2-[(5-{[4-(prop-2-én-1-yloxy)phényl]amino}pyrimidin-2-yl)oxy]éthyl}amino)éthoxy]-3,4-dihydroisoquinoléine-2(1H)-carboxylate de tert-butyle

Le composé du titre est obtenu selon le procédé décrit au Stade D de la Préparation 26b" en utilisant le composé du stade précédent.

**RMN ¹H** (500 MHz, dmso-d6) δ ppm 8.28 (s, 2 H), 7.8 (s, 1 H), 7.11 (t, 1 H), 6.93 (m, 2 H), 6.85 (m, 2 H), 6.8 (dd, 1 H), 6.72 (dd, 1 H), 6.03 (m, 1 H), 5.38/5.24 (dq, 2 H), 4.49 (dt, 2 H), 4.44 (brs, 2 H), 4.32 (t, 2 H), 4.04 (t, 2 H), 3.5 (t, 2 H), 2.84 (t, 2 H), 2.83 (t, 2 H), 2.59 (t, 2 H), 2.35 (s, 3 H), 1.4 (s, 9 H).

### Exemple 1 : 6-chloro-14-(4-hydroxyphényl)-10,11-diméthyl-20,23-dioxa-1,10,14-triazahexacyclo [26.3.1.1∼9,12∼.1∼15,19∼.0∼3,8∼.0∼24,29∼]tétratriaconta-3,5,7,9(34),11,15(33),16,18,24,26,28-undécaène-2,13-dione

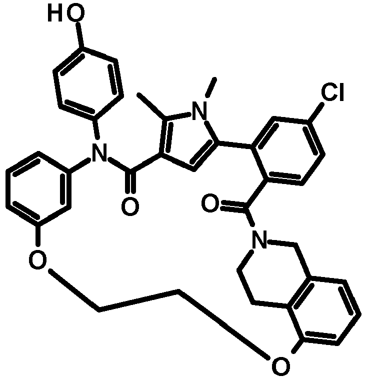

### Stade A : 5-(5-chloro-2-{[5-(prop-2-én-1-yloxy)-3,4-dihydroisoquinoléin-2(1H)-yl]carbonyl}phényl)-1,2-diméthyl-1H-pyrrole-3-carboxylate d'éthyle

A une solution du composé issu de la Préparation 1a (9,5 g ; 29,5 mmol) dans du dichlorométhane (110 mL), sont additionnés successivement le composé obtenu à la Préparation 1a' (7,32 g ; 32,4 mmol), le 1-hydroxybenzotriazole (4,78 g ; 35,4 mmol), le chlorhydrate du 1,(3-diméthylaminopropyl)-3-éthylcarbodiimide (6,1 g; 35,4 mmol) et la triéthylamine (20,6 mL ; 147,5 mmol). L'ensemble est ensuite agité pendant une nuit à température ambiante. Le milieu réactionnel est dilué avec un mélange de dichlorométhane et d'eau. Après décantation, la phase organique est lavée avec une solution aqueuse saturée en chlorure d'ammonium et une solution aqueuse saturée en chlorure de sodium, puis séchée sur sulfate de magnésium, filtrée et concentrée à sec. Le résidu est purifié par chromatographie sur gel de silice en utilisant l'éther de pétrole et l'acétate d'éthyle comme éluants pour obtenir le produit du titre.

**RMN ¹H** (400 MHz, dmso-d6, 300 K) δ ppm : 7.54/7.45 (2m, 3 H), 7.12/7 (2t, 1 H), 6.75/6.45 (2d, 1 H), 6.75/6.65 (2d, 1 H), 6.35/6.25 (2s, 1 H), 6.05 (m, 1 H), 5.4 (m, 1 H), 5.25 (m, 1 H), 4.55-3.95/2.95 (m, 8 H), 3.45/3.21 (2s, 3 H), 2.6-2.2 (m, 2 H), 2.49/2.05 (2s, 3 H), 1.22 (t, 3 H).

**IR :** v : >C=O : 1695 cm⁻¹.

### Stade B : acide 5-(5-chloro-2-{[5-(prop-2-én-1-yloxy)-3,4-dihydroisoquinoléin-2(1H)-yl]carbonyllphényl)-1,2-diméthyl-1H-pyrrole-3-carboxylique

A une solution du composé obtenu au Stade A (10,8 g; 21,9 mmol) dans le méthanol (100 mL), est additionnée la lithine (1,8 g ; 43,8 mmol) en solution dans 50 mL d'eau. L'ensemble est ensuite agité à 90 °C pendant 48 heures. Le milieu réactionnel est concentré pour éliminer le méthanol puis acidifié à pH=4 par ajout d'une solution aqueuse d'acide chlorhydrique 1 N et enfin extrait au dichlorométhane. La phase organique est séchée sur sulfate de magnésium, filtrée et concentrée à sec. Le produit du titre est obtenu sous forme d'une poudre et est utilisée au stade suivant sans purification.

**RMN** ¹**H** (400 MHz, dmso-d6, 300 K) δ ppm : 11.55 (br. s, 1 H), 7.52/7.45 (m, 3 H), 7.12/7 (2t, 1 H), 6.78/6.7/6.48 (3d, 2 H), 6.32/6.25 (2s, 1 H), 6.05 (m, 1 H), 5.4 (m, 1 H), 5.25 (m, 1 H), 4.8-3 (m, 6 H), 3.45/3.2 (2s, 3 H), 2.5 (m, 2 H), 2.5/2.05 (2s, 3 H).

### Stade C : N-(4-{[tert-butyl(diméthyl)silyl]oxy}phényl)-N-[3-(2-chloroéthoxy)phényl]-5-(5-chloro-2-{[5-(prop-2-én-1-yloxy)-3,4-dihydroisoquinoléin-2(1H)-yl]carbonyl}phényl)-1,2-diméthyl-1H-pyrrole-3-carboxamide

A une solution du composé obtenu au Stade B (1 g ; 2,15 mmol) dans le dichloroéthane (20 mL), est additionné le 1-chloro-*N**,**N*,2-triméthyl-prop-1-èn-1-amine (0,31 mL; 2,4 mmol). Le milieu réactionnel est agité à température ambiante pendant 2 heures, puis est ajouté le composé de la Préparation la" (1,6 g ; 4,3 mmol).L'ensemble est agité à 80 °C pendant 24 heures. Le milieu réactionnel est dilué dans un mélange de dichlorométhane et d'une solution aqueuse saturée en hydrogénocarbonate de sodium. Après extraction de la phase aqueuse au dichlorométhane, les phases organiques sont réunies, séchées sur sulfate de magnésium, filtrées et concentrées à sec. Le résidu est purifié par chromatographie sur gel de silice en utilisant le dichlorométhane et le méthanol comme éluants pour obtenir le produit du titre.

**RMN ¹H** (500 MHz, dmso-d6, 300 K) δ ppm : 7.5-7.25 (m, 3 H), 7.2 (m, 2 H), 6.95/6.65 (m, 4 H), 6.85-6.6 (m, 5 H), 6.05 (m, 1 H), 5.4/5.25 (m, 2 H), 5.35-5.2 (m, 1 H), 4.8-3.9 (m, 2 H), 4.5 (m, 2 H), 4.15 (m, 2 H), 3.85 (m, 2 H), 3.3-2.9 (m, 2 H), 3.3-3.2 (m, 3 H), 2.8-2.5 (m, 2 H), 2.35-2.2 (m, 3 H), 0.85 (m, 9 H), 0.1 (m, 6 H).

### Stade D : N-(4-{[tert-butyl(diméthyl)silyl]oxy}phényl)-N-[3-(2-chloroéthoxy)phényl]-5-{5-chloro-2-[(5-hydroxy-3,4-dihydroisoquinoléin-2(1H)-yl)carbonyl]phényl}-1,2-diméthyl-1H-pyrrole-3-carboxamide

A une solution du composé obtenu au Stade C (1,3 g ; 1,57 mmol) dans un mélange de dichlorométhane (6 mL) et de méthanol (3 mL), est additionné l'acide 1,3-diméthylbarbiturique (0,49 g; 3,15 mmol). Le milieu réactionnel est dégazé par bullage d'argon pendant 10 minutes et le tétrakis-(triphénylphosphine)palladium(0) (0,09 g ; 0,07 mmol) est ajouté. L'ensemble est chauffé à 40 °C pendant 19 heures. Après concentration du méthanol, le milieu réactionnel est dilué dans un mélange d'acétate d'éthyle et d'eau. Après décantation, la phase organique est lavée avec une solution aqueuse saturée en hydrogénocarbonate de sodium et une solution aqueuse saturée en chlorure de sodium, puis séchée sur sulfate de magnésium, filtrée et concentrée à sec. Le résidu est purifié par chromatographie sur gel de silice en utilisant le dichlorométhane et le méthanol comme éluants pour obtenir le produit du titre.

**RMN ¹H** (500 MHz, dmso-d6, 300 K) δ ppm : 9.45 (m, 1 H), 7.5-7.25 (m, 3 H), 7.2 (m, 1 H), 7-6.95 (m, 1 H), 6.8-6.6 (m, 2 H), 6.8-6.6 (m, 3 H), 6.75-6.6 (m, 4 H), 5.4-5.2 (m, 1 H), 4.8-4.1 (m, 2 H), 4.15 (m, 2 H), 3.9 (m, 2 H), 3.85 (m, 2 H), 3.3-3.2 (m, 3 H), 2.9-2.5 (m, 2 H), 2.35-2.2 (m, 3 H), 0.85 (m, 9 H), 0.1 (m, 6 H).

**IR :** v : -OH : 3260 cm⁻¹ ; >C=O : 1624 cm⁻¹.

### Stade E : N-(4-{[tert-butyl(diméthyl)silyl]oxy}phényl)-5-[5-chloro-2-[(5-hydroxy-3,4-dihydroisoquinoléin-2(1H)-yl)carbonyl]phényl}-N-[3-(2-iodoéthoxy)phényl]-1,2-diméthyl-1H-pyrrole-3-carboxamide

A une solution du composé obtenu au Stade D (0,88 g ; 1,17 mmol) dans l'acétone (15 mL), est additionné l'iodure de sodium (0,35 g ; 2,34 mmol). Le milieu réactionnel est agité à 80 °C pendant 24 heures. Après filtration de celui-ci, le filtrat est concentré. Le résidu obtenu est repris dans un mélange d'acétate d'éthyle et d'eau. Après extraction de la phase aqueuse à l'acétate d'éthyle, les phases organiques sont réunies, séchées sur sulfate de magnésium, filtrées et concentrées à sec. Le résidu obtenu est directement engagé dans l'étape suivante sans purification.

### Stade F : 14-(4-{[tert-butyl(diméthyl)silyl]oxy}phényl)-6-chloro-10,11-diméthyl-20,23-dioxa-1,10,14-triazahexacyclo[26.3.1.1∼9,12∼.1∼15,19∼.0-3,8∼.0∼24,29∼]tétratriaconta-3,5,7,9(34),11,15(33),16,18,24,26,28-undécaène-2,13-dione

A une solution du composé obtenu au Stade E (0,98 g; 1,12 mmol) dans l'acétonitrile (112 mL), est additionné en trois portions à une heure d'intervalle et à température ambiante le carbonate de césium (0,36 g ; 1,12 mmol). Le milieu réactionnel est ensuite chauffé à 50 °C pendant 4 heures puis concentré. Le résidu obtenu est repris dans un mélange de dichlorométhane et d'eau. Après décantation, la phase organique est lavée avec de l'eau et une solution aqueuse saturée en chlorure de sodium, puis séchée sur sulfate de magnésium, filtrée et concentrée. Le résidu obtenu est directement engagé dans l'étape suivante sans purification.

### Stade G : 6-chloro-14-(4-hydroxyphényl)-10,11-diméthyl-20,23-dioxa-1,10,14-triazahexacyclo[26.3.1.1∼9,12∼.1∼15,19~.0~3,8∼.0∼24,29~]tétratriaconta-3,5,7,9(34),11,15(33),16,18,24,26,28-undécaène-2,13-dione

A une solution du composé obtenu au Stade F (0,5 g ; 0,67 mmol) dans le tétrahydrofurane (10 mL), est additionnée une solution de fluorure de tétrabutylammonium dans le tétrahydrofurane 1 N (1 mL ; 1,01 mmol). L'ensemble est agité à température ambiante pendant 1 heure. Le milieu réactionnel est ensuite dilué dans un mélange de dichlorométhane et d'eau. Après décantation, la phase organique est lavée avec de l'eau, et une solution aqueuse saturée en chlorure de sodium, puis séchée sur sulfate de magnésium, filtrée et concentrée. Le résidu ainsi obtenu est purifié par chromatographie sur gel de silice en utilisant le dichlorométhane et le méthanol ammoniacal comme éluants, suivie d'une chromatographie sur colonne chirale IC en utilisant l'acétonitrile, l'isopropanol et la diéthylamine. Le solide ainsi obtenu est dissous dans un mélange eau / acétonitrile, filtré puis lyophilisé.

### Masse haute résolution (ESI+) :

Formule brute :

   C₃₇H₃₂ClN₃O₅
[M+H]+ calculé 634,2103,
[M+H]+ mesuré 634,2102.

### Exemple 2 : 6-chloro-14-(4-hydroxyphényl)-10,11-diméthyl-20,24-dioxa-1,10,14-triazahexacyclo[27.3.1.1-9,12-.1-15,19-.0-3,8-.0-25,30-]pentatriaconta-3,5,7,9(35),11,15(34),16,18,25,27,29-undécaène-2,13-dione

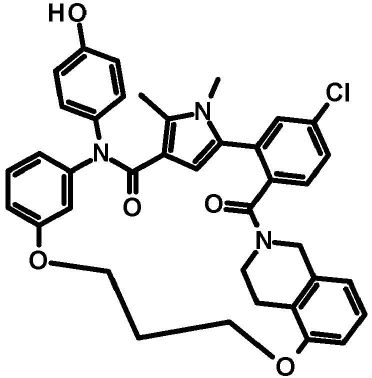

Le composé du titre est obtenu selon le procédé de l'Exemple 1 en utilisant l'acide de la Préparation la et le composé de la Préparation 1a' au Stade A, ainsi que le composé de la Préparation 2a" au Stade C.

### Microanalyse élémentaire : (% théorique : mesuré)

%C = 70.42 : 69.92 ; %H = 5.29 : 5.00 ; %N = 6.48 : 6.48.

### Masse haute résolution (ESI+) :

Formule brute :

   C₃₈H₃₄ClN₃O₅
[M+H]+ calculé 648,2259,
[M+H]+ mesuré 648,2260.

### Exemple 3 : 6-chloro-14-(4-hydroxyphényl)-10,11-diméthyl-20,25-dioxa-1,10,14-triazahexacyclo [28.3.1.1∼9,12∼.1~15,19∼.0~3,8~.0~26,31~]hexatriaconta-3,5,7,9(36),11,15(35),16,18,26,28,30-undécaène-2,13-dione

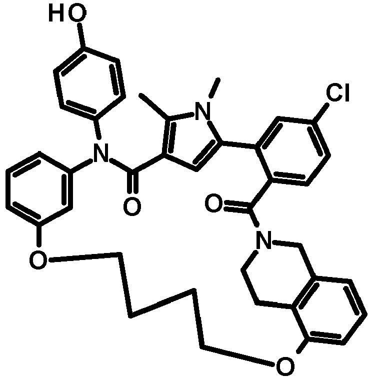

Le composé du titre est obtenu selon le procédé de l'Exemple 1 en utilisant l'acide de la Préparation 1a et le composé de la Préparation 1a' au Stade A, ainsi que le composé de la Préparation 3a" au Stade C.

### Microanalyse élémentaire : (% théorique : mesuré)

%C = 70.74 : 70.22 ; %H = 5.48 : 5.37 ; %N = 6.35 : 6.27.

### Masse haute résolution (ESI+) :

Formule brute :

   C₃₉H₃₆ClN₃O₅
[M+H]+ calculé 662,2416,
[M+H]+ mesuré 662,2422.

### Exemple 4 : 6-chloro-14-(4-hydroxyphényl)-10,11-diméthyl-2,13-dioxo-20,23-dioxa-1,10,14-triazahexacyclo [26.3.1.1~9,12~.1~15,19~.0~3,8~.0~24,29~]tétratriaconta-3,5,7,9(34),11,15(33),16,18,24,26,28-undécaène-17-carbonitrile

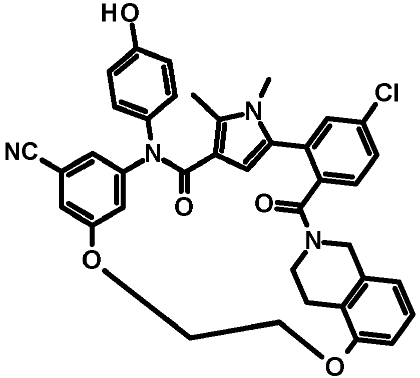

Le composé du titre est obtenu selon le procédé de l'Exemple 1 en utilisant l'acide de la Préparation 1a et le composé de la Préparation 1a' au Stade A, ainsi que le composé de la Préparation 4a" au Stade C.

### Microanalyse élémentaire : (% théorique : mesuré)

%C = 69.24 : 68.29 ; %H = 4.74 : 4.68 ; %N = 8.50 : 8.70 ; %Cl = 5.38 : 5.33.

### Masse haute résolution (ESI+) :

Formule brute :

   C₃₈H₃₁ClN₄O₅
[M+H]+ calculé 659,2057,
[M+H]+ mesuré 659,2056.

### Exemple 5 : 6-chloro-14-(4-hydroxyphényl)-10,11-diméthyl-2,13-dioxo-20,24-dioxa-1,10,14-triazahexacyclo[27.3.1.1~9,12~.1~15,19~.0~3,8~.0~25,30~]pentatriaconta-3,5,7,9(35),11,15(34),16,18,25,27,29-undécaène-17-carbonitrile

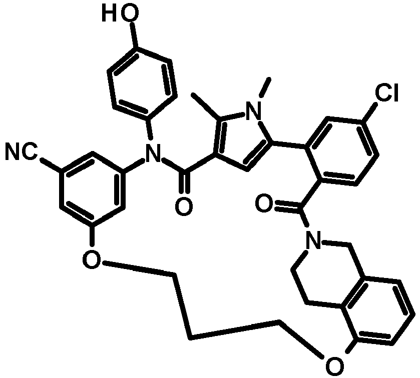

Le composé du titre est obtenu selon le procédé de l'Exemple 1 en utilisant l'acide de la Préparation 1a et le composé de la Préparation 1a' au Stade A, ainsi que le composé de la Préparation 5a" au Stade C.

### Masse haute résolution (ESI+) :

Formule brute :

   C₃₉H₃₃ClN₄O₅
[M+H]+ calculé 673,2212,
[M+H]+ mesuré 673,2211.

### Exemple 6 : 11-chloro-4-(4-hydroxyphényl)-1,7,8-triméthyl-4,16,17,23,24,25-hexahydro-1H,14H-15,18-méthano-6,9-(méthéno)dibenzo[b,h] pyrazolo[4,3-p][1,6,11,15]oxatriazacycloicosine-5,14(8H)-dione

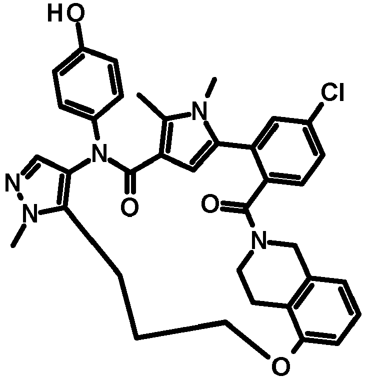

### Stade A : N-(4-{[tert-butyl(diméthyl)silyl]oxy}phényl)-5-(5-chloro-2-{[5-(prop-2-én-1-yloxy)-3,4-dihydroisoquinoléin-2(1H)-yl]carbonyl}phényl)-N-[5-(3-chloropropyl)-1-méthyl-1H-pyrazol-4-yl)-1,2-diméthyl-1H-pyrrole-3-carboxamide

A une solution du composé obtenu au Stade B de l'Exemple 1 (0,75 g ; 1,6 mmol) dans le dichloroéthane (40 mL), est additionnée le chlorure d'oxalyle (0,28 mL ; 3,2 mmol). Le milieu réactionnel est agité à température ambiante pendant 1 heure puis concentré. Le résidu est repris dans le dichloroéthane puis concentré, cette opération étant effectuée à deux reprises. Le résidu final est repris dans le dichloroéthane (20 mL), puis est ajouté à une solution de composé de la Préparation 6a" (0,61 g ; 1,6 mmol) et de pyridine (0,4 mL ; 4,8 mmol) dans le dichloroéthane (10 mL).L'ensemble est agité à 110 °C pendant 16 heures. Le milieu réactionnel est concentré, puis le résidu est repris dans un mélange de dichlorométhane et d'une solution aqueuse saturée en hydrogénocarbonate de sodium. Après extraction de la phase aqueuse au dichlorométhane, les phases organiques sont réunies, séchées sur sulfate de magnésium, filtrées et concentrées à sec. Le résidu est purifié par chromatographie sur gel de silice en utilisant le dichlorométhane et l'acétate d'éthyle comme éluants pour obtenir le produit du titre.

**RMN ¹H** (400 MHz, dmso-d6, 300 K) δ ppm : 7.5-7.25 (m, 3 H), 7.2-7.1 (m, 1 H), 7.15 (m, 1 H), 7.15-6.93 (m, 2 H), 6.82-6.7 (m, 2 H), 6.8 (m, 1 H), 6.62-6.5 (m, 1 H), 6.08 (m, 1 H), 5.5-5.2 (m, 2 H), 5.38-5.2 (m, 1 H), 4.75-2.9 (m, 2 H), 4.55 (m, 2 H), 4.25-3.9 (m, 2 H), 3.73 (m, 3 H), 3.55 (m, 2 H), 3.3-3.15 (m, 3 H), 2.7-2.3 (m, 2 H), 2.6 (m, 2 H), 2.3-2.15 (m, 3 H), 16 (m, 2 H), 0.8 (m, 9 H), 0.1 (m, 6 H).

### Stade B : 11-chloro-4-(4-hydroxyphényl)-1,7,8-triméthyl-4,16,17,23,24,25-hexahydro-1H,14H-15,18-méthano-6,9-(méthéno)dibenzo[b,h]pyrazolo[4,3-p][1,6,11,15] oxatriazacycloicosine-5,14(8H)-dione

Le composé du titre est obtenu selon les procédés décrits aux Stades D, E, F et G de l'Exemple 1, en utilisant le composé du stade précédent comme produit de départ.

### Microanalyse élémentaire : (% théorique : mesuré)

%C = 67.97 : 68.05 ; %H = 5.39 : 5.25 ; %N = 11.01 : 10.77.

### Masse haute résolution (ESI+) :

Formule brute :

   C₃₆H₃₄ClN₅O₄
[M+H]+ calculé 636,2372,
[M+H]+ mesuré 636,2369.

### Exemple 7 : 6-chloro-14-(4-hydroxyphényl)-10,11,33-triméthyl-2,13-dioxo-23-oxa-1,10,14,18-tétraazahexacyclo[26.3.1.1~9,12~.1~15,18~.0~3,8~.0~24,29~]tétratriaconta-3,5,7,9(34),11,15(33),16,24,26,28-décaène-17-carbonitrile

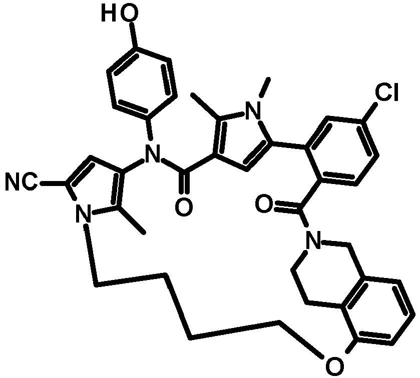

Le composé du titre est obtenu selon le procédé de l'Exemple 6 en utilisant l'acide obtenu au Stade B de l'Exemple 1 et l'amine de la Préparation 7a".

### Microanalyse élémentaire : (% théorique : mesuré)

%C = 69.48 : 69.01 ; %H = 5.38 : 5.34 ; %N = 10.39 : 10.19.

### Masse haute résolution (ESI+) :

Formule brute :

   C₃₉H₃₆ClN₅O₄
[M+H]+ calculé 674,2529,
[M+H]+ mesuré 674,2515.

### Exemple 8 : 6-chloro-14-(4-hydroxyphényl)-10,11,32-triméthyl-2,13-dioxo-22-oxa-1,10,14,18-tétraazahexacyclo[25.3.1.1~9,12~.1~15,18~.0~3,8~.0~23,28~]tritriaconta-3,5,7,9(33),11,15(32),16,23,25,27-décaène-17-carbonitrile

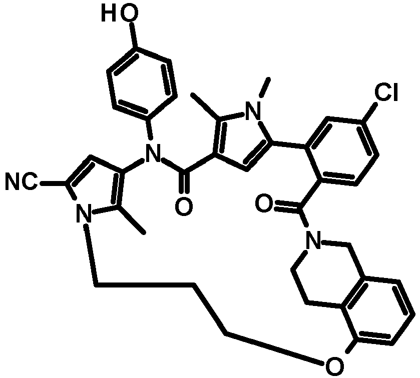

Le composé du titre est obtenu selon le procédé de l'Exemple 6 en utilisant l'acide obtenu au Stade B de l'Exemple let l'amine de la Préparation 8a".

### Masse haute résolution (ESI+) :

Formule brute :

   C₃₈H₃₄ClN₅O₄
[M+H]+ calculé 660.2372,
[M+H]+ mesuré 660.2377.

### Exemple 9 : 6-chloro-14-(4-hydroxyphényl)-10,11-diméthyl-2,13-dioxo-23-oxa-1,10,14-triazahexacyclo[26.3.1.1~9,12~.1~15,19~.0~3,8~.0~24,29~]tétratriaconta-3,5,7,9(34),11,15(33),16,18,24,26,28-undécaène-17-carbonitrile

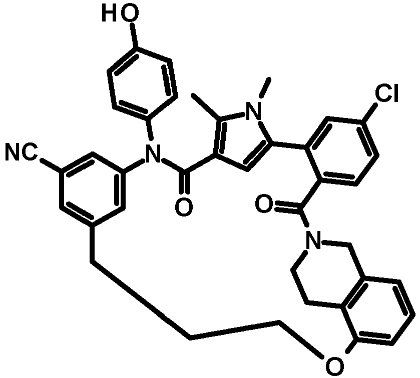

Le composé du titre est obtenu selon le procédé de l'Exemple 6 en utilisant l'acide obtenu au Stade B de l'Exemple 1 et l'amine de la Préparation 9a".

### Masse haute résolution (ESI+) :

Formule brute :

   C₃₉H₃₃ClN₄O₄
[M+H]+ calculé 657.2263,
[M+H]+ mesuré 657.2267.

### Exemple 10 : Chlorhydrate de 11-chloro-4-(4-hydroxyphényl)-1,7,8-triméthyl-1,4,16,17,23,24-hexahydro-14H-15,18-méthano-6,9-(méthéno)dibenzo[l,r]pyrazolo[3,4-d][1,6,10,15]oxatriazacyclononadécin-5(8H)-one

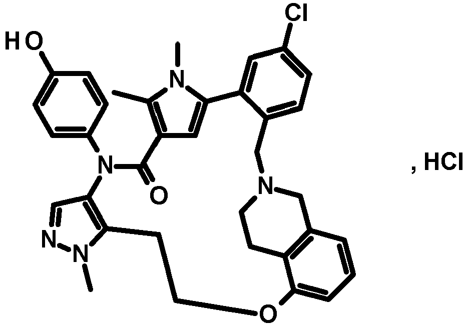

### Stade A : 5-(5-chloro-2-{[5-(prop-2-én-1-yloxy)-3,4-dihydroisoquinoléin-2(1H)-yllméthyllphényl)-1,2-diméthyl-1H-pyrrole-3-carboxylate d'éthyle

A une solution du composé issu du Stade B de la Préparation 1a (2 g ; 6,54 mmol) dans du dichlorométhane (25 mL), sont additionnés successivement le composé obtenu à la Préparation 1a' (1,5 g; 6,54 mmol) et le triacétoxyborohydrure de sodium (2,2 g ; 10 mmol). L'ensemble est ensuite agité pendant une nuit à température ambiante. Le milieu réactionnel est versé sur un mélange d'eau et de chlorure d'ammonium. Après décantation, la phase organique est séchée sur sulfate de sodium, filtrée et concentrée à sec. Le résidu est purifié par chromatographie sur gel de silice en utilisant l'heptane et l'éthanol ammoniacal comme éluants pour obtenir le produit du titre.

**RMN ¹H** (400 MHz, dmso-d6, 300 K) δ ppm : 7.6 (d, 1 H), 7.47 (dd, 1 H), 7.3 (d, 1 H), 7.03 (t, 1 H), 6.74 (d, 1 H), 6.57 (d, 1 H), 6.43 (s, 1 H), 6.03 (m, 1 H), 5.38 (m, 1 H), 5.23 (m, 1 H), 4.54 (m, 2 H), 4.16 (quad, 2 H), 3.42 (s, 2 H), 3.4 (s, 2 H), 3.22 (s, 3 H), 2.64 (m, 2 H), 2.56 (m, 2 H), 2.49 (s, 3 H), 1.25 (t, 3 H).

**IR :** v : >C=O : 1692 cm⁻¹ ; >C-O-C< : 1063 cm⁻¹

### Stade B : acide 5-(5-chloro-2-{[5-(prop-2-én-1-yloxy)-3,4-dihydroisoquinoléin-2(1H)-yl]méthyl}phényl)-1,2-diméthyl-1H-pyrrole-3-carboxylique

A une solution du composé obtenu au Stade A (2,1 g ; 4,4 mmol) dans le dioxane (12 mL), est additionnée une solution aqueuse de lithine 1 N (13 mL ; 13,1 mmol). L'ensemble est agité sous micro-onde (300 W) à 120 °C pendant 4,5 heures. Le milieu réactionnel est versé sur un mélange d'une solution aqueuse d'acide chlorhydrique 1 N et de glace. Le produit est extrait au dichlorométhane. Les phases organiques sont réunies, séchées sur sulfate de sodium, filtrées et concentrées pour fournir sans autre purification le produit du titre.

**RMN ¹H** (400 MHz, dmso-d6, 300 K) δ ppm : 7.61 (d, 1 H), 7.45 (dd, 1 H), 7.27 (br. s, 1 H), 7.03 (t, 1 H), 6.73 (d, 1 H), 6.57 (d, 1 H), 6.33 (s, 1 H), 6.04 (m, 1 H), 5.39 (d, 1 H), 5.23 (d, 1 H), 4.53 (m, 2 H), 3.45 (s, 2 H), 3.39 (s, 2 H), 3.2 (s, 3 H), 2.63 (m, 2 H), 2.55 (m, 2 H), 2.5 (s, 3 H).

**IR :** v : -OH : 3300-2200 cm⁻¹ ; >C=O : 1661 cm⁻¹ ; >C-O-C< : 1258 cm⁻¹.

### Stade C : N-(4-{[tert-butyl(diméthyl)silyl]oxy}phényl)-5-(5-chloro-2-{[5-(prop-2-én-1-yloxy)-3,4-dihydroisoquinoléin-2(1H)-yl]méthyl}phényl)-1,2-diméthyl-N-{1-méthyl-5-[2-(tétrahydro-2H-pyran-2-yloxy)éthyl]-1H-pyrazol-4-yl}-1H-pyrrole-3-carboxamide

A une solution du composé obtenu au Stade B (2 g ; 4,43 mmol) dans le dichloroéthane (50 mL), est additionné le 1-chloro-*N,N*,2-triméthyl-prop-1-èn-1-amine (0,59 mL ; 4,87 mmol). Le milieu réactionnel est agité à température ambiante pendant 2 heures, puis on ajoute le composé de la Préparation 10a" (3,2 g ; 7,4 mmol) et la pyridine (1,8 mL ; 22,1 mmol).L'ensemble est agité à 80 °C pendant 24 heures. Le milieu réactionnel est versé sur un mélange d'une solution aqueuse saturée en hydrogénocarbonate de sodium et de glace. Après extraction de la phase aqueuse au dichlorométhane, les phases organiques sont réunies, séchées sur sulfate de sodium, filtrées et concentrées à sec. Le résidu est purifié par chromatographie sur gel de silice en utilisant le dichlorométhane et l'isopropanol comme éluants pour obtenir le produit du titre.

**RMN ¹H** (500 MHz, dmso-d6, 300 K) δ ppm : 7.45 (d, 1 H), 7.3 (dd, 1 H), 7.15 (s, 1 H), 7.05 (d, 1 H), 7.02 (d, 1 H), 6.97 (t, 1 H), 6.9/6.6 (ab, 4 H), 6.5 (d, 1 H), 5.95 (m, 1 H), 5.3 (s, 1 H), 5.3/5.15 (m, 2 H), 4.48 (m, 2 H), 4.3 (m, 1 H), 3.65 (s, 3 H), 3.5-3.25 (m, 6 H), 3.2-3.1 (m, 4 H), 3.08 (s, 3 H), 2.8-2.5 (m, 2 H), 2.5 (m, 2 H), 2.3 (s, 3 H), 1.6-1.2 (m, 6 H), 0.87-0.8 (s, 9 H), 0.1-0 (s, 6 H).

### Stade D : N-(4-{[tert-butyl(diméthyl)silyl]oxy}phényl)-5-{5-chloro-2-[(5-hydroxy-3,4-dihydroisoquinoléin-2(1H)-yl)méthyl]phényl}-N-[5-(2-hydroxyéthyl)-1-méthyl-1H-pyrazol-4-yl]-1,2-diméthyl-1H-pyrrole-3-carboxamide

Le composé du titre est obtenu en deux étapes. Le composé du Stade C est d'abord soumis à une réaction de déprotection selon le procédé décrit au Stade D de l'Exemple 1 et est engagé dans l'étape suivante.

A une solution du produit ainsi obtenu (430 mg ; 0,52 mmol) dans le méthanol (15 mL), est additionné le *para*-toluènesulfonate de pyridine (140 mg ; 0,52 mmol). Le milieu réactionnel est agité à 80°C pendant une nuit, puis concentré à sec. Le résidu est purifié par chromatographie sur gel de silice en utilisant le dichlorométhane et le méthanol comme éluants pour obtenir le produit du titre.

**RMN ¹H** (500 MHz, dmso-d6, 300 K) δ ppm : 9.2 (s, 1 H), 4.62 (s, 1 H), 7.44 (d, 1 H), 7.3 (d, 1 H), 7.15 (s, 1 H), 7.02 (s, 1 H), 6.94-6.6 (m, 4 H), 6.8 (t, 1 H), 6.5 (d, 1 H), 6.35 (d, 1 H), 5.7-5.3 (s, 1 H), 3.65 (s, 3 H), 3.3 (m, 2 H), 3.2 (m, 2 H), 3.1 (m, 2 H), 3.05 (s, 3 H), 2.6 (m, 2 H), 2.5 (m, 2 H), 2.48 (m, 2 H), 2.3 (s, 3 H), 0.8 (s, 9 H), 0.15 (s, 6 H).

### Stade E : Chlorhydrate de 11-chloro-4-(4-hydroxyphényl)-1,7,8-triméthyl-1,4,16,17,23,24-hexahydro-14H-15,18-méthano-6,9-(méthéno)dibenzo[l,r]pyrazolo[3,4-d][1,6,10,15] oxatriazacyclononadécin-5(8H)-one

Une solution du composé obtenu au Stade D (250 mg ; 0,34 mmol) dans un mélange de tétrahydrofurane (80 mL) et de toluène (80 mL) est dégazée à l'argon pendant 10 minutes. Y est additionné le cyanométhylène tri-*n*-butylphosphorane (0,18 mL ; 0,75 mmol), puis l'ensemble est scellé et agité à 110 °C pendant 48 heures. Le milieu réactionnel est concentré puis dilué dans un mélange d'acétate d'éthyle et d'eau. Après décantation, la phase organique est lavée avec de l'eau et une solution aqueuse saturée en hydrogénocarbonate de sodium, séchée sur sulfate de magnésium, filtrée et concentrée à sec. Le résidu est purifié par chromatographie sur gel de silice en utilisant le dichlorométhane ammonical comme éluant puis est engagé dans l'étape suivante.

A une solution du produit obtenu dans le méthanol (5 mL), est additionnée une solution de potasse dans le méthanol 1 M (0,34 mL ; 0,34 mmol). Le milieu réactionnel est agité à température ambiante pendant 2 heures, puis est versé sur un mélange d'une solution aqueuse saturée en hydrogénocarbonate de sodium et de glace. Après extraction de la phase aqueuse au dichlorométhane, les phases organiques sont réunies, séchées sur sulfate de sodium, filtrées et concentrées à sec. Le résidu est purifié par chromatographie sur gel de silice en utilisant le dichlorométhane et l'éthanol ammoniacal comme éluants. Le solide ainsi obtenu est dissous dans un mélange d'une solution aqueuse d'acide chlorhydrique 1 N et d'acétonitrile, filtré puis lyophilisé pour obtenir le produit du titre.

### Masse haute résolution (ESI+) :

Formule brute :

   C₃₅H₃₄ClN₅O₃ . HCl
[M+H]+ calculé 608.2423,
[M+H]+ mesuré 608.2425.

### Exemple 11: 11-chloro-4-(4-hydroxyphényl)-1,7,8-triméthyl-4,16,17,23,24,25-hexahydro-1H,14H-15,18-méthano-6,9-(méthéno)dibenzo[b,h]pyrazolo[4,3-p] [1,6,11,15]oxatriazacycloicosin-5(8H)-one

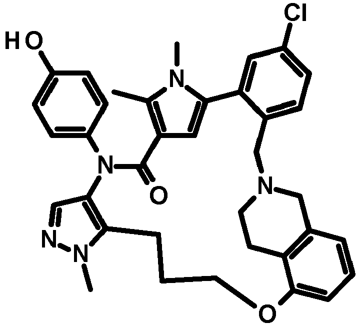

### Stade A : N-(4-{[tert-butyl(diméthyl)silyl]oxy}phényl)-5-(5-chloro-2-{[5-(prop-2-én-1-yloxy)-3,4-dihydroisoquinoléin-2(1H)-yl]méthyl}phényl)-N-[5-(3-chloropropyl)-1-méthyl-1H-pyrazol-4-yl]-1,2-diméthyl-1H-pyrrole-3-carboxamide

Le composé du titre est obtenu selon le procédé décrit au stade A de l'Exemple 6, en utilisant l'acide du Stade B de l'Exemple 10 et l'amine de la Préparation 6a".

**RMN ¹H** (500 MHz, dmso-d6, 300 K) δ ppm : 7.53 (d, 1 H), 7.41 (dd, 1 H), 7.28 (s, 1 H), 7.1 (d, 1 H), 7.05 (t, 1 H), 6.99 (d, 2 H), 6.75 (d, 1 H), 6.69 (d, 2 H), 6.59 (d, 1 H), 6.04 (m, 1 H), 5.4/5.24 (m+m, 1+1 H), 5.4 (s, 1 H), 4.55 (d, 2 H), 3.7 (s, 3 H), 3.59 (t, 2 H), 3.34 (s, 2 H), 3.22 (br. s, 2 H), 3.15 (s, 3 H), 2.63 (m, 2 H), 2.62 (m, 2 H), 2.5 (m, 2 H), 2.38 (s, 3 H), 1.73 (m, 2 H), 0.87 (s, 9 H), 0.08 (s, 6 H).

### Stade B : N-(4-{[tert-butyl(diméthyl)silyl]oxy}phényl)-5-{5-chloro-2-[(5-hydroxy-3,4-dihydroisoquinoléin-2(1H)-yl)méthyl]phényl}-N-[5-(3-chloropropyl)-1-méthyl-1H-pyrazol-4-yl]-1,2-diméthyl-1H-pyrrole-3-carboxamide

Le composé du titre est obtenu selon le procédé décrit au Stade D de l'Exemple 1 à partir du composé obtenu au stade précédent.

**RMN ¹H** (500 MHz, dmso-d6, 300 K) δ ppm 7.55 (d, 1 H), 7.4 (dd, 1 H), 7.3 (s, 1 H), 7.1 (d, 1 H), 7 (d, 2 H), 6.9 (t, 1 H), 6.7 (d, 2 H), 6.6 (d, 1 H), 6.4 (d, 1 H), 5.4 (s, 1 H), 3.7 (s, 3 H), 3.6 (t, 2 H), 3.3 (s, 2 H), 3.2 (br. s, 2 H), 3.15 (s, 3 H), 2.6 (m, 2 H), 2.55 (m, 2 H), 2.5 (m, 2 H), 2.35 (s, 3 H), 1.7 (quint, 2 H), 0.85 (s, 9 H), 0.1 (s, 6 H).

**IR :** v : -OH : 3500-2500 cm⁻¹ ; >C=O : 1635 et 1622 cm⁻¹.

### Stade C : N-(4-{[tert-butyl(diméthyl)silyl]oxy}phényl)-5-{5-chloro-2-[(5-hydroxy-3,4-dihydroisoquinoléin-2(1H)-yl)méthyl]phényl}-N-[5-(3-iodopropyl)-1-méthyl-1H-pyrazol-4-yl]-1,2-diméthyl-1H-pyrrole-3-carboxamide

Le composé du titre est obtenu selon le procédé décrit au Stade E de l'Exemple 1 à partir du composé obtenu au stade précédent. Le résidu obtenu est directement engagé dans l'étape suivante sans purification.

### Stade D : 11-chloro-4-(4-hydroxyphényl)-1,7,8-triméthyl-4,16,17,23,24,25-hexahydro-1H,14H-15,18-méthano-6,9-(méthéno)dibenzo[b,h]pyrazolo[4,3-p][1,6,11,15]oxatriazacycloicosin-5(8H)-one

Le composé du titre est obtenu selon les procédés décrits aux Stades F et G de l'Exemple 1 à partir du composé obtenu au stade précédent.

### Masse haute résolution (ESI+) :

Formule brute :

   C₃₆H₃₆ClN₅O₃
[M+H]+ calculé 622.2579,
[M+H]+ mesuré 622.2573.

### Exemple 12 : Phosphate disodique du 4-[6-chloro-17-cyano-10,11-diméthyl-2,13-dioxo-20,23-dioxa-1,10,14-triazahexacyclo[26.3.1.1~9,12~.1~15,19~.0~3,8~.0~24,29~]tétratriaconta-3,5,7,9(34),11,15(33),16,18,24,26,28-undécaén-14-yl]phényle

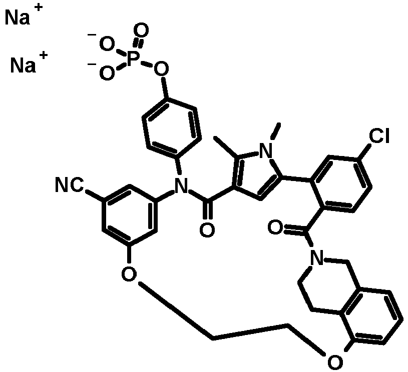

A une solution du composé de l'Exemple 4 (75 mg ; 0,113 mmol) dans le dichlorométhane (1,5 mL) sont additionnés le 1,8-diazabicyclo[5.4.0]undéc-7-ène (34 µL ; 0,227 mmol), puis le chlorure de bis(diméthylamino)phosphoryle (16,5 µL ; 0,113 mmol). Le milieu réactionnel est agité à température ambiante pendant 16 heures, puis est dilué dans un mélange d'acétate d'éthyle et d'une solution aqueuse saturée en hydrogénocarbonate de sodium. Après extraction au dichlorométhane, les phases organiques sont réunies et séchées sur sulfate de magnésium, filtrées et concentrées à sec.

A une solution du produit obtenu dans l'acétonitrile (2 mL), est additionnée une solution d'acide trifluoroacétique (1 mL) dans l'eau (0,5 mL). Le milieu réactionnel est agité à température ambiante pendant 24 heures puis concentré à sec. Le réside obtenu est repris dans l'acétonitrile, puis une solution aqueuse saturée en bicarbonate de sodium est ajoutée goutte à goutte jusqu'à pH=7. Le milieu est concentré à sec, puis repris dans l'éthanol et filtré. Le filtrat est concentré, puis le résidu obtenu est purifié par chromatographie sur phase Oasis^{®} en utilisant l'acétonitrile et l'eau comme éluants avant d'être lyophilisé pour obtenir le produit du titre.

### Masse haute résolution (ESI+) :

Formule brute :

   C₃₈H_{3O}ClN₄O₈P . 2 Na
[M+H]+ calculé 783.1358,
[M+H]+ mesuré 783.1361.

### Exemple 13 : 12-chloro-5-(4-hydroxyphényl)-8,9-diméthyl-6,15-dioxo-6,9,22,23-tétrahydro-5H,15H,17H-16,22-méthano-7,10-(méthéno)tribenzo[b,j,o] [1,4,8,13]oxatriazacyclooctadécine-3-carbonitrile

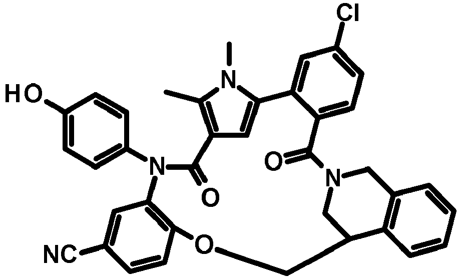

### Stade A : 5-(2-{[4-({[tert-butyl(diméthyl)silyl]oxy}méthyl)-3,4-dihydroisoquinoléin-2(1H)-yl]carbonyl}-5-chlorophényl)-1,2-diméthyl-1H-pyrrole-3-carboxylate d'éthyle

A une solution de la Préparation 2a' (2,95 g ; 18,1 mmol) dans le dichlorométhane (30 mL), sont additionnés l'imidazole (3,08 g ; 45,25 mmol), la 4-(diméthyl)-amino-pyridine (0,11 g ; 0,9 mmol) et le chloro-*tert*-butyl-diméthylsilane (3,28 g ; 21,72 mmol). L'ensemble est agité à température ambiante pendant 2 heures. Après hydrolyse, la phase organique est lavée avec de l'eau et séchée sur sulfate de magnésium, filtrée et concentrée à sec. Le résidu est purifié par chromatographie sur gel de silice en utilisant le dichlorométhane et le méthanol comme éluants, puis est engagé dans l'étape suivante sans purification.

Le composé du titre est obtenu selon le procédé décrit au Stade A de l'exemple 1, en utilisant le résidu intermédiaire ci-dessus et l'acide de la Préparation 1a.

**RMN ¹H** (500 MHz, dmso-d6, 300 K) δ ppm : 7.6-7.35 (m, 3 H), 7.2-6.85 (m, 4 H), 6.45-5.9 (4s, 1 H), 5.05-2.7 (m, 4 H), 4.1/3.95 (2m, 2 H), 3.85-3.2 (m, 2 H), 3.45/3.3 (2s, 3 H), 2.9/2.6 (2m, 1 H), 2.45/2.35/2.2 (3s, 3 H), 1.2/1.05 (2m, 3 H), 0.85/0.7 (2br. s, 9 H), 0.05-0 (3br. s, 6 H)
**IR :** v : >C=O : 1697 et 1634 cm⁻¹.

### Stade B : acide 5-(5-chloro-2-{[4-(hydroxyméthyl)-3,4-dihydroisoquinoléin-2(1H)-yl]carbonyl}phényl)-1,2-diméthyl-1H-pyrrole-3-carboxylique

Le composé du titre est obtenu selon le procédé décrit au Stade B de l'Exemple 1 en utilisant l'ester obtenu au stade précédent.

**RMN ¹H** (500 MHz, dmso-d6, 300 K) δ ppm : 7.6-7.4 (m, 3 H), 7.2-7 (m, 4 H), 6.5-6.2 (4br. s, 1 H), 5.05/4.3-3.95 (5m, 2 H), 4.3-2.8 (6m, 2 H), 3.75-2.8 (m, 2 H), 3.47/3.3 (2s, 3 H), 2.85/2.6 (3m, 1 H), 2.5/2.35/2.25 (3s, 3 H).

**IR :** v : >C=O : 1662 et 1613 cm⁻¹.

### Stade C: acide 5-(2-{[4-({[tert-butyl(diméthyl)silyl]oxy}méthyl)-3,4-dihydroisoquinoléin-2(1H)-yl]carbonyl}-5-chlorophényl)-1,2-diméthyl-1H-pyrrole-3-carboxylique

A une solution du composé obtenu au Stade B (3,65 g ; 8,32 mmol) dans l'acétonitrile (10 mL) sont additionnés le chloro-*tert*-butyl-diméthylsilane (3 g; 20 mmol) et la 4-(diméthyl)-amino-pyridine (0,11 g ; 0,9 mmol), puis l'ensemble est agité à température ambiante pendant 15 minutes. Le 2,3,4,6,7,8,9,10-octahydropyrimido[1,2-*a*]azépine (1,65 mL ; 10,8 mmol) est ensuite ajouté goutte à goutte à 0°C. Le milieu réactionnel est agité à 70°C pendant 24 heures. Après ajout d'une solution aqueuse d'acide chlorhydrique 0,1 N (10 mL), on laisse le milieu réactionnel sous agitation pendant 3 heures. Le milieu réactionnel est dilué dans un mélange d'eau et d'acétate d'éthyle. Après extraction à l'acétate d'éthyle, les phases organiques sont réunies, séchées sur sulfate de magnésium, filtrées et concentrées à sec. Le résidu est purifié par chromatographie sur gel de silice en utilisant le dichlorométhane, le méthanol et l'acide acétique comme éluants pour obtenir le produit du titre.

**RMN ¹H** (400 MHz, dmso-d6, 300 K) δ ppm : 7.5-7.1 (m, 7 H), 6.3 (m, 1 H), 3.6-3.4 (m, 2 H), 3.5 (m, 4 H), 3.4 (m, 3 H), 3 (s, 1 H), 2.5-2.3 (m, 3 H), 0.9 (s, 9 H), 0.1 (s, 6 H).

### Stade D : 5-(2-{[4-({[tert-butyl(diméthyl)silyl]oxy}méthyl)-3,4-dihydroisoquinoléin-2(1H)-yl]carbonyl}-5-chlorophényl)-N-(5-cyano-2-fluorophényl)-1,2-diméthyl-N-[4-(prop-2-én-1-yloxy)phényl]-1H-pyrrole-3-carboxamide

Le composé est obtenu selon le procédé décrit au Stade A de l'Exemple 6 en utilisant l'acide obtenu au stade précédent et l'amine de la Préparation 1 1a". Le résidu obtenu est directement engagé dans l'étape suivante sans purification.

### Stade E : 5-(5-chloro-2-{[4-(hydroxyméthyl)-3,4-dihydroisoquinoléin-2(1H)-yl]carbonyl}phényl)-N-(5-cyano-2-fluorophényl)-1,2-diméthyl-N-[4-(prop-2-én-1-yloxy)phényl]-1H-pyrrole-3-carboxamide

Le composé du titre est obtenu selon le procédé décrit au Stade G de l'Exemple 1, en utilisant le composé issu du stade précédent. Le résidu obtenu est directement engagé dans l'étape suivante sans purification.

### Stade F: 12-chloro-5-(4-hydroxyphényl)-8,9-diméthyl-6,15-dioxo-6,9,22,23-tétrahydro-5H,15H,17H-16,22-méthano-7,10-(méthéno)tribenzo[b,j,o][1,4,8,13]oxatriazacyclooctadécine-3-carbonitrile

A une solution du composé obtenu au Stade E (40 mg ; 0,058 mmol) dans le tétrahydrofurane (20 mL), est additionné à 0 °C l'hydrure de sodium (4,64 mg ; 0,012 mmol), puis le mélange est agité à 40 °C pendant 16 heures. A deux autres reprises, une quantité identique d'hydrure de sodium est ajoutée, puis le milieu réactionnel est agité à 40 °C pendant 16 heures avant d'être dilué dans une solution aqueuse saturée en chlorure d'ammonium. Après extraction à l'acétate d'éthyle, les phases organiques sont réunies, lavées avec une solution aqueuse saturée en chlorure de sodium, séchées sur sulfate de magnésium, filtrées et concentrées à sec. Le composé du titre est obtenu selon le procédé décrit au Stade D de l'Exemple 1 en utilisant le résidu ci-dessus.

### Masse haute résolution (ESI+) :

Formule brute :

   C₃₇H₂₉ClN₄O₄
[M+H]+ calculé 629.195,
[M+H]+ mesuré 629.194.

### Exemple 14 : 6-chloro-14-(4-hydroxyphényl)-10,11-diméthyl-2,13-dioxo-20-oxa-1,10,14-triazahexacyclo[26.3.1.1~9,12~.1~15,19~.0~3,8~.0~24,29~]tétratriaconta-3,5, 7,9(34),11,15(33),16,18,24,26,28-undécaène-17-carbonitrile

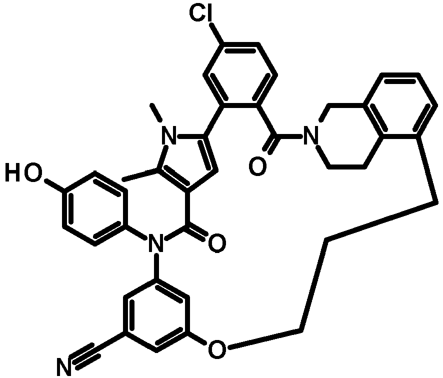

### Stade A : 5-[3-(3-cyano-5-{[4-(prop-2-én-1-yloxy)phényl)aminolphénoxy)propyl)-3,4-dihydroisoquinoléine-2(1H)-carboxylate de tert-butyle

A une solution du composé de la Préparation 1b" (1 g ; 1,63 mmol) dans le tétrahydrofurane (10 mL) à 0 °C, est additionnée une solution de fluorure de tétrabutylammonium dans le tétrahydrofurane 1 N (2,45 mL ; 2,45 mmol). L'ensemble est agité à température ambiante pendant 30 minutes. Le milieu réactionnel est concentré, puis dilué dans un mélange d'acétate d'éthyle et d'eau. Après décantation, la phase organique est lavée avec de l'eau et avec une solution aqueuse saturée en chlorure de sodium, puis séchée sur sulfate de magnésium, filtrée et concentrée.

A une solution du résidu obtenu dans l'acétonitrile, sont additionnés le bromure d'allyle (142 µL ; 1,63 mmol) puis le carbonate de césium (0,53 g : 1,63 mmol). L'ensemble est agité à température ambiante pendant 16 heures, puis pendant 2 heures à 60 °C après l'ajout de 0,2 équivalent de bromure d'allyle. Le milieu réactionnel est concentré puis dilué dans un mélange d'acétate d'éthyle et d'eau. Après décantation, la phase organique est lavée avec de l'eau, et avec une solution aqueuse saturée en chlorure de sodium, puis séchée sur sulfate de magnésium, filtrée et concentrée. Le résidu ainsi obtenu est purifié par chromatographie sur gel de silice en utilisant l'éther de pétrole et l'acétate d'éthyle comme éluants pour obtenir le produit du titre.

**RMN ¹H** (500 MHz, dmso-d6, 300 K) δ ppm : 8.24 (s, 1 H), 7.1 (dd, 1 H), 7.06 (dd, 2 H), 7 (d, 2 H), 6.94 (d, 2 H), 6.77-6.6 (3dd, 3 H), 6.04 (m, 1 H), 5.4/5.26 (2^{∗}d, 2 H), 4.54 (d, 2 H), 4.47 (s, 2 H), 3.96 (t, 2 H), 3.52 (t, 2 H), 2.76-2.64 (m, 4 H), 1.92 (m, 2 H), 1.42 (s, 9 H). **IR :** v : >NH : 3340 cm⁻¹ ; -CN : 2225 cm⁻¹ ; >C=O : 1624 cm⁻¹.

### Stade B : 5-[3-(3-{({5-[2-(tert-butoxycarbonyl)-5-chlorophényl]-1,2-diméthyl-1H-pyrrol-3-yl}carbonyl)[4-(prop-2-én-1-yloxy)phényllaminol-5-cyanophénoxy)propyl]-3,4-dihydroisoquinoléine-2(1H)-carboxylate de tert-butyle

A une solution du composé issu de la Préparation 1b (0,7 g ; 2 mmol) dans le dichlorométhane (20 mL) et le *N*,*N*-diméthylformamide (5 gouttes), est additionné le 1-chloro-*N,N*-2-triméthyl-prop-1-èn-1-amine (0,53 mL ; 4 mmol). Le milieu réactionnel est agité à température ambiante pendant 1 heure puis concentré. Le résidu est repris dans le dichloroéthane puis concentré, cette opération est effectuée à deux reprises. Le résidu final est repris dans le dichloroéthane (10 mL), puis est ajouté à une solution du composé obtenu au Stade A (1,08 g ; 2 mmol) et de pyridine (0,48 mL ; 6 mmol) dans le dichloroéthane (10 mL).L'ensemble est chauffé à 80 °C pendant 16 heures. Le milieu réactionnel est dilué dans un mélange de dichlorométhane et d'une solution aqueuse saturée en chlorure de sodium. Après extraction de la phase aqueuse au dichlorométhane, les phases organiques sont réunies, séchées sur sulfate de magnésium, filtrées et concentrées à sec. Le résidu est purifié par chromatographie sur gel de silice en utilisant l'éther de pétrole et l'acétate d'éthyle comme éluants pour obtenir le produit du titre.

**RMN ¹H** (500 MHz, dmso-d6, 300 K) δ ppm : 7.8 (d, 1 H), 7.55 (d, 1 H), 7.25 (s, 1 H), 7.15 (d, 2 H), 7.15/7.05/7 (3s, 3 H), 7.1-6.95 (m, 3 H), 6.9 (d, 2 H), 6 (m, 1 H), 5.35/5.2 (d+d, 1+1 H), 5.25 (s, 1 H), 4.55 (d, 2 H), 4.45 (br. s, 2 H), 4 (t, 2 H), 3.5 (t, 2 H), 3.15 (s, 3 H), 2.7 (m, 4 H), 2.45 (s, 3 H), 1.9 (quint, 2 H), 1.4 (s, 9 H), 1.3 (s, 9 H).

**IR :** v : -CN : 2230 cm⁻¹ ; >C=O : 1695 cm⁻¹ ; >C=O : 1645 cm⁻¹.

### Stade C : 6-chloro-14-(4-hydroxyphényl)-10,11-diméthyl-2,13-dioxo-20-oxa-1,10,14-triazahexacyclo[26.3.1.1~9,12~.1~15,19~.0~3,8~.0~24,29~]tétratriaconta-3,5,7,9(34),11,15(33),16,18,24,26,28-undécaène-17-carbonitrile

A une solution du composé obtenu au Stade B (0,58 g ; 0,67 mmol) dans le dioxane (15 mL), sont additionnés la triéthylamine (1,38 mL ; 10 mmol) et le trifluorométhanesulfonate de triméthylsilyle (1,8 mL ; 10 mmol). Le milieu réactionnel est agité à reflux pendant 2 heures puis versé dans de l'eau glacée. Après extraction à l'acétate d'éthyle, les phases organiques sont réunies, séchées sur sulfate de magnésium, filtrées et concentrés.

A une solution du résidu ainsi obtenu dans le dichlorométhane (200 mL), est additionnée successivement le 1-hydroxybenzotriazole (0,108 g ; 0,67 mmol), le chlorhydrate de 1-(3-diméthylaminopropyl)-3-éthylcarbodiimide (0,153 g; 0,8 mmol) et la diisopropyléthylamine (0,57 mL ; 3,33 mmol). L'ensemble est ensuite agité pendant 24 heures à température ambiante. Le milieu réactionnel est dilué avec un mélange de dichlorométhane et d'eau. Après décantation, la phase organique est séchée sur sulfate de magnésium, filtrée et concentrée à sec. Le résidu est purifié par chromatographie sur gel de silice en utilisant le dichlorométhane et l'acétate d'éthyle comme éluants.

A une solution du résidu ainsi obtenu dans un mélange de dichlorométhane (10 mL) et de méthanol (5 mL), est additionné l'acide 1,3-diméthylbarbiturique (0,036 g ; 0,23 mmol). Le milieu réactionnel est dégazé à l'argon pendant 10 minutes et est ajouté le tétrakis-(triphénylphosphine)palladium(0) (0,013 g ; 0,01 mmol). Le milieu réactionnel est chauffé à 40 °C pendant 45 minutes. Après concentration du méthanol, le milieu réactionnel est dilué dans un mélange d'acétate d'éthyle et d'eau. Après décantation, la phase organique est séchée sur sulfate de magnésium, filtrée et concentrée à sec. Le résidu est purifié par chromatographie sur phase Oasis^{®} en utilisant l'acétonitrile et l'eau comme éluants. Le solide ainsi obtenu est dissous dans un mélange d'eau et d'acétonitrile, filtré puis lyophilisé pour obtenir le produit du titre.

### Microanalyse élémentaire : (% théorique : mesuré)

%C = 71.28 : 70.81 ; %H = 5.06 : 4.94 ; %N = 8.53 : 8.51.

### Masse haute résolution (ESI+) :

Formule brute :

   C₃₉H₃₃ClN₄O₅
[M+H]+ calculé 673,2212,
[M+H]+ mesuré 673,2211.

### Exemple 15 : 6-chloro-14-(4-hydroxyphényl)-1C,11-diméthyl-23-oxa-1,10,14,17,18-pentaazahexacyclo[26.3.1.1~9,12~.1~15,18~.0~3,8~.0~24,29~]tétratriaconta-3,5,7,9(34),11,15(33),16,24,26,28-décaène-2,13-dione

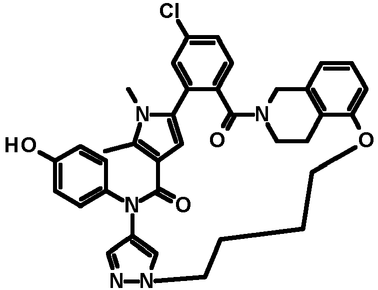

### Stade A : 5-(4-{4-[({5-[2-(tert-butoxycarbonyl)-5-chlorophényl]-1,2-diméthyl-1H-pyrrol-3-yl}carbonyl)(4-{[tert-butyl(diméthyl)silyl]oxy}phényl)amino]-1H-pyrazol-1-yl}butoxy)-3,4-dihydroisoquinoléine-2(1H)-carboxylate de tert-butyle

Le composé du titre est obtenu selon le procédé décrit au Stade B de l'Exemple 14, en utilisant le composé de la Préparation 1b (acide) et le composé de la Préparation 2b" (amine).

**RMN ¹H** (400 MHz, dmso-d6, 300 K) δ ppm : 7.83 (s, 1 H), 7.73 (d, 1 H), 7.51 (dd, 1 H), 7.15 (s, 1 H), 7.14-7.06 (m, 3 H), 7.1 (s, 1 H), 6.82 (d, 2 H), 6.73 (dd, 2 H), 4.97 (s, 1 H), 4.46 (s, 2 H), 4.1 (t, 2 H), 3.91 (t, 2 H), 3.53 (t, 2 H), 3.15 (s, 3 H), 2.59 (t, 2 H), 2.47 (s, 3 H), 1.9 (m, 2 H), 1.64 (m, 2 H), 1.41 (s, 9 H), 1.27 (s, 9 H), 0.86 (s, 9 H), 0.07 (s, 6 H).

**IR :** v : >C=O : 1695 cm⁻¹ ; >C=O : 1633 cm⁻¹.

### Stade B : 6-chloro-14-(4-hydroxyphényl)-10,11-diméthyl-23-oxa-1,10,14,17,18-pentaazahexacyclo[26.3.1.1~9,12~.1~15,18~.0~3,8~.0~24,29~]tétratriaconta-3,5,7,9(34),11,15(33),16,24,26,28-décaène-2,13-dione

A une solution du composé obtenu au Stade A (0,13 g ; 0,14 mmol) dans le dioxane (5 mL), sont additionnés la triéthylamine (0,1 mL ; 0,71 mmol) et le trifluorométhanesulfonate de triméthylsilyle (0,13 mL ; 0,71 mmol). Le milieu réactionnel est agité à reflux pendant 1 heure puis versé dans de l'eau glacée. Après extraction à l'acétate d'éthyle, les phases organiques sont réunies, séchées sur sulfate de magnésium, filtrées et concentrés.

A une solution du résidu obtenu dans le dichlorométhane (85 mL), sont additionnés successivement le 1-hydroxybenzotriazole (0,046 g ; 0,34 mmol), le chlorhydrate de 1-(3-diméthylaminopropyl)-3-éthylcarbodiimide (0,065 g ; 0,34 mmol) et la diisopropyléthylamine (0,243 mL ; 1,42 mmol). L'ensemble est ensuite agité pendant 20 heures à température ambiante. Le milieu réactionnel est dilué avec un mélange de dichlorométhane et d'eau. Après décantation, la phase organique est séchée sur sulfate de magnésium, filtrée et concentrée à sec. Le résidu est purifié par chromatographie sur gel de silice en utilisant le dichlorométhane et le méthanol comme éluants.

A une solution du résidu obtenu dans le méthanol (3 mL), est additionnée une solution de potasse 1 N dans le méthanol (0,11 mL ; 0,11 mmol). L'ensemble est ensuite agité pendant 18 heures à température ambiante. Après concentration du méthanol, le milieu réactionnel est dilué dans un mélange de dichlorométhane et d'eau. Après décantation, la phase organique est séchée sur sulfate de magnésium, filtrée et concentrée à sec. Le résidu est purifié par chromatographie sur phase Oasis^{®} en utilisant l'acétonitrile et l'eau comme éluants. Le solide ainsi obtenu est dissous dans un mélange d'eau et d'acétonitrile puis lyophilisé pour obtenir le produit du titre.

### Masse haute résolution (ESI+) :

Formule brute :

   C₃₆H₃₄ClN₅O₄
[M+H]+ calculé 636,2372,
[M+H]+ mesuré 636,2375.

### Exemple 16 : 11-chloro-4-(4-hydroxyphényl)-1,7,8-triméthyl-1,4,16,17,23,24-hexahydro-14H-15,18-méthano-6,9-(méthéno)dibenzo[l,r]pyrazolo[3,4-d][1,6,10,15]oxatriazacyclononadécine-5,14(8H)-dione

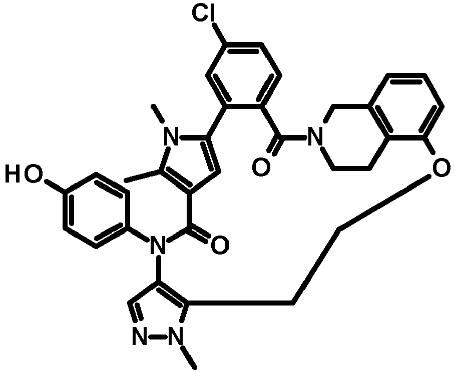

Le composé du titre est obtenu selon le procédé de l'Exemple 15 en utilisant l'acide de la Préparation 1b et l'amine de la Préparation 3b".

### Microanalyse élémentaire : (% théorique : mesuré)

%C = 67.57 : 67.70 ; %H = 5.18 : 4.89 ; %N = 11.26 : 11.16.

### Masse haute résolution (ESI+) :

Formule brute :

   C₃₅H₃₂ClN₅O₄
[M+H]+ calculé 622,2209,
[M+H]+ mesuré 622,2216.

### Exemple 17 : 11-chloro-4-(4-hydroxyphényl)-1,7,8-triméthyl-16-(morpholin-4-ylméthyl)-4,16,17,23,24,25-hexahydro-1H,14H-15,18-méthano-6,9-(méthéno)dibenzo[b,h]pyrazolo[4,3-p][1,6,11,15]oxatriazacycloicosine-5,14(8H)-dione

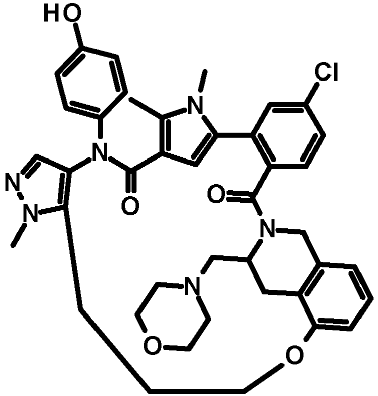

### Stade A : 2-{4-[(4-{[tert-butyl(diméthyl)silyl]oxy}phényl){1-méthyl-5-[3-(tétrahydro-2H-pyran-2-yloxy)propyl]-1H-pyrazol-4-yl}carbamoyl]-1,5-diméthyl-1H-pyrrol-2-yl}-4-chlorobenzoate de tert-butyle

Le composé du titre est obtenu selon le procédé décrit au Stade B de l'Exemple 14, en utilisant l'acide de la Préparation 1b et l'amine de la Préparation 4b".

**RMN ¹H** (400 MHz, dmso-d6, 300 K) δ ppm : 7.75 (d, 1 H), 7.52 (dd, 1 H), 7.21 (s, 1 H), 7.1 (d, 1 H), 7.02 (d, 2 H), 6.71 (d, 2 H), 6.19 (s, 1 H), 4.5 (t, 1 H), 3.71 (s, 3 H), 3.7-3.2 (4m, 4 H), 3.15 (s, 3 H), 2.55 (m, 2 H), 2.41 (s, 3 H), 1.7-1.2 (m, 8 H), 1.3 (s, 9 H), 0.85 (s, 9 H), 0.1 (s, 6 H).

**IR :** v : >C=O : 1706 cm⁻¹ ; >C=O : 1640 cm⁻¹.

### Stade B : 2-(4-{(4-{[tert-butyl(diméthyl)silyl]oxy}phényl)[5-(3-hydroxypropyl)-1-méthyl-1H-pyrazol-4-yl]carbamoyl}-1, 5-diméthyl-1H-pyrrol-2-yl)-4-chlorobenzoate de tert-butyle

A une solution du composé obtenu au Stade A (3,28 g ; 4,21 mmol) dans le méthanol (30 mL), est additionné le *para*-toluènesulfonate de pyridinium (1,5 g ; 0,42 mmol), puis l'ensemble est agité pendant 4 heures à 80 °C. Après retour à température ambiante, le milieu réactionnel est dilué avec du dichlorométhane et une solution aqueuse saturée en chlorure d'ammonium. Après décantation, la phase organique est lavée avec de l'eau, puis séchée sur sulfate de magnésium, filtrée et concentrée à sec. Le résidu est purifié par chromatographie sur gel de silice en utilisant le dichlorométhane et le méthanol comme éluants pour obtenir le produit du titre.

**RMN ¹H** (400 MHz, dmso-d6, 300 K) δ ppm : 7.75 (d, 1 H), 7.59 (dd, 1 H), 7.22 (s, 1 H), 7.12 (d, 1 H), 7.05 (d, 2 H), 6.75 (d, 2 H), 5.15 (s, 1 H), 4.5 (m, 1 H), 3.71 (s, 3 H), 3.3 (t, 2 H), 3.2 (s, 3 H), 2.5 (t, 2 H), 2.45 (s, 3 H), 1.5 (m, 2 H), 1.31 (s, 9 H), 0.9 (s, 9 H), 0.1 (s, 6 H).

**IR :** v : -OH : 3421 cm⁻¹ ; >C=O : 1708 cm⁻¹ ; >C=O : 1634 cm⁻¹.

### Stade C: 5-(3-{4-[({5-[2-(tert-butoxycarbonyl)-5-chlorophényl]-1,2-diméthyl-1H-pyrrol-3-yl}carbonyl)(4-{[tert-butyl(diméthyl)silyl]oxy}phényl)amtno]-1-méthyl-1H-pyrazol-5-yl}propoxy)-3-(morpholin-4-ylméthyl)-3,4-dihydroisoquinoléine-2(1H)-carboxylate de tert-butyle

A une solution du composé obtenu au Stade B (1,67 g ; 2,9 mmol), de triphénylphosphine (1,26 g; 3,5 mmol) et du composé de la Préparation 4' (0,84 g; 2,9 mmol) dans le tétrahydrofurane (20 mL), est additionnée goutte à goutte une solution d'azodicarboxylate de diisopropyle (1 mL ; 3,5 mmol) dans le tétrahydrofurane (20 mL). Le milieu réactionnel est agité à température ambiante pendant 2 heures puis dilué dans un mélange d'acétate d'éthyle et d'eau. Après extraction de la phase aqueuse à l'acétate d'éthyle, les phases organiques sont réunies, lavées avec une solution aqueuse saturée en chlorure d'ammonium, une solution aqueuse saturée en chlorure de sodium, puis séchées sur sulfate de magnésium, filtrées et concentrées à sec. Le résidu est purifié par chromatographie sur phase RP-18 en utilisant l'acétonitrile et l'eau comme éluants pour obtenir le produit du titre.

**RMN ¹H** (400 MHz, dmso-d6, 300 K) δ ppm : 7.74 (d, 1 H), 7.54 (d, 1 H), 7.26 (s, 1 H), 7.26 (s, 1 H), 7.1 (t, 1 H), 7.03 (d, 2 H), 6.74 (d, 1 H), 6.73 (d, 1 H), 6.69 (d, 2 H), 5.15 (s, 1 H), 4.64/4.08 (d+m, 1+1 H), 4.59/4.46 (m+m, 1 H), 3.9 (m, 2 H), 3.74 (s, 3 H), 3.49 (m, 4 H), 3.16 (s, 3 H), 2.73/2.58 (m+m, 1+1 H), 2.69 (m, 2 H), 2.5-2.2 (m, 4 H), 2.42 (s, 3 H), 2.24/1.98 (m+m, 1+1 H), 1.79 (m, 2 H), 1.42 (s, 9 H), 1.25 (s, 9 H), 0.85 (s, 9 H), 0.07 (s, 6 H).

**IR :** v : >C=O : 1693 cm⁻¹ ; >C=O : 1641 cm⁻¹.

### Stade D : 11-chloro-4-(4-hydroxyphényl)-1,7,8-triméthyl-16-(morpholin-4-ylméthyl)-4,16,17,23,24,25-hexahydro-1H,14H-15,18-méthano-6,9-(méthéno)dibenzo[b,h]pyrazolo[4,3-p][1,6,11,15]oxatriazacycloicosine-5,14(8H)-dione

A une solution du composé obtenu au Stade C (0,315 g ; 0,31 mmol) dans le dichloroéthane (12,5 mL), sont additionnés la triéthylamine (0,13 mL ; 0,92 mmol) et le bromure de zinc (0,34 g ; 1,53 mmol). Le milieu réactionnel est agité à 120°C pendant 2 heures sous micro-ondes (150 W) à 3 reprises, puis il est versé dans un mélange de dichlorométhane et d'eau glacée. Après extraction au dichlorométhane, les phases organiques sont réunies, séchées sur sulfate de magnésium, filtrées et concentrés.

A une solution du résidu ainsi obtenu dans le dichlorométhane (15 mL), sont additionnés successivement le 1-hydroxybenzotriazole (0,052 g ; 0,23 mmol), le chlorhydrate de 1-(3-diméthylaminopropyl)-3-éthylcarbodiimide (0,072 g ; 0,23 mmol) et la diisopropyléthylamine (0,2 mL ; 0,6 mmol). L'ensemble est ensuite agité pendant 16 heures à température ambiante. Le milieu réactionnel est dilué avec un mélange de dichlorométhane et d'eau. Après décantation, la phase organique est séchée sur sulfate de magnésium, filtrée et concentrée à sec.

A une solution du résidu ainsi obtenu dans le tétrahydrofurane (14 mL), est additionnée une solution de fluorure de tétrabutylammonium 1 M dans le tétrahydrofurane (0,5 mL ; 0,5 mmol). L'ensemble est ensuite agité pendant 30 minutes à température ambiante. Après concentration, le résidu est repris dans un mélange d'acétate d'éthyle et d'une solution aqueuse saturée en chlorure de sodium. Après décantation, la phase organique est séchée sur sulfate de magnésium, filtrée et concentrée à sec. Le résidu est purifié par chromatographie sur gel de silice en utilisant le dichlorométhane et le méthanol ammoniacal comme éluants. Le solide ainsi obtenu est dissous dans un mélange d'eau et d'acétonitrile, puis lyophilisé pour obtenir le produit du titre.

### Masse haute résolution (ESI+) :

Formule brute :

   C₄₁H₄₃ClN₆O₅
[M+H]+ calculé 735,3056,
[M+H]+ mesuré 735,3061.

### Exemple 18: 11-chloro-4-(4-hydroxyphényl)-1,7,8-triméthyl-5,14-dioxo-4,5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile

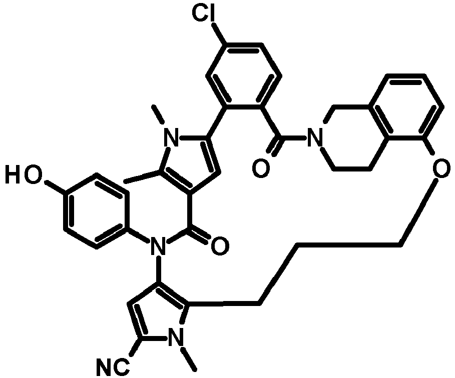

Le composé du titre est obtenu selon le procédé de l'Exemple 15 en utilisant l'acide de la Préparation 1b et l'amine de la Préparation 5b".

### Microanalyse élémentaire : (% théorique : mesuré)

%C = 69.14 : 68.70 ; %H = 5.19 : 5.16 ; %N = 10.61 : 9.97.

### Masse haute résolution (ESI+) :

Formule brute :

   C₃₈H₃₄ClN₅O₄
[M+H]+ calculé 660,2372,
[M+H]+ mesuré 660,2374.

### Exemple 19 : 11-chloro-4-(4-hydroxyphényl)-1,7,8-triméthyl-5,14-dioxo-1,4,5,8,16,17,23,24-octahydro-14H-15,18-méthano-6,9-(méthéno)dibenzo[l,r]pyrrolo[2,3-d][1,6,10,15]oxatriazacyclononadécine-2-carbonitrile

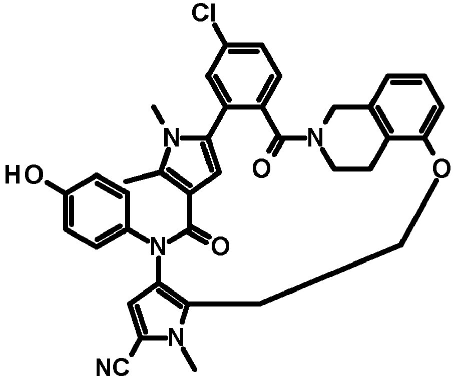

Le composé du titre est obtenu selon le procédé de l'Exemple 15 en utilisant l'acide de la Préparation 1b et l'amine de la Préparation 6b".

### Microanalyse élémentaire : (% théorique : mesuré)

%C = 68.78 : 68.64 ; %H = 4.99 : 5.01 ; %N = 10.84 : 10.66.

### Masse haute résolution (ESI+) :

Formule brute :

   C₃₇H₃₂ClN₅O₄
[M+H]+ calculé 646,2217,
[M+H]+ mesuré 646,2216.

### Exemple 20 : 11-fluoro-4-(4-hydroxyphényl)-1,7,8-triméthyl-1,4,16,17,23,24-hexahydro-14H-15,18-méthano-6,9-(méthéno)dibenzo[l,r]pyrazolo[3,4-d][1,6,10,15]oxatriazacyclononadécine-5,14(8H)-dione

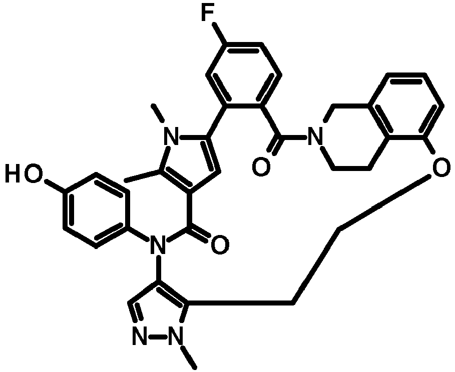

Le composé du titre est obtenu selon le procédé de l'Exemple 15 en utilisant l'acide de la Préparation 2b et l'amine de la Préparation 3b".

### Masse haute résolution (ESI+) :

Formule brute :

   C₃₅H₃₃FN₅O₄
[M+H]+ calculé 606.2511,
[M+H]+ mesuré 606.2517.

### Exemple 21 : 11-chloro-4-(4-hydroxyphényl)-1,7,8-triméthyl-1,4,16,17,23,24-hexahydro-14H-15,18-méthano-6,9-(méthéno)dibenzo[m,s]pyrazolo[3,4-e][1,4,7,11,16]dioxatriazacycloicosine-5,14(8H)-dione

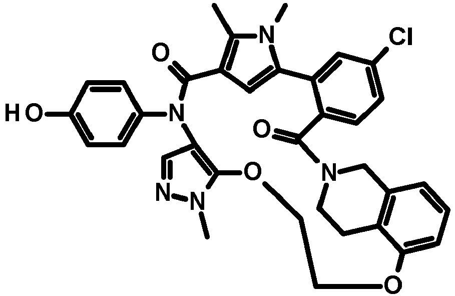

Le composé du titre est obtenu selon le procédé de l'Exemple 15 en utilisant l'acide de la Préparation 1b et l'amine de la Préparation 7b".

### Microanalyse élémentaire : (% théorique : mesuré)

%C = 65.88 : 65.61 ; %H = 5.05 : 4.98 ; %N = 10.60 : 10.94.

### Masse haute résolution (ESI+) :

Formule brute :

   C₃₅H₃₂ClN₅O₅
[M+H]+ calculé 638,2165,
[M+H]+ mesuré 638,2166.

### Exemple 22 : 11-chloro-4-(4-hydroxyphényl)-1,7,8-triméthyl-1,4,16,21,22,23-hexahydro-14H-15,21-méthano-6,9-(méthéno)dibenzo[j,o]pyrazolo[3,4-b][1,4,8,13]oxatriazacyclononadécine-5,14(8H)-dione

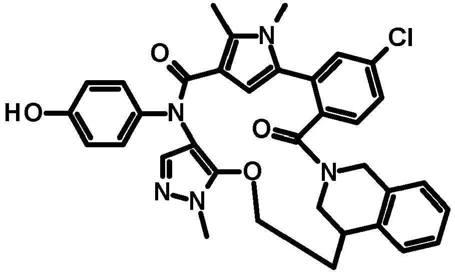

Le composé du titre est obtenu selon le procédé de l'Exemple 15 en utilisant l'acide de la Préparation 1b et l'amine de la Préparation 8b".

### Masse haute résolution (ESI+) :

Formule brute :

   C₃₅H₃₂ClN₅O₄
[M+H]+ calculé 622.2216,
[M+H]+ mesuré 622.2208.

### Exemple 23 : 11-chloro-4-(4-hydroxyphényl)-7,8-diméthyl-1-(tétrahydrofuran-3-yl)-1,4,16,17,23,24-hexahydro-14H-15,18-méthano-6,9-(méthéno)dibenzo[l,r]pyrazolo[3,4-d][1,6,10,15]oxatriazacyclononadécine-5,14(8H)-dione

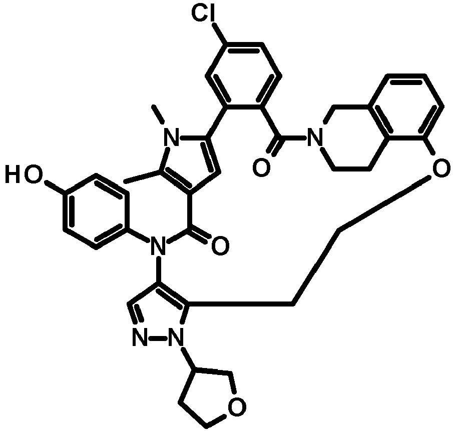

Le composé du titre est obtenu selon le procédé de l'Exemple 15 en utilisant l'acide de la Préparation 1b et l'amine de la Préparation 9b".

### Microanalyse élémentaire : (% théorique : mesuré)

%C = 67.30: 67.12 ; %H = 5.35 : 5.08 ; %N= 10.33 : 10.31.

### Masse haute résolution (ESI+) :

Formule brute :

   C₃₈H₃₆ClN₅O₅
[M+H]+ calculé 678,2478,
[M+H]+ mesuré 678,2481.

### Exemple 24 : 11-chloro-4-(4-hydroxyphényl)-1,7,8-triméthyl-1,4,22,23-tétrahydro-14H,16H-15,17-méthano-6,9-(méthéno)dibenzo[b,g]pyrazolo[4,3-o][1,5,10,14]oxatriazacyclooctadécine-5,14(8H)-dione

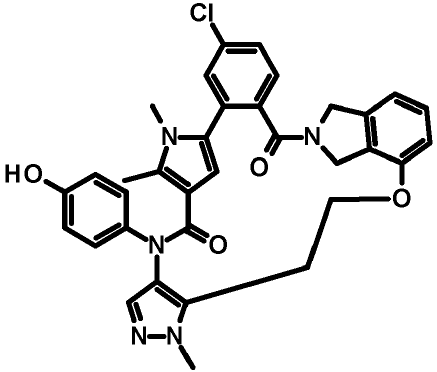

Le composé du titre est obtenu selon le procédé de l'Exemple 15 en utilisant l'acide de la Préparation 1b et l'amine de la Préparation 10b".

### Microanalyse élémentaire : (% théorique : mesuré)

%C = 67.16: 67.31 ; %H = 4.97 : 4.91 ; %N = 11.52 : 11.30.

### Masse haute résolution (ESI+) :

Formule brute :

   C₃₄H_{3O}ClN₅O₄
[M+H]+ calculé 608,2059,
[M+H]+ mesuré 608,2074.

### Exemple 25 : (16R ou S)-11-chloro-4-(4-hydroxyphényl)-1, 7,8,16-tétraméthyl-5,14-dioxo-4,5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile

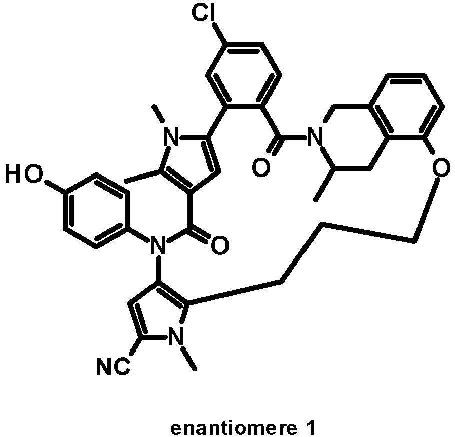

Le composé du titre est obtenu selon le procédé de l'Exemple 15 en utilisant l'acide de la Préparation 1b et l'amine de la Préparation 11b".

### Microanalyse élémentaire : (% théorique : mesuré)

%C = 69.48: 69.13 ; %H = 5.38 : 5.37 ; %N = 10.39 : 10.05.

### Masse haute résolution (ESI+) :

Formule brute :

   C₃₉H₃₆ClN₅O₄
[M+H]+ calculé 674,2529,
[M+H]+ mesuré 674,253.

### Exemple 26 : (16S ou R)-11-chloro-4-(4-hydroxyphényl)-1,7,8,16-tétraméthyl-5,14-dioxo-4,5,8,16,17,23,24,25-octahydro-1H,14H-15,18-méthano-6,9-(méthéno)dibenzo[b,hjpyrrolo[3,2-p][1,6,11,15]oxatriazacycloicosine-2-carbonitrile

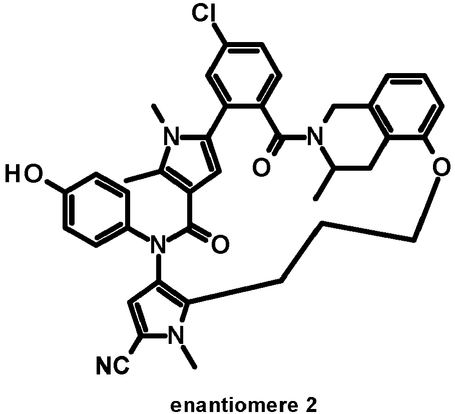

Le composé du titre est obtenu selon le procédé de l'Exemple 15 en utilisant l'acide de la Préparation 1b et l'amine de la Préparation 12b".

### Microanalyse élémentaire : (% théorique : mesuré)

%C = 69.48: 68.73 ; %H = 5.38 : 5.47 ; %N = 10.39 : 10.13.

### Masse haute résolution (ESI+) :

Formule brute :

   C₃₉H₃₆ClN₅O₄
[M+H]+ calculé 674,2529,
[M+H]+ mesuré 674,2532.

### Exemple 27 : 4-[15-chloro-3-hydroxy-18,19-diméthyl-12,21-dioxo-7,8,9,10,18,21-hexahydro-6H,12H,22H-8,11-éthano-20,17-(méthéno)dibenzo[b,j][1,4,8,13]oxatriazacyclooctadécin-22-yl]-1,5-diméthyl-1H-pyrrole-2-carbonitrile

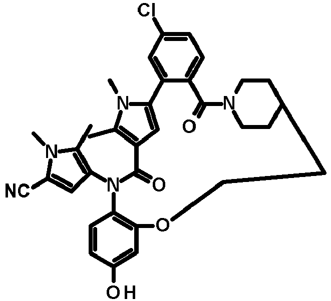

### Stade A : 4-(2-{5-(benzyloxy)-2-[({5-[2-(tert-butoxycarbonyl)-5-chlorophényl]-1,2-diméthyl-1H-pyrrol-3-yl}carbonyl)(5-cyano-1,2-diméthyl-1H-pyrrol-3-yl)amino]phénoxy}éthyl)pipéridine-1-carboxylate de tert-butyle

Le composé du titre est obtenu selon le procédé décrit au Stade B de l'Exemple 14 en utilisant l'acide de la Préparation 1b et l'amine de la Préparation 13b".

**RMN ¹H** (500 MHz, dmso-d6, 300 K) δ ppm : 7.8 (d, 1 H), 7.55 (dd, 1 H), 7.45-7.25 (m, 5 H), 7.05 (d, 1 H), 7.05 (s, 1 H), 6.65 (br. s, 1 H), 6.65 (s, 1 H), 6.5 (d, 1 H), 5.35 (br. s, 1 H), 5.05 (s, 2 H), 3.95 (br. s, 2 H), 3.85/2.55 (2br. s, 4H), 3.55 (s, 3 H), 3.15 (s, 3 H), 2.35 (s, 3 H), 2.1 (br. s, 3 H), 1.6/0.95 (m+m, 2+2 H), 1.55 (m, 1 H), 1.55 (m, 2 H), 1.35 (s, 9 H), 1.25 (s, 9 H).

**IR :** v : -CN : 2209 cm⁻¹ ; >C=O : 1715, 1688 et 1640 cm⁻¹.

### Stade B : 4-[15-chloro-3-hydroxy-18,19-diméthyl-12,21-dioxo-7,8,9,10,18,21-hexahydro-6H,12H,22H-8,11-éthano-20,17-(méthéno)dibenzo[bj][1,4,8,13]oxatriazacyclooctadécin-22-yl]-1,5-diméthyl-1H-pyrrole-2-carbonitrile

A une solution du composé obtenu au Stade A (1,3 g ; 1,29 mmol) dans le dichlorométhane (12 mL) est additionné le bromure de zinc (1,45 g ; 6,44 mmol). Le milieu réactionnel est agité à reflux pendant 17 heures, puis versé dans de l'eau. Le mélange réactionnel est ensuite agité pendant 2 heures et extrait au dichlorométhane. Les phases organiques sont réunies, séchées sur sulfate de magnésium, filtrées et concentrées. Le résidu est purifié par chromatographie sur gel de silice en utilisant le dichlorométhane et le méthanol comme éluants.

A une solution du résidu obtenu dans le dichlorométhane (470 mL), est additionnée successivement le 1-hydroxybenzotriazole (0,252 g ; 1,86 mmol), le chlorhydrate de 1-(3-diméthylaminopropyl)-3-éthylcarbodiimide (0,320 g; 1,86 mmol) et la diisopropyléthylamine (1,28 mL ; 7,77 mmol). L'ensemble est ensuite agité pendant 20 heures à température ambiante. Le milieu réactionnel est dilué avec un mélange de dichlorométhane et d'eau. Après décantation, la phase organique est séchée sur sulfate de magnésium, filtrée et concentrée à sec. Le résidu est purifié par chromatographie sur gel de silice en utilisant le dichlorométhane et le méthanol comme éluants.

A une solution du résidu ainsi obtenu dans un mélange de méthanol (25 mL) et d'acétate d'éthyle (25 mL), est additionné du palladium sur charbon (15 % massique), puis l'ensemble est hydrogéné pendant 4 heures à température ambiante sous 0,6 bar. Le milieu réactionnel est filtré et concentré à sec. Le résidu est purifié par chromatographie sur gel de silice en utilisant le dichlorométhane et le méthanol comme éluants, puis par chromatographie sur RP-18 en phase supercritique en utilisant l'éthanol avec 0,1 % de diéthylamine comme éluant. Le solide ainsi obtenu est dissous dans un mélange d'eau et d'acétonitrile, puis lyophilisé pour obtenir le produit du titre.

### Microanalyse élémentaire : (% théorique : mesuré)

%C = 66.71 : 66.25 ; %H = 5.60 : 5.52 ; %N = 11.44 : 11.37.

### Masse haute résolution (ESI+) :

Formule brute :

   C₃₄H₃₄ClN₅O₄
[M+H]+ calculé 612,2367,
[M+H]+ mesuré 612,2372.

### Exemple 28 : 4-[3-hydroxy-18,19-diméthyl-12,21-dioxo-7,8,9,10,18,21-hexahydro-6H,12H,22H-8,11-éthano-20,17-(méthéno)dibenzo[b,j][1,4,8,13]oxatriazacyclooctadécin-22-yl]-1,5-diméthyl-1H-pyrrole-2-carbonitrile

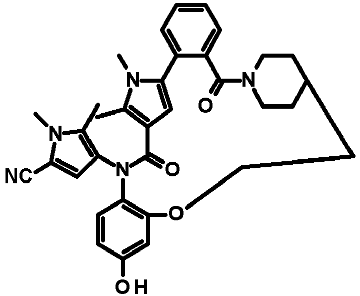

Le composé du titre est obtenu comme produit secondaire lors du Stade B de l'Exemple 27.

### Microanalyse élémentaire : (% théorique : mesuré)

%C = 70.69 : 70.15 ; %H = 6.11 : 6.13 ; %N = 12.12 : 12.86.

### Masse haute résolution (ESI+) :

Formule brute :

   C₃₄H₃₅N₅O₄
[M+H]+ calculé 578,2773,
[M+H]+ mesuré 578,2762.

### Exemple 29 : 4-[16-chloro-3-hydroxy-19,20-ditnéthyl-13,22-dioxo-6,7,8,9,10,11,19,22-octahydro-13H,23H- 8,12-méthano-21,18-(méthéno)dibenzo[b,j][1,4,8,13]oxatriazacyclononadécin-23-yl]-1,5-diméthyl-1H-pyrrole-2-carbonitrile

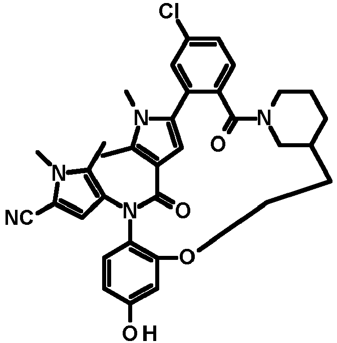

Le composé du titre est obtenu selon le procédé de l'Exemple 27 en utilisant l'acide de la Préparation 1b et l'amine de la Préparation 14b".

### Masse haute résolution (ESI+) :

Formule brute :

   C₃₄H₃₄ClN₅O₄
[M+H]+ calculé 612.2372,
[M+H]+ mesuré 612.2367.

### Exemple 30 : trifluorométhylsulfonate de 6-chloro-14-(4-hydroxyphényl)-10,11-diméthyl-2,13-dioxo-23-oxa-1,10,14-triaza-19-azoniahexacyclo[26.3.1.1~9,12~.1~15,19~.0~3,8~.0~24,29~]tétratriaconta-3, 5, 7, 9(34),11,15(33),16,18,24,26,28-undécaène

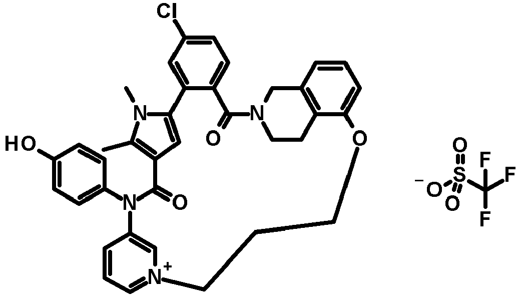

### Stade A : 3-[([5-[2-(tert-butoxycarbonyl)-5-chlorophényll-1,2-diméthyl-1H-pyrrol-3-yl}carbonyl)[4-(prop-2-én-1-yloxy)phényl]amino}-1-(3-{[2-(tert-butoxycarbonyl)-1,2,3,4-tétrahydroisoquinoléin-5-yl]oxy}propyl)pyridinium

A une solution du composé obtenu à la Préparation 1b (0,47 g ; 1,36 mmol) dans le dichlorométhane (34 mL), est additionnée le 1-chloro-*N*,*N*-2-triméthyl-prop-1-èn-1-amine (0,183 mL ; 1,49 mmol). Le milieu réactionnel est agité à température ambiante pendant 1 heure puis concentré. Le résidu est repris dans le tétrahydrofurane (14 mL).

En parallèle, on prépare une solution d'hydrure de sodium (65 mg ; 1,63 mmol) dans le tétrahydrofurane (5 mL) et de composé de la Préparation 15b" (960 mg ; 1,49 mmol) dans le tétrahydrofurane (15 mL), laquelle est agitée à température ambiante pendant 30 minutes. On y ajoute le résidu obtenu précédemment, et l'ensemble est agité pendant 3 heures. Le milieu réactionnel est dilué dans un mélange de dichlorométhane et d'eau. Après extraction de la phase aqueuse au dichlorométhane, les phases organiques sont réunies, séchées sur sulfate de magnésium, filtrées et concentrées à sec. Le résidu est purifié par chromatographie sur gel de silice en utilisant le dichloroméhane et le méthanol comme éluants pour obtenir le produit du titre.

**RMN ¹H** (400 MHz, dmso-d6, 300 K) δ ppm : 9.14 (br. s, 1 H), 8.87 (d, 1 H), 8.18 (d, 1 H), 8.06 (dd, 1 H), 7.81 (d, 1 H), 7.58 (d, 1 H), 7.17 (d, 2 H), 7.13 (t, 1 H), 7.01 (s, 1 H), 6.95 (d, 2 H), 6.77 (d, 1 H), 6.77 (d, 1 H), 6.01 (m, 1 H), 5.34 (d, 1 H), 5.21 (d, 1 H), 5.2 (s, 1 H), 4.8 (t, 2 H), 4.57 (d, 2 H), 4.45 (br. s, 2 H), 4.05 (t, 2 H), 3.48 (m, 2 H), 3.16 (s, 3 H), 2.46 (m, 2 H), 2.46 (s, 3 H), 2.4 (m, 2 H), 1.41/1.3 (2s, 18 H).

### Stade B : trifluorométhylsulfonate de 6-chloro-14-(4-hydroxyphényl)-10,11-diméthyl-2,13-dioxo-23-oxa-1,10,14-triaza-19-azoniahexacyclo[26.3.1.1~9,12~.1~15,19~.0~3,8~.0~24,29~]tétratriaconta-3, 5, 7, 9(34),11,15(33),16,18,24,26,28-undécaène

Le composé du titre est obtenu selon le procédé décrit au Stade C de l'Exemple 14 en utilisant le composé obtenu au stade précédent.

### Microanalyse élémentaire : (% théorique : mesuré)

%C = 58.27 : 58.74 ; %H = 4.38 : 4.35 ; %N = 7.15 : 7.71.

### Masse haute résolution (ESI+) :

Formule brute :

   C₃₈H₃₄ClF₃N₄O₇S
[M-CF3SO3]+ calculé 633,2249,
[M-CF3SO3]+ mesuré 633,2263.

### Exemple 31 : 6-chloro-14-(4-hydroxyphényl)-10,11,18-triméthyl-22-oxa-1,10,14,17,19-pentaazahexacyclo[25.3.1.1-9,12-.1-15,19-.0-3,8-.0-23,28-]tritriaconta-3,5,7,9(33),11,15,17,23,25,27-décaène-2,13,32-trione

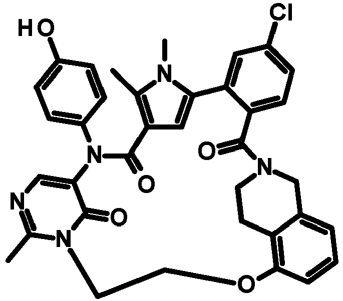

### Stade A : 4-chloro-2-[1,5-diméthyl-4-({2-méthyl-4-[2-(tétrahydro-2H-pyran-2-yloxy)éthoxy]pyrimidin-5-yl}[4-(prop-2-én-1-yloxy)phényl]carbamoyl)-1H-pyrrol-2-yl]benzoate de tert-butyle

Le composé du titre est obtenu selon le procédé décrit au Stade A de l'Exemple 30 en utilisant le composé de la Préparation 1b et celui de la Préparation 16b".

**RMN ¹H** (500 MHz, dmso-d6, 300 K) δ ppm : 8.36 (s, 1 H), 7.79 (d, 1 H), 7.56 (dd, 1 H), 7.21 (d, 2 H), 7.05 (d, 1 H), 6.87 (d, 2 H), 5.99 (m, 1 H), 5.33 (d, 1 H), 5.29 (s, 1 H), 5.2 (d, 1 H), 4.56 (m, 1 H), 4.53 (d, 2 H), 4.45 (m, 2 H), 3.83/3.61 (2m, 2 H), 3.69/3.35 (2m, 2 H), 3.15 (s, 3 H), 2.5 (s, 3 H), 2.39 (s, 3 H), 1.63/1.37 (2m, 2 H), 1.51/1.38 (2m, 2 H), 1.42/1.32 (2m, 2 H), 1.29 (s, 9 H).

**IR :** v : >C=O : 1703 cm⁻¹ ; >C=O : 1644 cm⁻¹.

### Stade B : 5-{2-[5-{({5-[2-(tert-butoxycarbonyl)-5-chlorophényl]-1,2-diméthyl-1H-pyrrol-3-yl}carbonyl)[4-(prop-2-én-1-yloxy)phényllaminol-2-méthyl-6-oxopyrimidin-1(6H)-yl]éthoxy}-3,4-dihydroisoquinoléine-2(1H)-carboxylate de tert-butyle

A une solution du composé obtenu au Stade A (0,97 g ; 1,35 mmol) dans le méthanol (7 mL), est additionnée le *para*-toluènesulfonate de pyridinium (0,34 g; 1,35 mmol), puis l'ensemble est agité pendant 4 heures à 80 °C. Après retour à température ambiante, le milieu réactionnel est concentré puis dilué avec de l'acétate d'éthyle et de l'eau. Après décantation, la phase organique est lavée avec de l'eau et une solution aqueuse saturée en chlorure de sodium, puis séchée sur sulfate de magnésium, filtrée et concentrée à sec.

A une solution du résidu obtenu (0,579 g ; 0,915 mmol) dans le dichlorométhane (5 mL), sont additionnés le chlorure de tosyle (0,349 g ; 1,83 mmol) et la triéthylamine (514 µL ; 3,66 mmol), puis l'ensemble est agité pendant 16 heures à température ambiante. Le milieu réactionnel est dilué avec du dichlorométhane et de l'eau. Après décantation, la phase organique est lavée avec de l'eau et une solution aqueuse saturée en chlorure de sodium, puis séchée sur sulfate de magnésium, filtrée et concentrée à sec.

A une solution du résidu obtenu (0,7 g ; 0,752 mmol) dans le *N,N*-diméthylformamide (5 mL), sont additionnés le composé de la Préparation 3b' (0,225 g ; 0,903 mmol) et le carbonate de potassium (0,313 g ; 2,26 mmol), puis l'ensemble est agité pendant 2 heures à 125 °C. Le milieu réactionnel est dilué avec de l'acétate d'éthyle et de l'eau. Après décantation, la phase organique est lavée avec de l'eau et une solution aqueuse saturée en chlorure de lithium, puis séchée sur sulfate de magnésium, filtrée et concentrée à sec. Le résidu est purifié par chromatographie sur gel de silice en utilisant le dichlorométhane et le méthanol comme éluants pour obtenir le produit du titre.

**RMN ¹H** (500 MHz, dmso-d6, 300 K) δ ppm : 7.88 (s, 1 H), 7.72 (d, 1 H), 7.5 (dd, 1 H), 7.15 (d, 2 H), 7.08 (t, 1 H), 7.07 (d, 1 H), 6.84 (d, 2 H), 6.74 (d, 1 H), 6.74 (d, 1 H), 5.99 (m, 1 H), 5.36 (s, 1 H), 5.33 (d, 1 H), 5.2 (d, 1 H), 4.52 (d, 2 H), 4.43 (br. s, 2 H), 4.39 (t, 2 H), 4.2 (t, 2 H), 3.48 (t, 2 H), 3.11 (s, 3 H), 2.62 (s, 3 H), 2.47 (t, 2 H), 2.36 (s, 3 H), 1.41/1.28 (2s, 18 H).

**IR :** v : >C=O : 1682 cm⁻¹ ; >C=O : 1639 cm⁻¹.

### Stade C: 6-chloro-14-(4-hydroxyphényl)-10,11,18-triméthyl-22-oxa-1,10,14,17,19-pentaazahexacyclo[25.3.1.1~9,12~.1~15,19~.0~3,8~.0~23,28~]tritriaconta-3,5,7,9(33),11,15,17,23,25,27-décaène-2,13,32-trione

Le composé du titre est obtenu selon le procédé décrit au Stade C de l'Exemple 14 en utilisant le composé du stade précédent.

### Microanalyse élémentaire : (% théorique : mesuré)

%C = 66.51 : 66.29 ; %H = 4.96 : 4.95 ; %N = 10.77 : 10.38.

### Masse haute résolution (ESI+) :

Formule brute :

   C₃₆H₃₂ClN₅O₅
[M+H]+ calculé 650,2169,
[M+H]+ mesuré 650,2165.

### Exemple 32 : 18-chloro-11-(4-hydroxyphényl)-2,14,15-triméthyl-1,12-dioxo-1,2,3,4,12,15-hexahydro-11H-6,10:13,16-di(méthéno)-5,2,11,15-benzoxatriazacyclooctadécine-8-carbonitrile

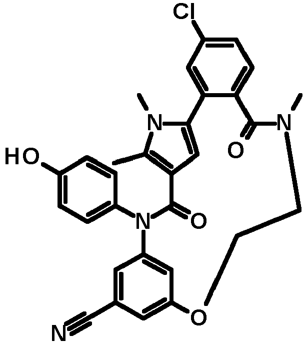

### Stade A : 2-(4-{(3-[2-[(tert-butoxycarbonyl)(méthyl)aminoléthoxyl-5-cyanophényl)[4-(prop-2-én-1-yloxy)phényl]carbamoyl}-1,5-diméthyl-1H-pyrrol-2-yl)-4-chlorobenzoate de tert-butyle

Le composé du titre est obtenu selon le procédé décrit au Stade B de l'Exemple 14 en utilisant le composé de la Préparation 1b et celui de la Préparation 17b".

**RMN ¹H** (400 MHz, dmso-d6, 300 K) δ ppm : 7.78 (d, 1 H), 7.56 (dd, 1 H), 7.28 (br. s, 1 H), 7.19 (br. s, 1 H), 7.14 (d, 2 H), 7.07 (d, 1 H), 6.99 (massif, 1 H), 6.92 (d, 2 H), 6 (m, 1 H), 5.34/5.21 (2dquad, 2 H), 5.26 (s, 1 H), 4.55 (m, 2 H), 4.11 (m, 2 H), 3.48 (t, 2 H), 3.16 (s, 3 H), 2.8 (s, 3 H), 2.43 (s, 3 H), 1.3 (m, 18 H).

**IR :** v : >CN : 2230 cm⁻¹ ; >C=O : 1697 et 1645 cm⁻¹.

### Stade B : 18-chloro-11-(4-hydroxyphényl)-2,14,15-triméthyl-1,12-dioxo-1,2,3,4,12,15-hexahydro-11H 6,10:13,16- di(méthéno)-5,2,11,15-benzoxatriazacyclooctadécine-8-carbonitrile

Le composé du titre est obtenu selon le procédé décrit au Stade C de l'Exemple 14 en utilisant le composé du stade précédent.

### Microanalyse élémentaire : (% théorique : mesuré)

%C = 66.60 : 65.92 ; %H = 4.66 : 4.62 ; %N = 10.36 : 10.08.

### Masse haute résolution (ESI+) :

Formule brute :

   C₃₀H₂₅ClN₄O₄
[M+H]+ calculé 541,1642,
[M+H]+ mesuré 541,1637.

### Exemple 33 : 21-chloro-6-(4-hydroxyphényl)-2,3,1 7-triméthyl-5,18-dioxo-5,6,13,14,15,16,17,18-octahydro-2H-4,1:11,7-di(méthéno)-1 2,2,6,1 7-benzoxatriazacycloicosine-9-carbonitrile

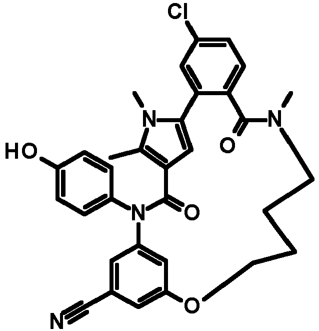

Le composé du titre est obtenu selon le procédé de l'Exemple 32 en utilisant l'acide de la Préparation 1b et l'amine de la Préparation 18b".

### Microanalyse élémentaire : (% théorique : mesuré)

%C = 67.54 : 67.16 ; %H = 5.14 : 5.05 ; %N = 9.85 : 9.27.

### Masse haute résolution (ESI+) :

Formule brute :

   C₃₂H₂₉ClN₄O₄
[M+H]+ calculé 569,1955,
[M+H]+ mesuré 569,1950.

### Exemple 34 : 18-chloro-11-(4-hydroxyphényl)-14,15-diméthyl-6,7,10,11,23,24-hexahydro-9H,21H-1,22-méthano-13,16-(méthéno)dibenzo[m,s][1,4,7,11,16]dioxatriazacycloicosine-12,21(15H)-dione

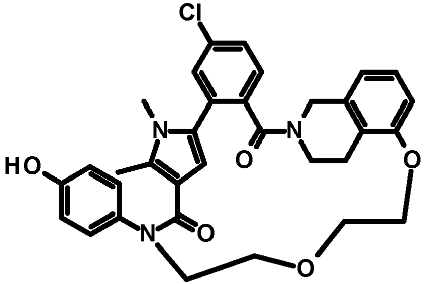

### Stade A : 5-(2-{2-[({5-[2-(tert-butoxycarbonyl)-5-chlorophényl]-1,2-diméthyl-1H-pyrrol-3-yl}carbonyl)(4-{[tert-butyl(diméthyl)silyl]oxy}phényl)anùno]éthoxy}éthoxy)-3,4-dihydroisoquinoléine-2(1H)-carboxylate de tert-butyle

Le composé du titre est obtenu selon le procédé décrit au Stade B de l'Exemple 14 en utilisant l'acide de la Préparation 1b et l'amine de la Préparation 19b".

**RMN ¹H** (500 MHz, dmso-d6, 300 K) δ ppm : 7.7 (d, 1 H), 7.5 (dd, 1 H), 7.11 (d, 1 H), 7.05 (m, 2 H), 6.8 (t, 1 H), 6.7 (d, 2 H), 6.65 (m, 2 H), 4.85 (s, 1 H), 4.42 (s, 2 H), 4.05 (m, 2 H), 3.87 (m, 2 H), 3.7 (m, 2 H), 3.65 (m, 2 H), 3.45 (m, 2 H), 3.15 (s, 3 H), 2.6 (m, 2 H), 2.4 (s, 3 H), 1.4-0.8 (s, 27 H), 0.1 (s, 6 H).

### Stade B : 18-chloro-11-(4-hydroxyphényl)-14,15-diméthyl-6,7,10,11,23,24-hexahydro-9H,21H-1,22-méthano-13,16-(méthéno)dibenzo[m,s][1,4,7,11,16]dioxatriazacycloicosine-12,21(15H)-dione

Le composé du titre est obtenu selon le procédé décrit au Stade D de l'Exemple 17 en utilisant le composé du stade précédent.

### Masse haute résolution (ESI+) :

Formule brute :

   C₃₃H₃₂ClN₃O₅
[M+H]+ calculé 586.2103,
[M+H]+ mesuré 586.2124.

### Exemple 35 : 21-chloro-29-(4-hydroxyphényl)-25,26-diméthyl-17,28-dioxo-2,3,4,16,25,29-hexaazaheptacyclo[28.3.1.1~2,5~.1~12,16~.1~24,27~.0~8,13~.0~18,23~]heptatriaconta-1(34),3,5(37),8,10,12,18,20,22,24(35),26,30,32-tridécaène-32-carbonitrile

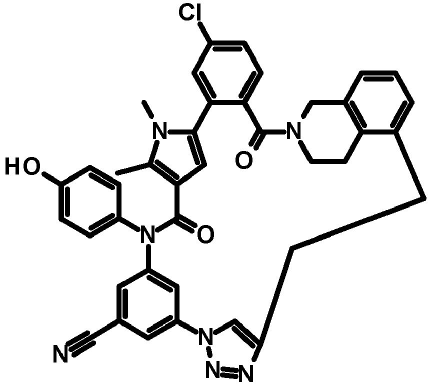

Le composé du titre est obtenu selon le procédé de l'Exemple 34 en utilisant l'acide de la Préparation 1b et l'amine de la Préparation 20b".

### Masse haute résolution (ESI+) :

Formule brute :

   C₄₀H₃₂ClN₇O₃
[M+H]+ calculé 694.2328,
[M+H]+ mesuré 694.233.

### Exemple 36 : 10-chloro-2-(4-hydroxyphényl)-5,6-diméthyl-23,26,29-trioxa-2,6,15,31,34-pentaazahexacyclo[28.2.2.1~4,7~.1~15,18~.0~8,13~.0~17,22~]hexatriaconta-1(32),4,7(36),8,10,12,17,19,21,30,33-undécaène-3,14-dione

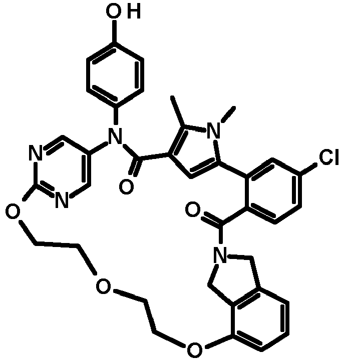

### Stade A : 4-(2-{2-[(5-{({5-[2-(tert-butoxycarbonyl)-5-chlorophényl]-1,2-diméthyl-1H-pyrrol-3-yl}carbonyl)[4-(prop-2-én-1-yloxy)phényl]amino}pyrimidin-2-yl)oxy]éthoxy}éthoxy)-1,3-dihydro-2H-isoindole-2-carboxylate de tert-butyle

Le composé du titre est obtenu selon le procédé décrit au Stade B de l'Exemple 14 en utilisant l'acide de la Préparation 1b et l'amine de la Préparation 21b".

**RMN ¹H** (500 MHz, dmso-d6, 300 K) δ ppm : 8.46/8.45 (s, 2 H), 7.77 (d, 1 H), 7.54 (dd, 1 H), 7.26-6.83 (m, 3 H), 7.21 (m, 2 H), 7.08 (d, 1 H), 6.92 (m, 2 H), 5.99 (m, 1 H), 5.33/5.2 (m+m, 2 H), 5.23/5.22 (s, 1 H), 4.6-4.41 (m, 4 H), 4.54 (m, 2 H), 4.45-3.75 (m, 8 H), 3.15 (s, 3 H), 2.43 (s, 3 H), 1.46-1.25 (s, 18 H).

### Stade B : acide 4-chloro-2-(4-{(2-{2-[2-(2,3-dihydro-1H-isoindol-4-yloxy)éthoxy]éthoxy}pyrimidin-5-yl)[4-(prop-2-én-1-yloxy)phényl]carbamoyl}-1,5-diméthyl-1H-pyrrol-2-yl)benzoïque

A une solution du composé obtenu au Stade A (1,2 g ; 1,36 mmol) dans le dioxane (18 mL), sont additionnés la triéthylamine (1,9 mL ; 13,6 mmol) et le trifluorométhanesulfonate de triméthylsilyle (2,46 mL ; 13,6 mmol). Le milieu réactionnel est agité à température ambiante pendant 20 minutes, puis versé dans de l'eau glacée. Le précipité obtenu est filtré, puis purifié par chromatographie sur phase RP-18 en utilisant l'acétonitrile, l'eau et l'acétate d'ammonium comme éluants pour obtenir le produit du titre.

**RMN ¹H** (500 MHz, dmso-d6, 300 K) δ ppm : 10.98 (brs, 2 H), 8.36 (s, 2 H), 7.58 (d, 1 H), 7.3 (dd, 1 H), 7.28 (t, 1 H), 7.16 (m, 2 H), 6.97 (d, 1 H), 6.95 (m, 2 H), 6.95 (d, 1 H), 6.92 (d, 1 H), 6.03 (m, 1 H), 5.41 (s, 1 H), 5.38/5.25 (m+m, 2 H), 4.57 (m, 2 H), 4.41 (brs, 2 H), 4.4-3.72 (m, 8 H), 4.33 (brs, 2 H), 3.15 (s, 3 H), 2.31 (s, 3 H).

### Stade C : 10-chloro-2-(4-hydroxyphényl)-5,6-diméthyl-23,26,29-trioxa-2,6,15,31,34-pentaazahexacyclo[28.2.2.1~4,7~.1~15,18~.0~8,13~.0~17,22~]hexatriaconta-1(32),4,7(36),8,10,12,17,19,21,30,33-undécaène-3,14-dione

A une solution du composé obtenu au Stade B (370 mg; 0,61 mmol) dans le dichlorométhane (50 mL), est additionné le tétrafluoroborate de 4-(4,6-diméthoxy-1,3,5-triazin-2-yl)-4-méthyl-morpholin-4-ium (237 mg; 0,7 mmol). Le milieu réactionnel est agité à température ambiante pendant 1 heure. Après un lavage avec de l'eau, la phase organique est séchée sur sulfate de magnésium, filtrée et concentrée.

A une solution du résidu ainsi obtenu dans un mélange de dichlorométhane (10 mL) et de méthanol (5 mL), est additionné l'acide 1,3-diméthylbarbiturique (0,159 g; 1 mmol). Le milieu réactionnel est dégazé à l'argon pendant 10 minutes, et on ajoute le tétrakis-(triphénylphosphine)palladium(0) (0,030 g ; 0,02 mmol). Le mélange est ensuite agité pendant 16 heures à température ambiante. Après concentration des solvants, le résidu est purifié par chromatographie sur phase RP-18 en utilisant l'acétonitrile, l'eau et l'acétate d'ammonium comme éluants pour obtenir le produit du titre.

### Masse haute résolution (ESI+) :

Formule brute :

   C₃₆H₃₂ClN₅O₆
[M+H]+ calculé 666.2198,
[M+H]+ mesuré 666.2108.

### Exemple 37 : 10-fluoro-2-(4-hydroxyphényl)-5,6-diméthyl-24,27,30-trioxa-2,6,15,32,35-pentaazahexacyclo[29.2.2.1~4,7~.1~15,19~.0~8,13~.0~18,23~]heptatriaconta-1(33),4,7(37),8,10,12,18,20,22,31,34-undécaène-3,14-dione

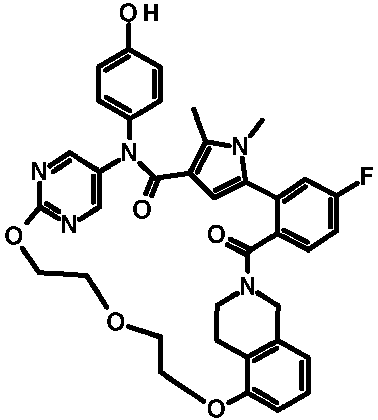

Le composé du titre est obtenu selon le procédé de l'Exemple 36 en utilisant l'acide de la Préparation 2b et l'amine de la Préparation 22b".

### Masse haute résolution (ESI+) :

Formule brute :

   C₃₇H₃₄FN₅O₆
[M+H]+ calculé 664.2559,
[M+H]+ mesuré 664.2571.

### Exemple 38 : 10-chloro-2-(4-hydroxyphényl)-5,6-diméthyl-24,27,30-trioxa-2,6,15,32,35-pentaazahexacyclo[29.2.2.1~4,7~.1~15,19~. 0~8,13~. 0~18,23~]heptatriaconta-1(33),4, 7(37),8,10,12,18,20,22,31,34-undécaène-3,14-dione

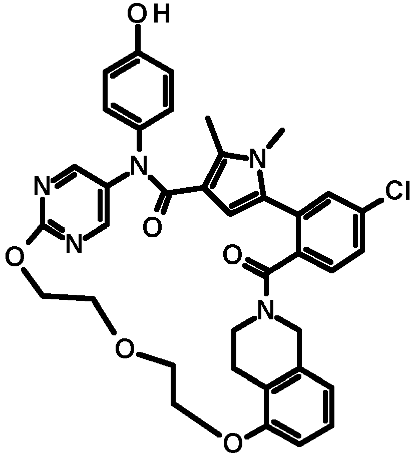

Le composé du titre est obtenu selon le procédé de l'Exemple 36 en utilisant l'acide de la Préparation 1b et l'amine de la Préparation 22b".

### Masse haute résolution (ESI+) :

Formule brute :

   C₃₇H₃₄ClN₅O₆
[M+H]+ calculé 680.2276,
[M+H]+ mesuré 680.2280.

### Exemple 39 : 10-chloro-2-(4-hydroxyphényl)-5,6-diméthyl-24,31-dioxa-2,6,15,33,36-pentaazahexacyclo[30.2.2.1~4,7~.1~15,1~.0~8,13~.0~18,23~]octatriaconta-1(34),4,7(38),8,10,12,18,20,22,32,35-undécaène-3,14-dione

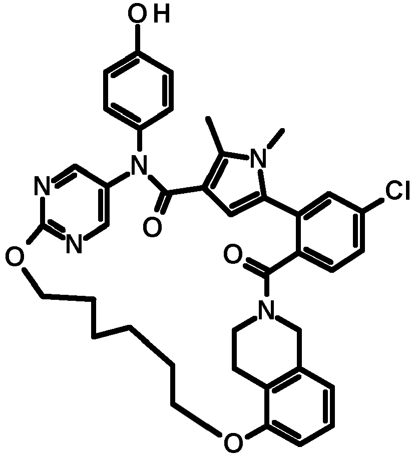

Le composé du titre est obtenu selon le procédé de l'Exemple 38 en utilisant l'amine de la Préparation 23b".

### Masse haute résolution (ESI+) :

Formule brute :

   C₃₉H₃₈ClN₅O₅
[M+H]+ calculé 692.2640,
[M+H]+ mesuré 692.2646.

### Exemple 40 : 10-chloro-2-(4-hydroxyphényl)-5,6-diméthyl-24,30-dioxa-2,6,15,32,35-pentaazahexacyclo[29.2.2.1~4,7~.1~15,19~.0~8,13~.0~18,23~]heptatriaconta-1(33),4,7(37),8,10,12,18,20,22,31,34-undécaène-3,14-dione

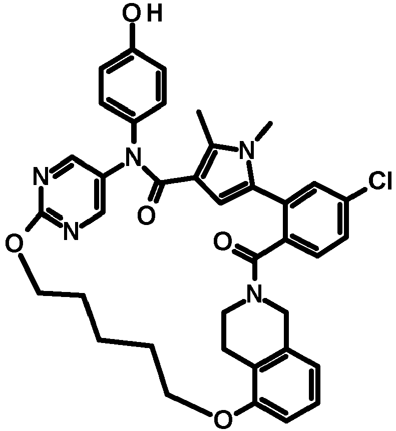

Le composé du titre est obtenu selon le procédé de l'Exemple 38 en utilisant l'amine de la Préparation 24b".

### Masse haute résolution (ESI+) :

Formule brute :

   C₃₈H₃₆ClN₅O₅
[M+H]+ calculé 678.2484,
[M+H]+ mesuré : 678.2488.

### Exemple 41 : 10-chloro-2-(4-hydroxyphényl)-5,6-diméthyl-24,29-dioxa-2,6,15,31,34-pentaazahexacyclo[28.2.2.1~4,7~.1~15,19~.0~8,13~.0~18,23~]hexatriaconta-1(32),4,7(36),8,10,12,18,20,22,30,33-undécaène-3,14-dione

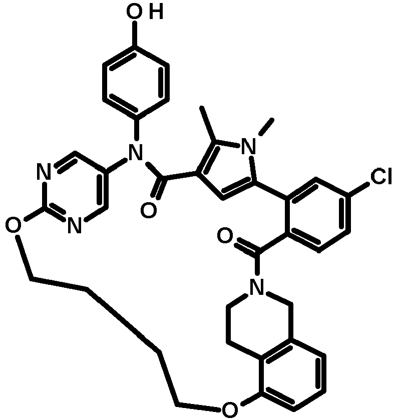

Le composé du titre est obtenu selon le procédé de l'Exemple 38 en utilisant l'amine de la Préparation 25b".

### Masse haute résolution (ESI+) :

Formule brute :

   C₃₇H₃₄ClN₅O₅
[M+H]+ calculé : 664.2328
[M+H]+ mesuré : 664.2331

### Exemple 42 : 10-chloro-2-(4-hydroxyphényl)-5,6-diméthyl-21,24,27-trioxa-2,6,15,29,32-pentaazapentacyclo[26.2.2.1~4,7~.1 ~15,19~.0~8,13~]tétratriaconta-1 (30),4, 7(34), 8,1 0,12,28,31-octaène-3,14-dione

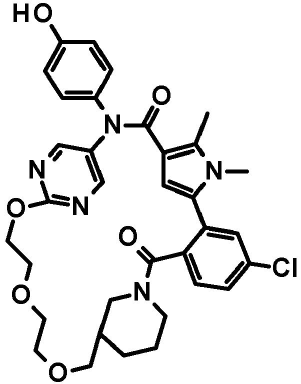

Le composé du titre est obtenu selon le procédé de l'Exemple 38 en utilisant l'amine de la Préparation 26b".

### Masse haute résolution (ESI+) :

Formule brute :

   C₃₄H₃₆ClN₅O₆
[M+H]+ calculé : 646.2433
[M+H]+ mesuré : 646.2427.

### Exemple 43 : 6-chloro-14-(4-hydroxyphényl)-10,11-diméthyl-19,22,25-trioxa-1,10,14,17,32-pentaazapentacyclo[25.2.2.2~15,18~.1~9,12~.0~3,8~]tétratriaconta-3,5,7,9(34),11,15,17,32-octaène-2,13-dione

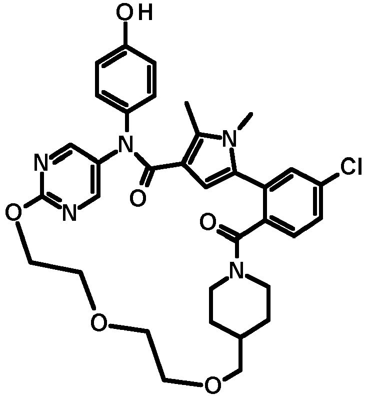

Le composé du titre est obtenu selon le procédé de l'Exemple 38 en utilisant l'amine de la Préparation 27b".

### Masse haute résolution (ESI+) :

Formule brute :

   C₃₄H₃₆ClN₅O₆
[M+H]+ calculé : 646.2433
[M+H]+ mesuré : 646.2426.

### Exemple 44 : 10-chloro-2-(4-hydroxyphényl)-5,6,27-triméthyl-24,30-dioxa-2,6,15,27,32,35-hexaazahexacyclo[29.2.2.1~4,7~.1~15,19~.0~8,13~.0~18,23~] heptatriaconta-1 (33), 4, 7(3 7),8,10,12,18,20,22,31,34-undécaène-3,14-dione

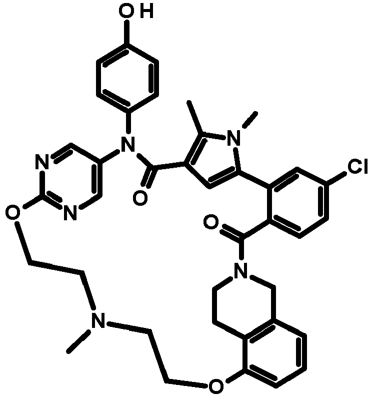

Le composé du titre est obtenu selon le procédé de l'Exemple 38 en utilisant l'amine de la Préparation 28b".

### Masse haute résolution (ESI+) :

Formule brute :

   C₃₈H₃₇ClN₆O₅
[M+H]+ calculé : 693.2593
[M+H]+ mesuré : 693.2583.

### ETUDE PHARMACOLOGIQUE

### EXEMPLE A: Inbibition de Bcl-2 par la technique de polarisation de fluorescence

Les essais de polarisation de fluorescence ont été réalisés en microplaques (384 puits). La protéine Bcl-2 étiquetée (histag-Bcl-2 tel que Bcl-2 correspond au numéro d'ordre primaire UniProtKB^{®} : P10415), à la concentration finale de 5×10⁻⁹ M, est mélangée avec un peptide fluorescent (Fluorescein-REIGAQLRRMADDLNAQY), à la concentration finale de 1,00x10⁻⁹M dans une solution tampon (Hepes 10 mM, NaCl 150 mM, Tween20 0,05%, pH 7,4), en présence ou en absence de concentrations croissantes de composés à tester. Après incubation de 2 heures, la polarisation de fluorescence est mesurée.

Les résultats sont exprimés en IC₅₀ (concentration en composé qui inhibe à 50 % la polarisation de fluorescence) et sont présentés dans le tableau 1 ci-dessous.

Les résultats montrent que les composés de l'invention inhibent l'interaction entre la protéine Bcl-2 et le peptide fluorescent décrit précédemment.

### EXEMPLE B : Cytotoxicité in vitro.

Les études de cytotoxicité ont été réalisées sur la lignée tumorale de leucémie RS4 ;11. Les cellules sont réparties dans des microplaques et exposées aux composés à tester pendant 48 heures. La viabilité cellulaire est ensuite quantifiée par un essai colorimétrique, le Microculture Tétrazolium Assay (Cancer Res., 1987, 47, 939-942).

Les résultats sont exprimés en IC₅₀ (concentration en composé qui inhibe à 50 % la viabilité cellulaire) et sont présentés dans le tableau 1 ci-dessous.

Les résultats montrent que les composés de l'invention sont cytotoxiques.

**Tableau 1 : IC₅₀ d'inhibition de Bcl-2 (test de polarisation de fluorescence) et de cytotoxicité pour les cellules RS4 ;11**

| | IC₅₀ (µM) Bcl-2 FP | IC₅₀ (M) MTT RS4;11 | | IC₅₀ (µM) Bcl-2 FP | IC₅₀ (M) MTT RS4;11 |
|---|---|---|---|---|---|
| **Exemple 1** | 0,0067 | 1,07E-07 | **Exemple 22** | 0,0153 | 1,18E-08 |
| **Exemple 2** | 0,0044 | 1,79E-07 | **Exemple 23** | 0,0143 | 7,33E-08 |
| **Exemple 3** | 0,0655 | 7,49E-07 | **Exemple 24** | 0,0149 | 1,06E-07 |
| **Exemple 4** | 0,0023 | 7,73E-09 | **Exemple 25** | 0,268 | 8,09E-07 |
| **Exemple 5** | 0,00205 | 2,14E-08 | **Exemple 26** | 0,0041 | 1,66E-09 |
| **Exemple 6** | 0,0052 | 2,66E-08 | **Exemple 27** | 0,0098 | 1,04E-07 |
| **Exemple 7** | 0,003 | 4,94E-09 | **Exemple 28** | 0,0288 | 4,01E-07 |
| **Exemple 8** | 0,00615 | 4,93E-08 | **Exemple 29** | not tested | 7,05E-08 |
| **Exemple 9** | 0,0023 | 5,24E-09 | **Exemple 30** | 0,0314 | >1,88E-06 |
| **Exemple 10** | 0,299 | >3E-07 | **Exemple 31** | 74,55% @1,1 µM | 1,33E-06 |
| **Exemple 11** | 0,157 | 4,48E-07 | **Exemple 34** | 0,0693 | 1,24E-06 |
| **Exemple 12** | not tested | not tested | **Exemple 36** | 41,5% @ 10 µM | >6E-07 |
| **Exemple 13** | 0,0332 | 2,39E-07 | **Exemple 37** | 0,0102 | 4,96E-08 |
| **Exemple 14** | 0,0147 | 1,46E-08 | **Exemple 38** | 0,00385 | 2,31E-08 |
| **Exemple 15** | 0,0037 | 1,16E-08 | **Exemple 39** | 0,0768 | 1,49E-07 |
| **Exemple 16** | 0,00555 | 5,33E-08 | **Exemple 40** | 0,0064 | 2,99E-08 |
| **Exemple 17** | 0,0123 | 6,28E-08 | **Exemple 41** | 0,0467 | 1,97E-07 |
| **Exemple 18** | 0,00365 | 8,36E-09 | **Exemple 42** | 39,2% @ 10 µM | >6E-07 |
| **Exemple 19** | 0,0055 | 1,03E-08 | **Exemple 43** | 34,8% @ 10 µM | >1,88E-06 |
| **Exemple 20** | 0,0123 | 8,12E-08 | **Exemple 44** | 0,00497 | 3,54E-08 |
| **Exemple 21** | 0,0148 | 1,31E-07 | | | |

### EXEMPLE C : Quantification de la forme clivée de la caspase 3 in vivo.

La capacité des composés de l'invention à activer la caspase 3 est évaluée dans un modèle de xénogreffe de cellules leucémiques RS4 ;11.

1.10⁷ cellules RS4 ; 11 sont greffées par voie sous-cutanée dans des souris immunodéprimées (souche SCID). 25 à 30 jours après la greffe, les animaux sont traités par voie orale par les différents composés. Après 2 heures de traitement, les masses tumorales sont récupérées, lysées et 1a forme clivée (activée) de la caspase 3 est quantifiée dans les lysats tumoraux.

Cette quantification est réalisée en utilisant l'essai « Meso Scale Discovery (MSD) ELISA platform » qui dose spécifiquement la forme clivée de la caspase 3. Elle est exprimée sous la forme d'un facteur d'activation correspondant au rapport entre la quantité de caspase 3 clivée chez les souris traitées divisée par la quantité de caspase 3 clivée chez les souris contrôles.

Les résultats montrent que les composés de l'invention sont capables d'induire l'apoptose dans les cellules tumorales RS4 ; 11 *in vivo.*

**Tableau 2 : Facteurs d'activation des caspases (caspase 3 clivée essai MSD dans les tumeurs des souris traitées versus souris contrôles) in vivo, après traitement par voie orale (doses précisées entre parenthèses)**

| Composé testé | Formulation | Dose (mg/kg) | Facteur d'activation ± SEM (versus contrôle) |
|---|---|---|---|
| Exemple 6 | PEG/EtOH/Water | 12,5 | 6,8 ± 4,2 |
| Exemple 6 | PEG/EtOH/Water | 25 | 32,3 ± 9,1 |
| Exemple 16 | PEG/EtOH/Water | 25 | 31,7 ± 2,9 |
| Exemple 16 | PEG/EtOH/Water | 50 | 75,1 ± 13,2 |
| Exemple 18 | PEG/EtOH/Phosal | 6,25 | 7,8 ± 2,5 |
| Exemple 18 | PEG/EtOH/Phosal | 12,5 | 31,7 ± 6,0 |
| Exemple 29 | PEG/EtOH/Phosal | 25 | 13,3 ± 6,3 |
| Exemple 29 | PEG/EtOH/Phosal | 50 | 54,4 ± 9,7 |

### EXEMPLE D : Composition pharmaceutique : Comprimés

| | |
|---|---|
| 1000 comprimés dosés à 5 mg d'un composé choisi parmi les exemples 1 à 44 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 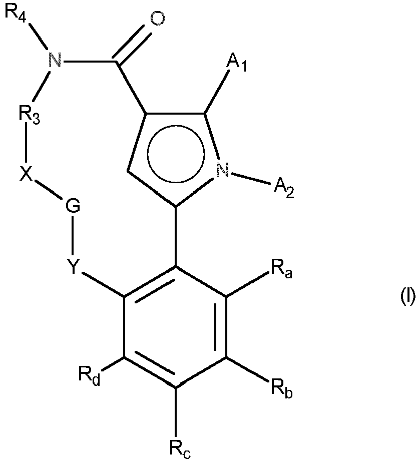 dans laquelle :
◆ A₁ et A₂ représentent chacun un groupement méthyle,
◆ G représente un groupe -NR₇-, un groupe 1,2,3,4-tétrahydroisoquinoléinylène éventuellement substitué par un groupement T, un groupe 2,3-dihydro-1*H-*isoindolylène éventuellement substitué par un groupement T, ou un groupe pipéridinylène,
◆ T représente un atome d'hydrogène, un alkyle (C₁-C₆) linéaire ou ramifié éventuellement substitué par un à trois atomes d'halogène, un groupement alkyl(C₁-C₄)-NR₁R₂, ou un groupement alkyl(C₁-C₄)-OR₆,
◆ X représente un groupe (C₂-C₈)alkylène, dont 1 à 3 chaînons peuvent être remplacés par un hétéroatome choisi parmi oxygène, soufre et N-R₅, ou par un groupe arylène ou hétéroarylène,
◆ Y représente un groupe -CH₂- ou -CO-,
◆ R₁ et R₂ représentent indépendamment l'un de l'autre un atome d'hydrogène ou un groupement alkyle (C₁-C₆) linéaire ou ramifié,
ou bien R₁ et R₂ forment avec l'atome d'azote qui les porte un hétérocycloalkyle,
◆ R₃ et R₄ sont tels que :
- l'un d'entre eux représente un groupement phényl de formule suivante: 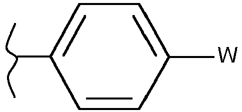 dans lequel W représente un groupe hydroxy ou un groupe phosphate choisi parmi - OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃), et -OPO(O⁻M₁⁺)(O[CH₂CH₂O]ₙCH₃), dans lesquels M et M' représentent indépendamment l'un de l'autre un atome d'hydrogène, un groupement alkyle (C₁-C₆) linéaire ou ramifié, un groupement alkényle (C₂-C₆) linéaire ou ramifié, un groupement alkynyle (C₂-C₆) linéaire ou ramifié, un cycloalkyle ou un hétérocycloalkyle, tous deux constitués de 5 à 6 chaînons, tandis que M₁⁺ et M₂⁺ représentent indépendamment l'un de l'autre un cation monovalent pharmaceutiquement acceptable, M₃²⁺ représente un cation divalent pharmaceutiquement acceptable et n est un nombre entier compris entre 1 et 5,
- tandis que l'autre représente un groupement aryle, hétéroaryle, hétérocycloalkyle, cycloalkyle ou alkyle (C₁-C₆) linéaire ou ramifié, étant entendu qu'un ou plusieurs atomes de carbone des groupements précédents, ou de leurs éventuels substituants, peut(vent) être deutéré(s),
◆ R₅ représente un atome d'hydrogène ou un groupement alkyle (C₁-C₆) linéaire ou ramifié,
◆ R₆ et R₇ représentent indépendamment les uns des autres un atome d'hydrogène ou un groupement alkyle (C₁-C₆) linéaire ou ramifié,
◆ Rₐ, R_{c} et R_{d} représentent chacun un atome d'hydrogène et R_{b} représente un atome d'hydrogène ou d'halogène
étant entendu que :
- par "aryle", on entend un groupement phényle, naphtyle, biphényle ou indényle,
- par "hétéroaryle", on entend tout groupement mono ou bi-cyclique constitué de 5 à 10 chaînons, possédant au moins une partie aromatique, et contenant de 1 à 4 hétéroatomes choisis parmi oxygène, soufre, azote et azote quaternaire,
- par "cycloalkyle", on entend tout groupement carbocyclique non aromatique, mono ou bi-cyclique, contenant 3 à 10 chaînons,
- par "hétérocycloalkyle", on entend tout groupement non aromatique mono ou bi-cyclique, fusionné ou spiro, constitué de 3 à 10 chaînons, et contenant de 1 à 3 hétéroatomes choisis parmi oxygène, soufre, SO, SO₂ ou azote,
- par arylène, hétéroarylène, 1,2,3,4-tétrahydroisoquinoléinylène, 2,3-dihydro-1*H-*isoindolylène ou pipéridinylène, on entend un groupe aryl, hétéroaryl, 1,2,3,4-tétrahydroisoquinoléine ou pipéridine divalent,
les groupements aryle, hétéroaryle, cycloalkyle et hétérocycloalkyle ainsi définis et les groupements alkyle, alkényle, alkynyle, alkoxy, pouvant être substitués par 1 à 3 groupements choisis parmi : alkyle (C₁-C₆) linéaire ou ramifié éventuellement substitué par un groupe hydroxy, morpholinyl, 3-3-difluoropiperidinyl ou 3-3-difluoropyrrolidinyl ; spiro (C₃-C₆) ; alkoxy (C₁-C₆) linéaire ou ramifié éventuellement substitué par un groupe morpholinyl ; (C₁-C₆)alkyl-S- ; hydroxy ; oxo ; *N*-oxyde ; nitro ; cyano ; -COOR' ; -OCOR' ; NR'R" ; polyhalogénoalkyle (C₁-C₆) linéaire ou ramifié ; trifluorométhoxy ; (C₁-C₆)alkylsulfonyl ; halogène ; aryle éventuellement substitué par un ou plusieurs atomes d'halogène ; hétéroaryle ; aryloxy ; arylthio ; cycloalkyle ; hétérocycloalkyle éventuellement substitué par un ou plusieurs atomes d'halogène ou groupements alkyles (C₁-C₆) linéaires ou ramifiés ; étant entendu que R' et R" représentent, indépendamment l'un de l'autre, un atome d'hydrogène ou un groupement alkyle (C₁-C₆) linéaire ou ramifié éventuellement substitué par un groupe méthoxy,
ses énantiomères, diastéréoisomères, ou ses sels d'addition à un acide ou à une base pharmaceutiquement acceptable.

2. Composé de formule (I) selon la revendication 1 dans lequel Y représente un groupe -CO-.

3. Composé de formule (I) selon la revendication 1 ou 2 dans lequel G représente un groupe choisi parmi les groupes suivants: 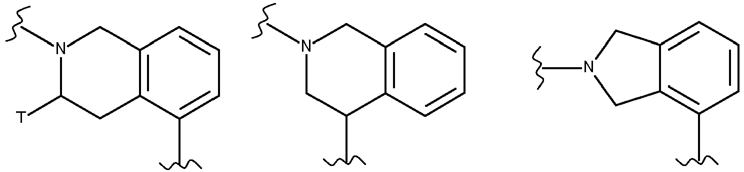 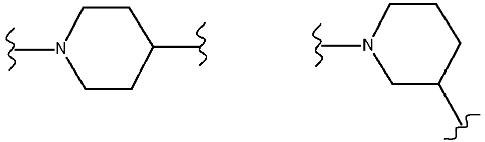 où T représente un groupe méthyle ou un groupe (4-morpholinyl)méthyle.

4. Composé de formule (I) selon l'une des revendications 1 à 3 dans lequel X représente un groupe choisi parmi les groupes suivants :

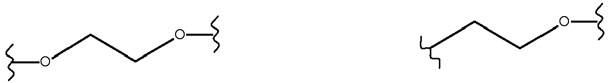

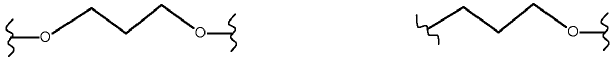

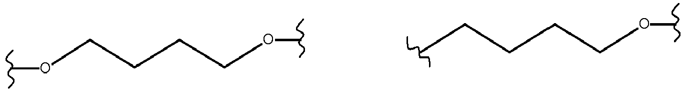

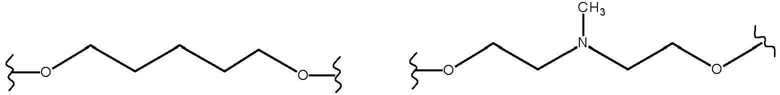

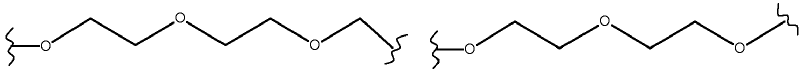

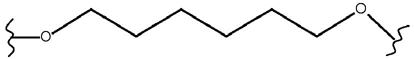

5. Composé de formule (I) selon l'une des revendications 1 à 4 dans lequel l'un des groupes R₃ ou R₄ représente un groupe 4-hydroxyphényl tandis que l'autre représente un groupe choisi dans la liste suivante :
- un groupe phényl éventuellement substituté par un groupement cyano,
- un groupe pyrazolyl,
- un groupe 1-méthyl-1*H*-pyrazolyl,
- un groupe 1-(tétrahydrofuran-3-yl)-1*H*-pyrazolyl,
- un groupe 5-méthyl-2-cyano-1*H*-pyrrolyl,
- un groupe 1-méthyl-2-cyano-1*H*-pyrrolyl,
- un groupe 1,2-diméthyl-1*H*-pyrrolyl,
- un groupe 1,5-diméthyl-2-cyano-1*H*-pyrrolyl,
- un groupe pyrimidinyl,
- un groupe éthyl,
- un groupe pyridinium.

6. Composé de formule (I) selon la revendication 5 dans lequel R₄ représente un groupe 4-hydroxyphényl.

7. Composé de formule (I) selon la revendication 5 dans lequel R₃ représente un groupe 4-hydroxyphényl.

8. Composé de formule (I) selon la revendication 7 dans lequel R₃ représente un groupe 4-hydroxyphényl et G représente un groupe pipéridinylène.

9. Composé de formule (I) selon la revendication 1 choisi parmi le groupe suivant :
- 6-chloro-14-(4-hydroxyphényl)-10,11-diméthyl-2,13-dioxo-20,23-dioxa-1,10,14-triazahexacyclo[26.3.1.1~9,12~.1~15,19~.0~3,8~.0~24,29~]tétratriaconta-3,5,7,9(34), 11,15(33), 16,18,24,26,28-undécaène-17-carbonitrile,
- 11-chloro-4-(4-hydroxyphényl)-1,7,8-triméthyl-4,16,17,23,24,25-hexahydro-1H, 14H-15, 18-méthano-6,9-(méthéno)dibenzo[b,h]pyrazolo[4,3-p][1,6,11,15]oxatriazacycloicosine-5,14(8H)-dione,
- 6-chloro-14-(4-hydroxyphényl)-10,11-diméthyl-2,13-dioxo-23-oxa-1,10,14-triazahexacyclo[26.3.1.1~9,12~1~15,19~0~3,8~0~24,29~]tétratriaconta-3,5,7,9(34), 11,15(33), 16,18,24,26,28-undécaène-17-carbonitrile,
- 11-chloro-4-(4-hydroxyphényl)-1,7,8-triméthyl-5,14-dioxo-4,5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile,
- 11-chloro-4-(4-hydroxyphényl)-1,7,8-tiiméthyl-5,14-dioxo-1,4,5,8,16,17,23,24-octahydro-14H-15,18-méthano-6,9-(méthéno)dibenzo[l,r]pyrrolo[2,3-d][1,6,10,15]oxatriazacyclononadécine-2-carbonitrile,
- 11-chloro-4-(4-hydroxyphényl)-1,7,8-triméthyl-1,4,16,21,22,23-hexahydro-14H-15,21-méthano-6,9-(méthéno)dibenzo[j,o]pyrazolo[3,4-b][1,4,8,13]oxatriazacyclononadécine-5,14(8H)-dione,
- (16S ou R)-11-chloro-4-(4-hydroxyphényl)-1,7,8,16-tétraméthyl-5,14-dioxo-4,5,8,16,17,23,24,25-octahydro-1H,14H-15,18-méthano-6,9-(méthéno)dibenzo[b,h]pyrrolo[3,2-p][1,6,11,15]oxatriazacycloicosine-2-carbonitrile,
- 4-[16-chloro-3-hydroxy-19,20-diméthyl-13,22-dioxo-6,7,8,9,10,11,19,22-octahydro-13H,23H-8,12-méthano-21,18-(méthéno)dibenzo[b,j][1,4,8,13]oxatriazacyclononadécin-23-yl]-1,5-diméthyl-1H-pyrrole-2-carbonitrile,
- 10-fluoro-2-(4-hydroxyphényl)-5,6-diméthyl-24,27,30-trioxa-2,6,15,32,35-pentaazahexacyclo[29.2.2.1~4,7~.1~15,19~.0~8,13~.0~18,23~]heptatriaconta-1(33),4,7(37),8,10,12,18,20,22,31,34-undécaène-3,14-dione,
- 10-chloro-2-(4-hydroxyphényl)-5,6-diméthyl-24,30-dioxa-2,6,15,32,35-pentaazahexacyclo[29.2.2.1~4,7~.1~15,19~.0~8,13~.0~18,23~]heptatriaconta-1(33),4,7(37),8,10,12,18,20,22,31,34-undécaène-3,14-dione,
- 10-chloro-2-(4-hydroxyphényl)-5,6,27-triméthyl-24,30-dioxa-2,6,15,27,32,35-hexaazahexacyclo[29.2.2.1~4,7~.1~15,19~.0~8,13~.0~18,23~]heptatriaconta-1(33),4,7(37),8,10,12,18,20,22,31,34-undécaène-3,14-dione,
- 11 -chloro-4-(4-hydroxyphényl)- 1,7,8-triméthyl- 1,4,16,17,23,24-hexahydro- 14H-15,18-méthano-6,9-(méthéno)dibenzo[l,r]pyrazolo[3,4-d][1,6,10,15]oxatriazacyclononadécine-5,14(8H)-dione.

10. Procédé de préparation d'un composé de formule (I) selon la revendication 1 **caractérisé en ce que** l'on utilise comme produit de départ le composé de formule (II): 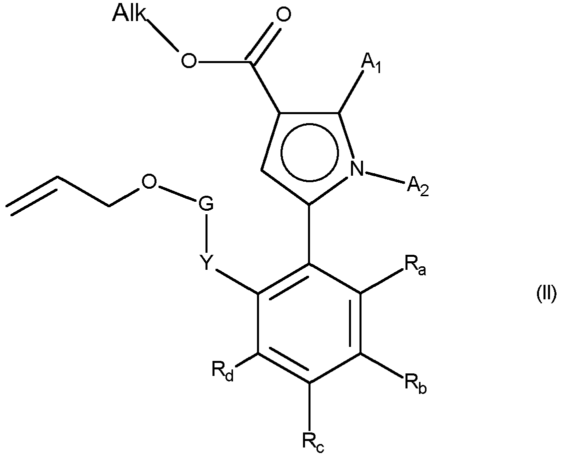
dans lequel A₁, A₂, Rₐ, R_{b}, R_{c}, Rₐ, Y et G ont la même signification que dans la formule (I) définie dans la revendication 1, et Alk représente un groupe (C₁-C₆)alkyl linéaire ou ramifié,
composé de formule (II) dont la fonction ester -OAlk est hydrolysée pour conduire à l'acide carboxylique ou au carboxylate correspondant, lequel peut être converti en le chlorure d'acyle ou l'anhydride correspondant, avant d'être couplé avec une amine NHR_{3A}R₄, dans laquelle R₄ a la même signification que dans la formule (I), et R_{3A} représente :
- un groupement R₃ tel que défini dans la formule (I)
- ou un groupement R₃-O-Alk'-Z, R₃-Alk'-Z ou R₃-Z dans lequel Alk' représente un groupe (C₁-C₆)alkyl linéaire ou ramifié, et Z représente un atome d'halogène ou un groupe -OH,
pour former le composé de formule (III) : 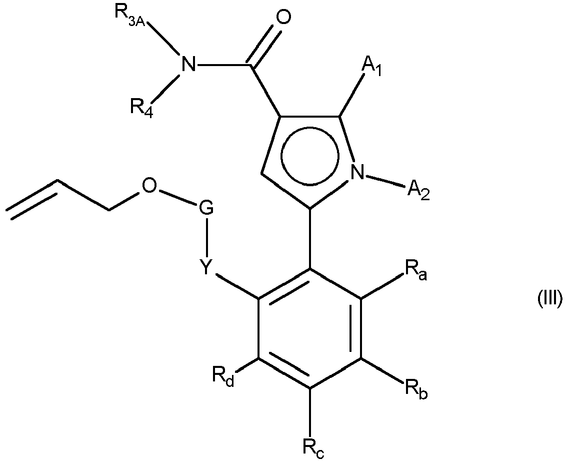
dans lequel A₁, A₂, Rₐ, R_{b}, R_{c}, Rₐ, R₄, Y et G ont la même signification que dans la formule (I),
lequel est soumis à une réaction de déprotection de la fonction alcool, suivie soit d'une substitution nucléophile intramoléculaire, soit d'une réaction de Mitsunobu, ou bien d'une substitution nucléophile aromatique,
pour conduire au composé de formule (I),
composé de formule (I) qui peut être purifié selon une technique classique de séparation, qui peut être transformé en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, les groupements hydroxy et amino des réactifs ou intermédiaires de synthèse peuvent être protégés puis déprotégés pour les besoins de la synthèse.

11. Procédé de préparation d'un composé de formule (I) selon la revendication 1 **caractérisé en ce que** l'on utilise comme produit de départ le composé de formule (IV): 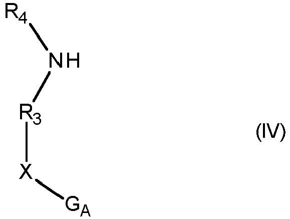
dans lequel R₃, R₄ et X ont la même signification que dans la formule (I), et G_{A} représente un groupe choisi parmi la liste suivante : 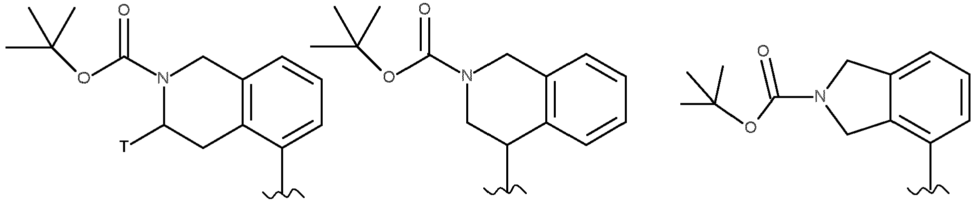 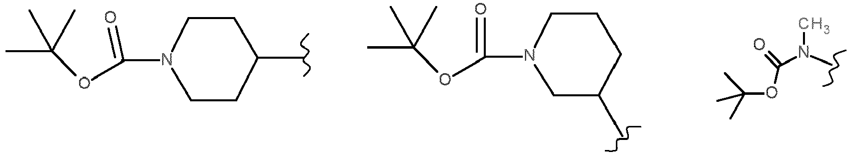
composé de formule (IV) qui est ensuite couplé à un composé de formule (V) : 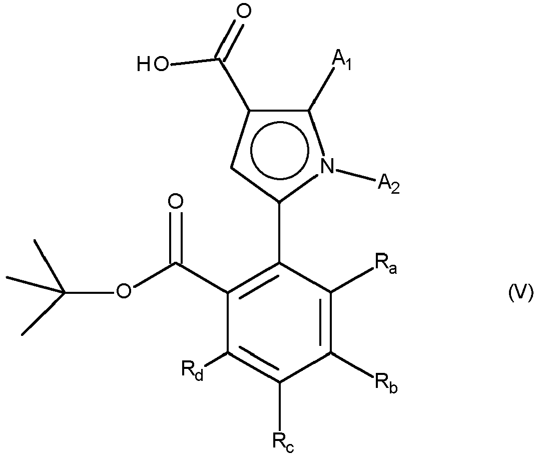
dans lequel A₁, A₂, Rₐ, R_{b}, R_{c}, et R_{d} ont la même signification que dans la formule (I), pour conduire au composé de formule (VI) : 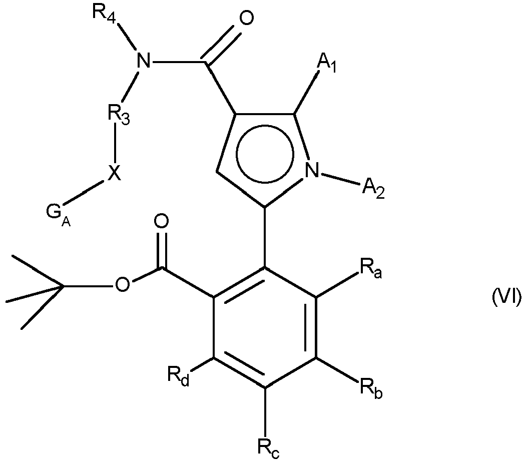
dans lequel A₁, A₂, Rₐ, R_{b}, R_{c}, R_{d}, R₃, R₄ et X ont la même signification que dans la formule (I),
lequel est soumis à une réaction de déprotection suivie d'un couplage intramoléculaire pour conduire au composé de formule (I),
composé de formule (I) qui peut être purifié selon une technique classique de séparation, que peut être transformé en ses sels d'addition à un acide ou à une base pharmaceutiquement acceptable et dont on sépare éventuellement les isomères selon une technique classique de séparation,
étant entendu qu'à tout moment jugé opportun au cours du procédé précédemment décrit, les groupements hydroxy et amino des réactifs ou intermédiaires de synthèse peuvent être protégés puis déprotégés pour les besoins de la synthèse.

12. Composition pharmaceutique contenant un composé de formule (I) selon l'une quelconque des revendications 1 à 9, ou un de ses sels d'addition avec un acide ou une base pharmaceutiquement acceptable, en combinaison avec un ou plusieurs excipients pharmaceutiquement acceptables.

13. Composition pharmaceutique selon la revendication 12 pour son utilisation en tant qu'agent pro-apoptotique.

14. Composition pharmaceutique selon la revendication 12 pour son utilisation dans le traitement des cancers, des maladies auto-immunes et du système immunitaire.

15. Composition pharmaceutique selon la revendication 14 dans laquelle le cancer est choisi parmi la liste suivante : cancer de la vessie, du cerveau, du sein, de l'utérus, leucémie lymphoïde chronique, cancer colorectal, cancer de l'oesophage, du foie, leucémie lymphoblastique, lymphome non hodgkinien, mélanome, hémopathie maligne, myélome, cancer de l'ovaire, cancer du poumon non à petites cellules, cancer de la prostate et cancer du poumon à petites cellules.

16. Utilisation d'un composé de formule (I) selon l'une des revendications 1 à 9 pour la fabrication d'un médicament utile en tant qu'agent pro-apoptotique.

17. Utilisation d'un composé de formule (I) selon l'une des revendications 1 à 9 pour la fabrication d'un médicament destiné au traitement des cancers, des maladies immunitaires et auto-immunes.

18. Utilisation d'un composé de formule (I) selon la revendication 17 dans laquelle le cancer est choisi parmi la liste suivante : cancer de la vessie, du cerveau, du sein, de l'utérus, leucémie lymphoïde chronique, cancer colorectal, cancer de l' oesophage, du foie, leucémie lymphoblastique, lymphome non hodgkinien, mélanome, hémopathie maligne, myélome, cancer de l'ovaire, cancer du poumon non à petites cellules, cancer de la prostate et cancer du poumon à petites cellules.

19. Association d'un composé de formule (I) selon l'une quelconque des revendications 1 à 9 avec un agent anticancéreux choisi parmi les agents génotoxiques, les poisons mitotiques, les anti-métabolites, les inhibiteurs du protéasome, les inhibiteurs de kinases ou les anticorps.

20. Composition pharmaceutique contenant une association selon la revendication 19 en combinaison avec un ou plusieurs excipients pharmaceutiquement acceptables.

21. Association selon la revendication 19 pour son utilisation dans le traitement des cancers.

22. Utilisation d'une association selon la revendication 19 pour la fabrication d'un médicament utile dans le traitement des cancers.

23. Composé de formule (I) selon l'une quelconque des revendications 1 à 9 pour son utilisation en association avec une radiothérapie dans le traitement des cancers.

## Patentansprüche

1. Verbindung der Formel (I): 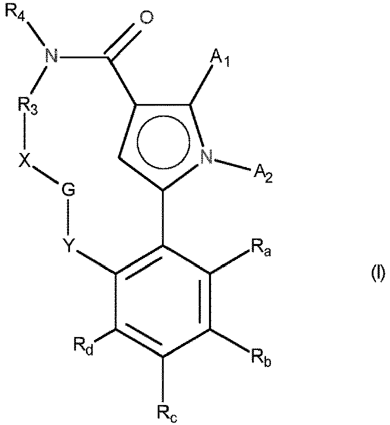 worin:
◆ A₁ und A₂ jeweils eine Methylgruppe darstellen,
◆ G eine -NR₇-Gruppe, eine 1,2,3,4-Tetrahydroisochinolinylengruppe, die gegebenenfalls durch eine Gruppe T substituiert ist, eine 2,3-Dihydro-1*H*-isoindolylengruppe, die gegebenenfalls durch eine Gruppe T substituiert ist, oder eine Piperidinylengruppe darstellt,
◆ T ein Wasserstoffatom, ein lineares oder verzweigtes (C₁-C₆)-Alkyl, das gegebenenfalls durch ein bis drei Halogenatome substituiert ist, eine Gruppe (C₁-C₄)-Alkyl-NR₁R₂ oder eine Gruppe (C₁-C₄)-Alkyl-OR₆ darstellt,
◆ X eine (C₂-C₈)-Alkylengruppe darstellt, von der 1 bis 3 Mitglieder durch ein Heteroatom, ausgewählt aus Sauerstoff, Schwefel und N-R₅, oder durch eine Arylen- oder Heteroarylengruppe ersetzt sein können,
◆ Y eine Gruppe -CH₂- oder -CO- darstellt,
◆ R₁ und R₂ unabhängig voneinander ein Wasserstoffatom oder eine lineare oder verzweigte (C₁-C₆)-Alkylgruppe darstellen,
oder alternativ R₁ und R₂ zusammen mit dem sie tragenden Stickstoffatom ein Heterocycloalkyl bilden,
◆ R₃ und R₄ so beschaffen sind, dass:
- einer von ihnen eine Phenylgruppe der folgenden Formel darstellt: 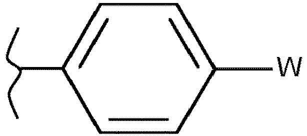 worin W eine Hydroxygruppe oder eine Phosphatgruppe, ausgewählt aus -OPO(OM)(OM'), -OPO(OM)(O⁻M₁⁺), -OPO(O⁻M₁⁺)(O⁻M₂⁺), -OPO(O⁻)(O⁻)M₃²⁺, -OPO(OM)(O[CH₂CH₂O]ₙCH₃) und -OPO(O⁻M₁⁺)(O[CH₂CH₂O]ₙCH₃), darstellt, wobei M und M' unabhängig voneinander ein Wasserstoffatom, eine lineare oder verzweigte (C₁-C₆)-Alkylgruppe, eine lineare oder verzweigte (C₂-C₆)-Alkenylgruppe, eine lineare oder verzweigte (C₂-C₆)-Alkinylgruppe, ein Cycloalkyl oder ein Heterocycloalkyl darstellen, wobei beide aus 5 bis 6 Mitgliedern bestehen, wohingegen M₁⁺ und M₂⁺ unabhängig voneinander ein pharmazeutisch verträgliches einwertiges Kation darstellen, M₃²⁺ ein pharmazeutisch verträgliches zweiwertiges Kation darstellt und n eine ganze Zahl zwischen 1 und 5 ist,
- wohingegen der andere eine Aryl-, Heteroaryl-, Heterocycloalkyl-, Cycloalkylgruppe oder eine lineare oder verzweigte (C₁-C₆)-Alkylgruppe darstellt, mit der Maßgabe, dass ein oder mehrere Kohlenstoffatome der vorstehenden Gruppen oder ihrer möglichen Substituenten deuteriert sein kann/können,
◆ R₅ ein Wasserstoffatom oder eine lineare oder verzweigte (C₁-C₆)-Alkylgruppe darstellt,
◆ R₆ und R₇ unabhängig voneinander ein Wasserstoffatom oder eine lineare oder verzweigte (C₁-C₆)-Alkylgruppe darstellen,
◆ Rₐ, R_{c} und R_{d} jeweils ein Wasserstoffatom darstellen und R_{b} ein Wasserstoff- oder Halogenatom darstellt
mit der Maßgabe, dass:
- unter "Aryl" eine Phenyl-, Naphthyl-, Biphenyl- oder Indenylgruppe zu verstehen ist
- unter "Heteroaryl" irgendeine mono- oder bizyklische Gruppe, die aus 5 bis 10 Mitgliedern besteht, mindestens einen aromatischen Teil aufweist und 1 bis 4 Heteroatome, ausgewählt aus Sauerstoff, Schwefel, Stickstoff und quartärem Stickstoff, enthält, zu verstehen ist,
- unter "Cycloalkyl" irgendeine carbozyklische nichtaromatische, mono- oder bizyklische Gruppe, die 3 bis 10 Mitglieder enthält, zu verstehen ist,
- unter "Heterocycloalkyl" irgendeine nichtaromatische mono- oder bizyklische, kondensierte oder Spirogruppe, die aus 3 bis 10 Mitgliedern besteht und 1 bis 3 Heteroatome, ausgewählt aus Sauerstoff, Schwefel, SO, SO₂ oder Stickstoff, enthält, zu verstehen ist,
- unter Arylen, Heteroarylen, 1,2,3,4-Tetrahydroisochinolinylen, 2,3-Dihydro-1*H*-isoindolylen oder Piperidinylen eine zweiwertige Aryl-, Heteroaryl-, 1,2,3,4-Tetrahydroisochinolinylen- oder Piperidingruppe zu verstehen ist,
wobei die so definierten Aryl-, Heteroaryl-, Cycloalkyl- und Heterocycloalkylgruppen und die Alkyl-, Alkenyl-, Alkinyl-, Alkoxygruppen, durch 1 bis 3 Gruppen substituiert sein können, ausgewählt aus: linearem oder verzweigtem (C₁-C₆)-Alkyl, das gegebenenfalls substituiert ist durch eine Hydroxy-, Morpholinyl-, 3-3-Difluorpiperidinyl- oder 3-3-Difluorpyrrolidinylgruppe; Spiro-(C₃-C₆); linearem oder verzweigtem (C₁-C₆)-Alkoxy, das gegebenenfalls substituiert ist durch eine Morpholinylgruppe; (C₁-C₆)-Alkyl-S-; Hydroxy; Oxo; N-Oxid; Nitro; Cyano; -COOR'; -OCOR'; NR'R"; linearem oder verzweigtem (C₁-C₆)-Polyhalogenalkyl; Trifluormethoxy; (C₁-C₆)-Alkylsulfonyl; Halogen; Aryl, das gegebenenfalls substituiert ist durch ein oder mehrere Halogenatome; Heteroaryl; Aryloxy; Arylthio; Cycloalkyl; Heterocycloalkyl, das gegebenenfalls substituiert ist durch ein oder mehrere Halogenatome oder lineare oder verzweigte (C₁-C₆)-Alkylgruppen; mit der Maßgabe, dass R' und R" unabhängig voneinander ein Wasserstoffatom oder eine lineare oder verzweigte (C₁-C₆)-Alkylgruppe darstellen, die gegebenenfalls durch eine Methoxygruppe substituiert ist,
ihre Enantiomere, Diastereoisomere oder ihre Additionssalze mit einer pharmazeutisch verträglichen Säure oder Base.

2. Verbindung der Formel (I) nach Anspruch 1, worin Y eine -CO-Gruppe darstellt.

3. Verbindung der Formel (I) nach Anspruch 1 oder 2, worin G eine Gruppe, ausgewählt aus den folgenden Gruppen, darstellt: 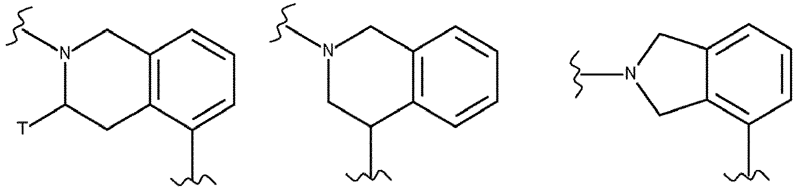 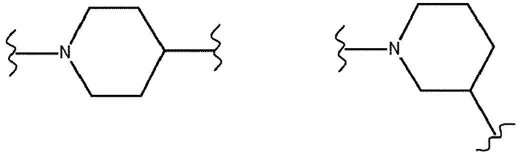 worin T eine Methylgruppe oder eine (4-Morpholinyl)methylgruppe darstellt.

4. Verbindung der Formel (I) nach einem der Ansprüche 1 bis 3, worin X eine Gruppe, ausgewählt aus den folgenden Gruppen, darstellt:

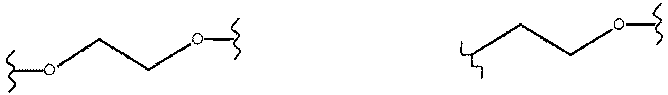

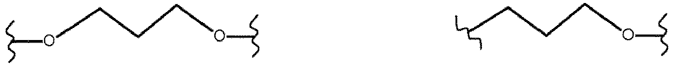

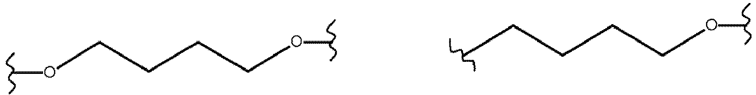

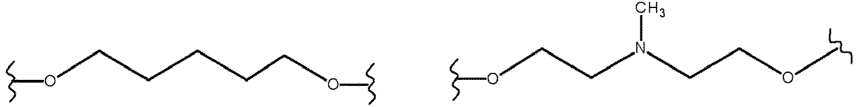

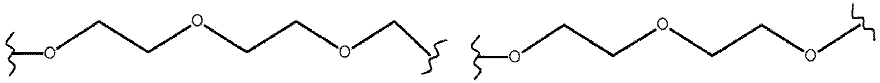

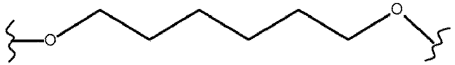

5. Verbindung der Formel (I) nach einem der Ansprüche 1 bis 4, worin eine der Gruppen R₃ oder R₄ eine 4-Hydroxyphenylgruppe darstellt, wohingegen die andere eine aus der folgenden Liste ausgewählte Gruppe darstellt:
- eine gegebenenfalls durch eine Cyanogruppe substituierte Phenylgruppe,
- eine Pyrazolylgruppe,
- eine 1-Methyl-1*H*-pyrazolylgruppe,
- eine 1-(Tetrahydrofuran-3-yl)-1*H*-pyrazolylgruppe,
- eine 5-Methyl-2-cyano-1*H*-pyrrolylgruppe,
- eine 1-Methyl-2-cyano-1*H*-pyrrolylgruppe,
- eine 1,2-Dimethyl-1*H*-pyrrolylgruppe,
- eine 1,5-Dimethyl-2-cyano-1*H*-pyrrolylgruppe,
- eine Pyrimidinylgruppe,
- eine Ethylgruppe,
- eine Pyridiniumgruppe.

6. Verbindung der Formel (I) nach Anspruch 5, worin R₄ eine 4-Hydroxyphenylgruppe darstellt.

7. Verbindung der Formel (I) nach Anspruch 5, worin R₃ eine 4-Hydroxyphenylgruppe darstellt.

8. Verbindung der Formel (I) nach Anspruch 7, worin R₃ eine 4-Hydroxyphenylgruppe darstellt und G eine Piperidinylengruppe darstellt.

9. Verbindung der Formel (I) nach Anspruch 1, ausgewählt aus der folgenden Gruppe:
- 6-Chlor-14-(4-hydroxyphenyl)-10,11-dimethyl-2,13-dioxo-20,23-dioxa-1,10,14-triazahexacyclo[26.3.1.1~9,12~.1~15,19~.0~3,8~.0~24,29~]tetratriaconta-3,5,7,9(34),11,15(33),16,18,24,26,28-undecaen-17-carbonitril,
- 11-Chlor-4-(4-hydroxyphenyl)-1,7,8-trimethyl-4,16,17,23,24,25-hexahydro-1H,14H-15,18-methano-6,9-(metheno)dibenzo[b,h]pyrazolo[4,3-p][1,6,11,15]oxatriazacycloicosin-5,14(8H)-dion,
- 6-Chlor-14-(4-hydroxyphenyl)-10,11-dimethyl -2,13-dioxo-23-oxa-1,10,14-triazahexacyclo[26.3.1.1~9,12~.1~15,19~.0~3,8~.0~24,29~]tetratriaconta-3,5,7,9(34),11,15(33),16,18,24,26,28-undecaen-17-carbonitril,
- 11-Chlor-4-(4-hydroxyphenyl)-1,7,8-trimethyl-5,14-dioxo-4,5,8,16,17,23,24,25-octahydro-1H,14H-15,18-methano-6,9-(metheno)dibenzo[b,h]pyrrolo[3,2-p][1,6,11,15]oxatriazacycloicosin-2-carbonitril,
- 11-Chlor-4-(4-hydroxyphenyl)-1,7,8-trimethyl-5,14-dioxo-1,4,5,8,16,17,23,24-octahydro-14H-15,18-methano-6,9-(metheno)dibenzo[1,r]pyrrolo[2,3-d][1,6,10,15]oxatriazacyclononadecin-2-carbonitril,
- 11-Chlor-4-(4-hydroxyphenyl)-1,7,8-trimethyl-1,4,16,21,22,23-hexahydro-14H-15,21-methano-6,9-(metheno)dibenzo[j,o]pyrazolo[3,4-b][1,4,8,13]oxatriazacyclononadecin-5,14(8H)-dion,
- (16S oder R)-11-Chlor-4-(4-hydroxyphenyl)-1,7,8,16-tetramethyl-5,14-dioxo-4,5,8,16,17,23,24,25-octahydro-1H,14H-15,18-methano-6,9-(metheno)dibenzo[b,h]pyrrolo[3,2-p][1,6,11,15]oxatriazacycloicosin-2-carbonitril,
- 4-[16-Chlor-3-hydroxy-19,20-dimethyl-13,22-dioxo-6,7,8,9,10,11,19,22-octahydro-13H,23H-8,12-methano-21,18-(metheno)dibenzo[b,j][1,4,8,13]oxatriazacyclononadecin-23-y1]-1, 5-dimethyl-1H-pyrrol-2-carbonitril,
- 10-Fluor-2-(4-hydroxyphenyl)-5,6-dimethyl-24,27,30-trioxa-2,6,15,32,35-pentaazahexacyclo[29.2.2.1~4,7~.1~15,19~.0~8,13~.0~18,23~]heptatriacon ta-1 (3 3),4,7(3 7), 8,10,12,18,20,22,31,34-undecaen-3,14-dion,
- 10-Chlor-2-(4-hydroxyphenyl)-5,6-dimethyl-24,30-dioxa-2,6,15,32,35-pentaazahexacyclo[29.2.2.1~4,7~.1~15,19~.0~8,13~.0~18,23~]heptatriacon ta-1 (3 3),4,7(3 7), 8,10,12,18,20,22,31,34-undecaen-3,14-dion,
- 10-Chlor-2-(4-hydroxyphenyl)-5,6,27-trimethyl-24,30-dioxa-2,6,15,27,32,35-hexaazahexacyclo[29.2.2.1~4,7~.1~15,19~.0~8,13~.0~18,23~]heptatriacont a-1(33),4,7(37),8,10,12,18,20,22,31,34-undecaen-3,14-dion,
- 11-Chlor-4-(4-hydroxyphenyl)-1,7,8-trimethyl-1,4,16,17,23,24-hexahydro-14H-15,18-methano-6,9-(metheno)dibenzo[1,r]pyrazolo[3,4-d][1,6,10,15]oxatriazacyclononadecin-5,14(8H)-dion.

10. Verfahren zur Herstellung einer Verbindung der Formel (I) nach Anspruch 1, **dadurch gekennzeichnet, dass** als Ausgangsprodukt die Verbindung der Formel (II) verwendet wird: 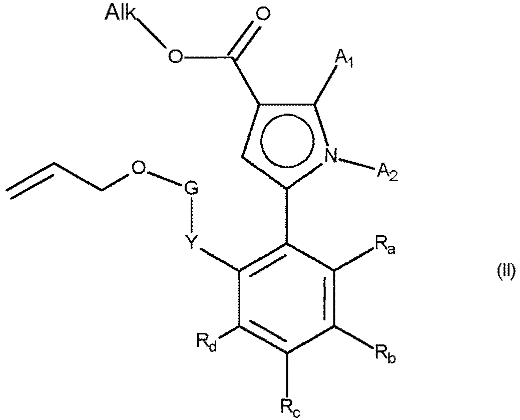
worin A₁, A₂, Rₐ, R_{b}, R_{c}, R_{d}, Y und G die gleiche Bedeutung wie in der in Anspruch 1 definierten Formel (I) haben und Alk eine lineare oder verzweigte (C₁-C₆)-Alkylgruppe darstellt,
Verbindung der Formel (II), deren Esterfunktion -OAlk hydrolysiert wird, um die Carbonsäure oder das entsprechende Carboxylat zu ergeben, welche(s) in das Acylchlorid oder entsprechende -anhydrid umgewandelt werden kann, bevor es mit einem Amin NHR_{3A}R₄ gekuppelt wird, worin R₄ die gleiche Bedeutung wie in Formel (I) hat und R_{3A} Folgendes darstellt:
- eine Gruppe R₃ wie in Formel (I) definiert
- oder eine Gruppe R₃-O-Alk'-Z, R₃-Alk'-Z oder R₃-Z, in der Alk' eine lineare oder verzweigte (C₁-C₆)-Alkylgruppe darstellt und Z ein Halogenatom oder eine -OH-Gruppe darstellt,
um die Verbindung der Formel (III) zu bilden: 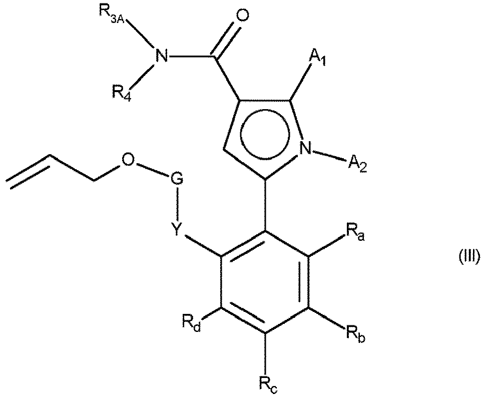
worin A₁, A₂, Rₐ, R_{b}, R_{c}, R_{d}, R₄, Y und G die gleiche Bedeutung wie in Formel (I) haben,
die einer Entschützungsreaktion der Alkoholfunktion unterzogen wird, gefolgt entweder von einer intramolekularen nukleophilen Substitution oder einer Mitsunobu-Reaktion oder einer aromatischen nukleophilen Substitution,
um zur Verbindung der Formel (I) zu führen,
Verbindung der Formel (I), die mittels eines herkömmlichen Trennverfahrens gereinigt werden kann, die in ihre Additionssalze mit einer pharmazeutisch verträglichen Säure oder Base umgewandelt werden kann und deren Isomere gegebenenfalls mittels eines herkömmlichen Trennverfahrens getrennt werden, wobei zu jedem als angemessen erachteten Zeitpunkt während des oben beschriebenen Verfahrens die Hydroxy- und Aminogruppen der Reagenzien oder Synthesezwischenprodukte geschützt und dann für die Zwecke der Synthese entschützt werden können.

11. Verfahren zur Herstellung einer Verbindung der Formel (I) nach Anspruch 1, **dadurch gekennzeichnet, dass** als Ausgangsprodukt die Verbindung der Formel (IV) verwendet wird: 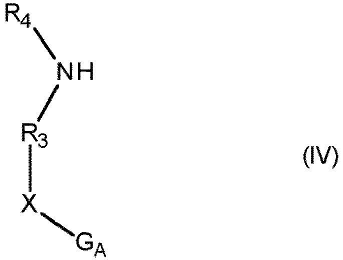
worin R₃, R₄ und X die gleiche Bedeutung wie in Formel (I) haben, und G_{A} eine aus der folgenden Liste ausgewählte Gruppe darstellt: 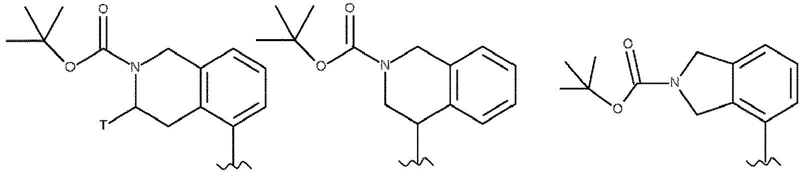 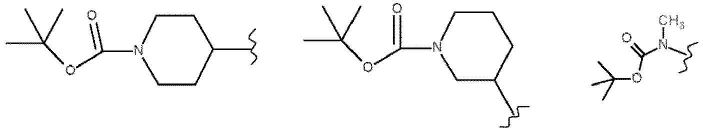
Verbindung der Formel (IV), die dann an eine Verbindung der Formel (V) gekuppelt wird: 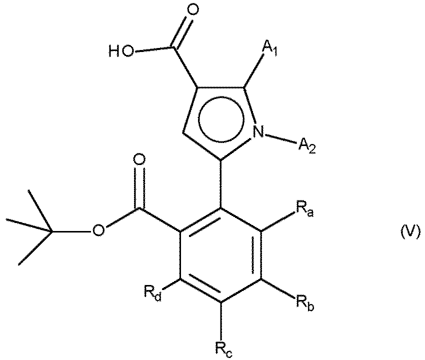
worin A₁, A₂, Rₐ, R_{b}, R_{c} und R_{d} die gleiche Bedeutung wie in Formel (I) haben, um zur Verbindung der Formel (VI) zu führen: 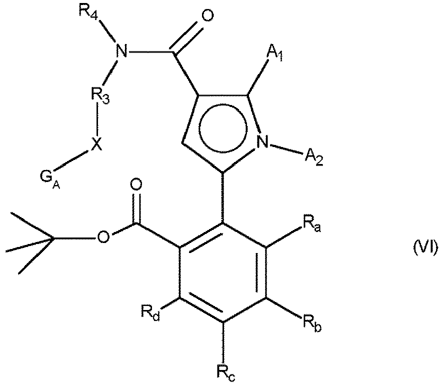
worin A₁, A₂, Rₐ, R_{b}, R_{c}, R_{d}, R₃, R₄ die gleiche Bedeutung wie in Formel (I) haben,
die einer Entschützungsreaktion unterzogen wird, gefolgt von einer intramolekularen Kupplung, um zur Verbindung der Formel (I) zu führen,
Verbindung der Formel (I), die mittels eines herkömmlichen Trennverfahrens gereinigt werden kann, die in ihre Additionssalze mit einer pharmazeutisch verträglichen Säure oder Base umgewandelt werden kann und deren Isomere gegebenenfalls mittels eines herkömmlichen Trennverfahrens getrennt werden, wobei zu jedem als angemessen erachteten Zeitpunkt während des oben beschriebenen Verfahrens die Hydroxy- und Aminogruppen der Reagenzien oder Synthesezwischenprodukte geschützt und dann für die Zwecke der Synthese entschützt werden können.

12. Pharmazeutische Zusammensetzung, enthaltend eine Verbindung der Formel (I) nach einem der Ansprüche 1 bis 9 oder ein Additionssalz davon mit einer pharmazeutisch verträglichen Säure oder Base in Kombination mit einem oder mehreren pharmazeutisch verträglichen Hilfsstoffen.

13. Pharmazeutische Zusammensetzung nach Anspruch 12 zur Verwendung als proapoptotisches Mittel.

14. Pharmazeutische Zusammensetzung nach Anspruch 12 zur Verwendung bei der Behandlung von Krebs, Autoimmunerkrankungen und Erkrankungen des Immunsystems.

15. Pharmazeutische Zusammensetzung nach Anspruch 14, wobei der Krebs aus der folgenden Liste ausgewählt ist: Blasenkrebs, Gehirntumor, Brustkrebs, Gebärmutterkrebs, chronische lymphatische Leukämie, Darmkrebs, Speiseröhrenkrebs, Leberkrebs, lymphoblastische Leukämie, Non-Hodgkin-Lymphom, Melanom, hämatologische Malignität, Myelom, Eierstockkrebs, nicht-kleinzelliger Lungenkrebs, Prostatakrebs und kleinzelliger Lungenkrebs.

16. Verwendung einer Verbindung der Formel (I) nach einem der Ansprüche 1 bis 9 zur Herstellung eines Medikaments, das als proapoptotisches Mittel nützlich ist.

17. Verwendung einer Verbindung der Formel (I) nach einem der Ansprüche 1 bis 9 zur Herstellung eines Medikaments zur Behandlung von Krebserkrankungen, Immun- und Autoimmunerkrankungen.

18. Verwendung einer Verbindung der Formel (I) nach Anspruch 17, wobei der Krebs aus der folgenden Liste ausgewählt ist: Blasenkrebs, Gehirntumor, Brustkrebs, Gebärmutterkrebs, chronische lymphatische Leukämie, Darmkrebs, Speiseröhrenkrebs, Leberkrebs, lymphoblastische Leukämie, Non-Hodgkin-Lymphom, Melanom, hämatologische Malignität, Myelom, Eierstockkrebs, nicht-kleinzelliger Lungenkrebs, Prostatakrebs und kleinzelliger Lungenkrebs.

19. Kombination einer Verbindung der Formel (I) nach einem der Ansprüche 1 bis 9 mit einem Antikrebsmittel, ausgewählt aus genotoxischen Mitteln, mitotischen Giften, Antimetaboliten, Proteasom-Inhibitoren, Kinase-Inhibitoren oder Antikörpern.

20. Pharmazeutische Zusammensetzung, enthaltend eine Kombination nach Anspruch 19 in Kombination mit einem oder mehreren pharmazeutisch verträglichen Hilfsstoffen.

21. Kombination nach Anspruch 19 zur Verwendung bei der Behandlung von Krebserkrankungen.

22. Verwendung einer Kombination nach Anspruch 19 zur Herstellung eines Medikaments, das bei der Behandlung von Krebserkrankungen nützlich ist.

23. Verbindung der Formel (I) nach einem der Ansprüche 1 bis 9 zur Verwendung in Kombination mit einer Strahlentherapie bei der Behandlung von Krebserkrankungen.

## Claims

1. Compound of a formula (I): 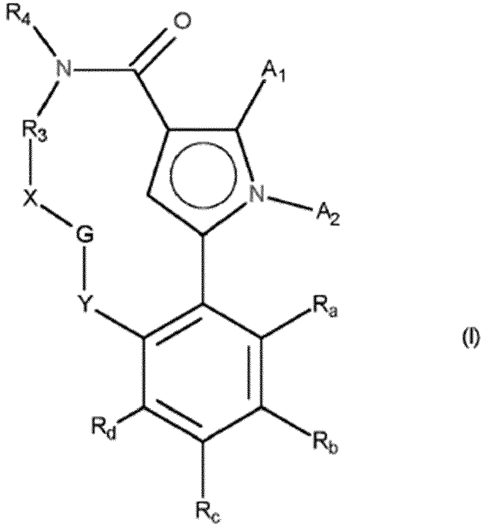 wherein:
▪ A₂ and A₂ each represent a methyl group,
▪ G represents a -NR₇- group, a 1,2,3,4-tetrahydroisoquinolinylene group optionally substituted by a T group, a 2,3-dihydro-1H-isoindolylene group optionally substituted by a T group, or a piperidinylene group,
▪ T represents a hydrogen atom, a linear or branched (C₁-C₆) alkyl optionally substituted by one to three halogen atoms, a (C₁-C₄)-alkyl-NR₁R₂ group, or a (C₁-C₄)-OR₆ alkyl group,
▪ X represents a (C₂-C₈) alkylene group, 1 to 3 chain members of which may be replaced by a heteroatom selected from oxygen, sulphur and N-R₅, or by an arylene or heteroarylene group,
▪ Y represents a -CH₂- or -CO- group,
▪ R₁ and R₂ independently of each another represent a hydrogen atom or a linear or branched (C₁-C₆) alkyl group,
or R₂ and R₂, together with the nitrogen atom carrying them, form a heterocycloalkyl,
▪ R₃ and R₄ are such that:
- one of them represents a phenyl group with the following formula: 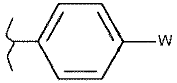 wherein W represents a hydroxy group or a phosphate group selected from - OPO (OM) (OM') -OPO (OM) (O⁻M₁⁺) , -OPO (O⁻M₁⁺) (O⁻M₂⁺) , -OPO (O⁻) (O⁻) M₃²⁺, -OPO (OM) (O[CH₂CH₂O]ₙCH₃), and -OPO(O⁻M₁⁺) (O[CH₂CH₂O]ₙCH₃), wherein M and M' represent, independently of each other, a hydrogen atom, a linear or branched (C₁-C₆) alkyl group, a linear or branched (C₂-C₆) alkenyl group, a linear or branched (C₂-C₆) alkynyl group, a cycloalkyl or a heterocycloalkyl, both composed of 5 or 6 ring members, whereas M₁⁺ and M₂⁺ independently of one another represent a pharmaceutically acceptable monovalent cation, M₃²⁺ represents a pharmaceutically acceptable divalent cation and n is an integer of between 1 and 5,
- while the other represents an aryl, heteroaryl, heterocycloalkyl, cycloalkyl or linear or branched (C₁-C₆) alkyl group, it being understood that one or more carbon atoms of the foregoing groups, or of any substituents thereof, may be deuterated,
▪ R₅ represents a hydrogen atom or a linear or branched (C₁-C₆) alkyl group,
▪ R₆ and R₇ independently of each another represent a hydrogen atom or a linear or branched (C₁-C₆) alkyl group,
▪ Rₐ, R_{c} and R_{d} each represent a hydrogen atom and R_{b} represents a hydrogen or halogen atom
it being understood that:
- the term "aryl" refers to a phenyl, naphthyl, biphenyl or indenyl group,
- the term "heteroaryl" refers to any mono- or bi-cyclic group composed of 5 to 10 ring members, having at least one aromatic part, and containing from 1 to 4 heteroatoms chosen from oxygen, sulphur, nitrogen and quaternary nitrogen,
- the term "cycloalkyl" refers to any non-aromatic, mono- or bi-cyclic carbocyclic group containing 3 to 10 ring members,
- the term "heterocycloalkyl" refers to any non-aromatic mono- or bi-cyclic, fused or spiro, composed of 3 to 10 ring members and containing 1 to 3 heteroatoms chosen from oxygen, sulphur, SO, SO₂ or nitrogen,
- the terms arylene, heteroarylene, 1,2,3,4-tetrahydroisoquinolinylene, 2,3-dihydro-1H-isoindolylene or piperidinylene refer to a divalent aryl, heteroaryl, 1,2,3,4-tetrahydroisoquinoline or piperidine group,
the aryl, heteroaryl, cycloalkyl and heterocycloalkyl groups thus defined and the alkyl, alkenyl, alkynyl and alkoxy groups, which may be substituted by 1 to 3 groups selected from: a linear or branched (C₁-C₆) alkyl optionally substituted by a hydroxy, morpholinyl, 3-3-difluoropiperidinyl or 3-3-difluoropyrrolidinyl group; spiro (C₃-C₆); linear or branched (C₁-C₆) alkoxy optionally substituted by a morpholinyl group; (C₁-C₆) alkyl-S-; hydroxy; oxo; N-oxide; nitro; cyano; -COOR'; - OCOR'; NR'R"; linear or branched (C₁-C₆) polyhaloalkyl; trifluoromethoxy; (C₁-C₆) alkylsulfonyl; halogen; aryl optionally substituted by one or more halogen atoms; heteroaryl; aryloxy; arylthio; cycloalkyl; heterocycloalkyl optionally substituted by one or more halogen atoms or linear or branched (C₁-C₆) alkyl groups; it being understood that R' and R" represent, independently of each other, a hydrogen atom or a linear or branched (C₁-C₆) alkyl group optionally substituted by a methoxy group,
its enantiomers, diastereoisomers or its addition salts with a pharmaceutically acceptable acid or base.

2. Compound of the formula (I) according to claim 1 wherein Y represents a -CO- group.

3. Compound of the formula (I) according to claim 1 or 2 wherein G represents a group chosen among the following groups: 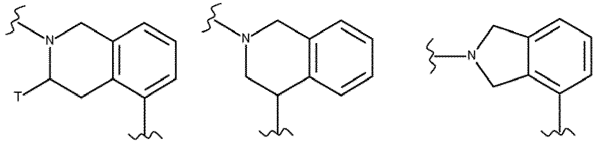 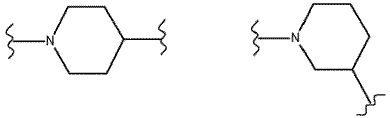 where T represents a methyl group or a (4-morpholinyl)methyl group.

4. Compound of the formula (I) according to any of claims 1 to 3 wherein X represents a group chosen among the following groups:

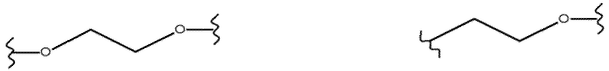

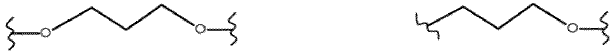

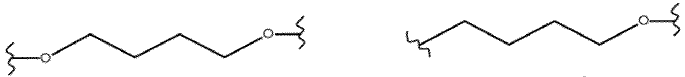

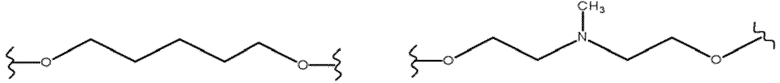

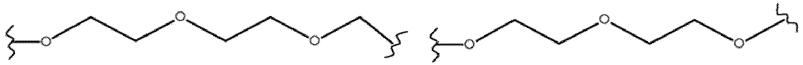

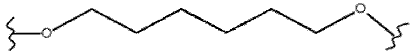

5. Compound of the formula (I) according to any of claims 1 to 4 wherein one of the groups R₃ or R₄ represent a 4-hydroxyphenyl group while the other represents a group chosen from the following list:
- a phenyl group optionally substituted by a cyano group,
- a pyrazolyl group
- an 1-methyl-1N-pyrazolyl group,
- a 1-(tetrahydrofuran-3-yl)-1H-pyrazolyl group,
- a 5-methyl-2-cyano-1H-pyrrolyl group,
- a 1-methyl-2-cyano-1H-pyrrolyl group,
- a 1,2-dimethyl-1H-pyrrolyl group,
- a 1,5-dimethyl-2-cyano-1H-pyrrolyl group,
- a pyrimidinyl group,
- an ethyl group,
- a pyridinium group.

6. Compound of the formula (I) according to claim 5 wherein R₄ represents a 4-hydroxyphenyl group.

7. Compound of the formula (I) according to claim 5 wherein R₃ represents a 4-hydroxyphenyl group.

8. Compound of the formula (I) according to claim 7 wherein R₃ represents a 4-hydroxyphenyl group and G represents a piperidinylene.

9. Compound of the formula (I) according to claim 1 chosen among the following group:
- 6-chloro-14-(4-hydroxyphenyl)-10,11-dimethyl-2,13-dioxo-20,23-dioxa-1,10,14-triazahexacyclo[26. 3. 1.1~9,12~.1~15,19~.0~3,8~.0~24,29~]tetratriaconta-3,5,7,9(34),11, 15(33),16,18,24,26,28-undecaene-17-carbonitrile,
- 11-chloro-4-(4-hydroxyphenyl)-1,7,8-trimethyl-4,16,17,23,24,25-hexahydro-1H,14H-15,18-methano-6,9-(metheno) dibenzo [b, h] pyrazolo [4, 3-p] [1,6,11,15]oxatriazacycloicosine-5,14(8H)-dione,
- 6-chloro-14-(4-hydroxyphenyl)-10,11-dimethyl-2,13-dioxo-23-oxa-1,10,14-triazahexacyclo[26.3.1.1~9,12~.1~15,19~.0~3,8~.0~24,29~]tetr atriaconta-3,5,7,9(34),11, 15(33),16,18,24,26,28-undecaene-17-carbonitrile,
- 11-chloro-4-(4-hydroxyphenyl)-1,7,8-trimethyl-5, 14-dioxo-4,5,8, 16,17,23,24,25- octahydro- 1H, 14H-15,18-methano-6,9-(metheno)dibenzo[b,h]pyrrolo[3,2-p] [1,6,11,15]oxatriazacycloicosine-2-carbonitrile,
- 11-chloro-4-(4-hydroxyphenyl)-1,7,8-trimethyl-5, 14-dioxo-1,4,5,8,16, 17,23,24- octahydro-14H-15, 18-methano-6,9-(metheno)dibenzo[1,r]pyrrolo[2,3-d] [1,6,10,15]oxatriazacyclononadecine-2-carbonitrile,
- 11-chloro-4-(4-hydroxyphenyl)-1,7,8-trimethyl-1,4, 16,21,22,23-hexahydro-14H- 15,21-methano-6,9-(metheno)dibenzo[j,o]pyrazolo[3,4-b] [1,4,8,13]oxatriazacyclononadecine-5,14(8H)-dione,
- (16S or R)-11-chloro-4-(4-hydroxyphenyl)-1,7,8,16-tetramethyl-5, 14-dioxo-4,5,8,16, 17,23,24,25-octahydro-1H, 14H-15,18-methano-6,9-(metheno)dibenzo[b,h]pyrrolo[3,2-p][1,6, 11,15]oxatriazacycloicosine-2-carbonitrile,
- 4-[16-chloro-3-hydroxy-19,20-dimethyl-13,22-dioxo-6,7,8,9,10, 11,19,22-octahydro-13H,23H-8,12-methano-21,18-(metheno)dibenzo[b,j] [1,4,8,13]oxatriazacyclononadecin-23-yl]-1,5-dimethyl-1H-pyrrole-2-carbonitrile,
- 10-fluoro-2-(4-hydroxyphenyl)-5,6-dimethyl-24,27,30-trioxa-2, 6, 15, 32, 35-pentaazahexacyclo[29.2.2.1~4,7~.1~15,19~.0~8,13~.0~18,23~]he ptatriaconta-1(33),4,7(37),8, 10,12,18,20,22,31,34-undecaene-3,14-dione,
- 10-chloro-2-(4-hydroxyphenyl)-5,6-dimethyl-24,30-dioxa-2,6,15,32,35-pentaazahexacyclo[29.2.2.1-4,7-.1-15,19-.0-8, 13~.0~18,23~]heptatriaconta-1(33),4,7(37),8,10,12,18,20,22,31,34-undecaene-3,14-dione,
- 10-chloro-2-(4-hydroxyphenyl)-5,6,27-trimethyl-24,30-dioxa-2,6,15,27,32,35-hexaazahexacyclo[29.2.2.1~4,7~.1~15,19~.0~8,13~.0~18,23~]hep tatriaconta-1(33),4,7(37),8,10, 12,18,20,22,31,34-undecaene-3,14-dione,
- 11-chloro-4-(4-hydroxyphenyl)-1,7,8-trimethyl-1,4, 16,17,23,24-hexahydro-14H-15,18-methano-6,9-(metheno)dibenzo[1,r]pyrazolo[3,4-d] [1,6,10,15]oxatriazacyclononadecine-5,14(8H)-dione.

10. A method for preparing a compound of a formula (I) according to claim 1 **characterised in that** the source material is the compound of a formula (II): 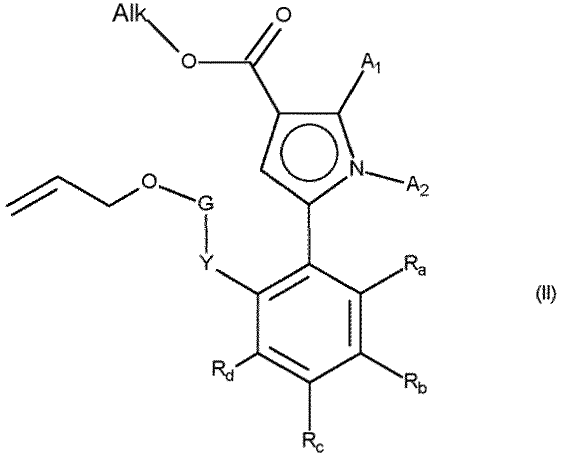
wherein A₁, A₂, Rₐ, R_{b}, R_{c}, R_{d}, Y and G have the same meaning as in formula (I) defined in claim 1, and Alk represents a linear or branched (C₁-C₆) alkyl group,
the compound of the formula (II) wherein which the ester function -OAlk is hydrolysed to give the carboxylic acid or the corresponding carboxylate, which may be converted into the corresponding acyl chloride or anhydride, before being coupled with an amine NHR_{3A}R₄, wherein R₄ has the same meaning as in formula (I), and R_{3A} represents:
- a R₃ group as defined in the formula (I)
- or a R₃-O-Alk'-Z, R₃-Alk'-Z or R₃-Z group in which Alk' represents a linear or branched (C₁-C₆) alkyl group, and Z represents a halogen atom or an -OH group,
to form the compound of a formula (III): 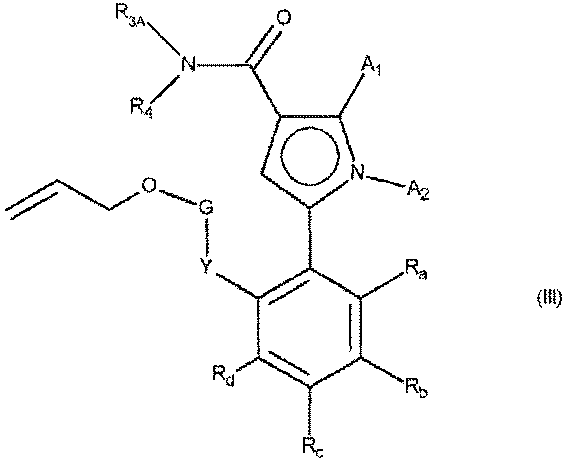
wherein A₁, A₂, Rₐ, R_{b}, R_{c}, R_{d}, R₄, Y and G have the same meaning as in formula (I),
which is subjected to a deprotection reaction of the alcohol function, followed either by an intramolecular nucleophilic substitution, or by a Mitsunobu reaction, or else by an aromatic nucleophilic substitution,
to give the compound of the formula (I),
which compound of the formula (I) can be purified by a conventional separation technique, which can be converted into its addition salts with a pharmaceutically acceptable acid or base and from which the isomers are optionally separated by a conventional separation technique,
it being understood that, at any time deemed appropriate during the process previously described, the hydroxy and amino groups of the synthesis reagents or intermediates can be protected and then deprotected for the purposes of the synthesis.

11. The method for preparing a compound of a formula (I) according to claim 1 **characterised in that** the source material is the compound of a formula (IV): 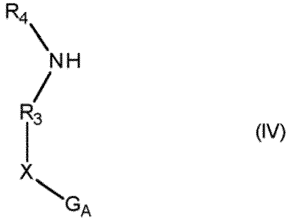
wherein R₃, R₄ and X have the same meaning as in the formula (I) and G_{A} represents a group chosen from the following list: 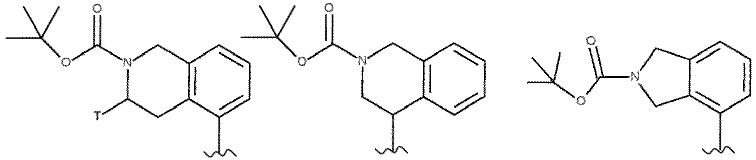 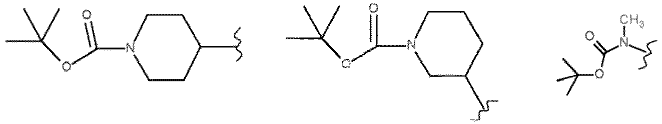
the compound of the formula (IV) that is then coupled to a compound of a formula (V) 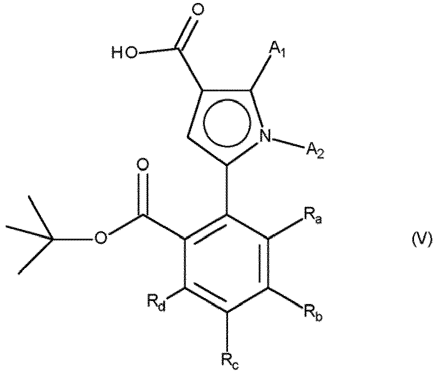
wherein A₁, A₂, Rₐ, R_{b}, R_{c} and R_{d}, have the same meaning as in the formula (I), to give the compound of the formula (VI), 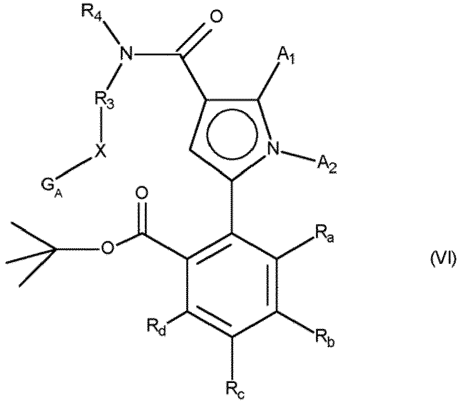
wherein A₁, A₂, Rₐ, R_{b}, R_{c}, R_{d}, R₃, R₄ and X have the same meaning as in the formula (I),
which is subjected to a deprotection reaction followed by intramolecular coupling to yield the compound of the formula (I),
the compound of the formula (I) which can be purified using a conventional separation technique, which can be converted into its addition salts with a pharmaceutically acceptable acid or base and from which the isomers are optionally separated using a conventional separation technique,
it being understood that, at any time deemed appropriate during the process described above, the hydroxy and amino groups of the synthesis reagents or intermediates can be protected and then deprotected for the purposes of the synthesis.

12. Pharmaceutical composition containing a compound of formula (I) according to any one of claims 1 to 9, or one of its addition salts with a pharmaceutically acceptable acid or base, in combination with one or more pharmaceutically acceptable excipients.

13. The pharmaceutical composition according to claim 12 for use as a pro-apoptotic agent.

14. The pharmaceutical composition according to claim 12 for use in the treatment of cancers, autoimmune diseases and diseases of the immune system.

15. The pharmaceutical composition according to claim 14 wherein the cancer is selected from the following list: bladder cancer, brain cancer, breast cancer, uterine cancer, chronic lymphocytic leukaemia, colorectal cancer, oesophageal cancer, liver cancer, lymphoblastic leukaemia, non-Hodgkin's lymphoma, melanoma, haematological malignancy, myeloma, ovarian cancer, non-small cell lung cancer, prostate cancer and small-cell lung cancer.

16. Use of a compound of the formula (I) according to any of claims 1 to 9 for the manufacture a medicament useful as a pro-apoptotic agent.

17. Use of a compound of the formula (I) according to any of claims 1 to 9 for the manufacture a medicament for the treatment of cancers, immune and autoimmune diseases.

18. Use of a compound of the formula (I) according to claim 17 wherein the cancer is selected from the following list: bladder cancer, brain cancer, breast cancer, uterine cancer, chronic lymphocytic leukaemia, colorectal cancer, oesophageal cancer, liver cancer, lymphoblastic leukaemia, non-Hodgkin's lymphoma, melanoma, haematological malignancy, myeloma, ovarian cancer, non-small cell lung cancer, prostate cancer and small-cell lung cancer.

19. Combination of a compound of the formula (I) according to any of claims 1 to 9 with an anticancer agent chosen from genotoxic agents, mitotic poisons, antimetabolites, proteasome inhibitors, kinase inhibitors or antibodies.

20. Pharmaceutical composition containing a combination according to claim 19 in combination with one or more pharmaceutically acceptable excipients.

21. A combination according to claim 19 for use in the treatment of cancer.

22. Use of a combination according to claim 19 for the manufacture a medicament useful in the treatment of cancer.

23. A compound of the formula (I) according to any of claims 1 to 9 for use in combination with radiotherapy in the treatment of cancer.
